# EUROPEAN PATENT SPECIFICATION

(11) **EP 1 071 826 B1**
(45) Date of publication and mention of the grant of the patent: **13.09.2006**
(21) Application number: 99922713.5
(22) Date of filing: 13.04.1999
(51) Int. Cl.: C12Q 1/68, G06F 19/00, C12N 15/11

(54) **IDENTIFICATION OF GENETIC TARGETS FOR MODULATION BY OLIGONUCLEOTIDES AND GENERATION OF OLIGONUCLEOTIDES FOR GENE MODULATION**
IDENTIFIZIERUNG VON GENETISCHEN ZIELEN ZUR MODULATION DURCH OLIGONUKLEOTIDE UND HERSTELLUNG VON OLIGONUKLEOTIDEN ZUR MODULATION VON GENEN
IDENTIFICATION DE CIBLES GENETIQUES DESTINEES A ETRE MODULEES PAR DES OLIGONUCLEOTIDES ET FORMATION D'OLIGONUCLEOTIDES DESTINES A SUBIR UNE MODULATION GENIQUE

(30) Priority: 13.04.1998 US 81483 P; 28.04.1998 US 67638
(43) Date of publication of application: 31.01.2001
(73) Proprietor: ISIS PHARMACEUTICALS, INC., Carlsbad, CA 92008 (US)
(72) Inventor: COWSERT, Lex, M., San Mateo, CA 94402 (US); BAKER, Brenda, F., Carlsbad, CA 92009 (US); MCNEIL, John, La Jolla, CA 92037 (US); FREIER, Susan, M., San Diego, CA 92122 (US); SASMOR, Henri, M., Encinitas, CA 92024 (US); BROOKS, Douglas, G., San Marcos, CA 92069 (US); OHASHI, Cara, San Fransisco, CA 94127 (US); WYATT, Jacqueline, R., Encinitas, CA 92024 (US); BORCHERS, Alexander, H., Encinitas, CA 92024 (US); VICKERS, Timothy, A., Oceanside, CA 92057 (US)
(74) Representative: Hallybone, Huw George
(86) International application number: PCT/US1999/008268
(87) International publication number: WO 1999/053101

(56) References cited:
- WO-A-93/04204
- WO-A-98/03533
- WO-A-98/37242
- WO-A1-89/10977
- US-A- 4 806 463
- US-A- 5 436 327
- US-A- 5 612 455
- US-A- 5 700 637
- US-A- 5 783 431
- US-A- 5 824 485
- STULL R A ET AL: "PREDICTING ANTISENSE OLIGONUCLEOTIDE INHIBITORY EFFICACY A COMPUTATIONAL APPROACH USING HISTOGRAMS AND THERMODYNAMIC INDICES" NUCLEIC ACIDS RESEARCH, vol. 20, no. 13, 1992, pages 3501-3508, XP001148805 ISSN: 0305-1048
- MITSUHASHI M: "STRATEGY FOR DESIGNING SPECIFIC ANTISENSE OLIGONUCLEOTIDE SEQUENCES" JOURNAL OF GASTROENTEROLOGY, SPRINGER VERLAG, TOKYO, JP, vol. 32, no. 2, 1997, pages 282-287, XP001053065 ISSN: 1340-9077
- HYNDMAN D ET AL: "SOFTWARE TO DETERMINE OPTIMAL OLIGONUCLEOTIDE SEQUENCES BASED ON HYBRIDIZATION SIMULATION DATA" BIOTECHNIQUES, EATON PUBLISHING, NATICK, US, vol. 20, no. 6, 1996, pages 1090-1097, XP002932984 ISSN: 0736-6205
- SCZAKIEL G ET AL: "Computer-aided search for effective antisense RNA target sequences of the human immunodeficiency virus type I" ANTISENSE RESEARCH AND DEVELOPMENT, MARY ANN LIEBERT, NEW YORK, US, US, vol. 3, no. 3, 1993, pages 45-52, XP002119904 ISSN: 1050-5261
- GHOSH M K ET AL: "Phosphorothioate - phosphodiester oligonucleotide co - polymers: assessment for antisense application" ANTI-CANCER DRUG DESIGN, BASINGSTOKE, GB, vol. 8, no. 1, February 1993 (1993-02), pages 15-32, XP002110959 ISSN: 0266-9536
- GHOSH et al., "Evaluation of Some Properties of a Phosphorodithioate Oligodeoxyribonucleotide for Antisense Application", NUCLEIC ACIDS RESEARCH, 1993, Volume 21, Number 24, pages 5761-5766, XP002921397.
- MIRABELLI et al., "In Vitro and In Vivo Pharmacologic Activities of Antisense Oligonucleotides", ANTI-CANCER DRUG DESIGN, December 1991, Volume 6, pages 647-661, XP002921398.
- UHLMANN et al., "Antisense Oligonucleotides: A New Therapeutic Principle", CHEMICAL REVIEWS, June 1990, Volume 90, Number 4, pages 543-584, XP000141412.

## Description

### FIELD OF THE INVENTION

The present invention relates generally to the generation and identification of synthetic compounds having defined physical, chemical or bioactive properties. More particularly, the present invention relates to the automated generation of oligonucleotide compounds targeted to a given nucleic acid sequence via computer-based, iterative robotic synthesis of synthetic oligonucleotide compounds and robotic or robot-assisted analysis of the activities of such compounds. Information gathered from assays of such compounds is used to identify nucleic acid sequences that are tractable to a variety of nucleotide sequence-based technologies, for example, antisense drug discovery and target validation.

### BACKGROUND OF THE INVENTION

### 1. Oligonucleotide Technology

Synthetic oligonucleotides of complementarity to targets are known to hybridize with particular, target nucleic acids in a sequence-specific manner. In one example, compounds complementary to the "sense" strand of nucleic acids that encode polypeptides, are referred to as "antisense oligonucleotides." A subset of such compounds may be capable of modulating the expression of a target nucleic acid; such synthetic compounds are described herein as "active oligonucleotide compounds."

Oligonucleotide compounds are commonly used *in vitro* as research reagents and diagnostic aids, and *in vivo* as therapeutic and bioactive agents. Oligonucleotide compounds can exert their effect by a variety of means. One such means takes advantage of an endogenous nuclease, such as RNase H in eukaryotes or RNase P in prokaryotes, to degrade the DNA/RNA hybrid formed between the oligonucleotide sequence and mRNA (Chiang *et al., J. Biol. Chem*., 1991, *266*, 18162; Forster *et al., Science,* 1990, *249*, 783). Another means involves covalently linking of a synthetic moiety having nuclease activity to an oligonucleotide having an antisense sequence. This does not rely upon recruitment of an endogenous nuclease to modulate target activity. Synthetic moieties having nuclease activity include, but are not limited to, enzymatic RNAs, lanthanide ion complexes, and other reactive species. (Haseloff *et al*., Nature, 1988, *334*, 585; Baker *et al., J. Am. Chem. Soc*., 1997, *119*, 8749).

Despite the advances made in utilizing antisense technology to date, it is still common to identify target sequences amenable to antisense technologies through an empirical approach (Szoka, *Nature Biotechnology,* 1997, *15*, 509). Accordingly, the need exists for systems and methods for efficiently and effectively identifying target nucleotide sequences that are suitable for antisense modulation. The present disclosure answers this need by providing systems and methods for automatically identifying such sequences via *in silico,* robotic or other automated means.

### 2. Identification of Active Oligonucleotide Compounds

Traditionally, new chemical entities with useful properties are generated by (1) identifying a chemical compound (called a "lead compound") with some desirable property or activity, (2) creating variants of the lead compound, and (3) evaluating the property and activity of such variant compounds. The process has been called "SAR," i.e., structure activity relationship. Although "SAR" and its handmaiden, rational drug design, has been utilized with some degree of success, there are a number of limitations to these approaches to lead compound generation, particularly as it pertains to the discovery of bioactive oligonucleotide compounds. In attempting to use SAR with oligonucleotides, it has been recognized that RNA structure can inhibit duplex formation with antisense compounds, so much so that "moving" the target nucleotide sequence even a few bases can drastically decrease the activity of such compounds (Lima *et al., Biochemistry*, 1992, *31,* 12055).

Heretofore, the preferred method of searching for lead antisense compounds has been the manual synthesis and analysis of such compounds. Consequently, a fundamental limitation of the conventional approach is its dependence upon the availability, number and cost of antisense compounds produced by manual, or at best semi-automated, means. Moreover, the assaying of such compounds has traditionally been performed by tedious manual techniques. Thus, the traditional approach to generating active antisense compounds is limited by the relatively high cost and long time required to synthesize and screen a relatively small number of candidate antisense compounds.

Accordingly, the need exists for systems and methods for efficiently and effectively generating new active antisense and other oligonucleotide compounds targeted to specific nucleic acid sequences. The present disclosure answers this need by providing systems and methods for automatically generating and screening active antisense compounds via robotic and other automated means.

### 3. Gene Function Analysis

Efforts such as the Human Genome Project are making an enormous amount of nucleotide sequence information available in a variety of forms, e.g., genomic sequences, cDNAs, expressed sequence tags (ESTs) and the like. This explosion of information has led one commentator to state that "genome scientists are producing more genes than they can put a function to" (Kahn, *Science,* 1995, *270,* 369). Although some approaches to this problem have been suggested, no solution has yet emerged. For example, methods of looking at gene expression in different disease states or stages of development only provide, at best, an association between a gene and a disease or stage of development (Nowak, *Science,* 1995, *270*, 368). Another approach, looking at the proteins encoded by genes, is developing but "this approach is more complex and big obstacles remain" (Kahn, *Science*, 1995, *270*, 369). Furthermore, neither of these approaches allows one to directly utilize nucleotide sequence information to perform gene function analysis.

In contrast, antisense technology does allow for the direct utilization of nucleotide sequence information for gene function analysis. Once a target nucleic acid sequence has been selected, antisense sequences hybridizable to the sequence can be generated using techniques known in the art. Typically, a large number of candidate antisense oligonucleotides (ASOs) are synthesized having sequences that are more-or-less randomly spaced across the length of the target nucleic acid sequence (e.g., a "gene walk") and their ability to modulate the expression of the target nucleic acid is assayed. Cells or animals can then be treated with one or more active antisense oligonucleotides, and the resulting effects determined in order to determine the function(s) of the target gene. Although the practicality and value of this empirical approach to determining gene function has been acknowledged in the art, it has also been stated that this approach "is beyond the means of most laboratories and is not feasible when a new gene sequence is identified, but whose function and therapeutic potential are unknown" (Szoka, *Nature Biotechnology,* 1997, 15, 509).

Accordingly, the need exists for systems and methods for efficiently and effectively determining the function of a gene that is uncharacterized except that its nucleotide sequence, or a portion thereof, is known. The present disclosure answers this need by providing systems and methods for automatically generating active antisense compounds to a target nucleotide sequence via robotic means. Such active antisense compounds are contacted with cells, cell-free extracts, tissues or animals capable of expressing the gene of interest and subsequent biochemical or biological parameters are measured. The results are compared to those obtained from a control cell culture, cell-free extract, tissue or animal which has not been contacted with an active antisense compound in order to determine the function of the gene of interest.

### 4. Target Validation

Determining the nucleotide sequence of a gene is no longer an end unto itself; rather, it is "merely a means to an end. The critical next step is to validate the gene and its [gene] product as a potential drug target" (Glasser, *Genetic Engineering News,* 1997, *17*, 1). This process, i.e., confirming that modulation of a gene that is suspected of being involved in a disease or disorder actually results in an effect that is consistent with a causal relationship between the gene and the disease or disorder, is known as target validation.

Efforts such as the Human Genome Project are yielding a vast number of complete or partial nucleotide sequences, many of which might correspond to or encode targets useful for new drug discovery efforts. The challenge represented by this plethora of information is how to use such nucleotide sequences to identify and rank valid targets for drug discovery. Antisense technology provides one means by which this might be accomplished; however, the many manual, labor-intensive and costly steps involved in traditional methods of developing active antisense compounds has limited their use in target validation (Szoka, *Nature Biotechnology,* 1997, *15*, 509). Nevertheless, the great target specificity that is characteristic of antisense compounds makes them ideal choices for target validation, especially when the functional roles of proteins that are highly related are being investigated (Albert *et al., Trends in Pharm. Sci.,* 1994, *15*, 250).

Accordingly, the need exists for systems and methods for developing compounds efficiently and effectively that modulate a gene, wherein such compounds can be directly developed from nucleotide sequence information. Such compounds are needed to confirm that modulation of a gene that is thought to be involved in a disease or disorder will in fact cause an *in vitro* or *in vivo* effect indicative of the origin, development, spread or growth of the disease or disorder.

The present disclosure answers this need by providing systems and methods for automatically generating active oligonucleotide, especially antisense compounds, to a target nucleotide sequence via robotic or other automated means. Such active compounds are contacted with a cell culture, cell-free extract, tissue or animal capable of expressing the gene of interest, and subsequent biochemical or biological parameters indicative of the potential gene product function are measured. These results are compared to those obtained with a control cell system, cell-free extract, tissue or animal which has not been contacted with an active antisense compound in order to determine whether or not modulation of the gene of interest affects a specific cellular function. The resulting active antisense compounds may be used as positive controls when other, non antisense-based agents directed to the same target nucleic acid, or to its gene product, are screened.

U.S. Patent 5,563,036 to Peterson *et al*. describes systems and methods of screening for compounds that inhibit the binding of a transcription factor to a nucleic acid. In a preferred embodiment, an assay portion of the process is stated to be performed by a computer controlled robot.

U.S. Patent 5,708,158 to Hoey describes systems and methods for identifying pharmacological agents stated to be useful for diagnosing or treating a disease associated with a gene the expression of which is modulated by a human nuclear factor of activated T cells. The methods are stated to be particularly suited to high-thoughput screening wherein one or more steps of the process are performed by a computer controlled robot.

U.S. Patents 5,693,463 and 5,716,780 to Edwards *et al.* describe systems and methods for identifying non-oligonucleotide molecules that specifically bind to a DNA molecule based on their ability to compete with a DNA-binding protein that recognizes the DNA molecule.

U.S. patents 5,463,564 and 5,684,711 to Agrafiotis et al. describe computer based iterative processes for generating chemical entities with defined physical, chemical and/or bioactive properties. WO 98/03533 describes the identification of antisense oligonucleotides using computer-related means and the synthesis of such oligonucleotides on an automatic oligonucleotide synthesis.

### SUMMARY OF THE INVENTION

The present invention is directed to automated systems and methods for defining sets of oligonucleotides that modulate the expression of target nucleic acid sequences, and generating sets of oligonucleotides that modulate the expression of target nucleic acid sequences. The present invention is also directed to identifying nucleic acid sequences amenable to antisense binding of oligonucleotides to those nucleic acid sequences by the systems and methods of the invention. For purposes of illustration, the present invention is described herein with respect to the production and identification of active antisense oligonucleotides; however, the present invention is not limited to this embodiment. According to present invention there is provided the method of claim 1.

The present invention is directed to iterative processes for defining oligonucleotides with prescribed sets of physical, chemical and/or biological properties, and to systems for implementing these processes. During each iteration of a process as contemplated herein, a target nucleic acid sequence is provided or selected, and a library of (candidate) virtual compounds is generated *in silico* (that is in a computer manipulatible and reliable form) according to defined criteria. A library of virtual compounds is generated. These virtual compounds are reviewed and compounds predicted to have particular desired properties are selected. The selected compounds are synthesized, in a robotic, batchwise manner, and then they are robotically assayed for a desired physical, chemical or biological activity in order to identify compounds with the desired properties. Active compounds are, thus, generated and, at the same time, preferred sequences and regions of the target nucleic acid that are amenable to modulation are identified.

In subsequent iterations of the process, second libraries of candidate compounds are generated and/or selected to give rise to a second virtual compound library. Through multiple iterations of the process, a library of target nucleic acid sequences that are tractable to modulation via binding of these compounds to the nucleic acid sequence are identified. Such modulation includes, but is not limited to, antisense technology, gene function analysis and target validation.

Further features and advantages of the present invention, as well as the structure and operation of various embodiments of the present invention, are described in detail below with reference to the accompanying drawings. In the drawings, like reference numbers indicate identical or functionally similar elements.

### BRIEF DESCRIPTION OF THE DRAWINGS

The present invention will be described with reference to the accompanying drawings, wherein:
Figures 1 and 2 are a flow diagram of one method according to the present invention depicting the overall flow of data and materials among various elements of the invention.
**Figure** 3 is a flow diagram depicting the flow of data and materials among elements of step **200** of Figure 1.
Figures 4 and 5 are a flow diagram depicting the flow of data and materials among elements of step **300** of Figure 1.
Figure 6 is a flow diagram depicting the flow of data and materials among elements of step **306** of Figure 4.

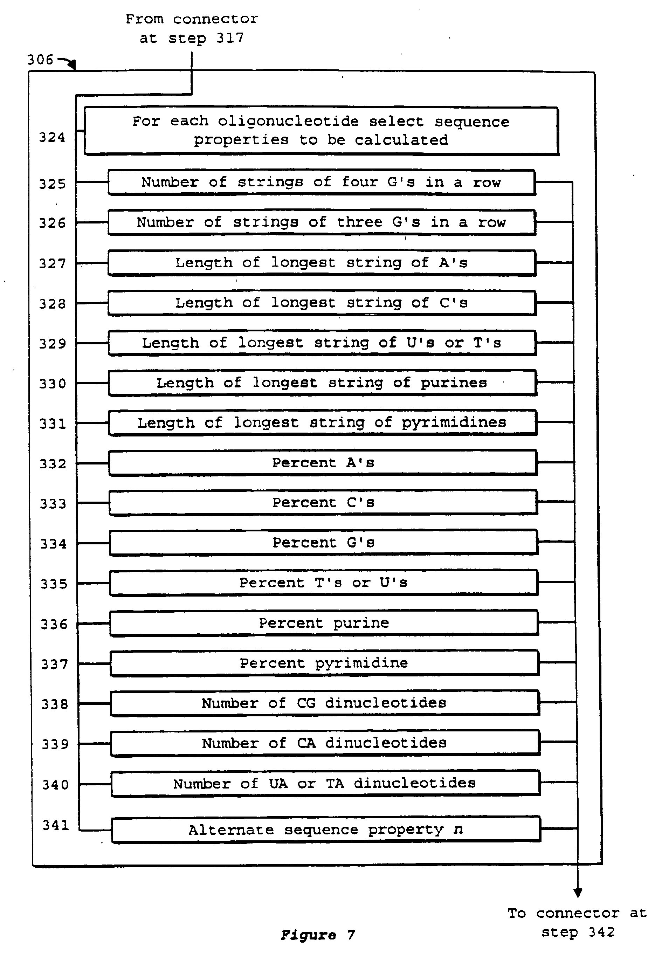

Figure 7 is another flow diagram depicting the flow of data and materials among elements of step **306** of Figure 4.

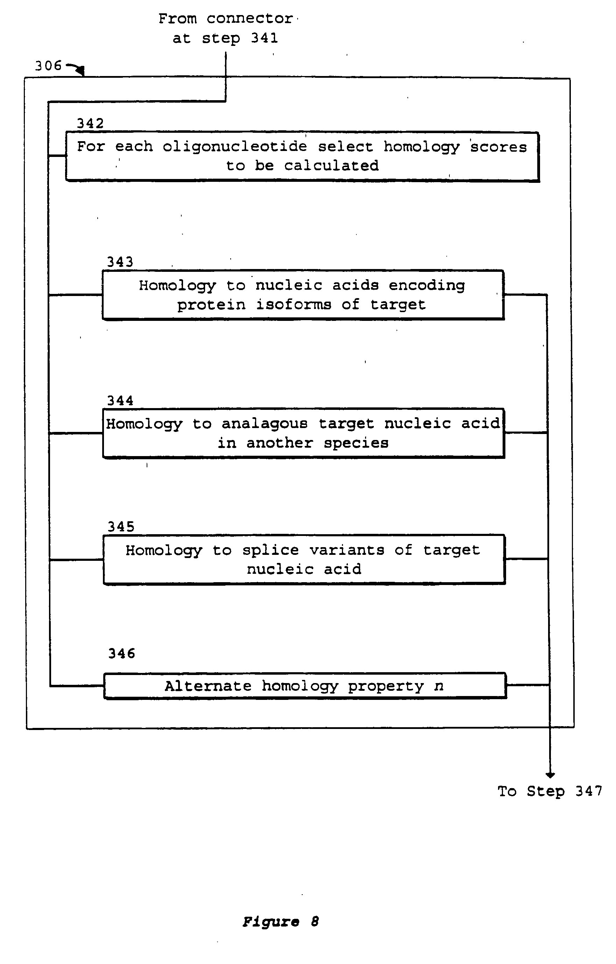

Figure 8 is a another flow diagram depicting the flow of data and materials among elements of step **306** of Figure 4.
Figure 9 is a flow diagram depicting the flow of data and materials among elements of step **350** of Figure 5.
Figures 10 and 11 are flow diagrams depicting a logical analysis of data and materials among elements of step **400** of Figure 1.
Figure 12 is a flow diagram depicting the flow of data and materials among the elements of step **400** of Figure 1.
Figures 13 and 14 are flow diagrams depicting the flow of data and materials among elements of step **500** of Figure 1.
Figure 15 is a flow diagram depicting the flow of data and materials among elements of step **600** of Figure 1.
Figure 16 is a flow diagram depicting the flow of data and materials among elements of step **700** of Figure 1.
Figure 17 is a flow diagram depicting the flow of data and materials among the elements of step **1100** of Figure 2.
Figure 18 is a block diagram showing the interconnecting of certain devices utilized in conjunction with a preferred method of the invention;
Figure 19 is a flow diagram showing a representation of data storage in a relational database utilized in conjunction with one method of the invention;
Figure 20 is a flow diagram depicting the flow of data and materials in effecting a preferred embodiment of the invention as set forth in Example 14;
Figure 21 is a flow diagram depicting the flow of data and materials in effecting a preferred embodiment of the invention as set forth in Example 15;
Figure 22 is a flow diagram depicting the flow of data and materials in effecting a preferred embodiment of the invention as set forth in Example 2;
Figure 23 is a pictorial elevation view of a preferred apparatus used to robotically synthesize oligonucleotides; and
Figure 24 is a pictorial plan view of an apparatus used to robotically synthesize oligonucleotides.

### DETAILED DESCRIPTION OF THE INVENTION

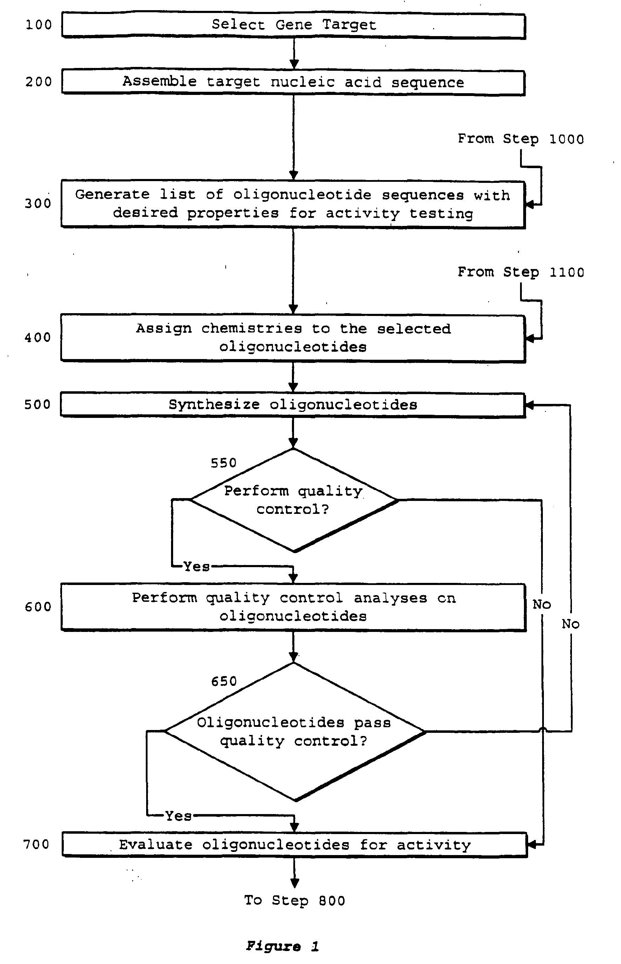

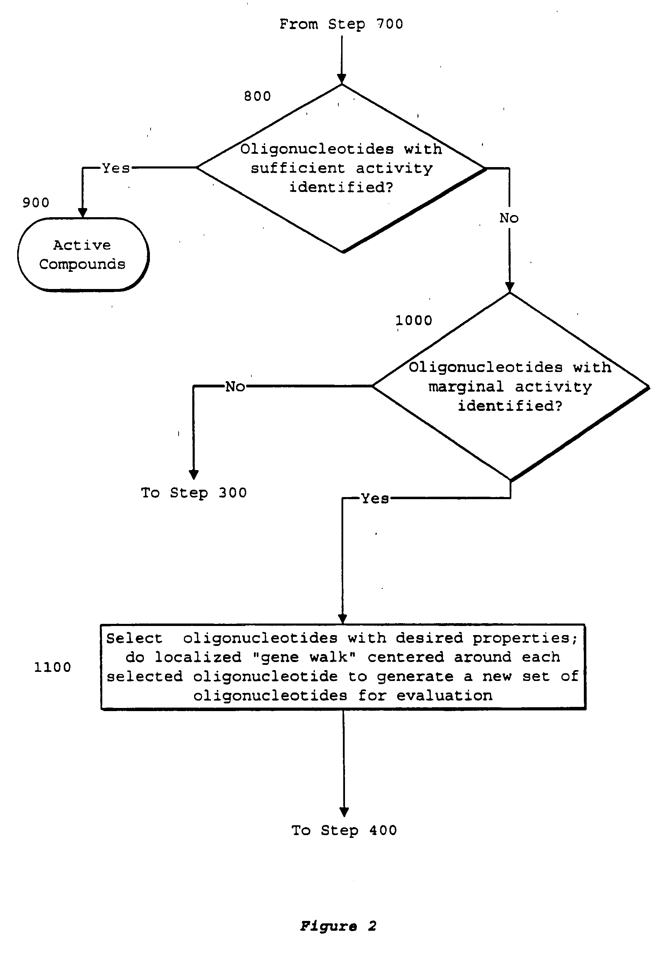

Certain preferred methods of this invention are now described with reference to the flow diagram of Figures 1 and 2.

**1. Target Nucleic Acid Selection.** The target selection process, process step **100,** provides a target nucleotide sequence that is used to help guide subsequent steps of the process. It is generally desired to modulate the expression of the target nucleic acid for any of a variety of purposes, such as, e.g., drug discovery, target validation and/or gene function analysis.

One of the primary objectives of the target selection process, step **100,** is to identify molecular targets that represent significant therapeutic opportunities, to provide new and efficacious means of drug discovery and to determine the function of genes that are uncharacterized except for nucleotide sequence. To meet these objectives, genes are classified based upon specific sets of selection criteria.

One such set of selection criteria concerns the quantity and quality of target nucleotide sequence. There must be sufficient target nucleic acid sequence information available for oligonucleotide design. Moreover, such information must be of sufficient quality to give rise to an acceptable level of confidence in the data to perform the methods described herein. Thus, the data must not contain too many missing or incorrect base entries. In the case of a target sequence that encodes a polypeptide, such errors can often be detected by virtually translating all three reading frames of the sense strand of the target sequence and confirming the presence of a continuous polypeptide sequence having predictable attributes, e.g., encoding a polypeptide of known size, or encoding a polypeptide that is about the same length as a homologous protein. In any event, only a very high frequency of sequence errors will frustrate the methods of the invention; most oligonucleotides to the target sequence will avoid such errors unless such errors occur frequently throughout the entire target sequence.

Another preferred criterion is that appropriate culturable cell lines or other source of reproducible genetic expression should be available. Such cell lines express, or can be induced to express, the gene comprising the target nucleic acid sequence. The oligonucleotide compounds generated by the process of the invention are assayed using such cell lines and, if such assaying is performed robotically, the cell line is preferably tractable to robotic manipulation such as by growth in 96 well plates. Those skilled in the art will recognize that if an appropriate cell line does not exist, it will nevertheless be possible to construct an appropriate cell line. For example, a cell line can be transfected with an expression vector comprising the target gene in order to generate an appropriate cell line for assay purposes.

For gene function analysis, it is possible to operate upon a genetic system having a lack of information regarding, or incomplete characterization of, the biological function(s) of the target nucleic acid or its gene product(s). This is a powerful agent of the invention. A target nucleic acid for gene function analysis might be absolutely uncharacterized, or might be thought to have a function based on minimal data or homology to another gene. By application of the process of the invention to such a target, active compounds that modulate the expression of the gene can be developed and applied to cells. The resulting cellular, biochemical or molecular biological responses are observed, and this information is used by those skilled in the art to elucidate the function of the target gene.

For target validation and drug discovery, another selection criterion is disease association. Candidate target genes are placed into one of several broad categories of known or deduced disease association. Level 1 Targets are target nucleic acids for which there is a strong correlation with disease. This correlation can come from multiple scientific disciplines including, but not limited to, epidemiology, wherein frequencies of gene abnormalities are associated with disease incidence; molecular biology, wherein gene expression and function are associated with cellular events correlated with a disease; and biochemistry, wherein the *in vitro* activities of a gene product are associated with disease parameters. Because there is a strong therapeutic rationale for focusing on Level 1 Targets, these targets are most preferred for drug discovery and/or target validation.

Level 2 Targets are nucleic acid targets for which the combined epidemiological, molecular biological, and/or biochemical correlation with disease is not so clear as for Level 1. Level 3 Targets are targets for which there is little or no data to directly link the target with a disease process, but there is indirect evidence for such a link, i.e., homology with a Level 1 or Level 2 target nucleic acid sequence or with the gene product thereof. In order not to prejudice the target selection process, and to ensure that the maximum number of nucleic acids actually involved in the causation, potentiation, aggravation, spread, continuance or after-effects of disease states are investigated, it is preferred to examine a balanced mix of Level 1, 2 and 3 target nucleic acids.

In order to carry out drug discovery, experimental systems and reagents shall be available in order for one to evaluate the therapeutic potential of active compounds generated by the process of the invention. Such systems may be operable *in vitro* (e.g., *in vitro* models of cell:cell association) or *in vivo* (e.g., animal models of disease states). It is also desirable, but not obligatory, to have available animal model systems which can be used to evaluate drug pharmacology.

Candidate targets nucleic acids can also classified by biological processes. For example, programmed cell death ("apoptosis") has recently emerged as an important biological process that is perturbed in a wide variety of diseases. Accordingly, nucleic acids that encode factors that play a role in the apoptotic process are identified as candidate targets. Similarly, potential target nucleic acids can be classified as being involved in inflammation, autoimmune disorders, cancer, or other pathological or dysfunctional processes.

Moreover, genes can often be grouped into families based on sequence homology and biological function. Individual family members can act redundantly, or can provide specificity through diversity of interactions with downstream effectors, or through expression being restricted to specific cell types. When one member of a gene family is associated with a disease process then the rationale for targeting other members of the same family is reasonably strong. Therefore, members of such gene families are preferred target nucleic acids to which the methods and systems of the invention may be applied. Indeed, the potent specificity of antisense compounds for different gene family members makes the invention particularly suited for such targets (Albert *et al., Trends Pharm. Sci.,* 1994, *15*, 250). Those skilled in the art will recognize that a partial or complete nucleotide sequence of such family members can be obtained using the polymerase chain reaction (PCR) and "universal" primers, i.e., primers designed to be common to all members of a given gene family.

PCR products generated from universal primers can be cloned and sequenced or directly sequenced using techniques known in the art. Thus, although nucleotide sequences from cloned DNAs, or from complementary DNAs (cDNAs) derived from mRNAs, may be used in the process of the invention, there is no requirement that the target nucleotide sequence be isolated from a cloned nucleic acid. Any nucleotide sequence, no matter how determined, of any nucleic acid, isolated or prepared in any fashion, may be used as a target nucleic acid in the process of the invention.

Furthermore, although polypeptide-encoding nucleic acids provide the target nucleotide sequences in one embodiment of the invention, other nucleic acids may be targeted as well. Thus, for example, the nucleotide sequences of structural or enzymatic RNAs may be utilized for drug discovery and/or target validation when such RNAs are associated with a disease state, or for gene function analysis when their biological role is not known.

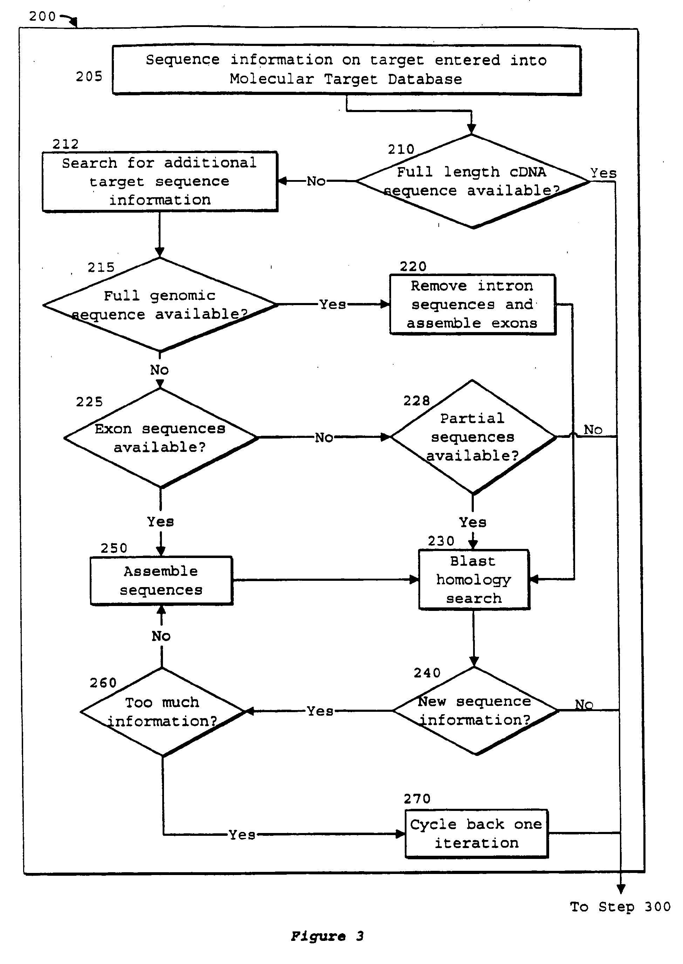

**2. Assembly of Target Nucleotide Sequence.** Figure 3 is a block diagram detailing the steps of the target nucleotide sequence assembly process, process step **200** in acccordance with one embodiment of the invention. The oligonucleotide design process, process step **300**, is facilitated by the availability of accurate target sequence information. Because of limitations of automated genome sequencing technology, gene sequences are often accumulated in fragments. Further, because individual genes are often being sequenced by independent laboratories using different sequencing strategies, sequence information corresponding to different fragments is often deposited in different databases. The target nucleic acid assembly process take advantage of computerized homology search algorithms and sequence fragment assembly algorithms to search available databases for related sequence information and incorporate available sequence information into the best possible representation of the target nucleic acid molecule, for example a RNA transcript. This representation is then used to design oligonucleotides, process step **300,** which can be tested for biological activity in process step **700.**

In the case of genes directing the synthesis of multiple transcripts, i.e. by alternative splicing, each distinct transcript is a unique target nucleic acid for purposes of step **300.** In one embodiment of the invention, if active compounds specific for a given transcript isoform are desired, the target nucleotide sequence is limited to those sequences that are unique to that transcript isoform. In another embodiment of the invention, if it is desired to modulate two or more transcript isoforms in concert, the target nucleotide sequence is limited to sequences that are shared between the two or more transcripts.

In the case of a polypeptide-encoding nucleic acid, it is generally preferred that full-length cDNA be used in the oligonucleotide design process step **300** (with full-length cDNA being defined as reading from the 5' cap to the poly A tail). Although full-length cDNA is preferred, it is possible to design oligonucleotides using partial sequence information. Therefore it is not necessary for the assembly process to generate a complete cDNA sequence. Further in some cases it may be desirable to design oligonucleotides targeting introns. In this case the process can be used to identify individual introns at process step **220.**

The process can be initiated by entering initial sequence information on a selected molecular target at process step **205.** In the case of a polypeptide-encoding nucleic acid, the full-length cDNA sequence is generally preferred for use in oligonucleotide design strategies at process step **300.** The first step is to determine if the initial sequence information represents the full-length cDNA, decision step **210.** In the case where the full-length cDNA sequence is available the process advances directly to the oligonucleotide design step **300.** When the full-length cDNA sequence is not available, databases are searched at process step **212** for additional sequence information.

The algorithm preferably used in process steps **212** and **230** is BLAST (Altschul, *et al., J. Mol. Biol*., **1990, *215*, 403),** or "Gapped BLAST" (Altschul *et al., Nucl. Acids Res.,* 1997, *25*, 3389). These are database search tools based on sequence homology used to identify related sequences in a sequence database. The BLAST search parameters are set to only identify closely related sequences. Some preferred databases searched by BLAST are a combination of public domain and proprietary databases. The databases, their contents, and sources are listed in Table 1.

**Table 1: Database Sources of Target Sequences**

| **Database** | **Contents** | **Source** |
|---|---|---|
| NR | All non-redundant GenBank, | National Center for Biotechnology |
| Month | EMBL, DDBJ and PDB sequences All new or revised GenBank, | Information at the National Institutes of Health National Center for Biotechnology |
| | EMBL, DDBJ and PDB sequences released in the last 30 days | Information at the National Institutes of Health |
| Dbest | Non-redundant database of GenBank, EMBL, DDBJ and EST divisions | National Center for Biotechnology Information at the National Institutes of Health |
| Dbsts | Non-redundant database of GenBank, EMBL, DDBJ and STS divisions | National Center for Biotechnology Information at the National Institutes of Health |
| Htgs | High throughput genomic sequences | National Center for Biotechnology Information at the National Institutes of Health |

When genomic sequence information is available at decision step **215,** introns are removed and exons are assembled into continuous sequence representing the cDNA sequence in process step **220.** Exon assembly occurs using the Phragment Assembly Program "Phrap" (Copyright University of Washington Genome Center, Seattle, WA). The Phrap algorithm analyzes sets of overlapping sequences and assembles them into one continuous sequence referred to as a "contig." The resulting contig is preferably used to search databases for additional sequence information at process step **230.** When genomic information is not available, the results of process step **212** are analyzed for individual exons at decision step **225.** Exons are frequently recorded individually in databases. If multiple complete exons are identified, they are prferably assembled into a contig using Phrap at process step **250.** If multiple complete exons are not identified at decision step **225,** then sequences can be analyzed for partial sequence information in decision step **228.** ESTs identified in the database dbEST are examples of such partial sequence information. If additional partial information is not found, then the process is advanced to process step **230** at decision step **228.** If partial sequence information is found in process **212** then that information is advanced to process step **230** via decision step **228.**

Process step **230,** decision step **240,** decision step **260** and process step **250** define a loop designed to extend iteratively the amount of sequence information available for targeting. At the end of each iteration of this loop, the results are analyzed in decision steps **240** and **260.** If no new information is found then the process advances at decision step **240** to process step **300.** If there is an unexpectedly large amount of sequence information identified, suggesting that the process moved outside the boundary of the gene into repetitive genomic sequence, then the process is preferably cycled back one iteration and that sequence is advanced at decision step **240** to process step **300.** If a small amount of new sequence information is identified, then the loop is iterated such as by taking the 100 most 5-prime (5') and 100 most 3-prime (3') bases and interating them through the BLAST homology search at process step **230.** New sequence information is added to the existing contig at process step **250.**

### 3. In Silico Generation of a Set of Nucleobase Sequences and Virtual Oligonucleotides.

For the following steps **300** and **400,** they may be performed in the order described below, i.e., step **300** before step **400,** or, in an alternative embodiment of the invention, step **400** before step **300.** In this alternate embodiment, each oligonucleotide chemistry is first assigned to each oligonucleotide sequence. Then, each combination of oligonucleotide chemistry and sequence is evaluated according to the parameters of step **300.** This embodiment has the desirable feature of taking into account the effect of alternative oligonucleotide chemistries on such parameters. For example, substitution of 5-methyl cytosine (5MeC or m5c) for cytosine in an antisense compound may enhance the stability of a duplex formed between that compound and its target nucleic acid. Other oligonucleotide chemistries that enhance oligonucleotide:[target nucleic acid] duplexes are known in the art (see for example, Freier *et al., Nucleic Acids Research,* 1997, *25*, 4429). As will be appreciated by those skilled in the art, different oligonucleotide chemistries may be preferred for different target nucleic acids. That is, the optimal oligonucleotide chemistry for binding to a target DNA might be suboptimal for binding to a target RNA having the same nucleotide sequence.

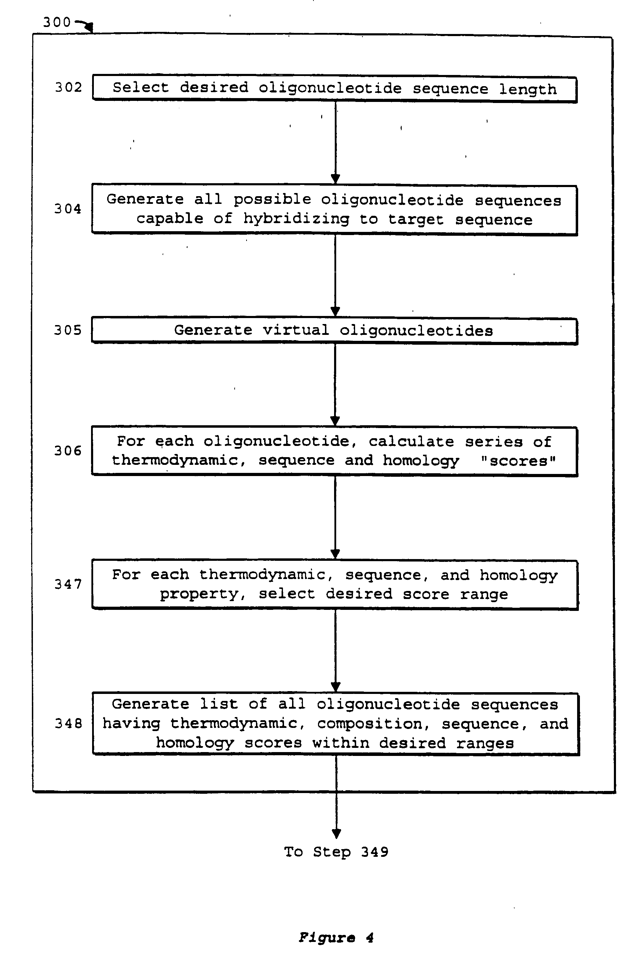

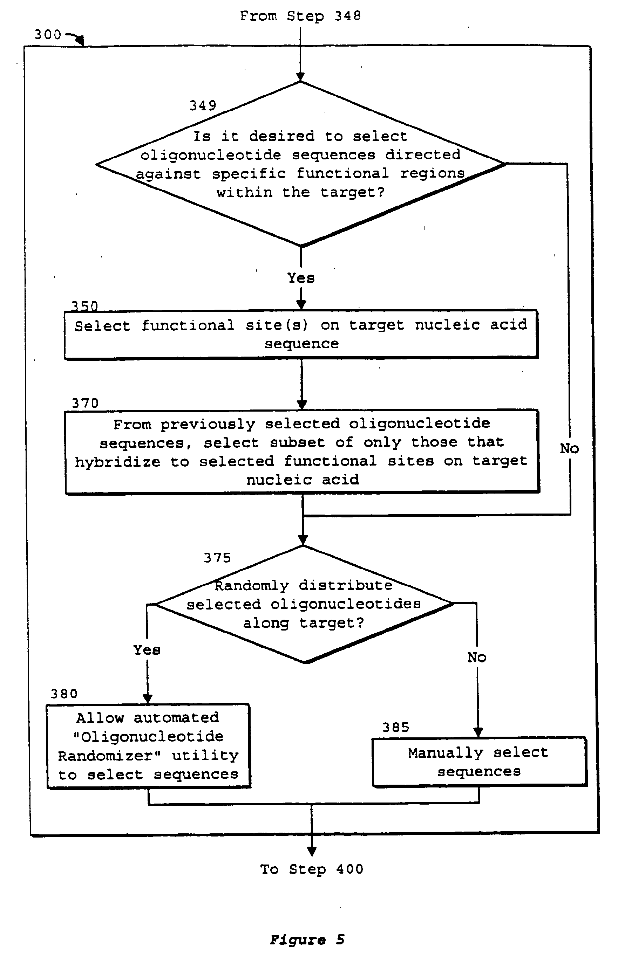

In effecting the process of the invention in the order step **300** before step **400** as seen in Figure 1, from a target nucleic acid sequence assembled at step **200,** a list of oligonucleotide sequences is generated as represented in the flowchart shown in Figures 4 and 5. In step **302,** the desired oligonucleotide length is chosen. In a preferred embodiment, oligonucleotide length is between from about 8 to about 30, more preferably from about 12 to about 25, nucleotides. In step **304,** all possible oligonucleotide sequences of the desired length capable of hybridizing to the target sequence obtained in step **200** are generated. In this step, a series of oligonucleotide sequences are generated, simply by determining the most 5' oligonucleotide possible and "walking" the target sequence in increments of one base until the 3' most oligonucleotide possible is reached.

In step **305,** a virtual oligonucleotide chemistry is applied to the nucleobase sequences of step **304** in order to yield a set of virtual oligonucleotides that can be evaluated *in silico.* Default virtual oligonucleotide chemistries include those that are well-characterized in terms of their physical and chemical properties, e.g., 2'-deoxyribonucleic acid having naturally occurring bases (A, T, C and G), unmodified sugar residues and a phosphodiester backbone.

### 4. In Silico Evaluation of Thermodynamic Properties of Virtual Oligonucleotides.

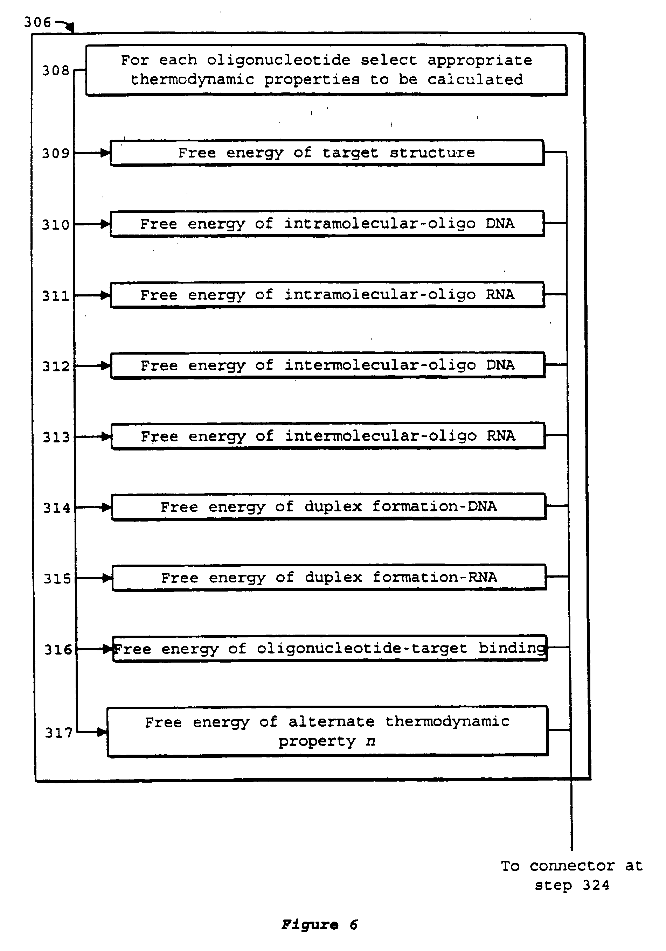

In step **306,** a series of thermodynamic, sequence, and homology scores are preferably calculated for each virtual oligonucleotide obtained from step **305.** Thermodynamic properties are calculated as represented in Figure 6. In step **308,** the desired thermodynamic properties are selected. As many or as few as desired can be selected; optionally, none will be selected. The desired properties will typically include step **309,** calculation of the free energy of the target structure. If the oligonucleotide is a DNA molecule, then steps **310, 312,** and **314** are performed. If the oligonucleotide is an RNA molecule, then steps **311, 313** and **315** are performed. In both cases, these steps correspond to calculation of the free energy of intramolecular oligonucleotide interactions, intermolecular interactions and duplex formation. In addition, a free energy of oligonucleotide-target binding is preferably calculated at step **316**.

Other thermodynamic and kinetic properties may be calculated for oligonucleotides as represented at step **317.** Such other thermodynamic and kinetic properties may include melting temperatures, association rates, dissociation rates, or any other physical property that may be predictive of oligonucleotide activity.

The free energy of the target structure is defined as the free energy needed to disrupt any secondary structure in the target binding site of the targeted nucleic acid. This region includes any intra-target nucleotide base pairs that need to be disrupted in order for an oligonucleotide to bind to its complementary sequence. The effect of this localized disruption of secondary structure is to provide accessibility by the oligonucleotide. Such structures will include double helices, terminal unpaired and mismatched nucleotides, loops, including hairpin loops, bulge loops, internal loops and multibranch loops (Serra *et al., Methods in Enzymology,* 1995, *259,* 242).

The intermolecular free energies refer to inherent energy due to the most stable structure formed by two oligonucleotides; such structures include dimer formation. Intermolecular free energies should also be taken into account when, for example, two or more oligonucleotides, of different sequence are to be administered to the same cell in an assay.

The intramolecular free energies refer to the energy needed to disrupt the most stable secondary structure within a single oligonucleotide. Such structures include, for example, hairpin loops, bulges and internal loops. The degree of intramolecular base pairing is indicative of the energy needed to disrupt such base pairing.

The free energy of duplex formation is the free energy of denatured oligonucleotide binding to its denatured target sequence. The oligonucleotide-target binding is the total binding involved, and includes the energies involved in opening up intra- and inter- molecular oligonucleotide structures, opening up target structure, and duplex formation.

The most stable RNA structure is predicted based on nearest neighbor analysis (Xia, T., *et al., Biochemistry,* 1998, *37,* 14719-14735; Serra *et al., Methods in Enzymology,* 1995, *259*, 242). This analysis is based on the assumption that stability of a given base pair is determined by the adjacent base pairs. For each possible nearest neighbor combination, thermodynamic properties have been determined and are provided. For double helical regions, two additional factors need to be considered, an entropy change required to initiate a helix and a entropy change associated with self-complementary strands only. Thus, the free energy of a duplex can be calculated using the equation: $Δ G {°}_{T} = Δ H ° - T Δ S °$
where:
*ΔG* is the free energy of duplex formation,
*ΔH* is the enthalpy change for each nearest neighbor,
*ΔS* is the entropy change for each nearest neighbor, and *T* is temperature.

The *ΔH* and *ΔS* for each possible nearest neighbor combination have been experimentally determined. These letter values are often available in published tables. For terminal unpaired and mismatched nucleotides, enthalpy and entropy measurements for each possible nucleotide combination are also available in published tables. Such results are added directly to values determined for duplex formation. For loops, while the available data is not as complete or accurate as for base pairing, one known model determines the free energy of loop formation as the sum of free energy based on loop size, the closing base pair, the interactions between the first mismatch of the loop with the closing base pair, and additional factors including being closed by AU or UA or a first mismatch of GA or UU. Such equations may also be used for oligoribonucleotide-target RNA interactions.

The stability of DNA duplexes is used in the case of intra- or intermolecular oligodeoxyribonucleotide interactions. DNA duplex stability is calculated using similar equations as RNA stability, except experimentally determined values differ between nearest neighbors in DNA and RNA and helix initiation tends to be more favorable in DNA than in RNA (SantaLucia *et al., Biochemistry,* 1996, *35,* 3555).

Additional thermodynamic parameters are used in the case of RNA/DNA hybrid duplexes. This would be the case for an RNA target and oligodeoxynucleotide. Such parameters were determined by Sugimoto *et al. (Biochemistry*, 1995, *34*, 11211). In addition to values for nearest neighbors, differences were seen for values for enthalpy of helix initiation.

### 5. In Silico Evaluation of Target Accessibility

Target accessibility is believed to be an important consideration in selecting oligonucleotides. Such a target site will possess minimal secondary structure and thus, will require minimal energy to disrupt such structure. In addition, secondary structure in oligonucleotides, whether inter- or intra-molecular, is undesirable due to the energy required to disrupt such structures. Oligonucleotide-target binding is dependent on both these factors. It is desirable to minimize the contributions of secondary structure based on these factors. The other contribution to oligonucleotide-target binding is binding affinity. Favorable binding affinities based on tighter base pairing at the target site is desirable.

Following the calculation of thermodynamic properties ending at step **317,** the desired sequence properties to be scored are selected at step **324.** As many or as few as desired can be selected; optionally, none will be selected. These properties include the number of strings of four guanosine residues in a row at step **325** or three guanosine in a row at step **326,** the length of the longest string of adenosines at step **327,** cytidines at step **328** or uridines or thymidines at step **329,** the length of the longest string of purines at step **330** or pyrimidine at step **331,** the percent composition of adenosine at step **332,** cytidine at step **333,** guanosine at step **334** or uridines or thymidines at step **335,** the percent composition of purines at step **336** or pyrimidines at step **337,** the number of CG dinucleotide repeats at step **338,** CA dinucleotide repeats at step **339** or UA or TA dinucleotide repeats at step **340.** In addition, other sequence properties may be used as found to be relevant and predictive of antisense efficacy, as represented at step **341.**

These sequence properties may be important in predicting oligonucleotide activity, or lack thereof. For example, U.S. Patent 5,523,389 discloses oligonucleotides containing stretches of three or four guanosine residues in a row. Oligonucleotides having such sequences may act in a sequence-independent manner. For an antisense approach, such a mechanism is not usually desired. In addition, high numbers of dinucleotide repeats may be indicative of low complexity regions which may be present in large numbers of unrelated genes. Unequal base composition, for example, 90% adenosine, can also give non-specific effects. From a practical standpoint, it may be desirable to remove oligonucleotides that possess long stretches of other nucleotides due to synthesis considerations. Other sequences properties, either listed above or later found to be of predictive value may be used to select oligonucleotide sequences.

Following step **341,** the homology scores to be calculated are selected in step **342.** Homology to nucleic acids encoding protein isoforms of the target, as represented at step **343,** may be desired. For example, oligonucleotides specific for an isoform of protein kinase C can be selected. Also, oligonucleotides can be selected to target multiple isoforms of such genes. Homology to analogous target sequences, as represented at step **344,** may also be desired. For example, an oligonucleotide can be selected to a region common to both humans and mice to facilitate testing of the oligonucleotide in both species. Homology to splice variants of the target nucleic acid, as represented at step **345,** may be desired. In addition, it may be desirable to determine homology to other sequence variants as necessary, as represented in step **346.**

Following step **346,** from which scores were obtained in each selected parameter, a desired range is selected to select the most promising oligonucleotides, as represented at step **347.** Typically, only several parameters will be used to select oligonucleotide sequences. As structure prediction improves, additional parameters may be used. Once the desired score ranges are chosen, a list of all oligonucleotides having parameters falling within those ranges will be generated, as represented at step **348.**

### 6. Targeting Oligonucleotides to Functional Regions of a Nucleic Acid.

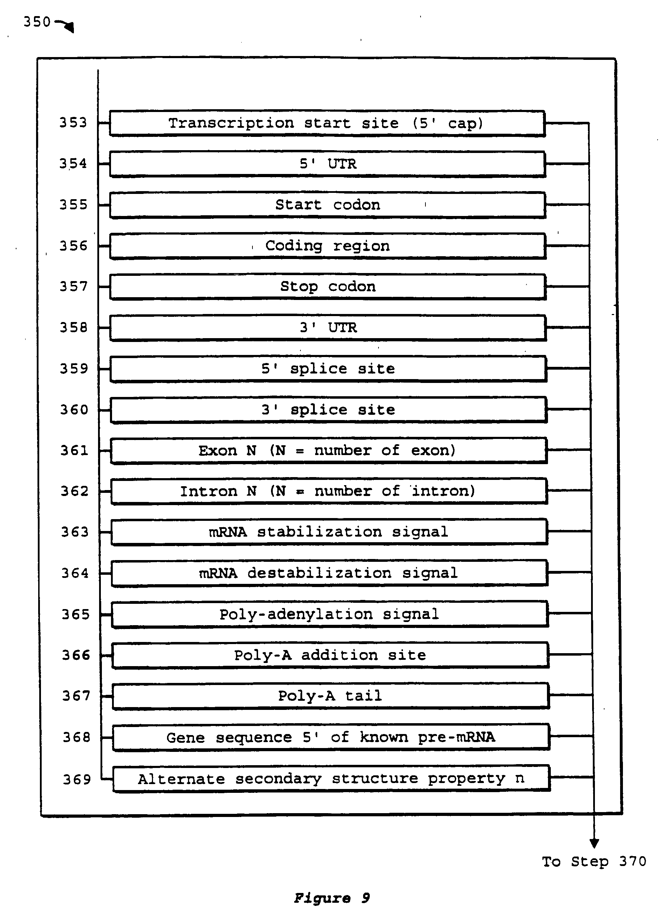

It may be desirable to target oligonucleotide sequences to specific functional regions of the target nucleic acid. A decision is made whether to target such regions, as represented in decision step **349.** If it is desired to target functional regions then process step **350** occurs as seen in greater detail in Figure 9. If it is not desired then the process proceeds to step **375.**

In step **350,** as seen in Figure 9, the desired functional regions are selected. Such regions include the transcription start site or 5' cap at step **353,** the 5' untranslated region at step **354,** the start codon at step **355,** the coding region at step **356,** the stop codon at step **357,** the 3' untranslated region at step **358,** 5' splice sites at step **359** or 3' splice sites at step **360,** specific exons at step **361** or specific introns at step **362,** mRNA stabilization signal at step **363,** mRNA destabilization signal at step **364,** poly-adenylation signal at step **365,** poly-A addition site at step **366,** poly-A tail at step **367,** or the gene sequence 5' of known pre-mRNA at step **368.** In addition, additional functional sites may be selected, as represented at step **369.**

Many functional regions are important to the proper processing of the gene and are attractive targets for antisense approaches. For example, the AUG start codon is commonly targeted because it is necessary to initiate translation. In addition, splice sites are thought to be attractive targets because these regions are important for processing of the mRNA. Other known sites may be more accessible because of interactions with protein factors or other regulatory molecules.

After the desired functional regions are selected and determined, then a subset of all previously selected oligonucleotides are selected based on hybridization to only those desired functional regions, as represented by step 370.

### 7. Uniform Distribution of Oligonucleotides.

Whether or not targeting functional sites is desired, a large number of oligonucleotide sequences may result from the process thus far. In order to reduce the number of oligonucleotide sequences to a manageable number, a decision is made whether to uniformly distribute selected oligonucleotides along the target, as represented in step 375. A uniform distribution of oligonucleotide sequences will aim to provide complete coverage throughout the complete target nucleic acid or the selected functional regions. A computer-based program is used to automate the distribution of sequences, as represented in step **380.** Such a program factors in parameters such as length of the target nucleic acid, total number of oligonucleotide sequences desired, oligonucleotide sequences per unit length, number of oligonucleotide sequences per functional region. Manual selection of oligonucleotide sequences is also provided for by step **385.** In some cases, it may be desirable to manually select oligonucleotide sequences. For example, it may be useful to determine the effect of small base shifts on activity. Once the desired number of oligonucleotide sequences is obtained either from step **380** or step **385,** then these oligonucleotide sequences are passed onto step 400 of the process, where oligonucleotide chemistries are assigned.

### 8. Assignment of Actual Oligonucleotide Chemistry.

Once a set of select nucleobase sequences has been generated according to the preceding process and decision steps, actual oligonucleotide chemistry is assigned to the sequences. An "actual oligonucleotide chemistry" or simply "chemistry" is a chemical motif that is common to a particular set of robotically synthesized oligonucleotide compounds. Preferred chemistries include, but are not limited to, oligonucleotides in which every linkage is a phosphorothioate linkage, and chimeric oligonucleotides in which a defined number of 5' and/or 3' terminal residues have a 2'-methoxyethoxy modification.

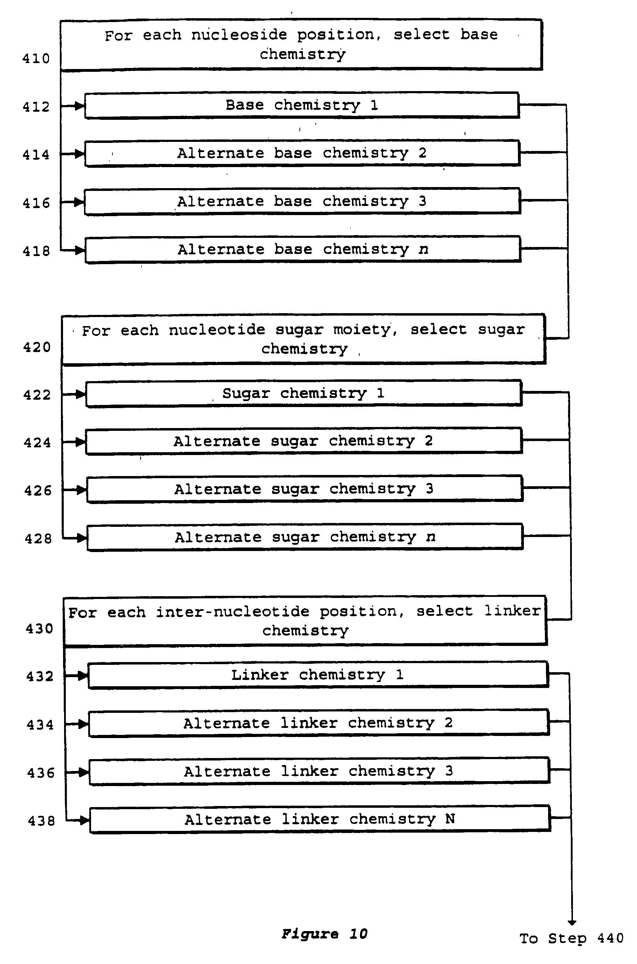

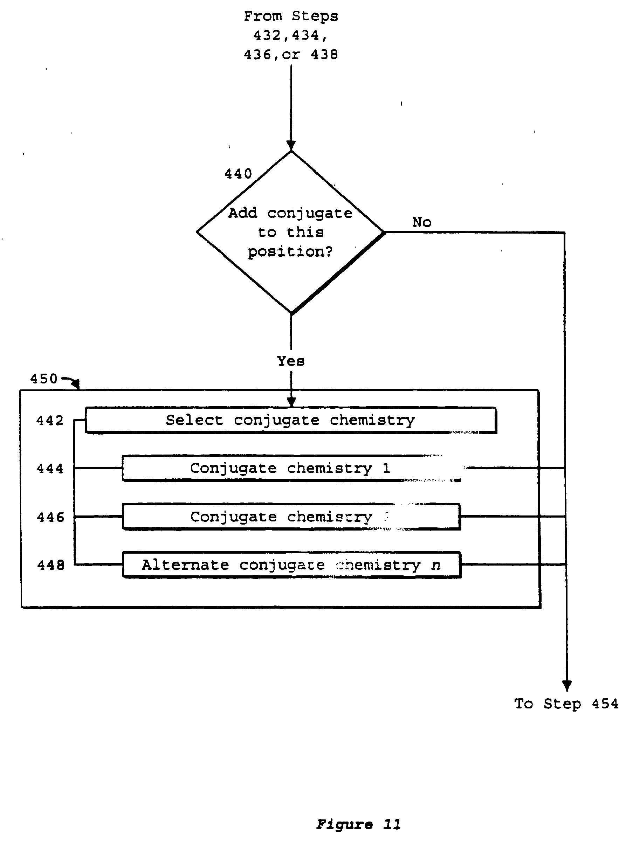

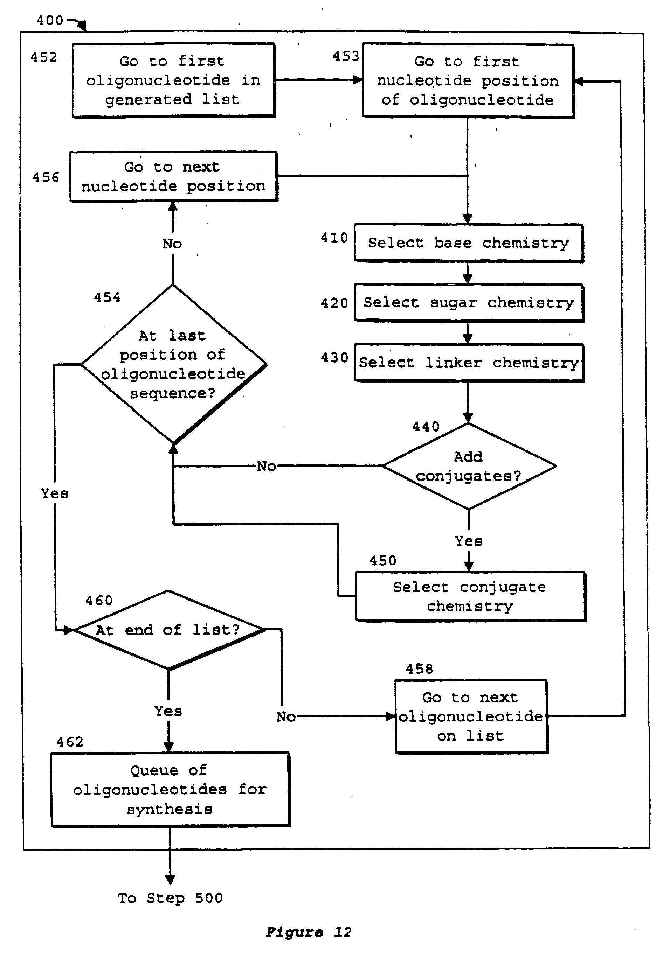

Chemistries can be assigned to the nucleobase sequences during general procedure step **400** (Figure 1). The logical basis for chemistry assignment is illustrated in Figures 10 and 11 and an iterative routine for stepping through an oligonucleotide nucleoside by nucleoside is illustrated in Figure 12. Chemistry assignment can be effected by assignment directly into a word processing program, via an interactive word processing program or via automated programs and devices. In each of these instances, the output file is selected to be in a format that can serve as an input file to automated synthesis devices.

### 9. Oligonucleotide Compounds.

In the context of this invention, in reference to oligonucleotides, the term "oligonucleotide" is used to refer to an oligomer or polymer of ribonucleic acid (RNA) or deoxyribonucleic acid (DNA) or mimetics thereof. Thus this term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms, i.e., phosphodiester linked A, C, G, T and U nucleosides, because of desirable properties such as, for example, enhanced cellular uptake, enhanced affinity for nucleic acid target and increased stability in the presence of nucleases.

The oligonucleotide compounds in accordance with this invention can be of various lengths depending on various parameters, including but not limited to those discussed above in reference to the selection criteria of general procedure **300**. For use as antisense oligonucleotides compounds of the invention preferably are from about 8 to about 30 nucleobases in length (i.e. from about 8 to about 30 linked nucleosides). Particularly preferred are antisense oligonucleotides comprising from about 12 to about 25 nucleobases. A discussion of antisense oligonucleotides and some desirable modifications can be found in De Mesmaeker *et al., Acc. Chem. Res.,* 1995, *28,* 366. Other lengths of oligonucleotides might be selected for non-antisense targeting strategies, for instance using the oligonucleotides as ribozymes. Such ribozymes normally require oligonucleotides of longer length as is known in the art.

A nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a normal (where normal is defined as being found in RNA and DNA) pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure, however, open linear structures are generally preferred. Within the oligonucleotide structure, the phosphate groups are commonly referred to as forming the internucleoside backbone of the oligonucleotide. The normal linkage or backbone of RNA and DNA is a 3' to 5' phosphodiester linkage.

Specific examples of preferred oligonucleotides useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

### 10. Selection of Oligonucleotide Chemistries.

In a general logic scheme as illustrated in Figures 10 and 11, for each nucleoside position, the user or automated device is interrogated first for a base assignment, followed by a sugar assignment, a linker assignment and finally a conjugate assignment. Thus for each nucleoside, at process step **410** a base is selected. In selecting the base, base chemistry 1 can be selected at process step **412** or one or more alternative bases are selected at process steps **414, 416** and **418.** After base selection is effected, the sugar portion of the nucleoside is selected. Thus for each nucleoside, at process step **420** a sugar is selected that together with the select base will complete the nucleoside. In selecting the sugar, sugar chemistry 1 can be selected at process **422** or one or more alternative sugars are selected at process steps **424, 426** and **428.** For each two adjacent nucleoside units, at process step **430,** the internucleoside linker is selected. The linker chemistry for the internucleoside linker can be linker chemistry 1 selected at process step **432** or one or more alternative internucleoside linker chemistries are selected at process steps **434, 436** and **438.**

In addition to the base, sugar and internucleoside linkage, at each nucleoside position, one or more conjugate groups can be attached to the oligonucleotide via attachment to the nucleoside or attachment to the internucleoside linkage. The addition of a conjugate group is integrated at process step **440** and the assignment of the conjugate group is effected at process step **450.**

For illustrative purposes in Figures 10 and 11, for each of the bases, the sugars, the internucleoside linkers, or the conjugates, chemistries 1 though n are illustrated. As described in this specification, it is understood that the number of alternate chemistries between chemistry 1 and alternative chemistry n, for each of the bases, the sugars, the internucleoside linkages and the conjugates, is variable and includes, but is not limited to, each of the specific alternative bases, sugar, internucleoside linkers and conjugates identified in this specification as well as equivalents known in the art.

Utilizing the logic as described in conjunction with Figures 10 and 11, chemistry is assigned, as is shown in Figure 12, to the list of oligonucleotides from general procedure **300.** In assigning chemistries to the oligonucleotides in this list, a pointer can be set at process step **452** to the first oligonucleotide in the list and at step **453** to the first nucleotide of that first oligonucleotide. The base chemistry is selected at step **410,** as described above, the sugar chemistry is selected at step **420,** also as described above, followed by selection of the internucleoside linkage at step **430,** also as described above. At decision **440,** the process branches depending on whether a conjugate will be added at the current nucleotide position. If a conjugate is desired, the conjugate is selected at step **450,** also as described above.

Whether or not a conjugate was added at decision step **440,** an inquiry is made at decision step **454.** This inquiry asks if the pointer resides at the last nucleotide in the current oligonucleotide. If the result at decision step **454** is "No," the pointer is moved to the next nucleotide in the current oligonucleotide and the loop including steps **410, 420, 430, 440** and **454** is repeated. This loop is reiterated until the result at decision step **454** is "Yes."

When the result at decision step **454** is "Yes," a query is made at decision step **460** concerning the location of the pointer in the list of oligonucleotides. If the pointer is not at the last oligonucleotide of the list, the "No" path of the decision step **460** is followed and the pointer is moved to the first nucleotide of the next oligonucleotide in the list at process step **458.** With the pointer set to the next oligonucleotide in the list, the loop that starts at process steps **453** is reiterated. When the result at decision step **460** is "Yes," chemistry has been assigned to all of the nucleotides in the list of oligonucleotides.

### 11. Description of Oligonucleotide Chemistries.

As is illustrated in Figure 10, for each nucleoside of an oligonucleotide, chemistry selection includes selection of the base forming the nucleoside from a large palette of different base units available. These may be "modified" or "natural" bases (also reference herein as nucleobases) including the natural purine bases adenine (A) and guanine (G), and the natural pyrimidine bases thymine (T), cytosine (C) and uracil (U). They further can include modified nucleobases including other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo uracils and cytosines particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Further nucleobases include those disclosed in United States Patent No. 3,687,808, those disclosed in the *Concise Encyclopedia Of Polymer Science And Engineering,* pages 858-859, Kroschwitz, J.I., ed. John Wiley & Sons, 1990, those disclosed by Englisch *et al., Angewandte Chemie, International Edition,* 1991, 30, 613, and those disclosed by Sanghvi, Y.S., Chapter 15, *Antisense Research and Applications,* pages 289-302, Crooke, S.T. and Lebleu, B., ed., CRC Press, 1993. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C (Sanghvi, Y.S., Crooke, S.T. and Lebleu, B., eds., *Antisense Research and Applications,* CRC Press, Boca Raton, 1993, pp. 276-278) and are presently preferred for selection as the base. These are particularly useful when combined with a 2'-O-methoxyethyl sugar modifications, described below.

Representative United States patents that teach the preparation of certain of the above noted modified nucleobases as well as other modified nucleobases include, but are not limited to, the above noted U.S. Patent 3,687,808, as well as U.S. Patents 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941. Reference is also made to allowed United States patent application 08/762,488, filed on December 10, 1996, commonly owned with the present application.

In selecting the base for any particular nucleoside of an oligonucleotide, consideration is first given to the need of a base for a particular specificity for hybridization to an opposing strand of a particular target. Thus if an "A" base is required, adenine might be selected however other alternative bases that can effect hybridization in a manner mimicking an "A" base such as 2-aminoadenine might be selected should other consideration, e.g., stronger hybridization (relative to hybridization achieved with adenine), be desired.

As is illustrated in Figure 10, for each nucleoside of an oligonucleotide, chemistry selection includes selection of the sugar forming the nucleoside from a large palette of different sugar or sugar surrogate units available. These may be modified sugar groups, for instance sugars containing one or more substituent groups. Preferred substituent groups comprise the following at the 2' position: OH; F; O-, S-, or N-alkyl; O-, S-, or N-alkenyl; or O, S- or N-alkynyl; wherein the alkyl, alkenyl and alkynyl may be substituted or unsubstituted C₁ to C₁₀ alkyl or C₂ to C₁₀ alkenyl and alkynyl. Particularly preferred are O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. Other preferred substituent groups comprise one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-CH₂CH₂OCH₃, also known as 2'-O-(2-methoxyethyl), 2'-O-methoxyethyl, or 2'-MOE) (Martin *et al., Helv. Chim. Acta,* 1995, *78*, 486) i.e., an alkoxyalkoxy group. A further preferred modification includes 2'-dimethylamino oxyethoxy, i.e., a O(CH₂)₂ON(CH₃)₂ group, also known as 2'-DMAOE, as described in co-owned United States patent application Serial Number 09/016,520, filed on January 30, 1998, which is incorporated herein by reference in its entirety.

Other preferred modifications include 2'-methoxy (2'-O-CH₃), 2'-aminopropoxy (2'-OCH₂CH₂CH₂NH₂) and 2'-fluoro (2'-F). Similar modifications may also be made at other positions on the sugar group, particularly the 3' position of the sugar on the 3' terminal nucleotide or in 2'-5' linked oligonucleotides and the 5' position of 5' terminal nucleotide. The nucleosides of the oligonucleotides may also have sugar mimetics such as cyclobutyl moieties in place of the pentofuranosyl sugar.

Representative United States patents that teach the preparation of such modified sugars structures include, but are not limited to, U.S. Patents 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053 5,639,873; 5,646,265; 5,658,873; 5,670,633; and 5,700,920, certain of which are commonly owned with the present application, together with allowed United States patent application 08/468,037, filed on June 5, 1995, which is commonly owned with the present application.

As is illustrated in Figure 10, for each adjacent pair of nucleosides of an oligonucleotide, chemistry selection includes selection of the internucleoside linkage. These internucleoside linkages are also referred to as linkers, backbones or oligonucleotide backbones. For forming these nucleoside linkages, a palette of different internucleoside linkages or backbones is available. These include modified oligonucleotide backbones, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalklyphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Various salts, mixed salts and free acid forms are also included.

Representative United States patents that teach the preparation of the above phosphorus containing linkages include, but are not limited to, U.S. Patents 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,625,050; and 5,697,248, certain of which are commonly owned with this application.

Preferred internucleoside linkages for oligonucleotides that do not include a phosphorus atom therein, i.e., for oligonucleosides, have backbones that are formed by short chain alkyl or cycloalkyl intersugar linkages, mixed heteroatom and alkyl or cycloalkyl intersugar linkages, or one or more short chain heteroatomic or heterocyclic intersugar linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

Representative United States patents that teach the preparation of the above oligonucleosides include, but are not limited to, U.S. Patents 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, certain of which are commonly owned with this application.

In other preferred oligonucleotides, i.e., oligonucleotide mimetics, both the sugar and the intersugar linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-phosphate backbone of an oligonucleotide is replaced with an amide-containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S.: 5,539,082; 5,714,331; and 5,719,262. Further teaching of PNA compounds can be found in Nielsen *et al., Science,* 1991, *254,* 1497.

For the internucleoside linkages, the most preferred embodiments of the invention are oligonucleotides with phosphorothioate backbones and oligonucleosides with heteroatom backbones, and in particular -CH₂-NH-O-CH₂-, -CH₂-N(CH₃)-O-CH₂- [known as a methylene (methylimino) or MMI backbone], -CH₂-O-N(CH₃)-CH₂-,- CH₂-N(CH₃)-N(CH₃)-CH₂- and -O-N(CH₃)-CH₂-CH₂- (wherein the native phosphodiester backbone is represented as -O-P-O-CH₂-) of the above referenced U.S. patent 5,489,677, and the amide backbones of the above referenced U.S. patent 5,602,240. Also preferred are oligonucleotides having morpholino backbone structures of the above-referenced U.S. Patent 5,034,506.

In attaching a conjugate group to one or more nucleosides or internucleoside linkages of an oligonucleotide, various properties of the oligonucleotide are modified. Thus modification of the oligonucleotides of the invention to chemically link one or more moieties or conjugates to the oligonucleotide are intended to enhance the activity, cellular distribution or cellular uptake of the oligonucleotide. Such moieties include but are not limited to lipid moieties such as a cholesterol moiety (Letsinger *et al., Proc. Natl. Acad. Sci. USA*, 1989, *86*, 6553), cholic acid (Manoharan *et al., Bioorg. Med. Chem. Let.,* 1994, *4*, 1053), a thioether, e.g., hexyl-*S*-tritylthiol (Manoharan *et al., Ann. N.Y. Acad. Sci.,* 1992, *660*, 306; Manoharan *et al., Bioorg. Med. Chem. Let*., 1993, *3*, 2765), a thiocholesterol (Oberhauser *et al., Nucl. Acids Res.,* 1992, *20*, 533), an aliphatic chain, e.g., dodecandiol or undecyl residues (Saison-Behmoaras *et al., EMBO J.,* 1991, *10*, 111; *Kabanov et al., FEBS Lett.,* 1990, 259, 327; Svinarchuk *et al., Biochimie,* 1993, *75*, 49), a phospholipid, e.g., di-hexadecyl-*rac*-glycerol or triethylammonium 1,2-di-O-hexadecyl-*rac*-glycero-3-H-phosphonate (Manoharan *et al., Tetrahedron Lett*., 1995, *36,* 3651; Shea *et al., Nucl. Acids Res.,* 1990, *18*, 3777), a polyamine or a polyethylene glycol chain (Manoharan *et al., Nucleosides & Nucleotides,* 1995, *14*, 969), or adamantane acetic acid (Manoharan *et al., Tetrahedron Lett.,* 1995, *36,* 3651), a palmityl moiety (Mishra *et al., Biochim. Biophys. Acta,* 1995, *1264,* 229), or an octadecylamine or hexylamino-carbonyloxycholesterol moiety (Crooke *et al., J. Pharmacol. Exp. Ther.,* 1996, *277*, 923).

Representative United States patents that teach the preparation of such oligonucleotide conjugates include, but are not limited to, U.S. Patents 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941, certain of which are commonly owned with the present application.

### 12. Chimeric Compounds.

It is not necessary for all positions in a given compound to be uniformly modified. In fact, more than one of the aforementioned modifications may be incorporated in a single compound or even at a single nucleoside within an oligonucleotide. The present invention also includes compounds which are chimeric compounds. "Chimeric" compounds or "chimeras," in the context of this invention, are compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids.

By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds of the invention may be formed as composite structures representing the union of two or more oligonucleotides, modified oligonucleotides, oligonucleosides and/or oligonucleotide mimetics as described above. Such compounds have also been referred to in the art as "hybrids" or "gapmers". Representative United States patents that teach the preparation of such hybrid structures include, but are not limited to, U.S. Patents 5,013,830; 5,149,797; 5,220,007; 5,256,775; 5,366,878; 5,403,711; 5,491,133; 5,565,350; 5,623,065; 5,652,355; 5,652,356; and 5,700,922, certain of which are commonly owned with the present application together with commonly owned and allowed United States patent application serial number 08/465,880, filed on June 6, 1995.

### 13. Description of Automated Oligonucleotide Synthesis.

In the next step of the overall process (illustrated in Figures 1 and 2), oligonucleotides are, synthesized on an automated synthesizer. Although many devices may be employed, the synthesizer is preferably a variation of the synthesizer described in United States patents 5,472,672 and 5,529,756, each of which is incorporated herein by reference in its entirety. The synthesizer described in those patents is modified to include movement in along the Y axis in addition to movement along the X axis. As so modified, a 96-well array of compounds can be synthesized by the synthesizer. The synthesizer further includes temperature control and the ability to maintain an inert atmosphere during all phases of synthesis. The reagent array delivery format employs orthogonal X-axis motion of a matrix of reaction vessels and Y-axis motion of an array of reagents. Each reagent has its own dedicated plumbing system to eliminate the possibility of cross-contamination of reagents and line flushing and/or pipette washing. This in combined with a high delivery speed obtained with a reagent mapping system allows for the extremely rapid delivery of reagents. This further allows long and complex reaction sequences to be performed in an efficient and facile manner.

The software that operates the synthesizer allows the straightforward programming of the parallel synthesis of a large number of compounds. The software utilizes a general synthetic procedure in the form of a command (.cmd) file, which calls upon certain reagents to be added to certain wells ***via*** lookup in a sequence (.seq) file. The bottle position, flow rate, and concentration of each reagent is stored in a lookup table (.tab) file. Thus, once any synthetic method has been outlined, a plate of compounds is made by permutating a set of reagents, and writing the resulting output to a text file. The text file is input directly into the synthesizer and used for the synthesis of the plate of compounds. The synthesizer is interfaced with a relational database allowing data output related to the synthesized compounds to be registered in a highly efficient manner.

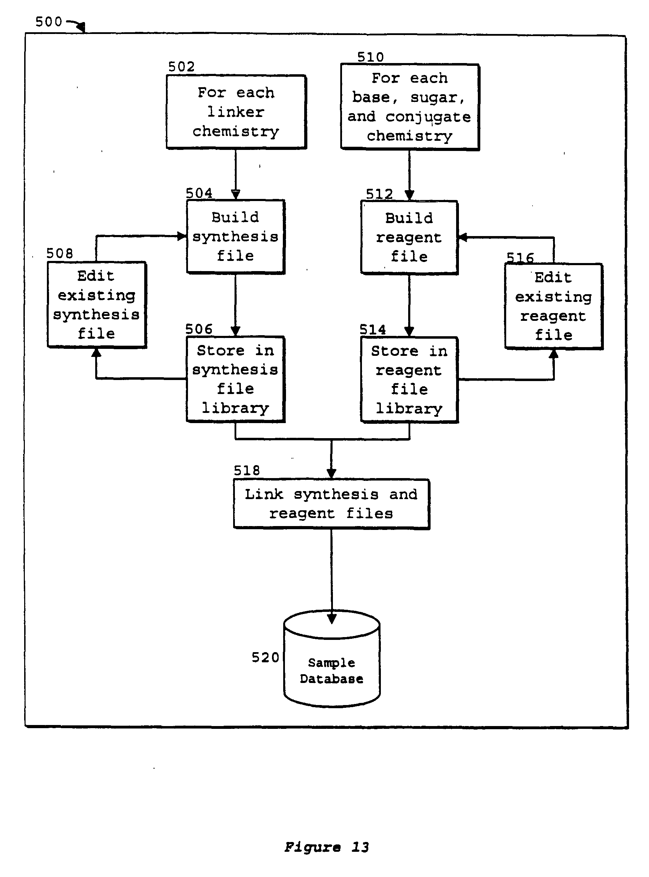

Building of the .seq, .cmd and .tab files is illustrated in Figure 13. Thus as a part of the general oligonucleotide synthesis procedure **500,** for each linker chemistry at process step **502,** a synthesis file, i.e., a .cmd file, is built at process step **504.** This file can be built fresh to reflect a completely new set of machine commands reflecting a set of chemical synthesis steps or it can modify an existing file stored at process step **504** by editing that stored file in process step **508.** The .cmd files are built using a word processor and a command set of instructions as outlined below.

It will be appreciated that the preparation of control software and data files is within the routine skill of persons skilled in annotated nucleotide synthesis. The same will depend upon the hardware employed, the chemistries adopted and the design paradigm selected by the operator.

In a like manner to the building the .cmd files, .tab files are built to reflect the necessary reagents used in the automatic synthesizer for the particular chemistries that have been selected for the linkages, bases, sugars and conjugate chemistries. Thus for each of a set of these chemistries at process step **510,** a .tab file is built at process step **512** and stored at process step **514.** As with the .cmd files, an existing .tab file can be edited at process step **516.**

Both the .cmd files and the .tab files are linked together at process step **518** and stored for later retrieval in an appropriate sample database **520.** Linking can be as simple as using like file names to associate a .cmd file to its appropriate .tab file, e.g., synthesis_1.cmd is linked to synthesis_1.tab by use of the same preamble in their names.

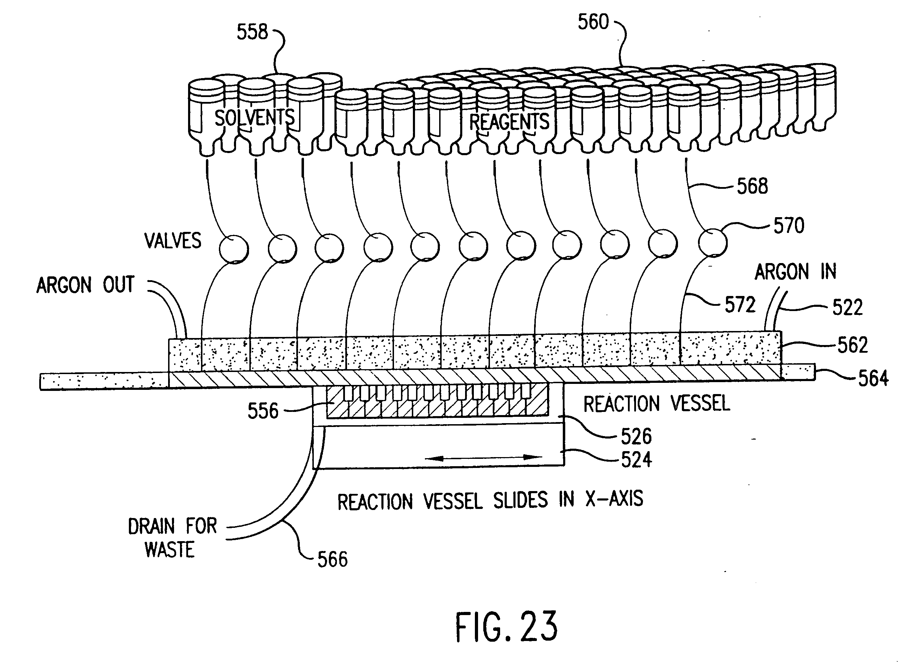

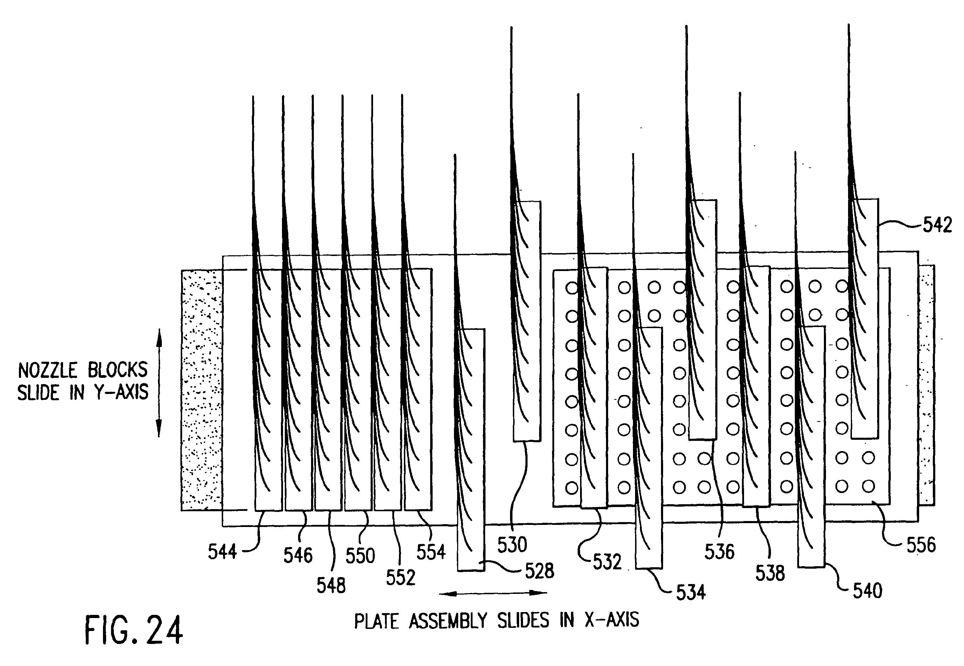

The automated, multi-well parallel array synthesizer employs a reagent array delivery format, in which each reagent utilized has a dedicated plumbing system. As seen in Figures 23 and 24, an inert atmosphere **522** is maintained during all phases of a synthesis. Temperature is controlled via a thermal transfer plate **524,** which holds an injection molded reaction block **526.** The reaction plate assembly slides in the X-axis direction, while for example eight nozzle blocks **(528, 530, 532, 534, 536, 538, 540** and **542)** holding the reagent lines slide in the Y-axis direction, allowing for the extremely rapid delivery of any of 64 reagents to 96 wells. In addition, there are for example, six banks of fixed nozzle blocks **(544, 546, 548, 550, 552** and **554)** which deliver the same reagent or solvent to eight wells at once, for a total of 72 possible reagents.

In synthesizing oligonucleotides for screening, the target reaction vessels, a 96 well plate **556** (a 2-dimensional array), moves in one direction along the X axis, while the series of independently controlled reagent delivery nozzles **(528, 530, 532, 534, 536, 538, 540** and **542)** move along the Y-axis relative to the reaction vessel **558.** As the reaction plate **556** and reagent nozzles **(528, 530, 532, 534, 536, 538, 540** and **542)** can be moved independently at the same time, this arrangement facilitates the extremely rapid delivery of up to 72 reagents independently to each of the 96 reaction vessel wells.

The system software allows the straightforward programming of the synthesis of a large number of compounds by supplying the general synthetic procedure in the form of the command file to call upon certain reagents to be added to specific wells via lookup in the sequence file with the bottle position, flow rate, and concentration of each reagent being stored in the separate reagent table file. Compounds can be synthesized on various scales. For oligonucleotides, a 200 nmole scale is typically selected while for other compounds larger scales, as for example a 10 µmole scale (3-5 mg), might be utilized. The resulting crude compounds are generally >80% pure, and are utilized directly for high throughput screening assays. Alternatively, prior to use the plates can be subjected to quality control (see general procedure 600 and Example 9) to ascertain their exact purity. Use of the synthesizer results in a very efficient means for the parallel synthesis of compounds for screening.

The software inputs accept tab delimited text files (as discussed above for file **504** and **512)** from any text editor. A typical command file, a .cmd file, is shown in Example 3 at Table 2. Typical sequence files, .seq files, are shown in Example 3 at Tables 3 and 4 (.SEQ file), and a typical reagent file, a .tab file, is shown in Example 3 at Table 5. Table 3 illustrates the sequence file for an oligonucleotide having 2'-deoxy nucleotides at each position with a phosphorothioate backbone throughout. Table 4 illustrates the sequence file for an oligonucleotide, again having a phosphorothioate backbone throughout, however, certain modified nucleoside are utilized in portions of the oligonucleotide. As shown in this table, 2'-O-(2-methoxyethyl) modified nucleosides are utilized in a first region (a wing) of the oligonucleotide, followed by a second region (a gap) of 2'-deoxy nucleotides and finally a third region (a further wing) that has the same chemistry as the first region. Typically some of the wells of the 96 well plate **556** may be left empty (depending on the number of oligonucleotides to be made during an individual synthesis) or some of the wells may have oligonucleotides that will serve as standards for comparison or analytical purposes.

Prior to loading reagents, moisture sensitive reagent lines are purged with argon at **522** for 20 minutes. Reagents are dissolved to appropriate concentrations and installed on the synthesizer. Large bottles, collectively identified as **558** in Figure 23 (containing 8 delivery lines) are used for wash solvents and the delivery of general activators, trityl group cleaving reagents and other reagents that may be used in multiple wells during any particular synthesis. Small septa bottles, collectively identified as **560** in Figure 23, are utilized to contain individual nucleotide amidite precursor compounds. This allows for anhydrous preparation and efficient installation of multiple reagents by using needles to pressurize the bottle, and as a delivery path. After all reagents are installed, the lines are primed with reagent, flow rates measured, then entered into the reagent table (.tab file). A dry resin loaded plate is removed from vacuum and installed in the machine for the synthesis.

The modified 96 well polypropylene plate **556** is utilized as the reaction vessel. The working volume in each well is approximately 700 µl. The bottom of each well is provided with a pressed-fit 20 µm polypropylene frit and a long capillary exit into a lower collection chamber as is illustrated in Figure 5 of the above referenced United States Patent 5,372,672. The solid support for use in holding the growing oligonucleotide during synthesis is loaded into the wells of the synthesis plate **556** by pipetting the desired volume of a balanced density slurry of the support suspended in an appropriate solvent, typically an acetonitrile-methylene chloride mixture. Reactions can be run on various scales as for instance the above noted 200 nmole and 10 µmol scales. For oligonucleotide synthesis a CPG support is preferred, however other medium loading polystyrene-PEG supports such as TENTAGEL^{™} or ARGOGEL^{™} can also be used.

As seen in Figure 24, the synthesis plate is transported back and forth in the X-direction under an array of 8 moveable banks **(530, 532, 534, 536, 538, 540, 542** and **544)** of 8 nozzles (64 total) in the Y-direction, and 6 banks **(544, 546, 548, 550, 552** and **554)** of 48 fixed nozzles, so that each well can receive the appropriate amounts of reagents and/or solvents from any reservoir (large bottle or smaller septa bottle). A sliding balloon-type seal **562** surrounds this nozzle array and joins it to the reaction plate headspace **564.** A slow sweep of nitrogen or argon **522** at ambient pressure across the plate headspace is used to preserve an anhydrous environment.

The liquid contents in each well do not drip out until the headspace pressure exceeds the capillary forces on the liquid in the exit nozzle. A slight positive pressure in the lower collection chamber can be added to eliminate residual slow leakage from filled wells, or to effect agitation by bubbling inert gas through the suspension. In order to empty the wells, the headspace gas outlet valve is closed and the internal pressure raised to about 2 psi. Normally, liquid contents are blown directly to waste **566.** However, a 96 well microtiter plate can be inserted into the lower chamber beneath the synthesis plate in order to collect the individual well eluents for spectrophotometric monitoring (trityl, etc.) of reaction progress and yield.

The basic plumbing scheme for the machine is the gas-pressurized delivery of reagents. Each reagent is delivered to the synthesis plate through a dedicated supply line, collectively identified at **568,** solenoid valve collectively identified at **570** and nozzle, collectively identified at **572.** Reagents never cross paths until they reach the reaction well. Thus, no line needs to be washed or flushed prior to its next use and there is no possibility of cross-contamination of reagents. The liquid delivery velocity is sufficiently energetic to thoroughly mix the contents within a well to form a homogeneous solution, even when employing solutions having drastically different densities. With this mixing, once reactants are in homogeneous solution, diffusion carries the individual components into and out of the solid support matrix where the desired reaction takes place. Each reagent reservoir can be plumbed to either a single nozzle or any combination of up to 8 nozzles. Each nozzle is also provided with a concentric nozzle washer to wash the outside of the delivery nozzles in order to eliminate problems of crystallized reactant buildup due to slow evaporation of solvent at the tips of the nozzles. The nozzles and supply lines can be primed into a set of dummy wells directly to waste at any time.

The entire plumbing system is fabricated with teflon tubing, and reagent reservoirs are accessed *via* syringe needle/septa or direct connection into the higher capacity bottles. The septum vials **560** are held in removable 8-bottle racks to facilitate easy setup and cleaning. The priming volume for each line is about 350 µl. The minimum delivery volume is about 2 µl, and flow rate accuracy is ±5%. The actual amount of material delivered depends on a timed flow of liquid. The flow rate for a particular solvent will depend on its viscosity and wetting characteristics of the teflon tubing. The flow rate (typically 200-350 µl per sec) is experimentally determined, and this information is contained in the reagent table setup file.

Heating and cooling of the reaction block **526** is effected utilizing a recirculating heat exchanger plate **524,** similar to that found in PCR thermocyclers, that nests with the polypropylene synthesis plate **556** to provide good thermal contact. The liquid contents in a well can be heated or cooled at about 10°C per minute over a range of +5 to +80°C, as polypropylene begins to soften and deform at about 80°C. For temperatures greater than this, a non-disposable synthesis plate machined from stainless steel or monel with replaceable frits can be utilized.

The hardware controller can be any of a wide variety, but conveniently can be designed around a set of three 1 MHz 86332 chips. This controller is used to drive the single X-axis and 8 Y-axis stepper motors as well as provide the timing functions for a total of 154 solenoid valves. Each chip has 16 bidirectional timer I/O and 8 interrupt channels in its timer processing unit (TPU). These are used to provide the step and direction signals, and to read 3 encoder inputs and 2 limit switches for controlling up to three motors per chip. Each 86332 chip also drives a serial chain of 8 UNC5891A darlington array chips to provide power to 64 valves with msec resolution. The controller communicates with the Windows software interface program running on a PC via a 19200 Hz serial channel, and uses an elementary instruction set to communicate valve_number, time_open, motor_number and position_data.

The three components of the software program that run the array synthesizer are the generalized procedure or command (.cmd) file which specifies the synthesis instructions to be performed, the sequence (.seq) file which specifies the scale of the reaction and the order in which variable groups will be added to the core synthon, and the reagent table (.tab) file which specifies the name of a chemical, its location (bottle number), flow rate, and concentration are utilized in conjunction with a basic set of command instructions.

One basic set of command instructions can be:

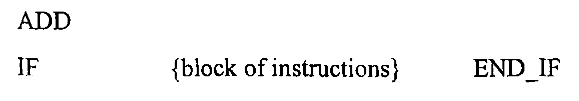

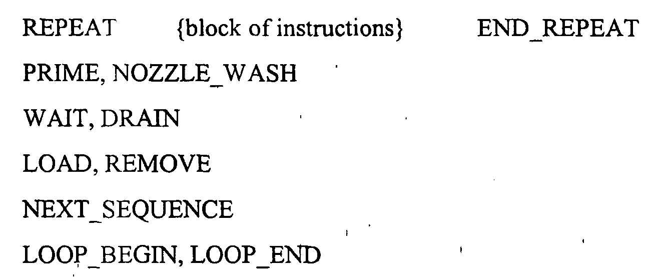

The ADD instruction has two forms, and is intended to have the look and feel of a standard chemical equation. Reagents are specified to be added by a molar amount if the number proceeds the name identifier, or by an absolute volume in microliters if the number follows the identifier. The number of reagents to be added is a parsed list, separated by the "+" sign. For variable reagent identifiers, the key word, <seq>, means look in the sequence table for the identity of the reagent to be added, while the key word, <act>, means add the reagent which is associated with that particular <seq>. Reagents are delivered in the order specified in the list.
Thus:
ADD ACN 300
   means: Add 300 µl of the named reagent acetonitrile; ACN to each well of active synthesis
ADD <seq> 300
   means: If the sequence pointer in the .seq file is to a reagent in the list of reagents, independent of scale, add 300 µl of that particular reagent specified for that well.
ADD 1.1 PYR + 1.0 <seq> + 1.1 <act1>
   means: If the sequence pointer in the .seq file is to a reagent in the list of acids in the Class ACIDS_1, and PYR is the name of pyridine, and ethyl chloroformate is defined in the .tab file to activate the class, ACIDS_1, then this instruction means:
   Add 1.1 equiv. pyridine
      1.0 equiv. of the acid specified for that well and
      1.1 equiv. of the activator, ethyl chloroformate
   The IF command allows one to test what type of reagent is specified in the <seq> variable and process the succeeding block of commands accordingly.
   Thus: 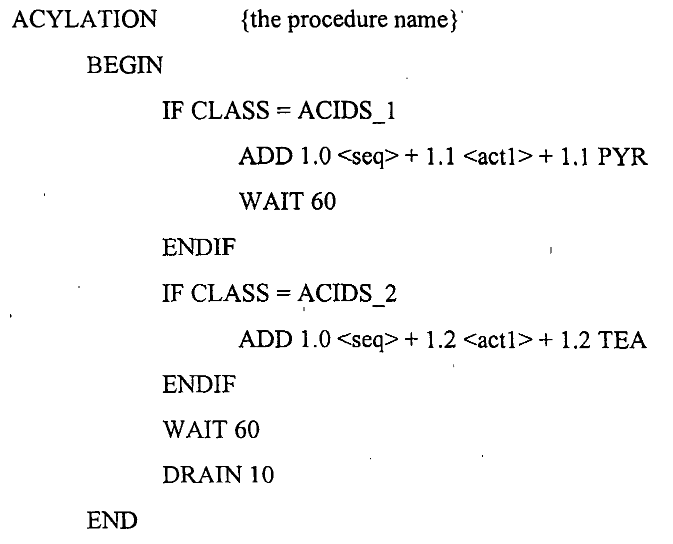 means: Operate on those wells for which reagents contained in the Acid_1 class are specified, WAIT 60 sec, then operate on those wells for which reagents contained in the Acid_2 class are specified, then WAIT 60 sec longer, then DRAIN the whole plate. Note that the Acid_1 group has reacted for a total of 120 sec, while the Acid_2 group has reacted for only 60 sec.

The REPEAT command is a simple way to execute the same block of commands multiple times.
Thus: 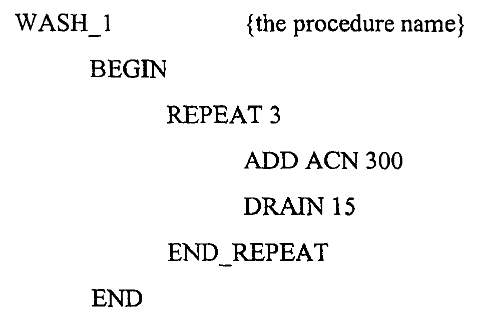 means: repeats the add acetonitrile and drain sequence for each well three times.

The PRIME command will operate either on specific named reagents or on nozzles which will be used in the next associated <seq> operation. The µl amount dispensed into a prime port is a constant that can be specified in a config.dat file.

The NOZZLE_WASH command for washing the outside of reaction nozzles free from residue due to evaporation of reagent solvent will operate either on specific named reagents or on nozzles which have been used in the preceding associated <seq> operation. The machine is plumbed such that if any nozzle in a block has been used, all the nozzles in that block will be washed into the prime port.

The WAIT and DRAIN commands are by seconds, with the drain command applying a gas pressure over the top surface of the plate in order to drain the wells.

The LOAD and REMOVE commands are instructions for the machine to pause for operator action.

The NEXT_SEQUENCE command increments the sequence pointer to the next group of substituents to be added in the sequence file. The general form of a .seq file entry is the definition:
**Well_No Well_ID Scale Sequence**

The sequence information is conveyed by a series of columns, each of which represents a variable reagent to be added at a particular position. The scale (µmole) variable is included so that reactions of different scale can be run at the same time if desired. The reagents are defined in a lookup table (the .tab file), which specifies the name of the reagent as referred to in the sequence and command files, its location (bottle number), flow rate, and concentration. This information is then used by the controller software and hardware to determine both the appropriate slider motion to position the plate and slider arms for delivery of a specific reagent, as well as the specific valve and time required to deliver the appropriate reagents. The adept classification of reagents allows the use of conditional IF loops from within a command file to perform addition of different reagents differently during a "single step" performed across 96 wells simultaneously. The special class ACTIVATORS defines certain reagents that always get added with a particular class of reagents (for example tetrazole during a phosphitylation reaction in adding the next nucleotide to a growing oligonucleotide).

The general form of the .tab file is the definition:
**Class Bottle Reagent Name Flow_rate Conc.**

The LOOP_BEGIN and LOOP_END commands define the block of commands which will continue to operate until a NEXT_SEQUENCE command points past the end of the longest list of reactants in any well.

Not included in the command set is a MOVE command. For all of the above commands, if any plate or nozzle movement is required, this is automatically executed in order to perform the desired solvent or reagent delivery operation. This is accomplished by the controller software and hardware, which determines the correct nozzle(s) and well(s) required for a particular reagent addition, then synchronizes the position of the requisite nozzle and well prior to adding the reagent.

A MANUAL mode can also be utilized in which the synthesis plate and nozzle blocks can be "homed" or moved to any position by the operator, the nozzles primed or washed, the various reagent bottles depressurized or washed with solvent, the chamber pressurized, etc. The automatic COMMAND mode can be interrupted at any point, MANUAL commands executed, and then operation resumed at the appropriate location. The sequence pointer can be incremented to restart a synthesis anywhere within a command file.

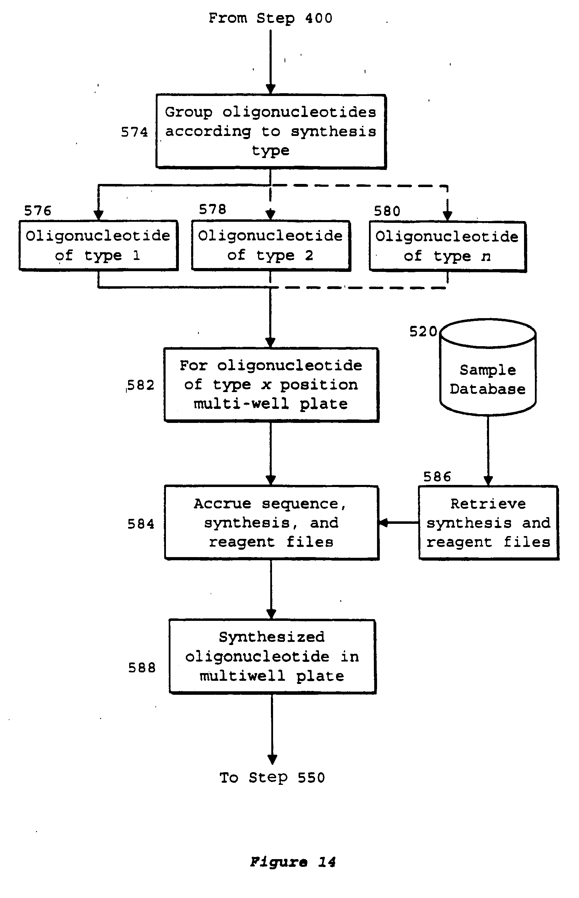

In reference to Figure 14, the list of oligonucleotides for synthesis can be rearranged or grouped for optimization of synthesis. Thus at process step **574**, the oligonucleotides are grouped according to a factor on which to base the optimization of synthesis. As illustrated in the Examples below, one such factor is the 3' most nucleoside of the oligonucleotide. Using the amidite approach for oligonucleotide synthesis, a nucleotide bearing a 3' phosphoramite is added to the 5' hydroxyl group of a growing nucleotide chain. The first nucleotide (at the 3' terminus of the oligonucleotide - the 3' most nucleoside) is first connected to a solid support. This is normally done batchwise on a large scale as is standard practice during oligonucleotide synthesis.

Such solid supports pre-loaded with a nucleoside are commercially available. In utilizing the multi well format for oligonucleotide synthesis, for each oligonucleotide to be synthesized, an aliquot of a solid support bearing the proper nucleoside thereon is added to the well for synthesis. Prior to loading the sequence of oligonucleotides to be synthesized in the .seq file, they are sorted by the 3' terminal nucleotide. Based on that sorting, all of the oligonucleotide sequences having an "A" nucleoside at their 3' end are grouped together, those with a "C" nucleoside are grouped together as are those with "G" or "T" nucleosides. Thus in loading the nucleoside-bearing solid support into the synthesis wells, machine movements are conserved.

The oligonucleotides can be grouped by the above described parameter or other parameters that facilitate the synthesis of the oligonucleotides. Thus in Figure 14, sorting is noted as being effected by some parameter of type 1, as for instance the above described 3' most nucleoside, or other types of parameters from type 2 to type n at process steps **576, 578** and **580.** Since synthesis will be from the 3' end of the oligonucleotides to the 5' end, the oligonucleotide sequences are reverse sorted to read 3' to 5'. The oligonucleotides are entered in the .seq file in this form, i.e., reading 3' to 5'.

Once sorted into types, the position of the oligonucleotides on the synthesis plates is specified at process step **582** by the creation of a .seq file as described above. The .seq file is associated with the respective .cmd and .tab files needed for synthesis of the particular chemistries specified for the oligonucleotides at process step **584** by retrieval of the .cmd and .tab files at process step **586** from the sample database **520.** These files are then input into the multi well synthesizer at process step **588** for oligonucleotide synthesis. Once physically synthesized, the list of oligonucleotides again enters the general procedure flow as indicated in Figure 1. For shipping, storage or other handling purposes, the plates can be lyophilized at this point if desired. Upon lyophilization, each well contains the oligonucleotides located therein as a dry compound.

### 14. Quality Control.

In an optional step, quality control is performed on the oligonucleotides at process step **600** after a decision is made (decision step **550**) to perform quality control. Although optional, quality control may be desired when there is some reason to think that some aspect of the synthetic process step **500** has been compromised. Alternatively, samples of the oligonucleotides may be taken and stored in the event that the results of assays conducted using the oligonucleotides (process step **700)** yield confusing results or suboptimal data. In the latter event, for example, quality control might be performed after decision step **800** if no oligonucleotides with sufficient activity are identified. In either event, decision step **650** follows quality control step process **600.** If one or more of the oligonucleotides do not pass quality control, process step **500** can be repeated, i.e., the oligonucleotides are synthesized for a second time.

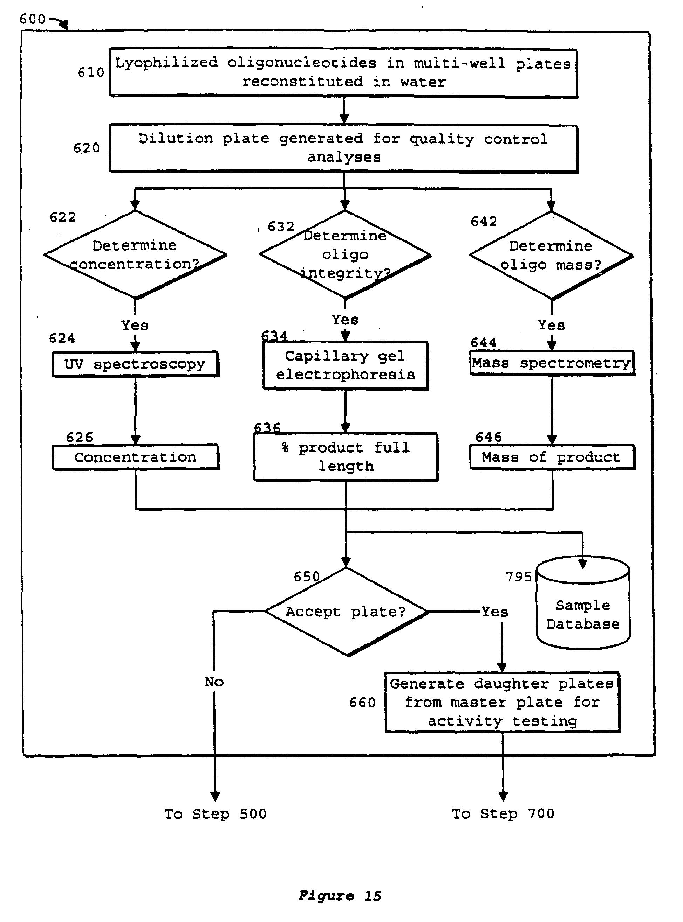

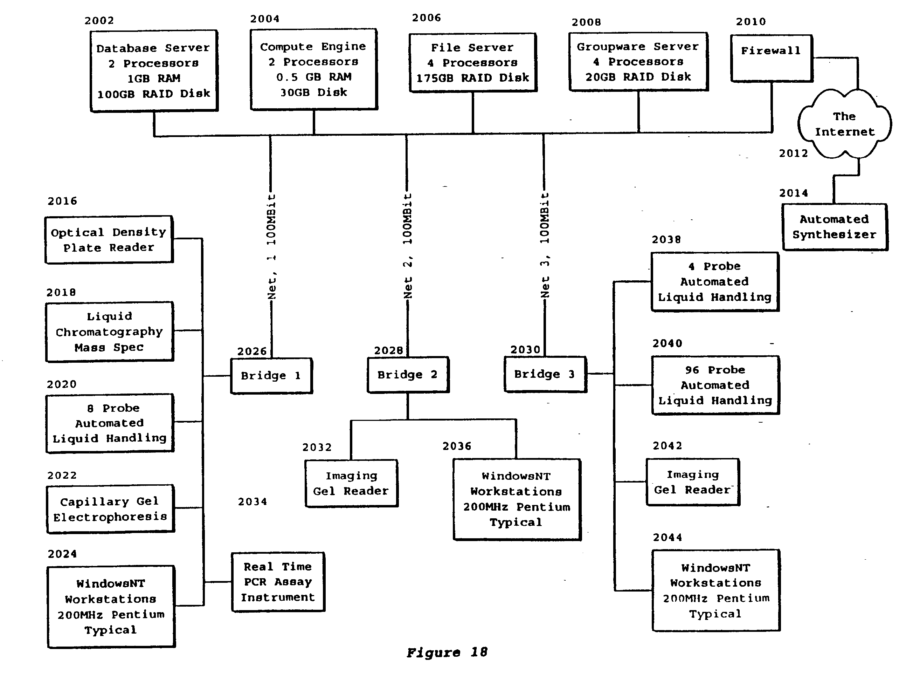

The operation of the quality control system general procedure **600** is detailed in steps **610-660** of Figure 15. Also referenced in the following discussion are the robotics and associated analytical instrumentation as shown in Figure 18.

During step **610** (Figure 15), sterile, double-distilled water is transferred by an automated liquid handler **(2040** of Figure 18) to each well of a multi-well plate containing a set of lyophilized antisense oligonucleotides. The automated liquid handler **(2040** of Figure 18) reads the barcode sticker on the multi-well plate to obtain the plate's identification number. Automated liquid handler **2040** then queries Sample Database 520 (which resides in Database Server **2002** of Figure 18) for the quality control assay instruction set for that plate and executes the appropriate steps. Three quality control processes are illustrated, however, it is understood that other quality control processes or steps maybe practiced in addition to or in place of the processes illustrated.

The first illustrative quality control process (steps **622** to **626)** quantitates the concentration of oligonucleotide in each well. If this quality control step is performed, an automated liquid handler **(2040** of Figure 18) is instructed to remove an aliquot from each well of the master plate and generate a replicate daughter plate for transfer to the UV spectrophotometer (**2016** of Figure 18). The UV spectrophotometer (**2016** of Figure 18) then measures the optical density of each well at a wavelength of 260 nanometers. Using standardized conversion factors, a microprocessor within UV spectrophotometer **(2016** of Figure 18) then calculates a concentration value from the measured absorbance value for each well and output the results to Sample Database **520.**

The second illustrative quality control process steps **632** to **636**) quantitates the percent of total oligonucleotide in each well that is full length. If this quality control step is performed, an automated liquid handler **(2040** of Figure 18) is instructed to remove an aliquot from each well of the master plate and generate a replicate daughter plate for transfer to the multichannel capillary gel electrophoresis apparatus **(2022** of Figure 18). The apparatus electrophoretically resolves in capillary tube gels the oligonucleotide product in each well. As the product reaches the distal end of the tube gel during electrophoresis, a detection window dynamically measures the optical density of the product that passes by it. Following electrophoresis, the value of percent product that passed by the detection window with respect to time is utilized by a built in microprocessor to calculate the relative size distribution of oligonucleotide product in each well. These results are then output to the Sample Database **(520.**

The third illustrative quality control process steps **632** to **636)** quantitates the mass of the oligonucleotide in each well that is full length. If this quality control step is performed, an automated liquid handler **(2040** of Figure 18) is instructed to remove an aliquot from each well of the master plate and generate a replicate daughter plate for transfer to the multichannel liquid electrospray mass spectrometer **(2018** of Figure 18). The apparatus then uses electrospray technology to inject the oligonucleotide product into the mass spectrometer. A built in microprocessor calculates the mass-to-charge ratio to arrive at the mass of oligonucleotide product in each well. The results are then output to Sample Database **520.**

Following completion of the selected quality control processes, the output data is manually examined or is examined using an appropriate algorithm and a decision is made as to whether or not the plate receives "Pass" or "Fail" status. The current criteria for acceptance, for 18 mer oligonucleotides, is that at least 85% of the oligonucleotides in a multi-well plate must be 85% or greater full length product as measured by both capillary gel electrophoresis and mass spectrometry. An input (manual or automated) is then made into Sample Database **520** as to the pass/fail status of the plate. If a plate fails, the process cycles back to step **500,** and a new plate of the same oligonucleotides is automatically placed in the plate synthesis request queue (process **554** of Figure 15). If a plate receives "Pass" status, an automated liquid handler **(2040** of Figure 18) is instructed to remove appropriate aliquots from each well of the master plate and generate two replicate daughter plates in which the oligonucleotide in each well is at a concentration of 30 micromolar. The plate then moves on to process **700** for oligonucleotide activity evaluation.

**15. Cell Lines for Assaying Oligonucleotide Activity.** The effect of antisense compounds on target nucleic acid expression can be tested in any of a variety of cell types provided that the target nucleic acid, or its gene product, is present at measurable levels. This can be routinely determined using, for example, PCR or Northern blot analysis. The following four cell types are provided for illustrative purposes, but other cell types can be routinely used.

**T-24 cells:** The transitional cell bladder carcinoma cell line T-24 is obtained from the American Type Culture Collection (ATCC) (Manassas, VA). T-24 cells were routinely cultured in complete McCoy's 5A basal media (Life Technologies, Gaithersburg, MD) supplemented with 10% fetal calf serum, penicillin 100 units per milliliter, and streptomycin 100 micrograms per milliliter (all from Life Technologies). Cells are routinely passaged by trypsinization and dilution when they reach 90% confluence. Cells are routinely seeded into 96-well plates (Falcon-Primaria #3872) at a density of 7000 cells/well for use in RT-PCR analysis. For Northern blotting or other analysis, cells are seeded onto 100 mm or other standard tissue culture plates and treated similarly, using appropriate volumes of medium and oligonucleotide.

**A549 cells:** The human lung carcinoma cell line A549 is obtained from the ATCC (Manassas, VA). A549 cells were routinely cultured in DMEM basal media (Life Technologies) supplemented with 10% fetal calf serum, penicillin 100 units per milliliter, and streptomycin 100 micrograms per milliliter (all from Life Technologies). Cells are routinely passaged by trypsinization and dilution when they reach 90% confluence.

**NHDF cells:** Human neonatal dermal fibroblast (NHDF) were obtained from the Clonetics Corporation (Walkersville, MD). NHDFs were routinely maintained in Fibroblast Growth Medium (Clonetics Corp.) as provided by the supplier. Cells are maintained for up to 10 passages as recommended by the supplier.

**HEK cells:** Human embryonic keratinocytes (HEK) were obtained from the Clonetics Corp. HEKs were routinely maintained in Keratinocyte Growth Medium (Clonetics Corp.) as provided by the supplier. Cell are routinely maintained for up to 10 passages as recommended by the supplier.

### 16. Treatment of Cells with Candidate Compounds:

When cells reach about 80% confluency, they are treated with oligonucleotide. For cells grown in 96-well plates, wells are washed once with 200 µl OPTI-MEM-1^{™} reduced-serum medium (Life Technologies) and then treated with 130 µl of OPTI-MEM-1^{™} containing 3.75 µg/ml LIPOFECTIN^{™} (Life Technologies) and the desired oligonucleotide at a final concentration of 150 nM. After 4 hours of treatment, the medium was replaced with fresh medium. Cells were harvested 16 hours after oligonucleotide treatment.

Alternatively, for cells resistant to cationic mediated transfection, oligonucleotides can be introduced by electroporation. Electroporation conditions must be optimized for every cell type. In general, oligonucleotide is added directly to complete growth media to a final concentration between 1 and 20 micromolar. An electronic pulse is delivered to the cells using a BTX T820 ELECTRO SQUARE PORATOR^{™} using a Multi-coaxial 96-well electrode (BT840) (BTX Corporation, San Diego, California). Following electroporation, the cells are returned to the incubator for 16 hours.

### 17. Assaying Oligonucleotide Activity:

Oligonucleotide-mediated modulation of expression of a target nucleic acid can be assayed in a variety of ways known in the art. For example, target RNA levels can be quantitated by, e.g., Northern blot analysis, competitive PCR, or reverse transcriptase polymerase chain reaction (RT-PCR). RNA analysis can be performed on total cellular RNA or, preferably in the case of polypeptide-encoding nucleic acids, poly(A)+ mRNA. For RT-PCR, poly(A)+ mRNA is preferred. Methods of RNA isolation are taught in, for example, Ausubel *et al.* (*Short Protocols in Molecular Biology,* 2nd Ed., pp. 4-1 to 4-13, Greene Publishing Associates and John Wiley & Sons, New York, 1992). Northern blot analysis is routine in the art (*Id*., pp. 4-14 to 4-29).

Alternatively, total RNA can be prepared from cultured cells or tissue using the QIAGEN RNeasy®-96 kit for the high throughput preparation of RNA (QIAGEN, Inc., Valencia, CA). Essentially, protocols are carried out according to the manufacturer's directions. Optionally, a DNase step is included to remove residual DNA prior to RT-PCR.

To improve efficiency and accuracy the repetitive pipeting steps and elution step have been automated using a QIAGEN Bio-Robot 9604. Essentially after lysing of the oligonucleotide treated cell cultures in situ, the plate is transferred to the robot deck where the pipeting, DNase treatment, and elution steps are carried out.

Reverse transcriptase polymerase chain reaction (RT-PCR) can be conveniently accomplished using the commercially available ABI PRISM® 7700 Sequence Detection System (PE-Applied Biosystems, Foster City, CA) according to the manufacturer's instructions. Other methods of PCR are also known in the art.

Target protein levels can be quantitated in a variety of ways well known in the art, such as immunoprecipitation, Western blot analysis (immunoblotting), Enzyme-linked immunosorbent assay (ELISA) or fluorescence-activated cell sorting (FACS). Antibodies directed to a protein encoded by a target nucleic acid can be identified and obtained from a variety of sources, such as the MSRS catalog of antibodies, (Aerie Corporation, Birmingham, MI or via the internet at http://www.ANTIBODIES-PROBES.com/), or can be prepared via conventional antibody generation methods. Methods for preparation of polyclonal, monospecific ("antipeptide") and monoclonal antisera are taught by, for example, Ausubel *et al. (Short Protocols in Molecular Biology,* 2nd Ed., pp. 11-3 to 11-54, Greene Publishing Associates and John Wiley & Sons, New York, 1992).

Immunoprecipitation methods are standard in the art and are described by, for example, Ausubel *et al.* (*Id*., pp. 10-57 to 10-63). Western blot (immunoblot) analysis is standard in the art (*Id*., pp. 10-32 to 10-10-35). Enzyme-linked immunosorbent assays (ELISA) are standard in the art (*Id*., pp. 11-5 to 11-17).

Because it is preferred to assay the compounds of the invention in a batchwise fashion, i.e., in parallel to the automated synthesis process described above, preferred means of assaying are suitable for use in 96-well plates and with robotic means. Accordingly, automated RT-PCR is preferred for assaying target nucleic acid levels, and automated ELISA is preferred for assaying target protein levels.

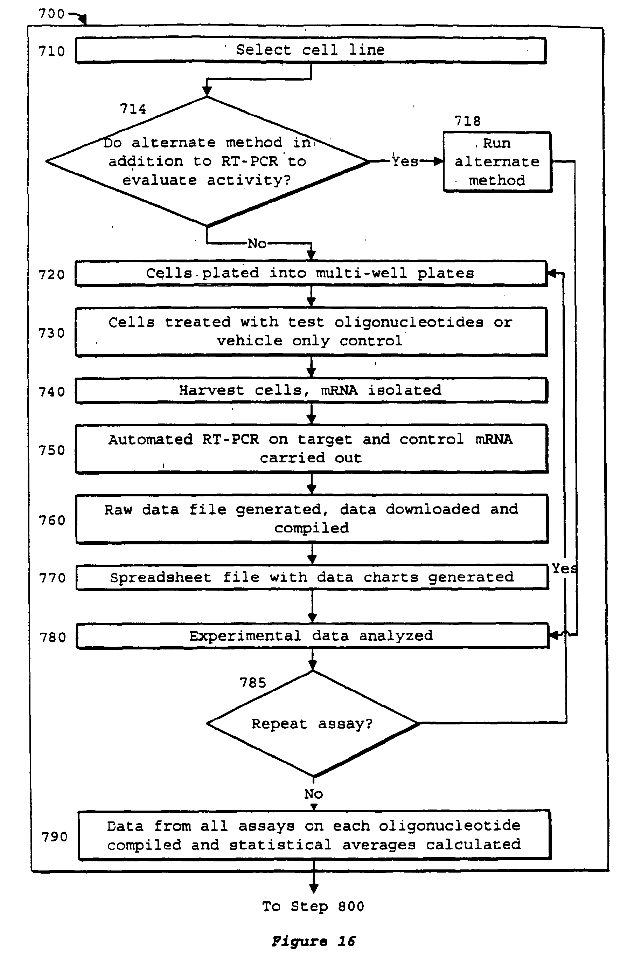

The assaying step, general procedure step **700,** is described in detail in Figure 16. After an appropriate cell line is selected at process step **710,** a decision is made at decision step **714** as to whether RT-PCR will be the only method by which the activity of the compounds is evaluated. In some instances, it is desirable to run alternative assay methods at process step **718;** for example, when it is desired to assess target polypeptide levels as well as target RNA levels, an immunoassay such as an ELISA is run in parallel with the RT-PCR assays. Preferably, such assays are tractable to semi-automated or robotic means.

When RT-PCR is used to evaluate the activities of the compounds, cells are plated into multi-well plates (typically, 96-well plates) in process step **720** and treated with test or control oligonucleotides in process step **730.** Then, the cells are harvested and lysed in process step **740** and the lysates are introduced into an apparatus where RT-PCR is carried out in process step **750.** A raw data file is generated, and the data is downloaded and compiled at step **760.** Spreadsheet files with data charts are generated at process step **770,** and the experimental data is analyzed at process step **780.** Based on the results, a decision is made at process step **785** as to whether it is necessary to repeat the assays and, if so, the process begins again with step **720.** In any event, data from all the assays on each oligonucleotide are compiled and statistical parameters are automatically determined at process step **790.**

### 18. Classification of Compounds Based on Their Activity:

Following assaying, general procedure step **700**, oligonucleotide compounds are classified according to one or more desired properties. Typically, three classes of compounds are used: active compounds, marginally active (or "marginal") compounds and inactive compounds. To some degree, the selection criteria for these classes vary from target to target, and members of one or more classes may not be present for a given set of oligonucleotides.

However, some criteria are constant. For example, inactive compounds will typically comprise those compounds having 5% or less inhibition of target expression (relative to basal levels). Active compounds will typically cause at least 30% inhibition of target expression, although lower levels of inhibition are acceptable in some instances. Marginal compounds will have activities intermediate between active and inactive compounds, with preferred marginal compounds having activities more like those of active compounds.

### 19. Optimization of Lead Compounds by Sequence.

One means by which oligonucleotide compounds are optimized for activity is by varying their nucleobase sequences so that different regions of the target nucleic acid are targeted. Some such regions will be more accessible to oligonucleotide compounds than others, and "sliding" a nucleobase sequence along a target nucleic acid only a few bases can have significant effects on activity. Accordingly, varying or adjusting the nucleobase sequences of the compounds of the invention is one means by which suboptimal compounds can be made optimal, or by which new active compounds can be generated.

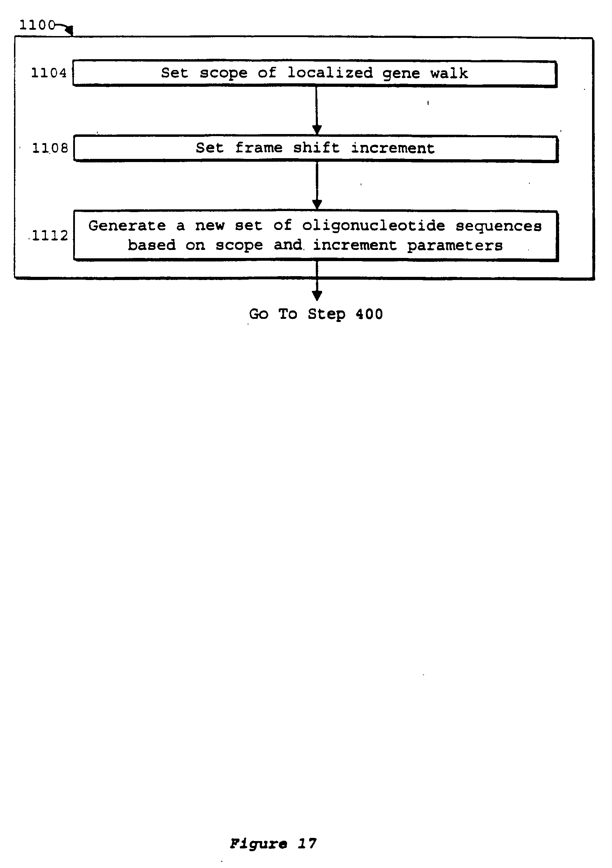

The operation of the gene walk process **1100** detailed in steps **1104-1112** of Figure 17 is detailed as follows. As used herein, the term "gene walk" is defined as the process by which a specified oligonucleotide sequence *x* that binds to a specified nucleic acid target *y* is used as a frame of reference around which a series of new oligonucleotides sequences capable of hybridizing to nucleic acid target *y* are generated that are sequence shifted increments of oligonucleotide sequence *x*. Gene walking can be done "downstream", "upstream" or in both directions from a specified oligonucleotide.

During step **1104** the user manually enters the identification number of the oligonucleotide sequence around which it is desired to execute gene walk process 1100 and the name of the corresponding target nucleic acid. The user then enters the scope of the gene walk at step **1104,** by which is meant the number of oligonucleotide sequences that it is desired to generate. The user then enters in step **1108** a positive integer value for the sequence shift increment. Once this data is generated, the gene walk is effected. This causes a subroutine to be executed that automatically generates the desired list of sequences by walking along the target sequence. At that point, the user proceeds to process **400** to assign chemistries to the selected oligonucleotides.

Example 16 below, details a gene walk. In subsequent steps, this new set of nucleobase sequences generated by the gene walk is used to direct the automated synthesis at general procedure step **500** of a second set of candidate oligonucleotides. These compounds are then taken through subsequent process steps to yield active compounds or reiterated as necessary to optimize activity of the compounds.

### 20. Optimization of Lead Compounds by Chemistry.

Another means by which oligonucleotide compounds of the invention are optimized is by reiterating portions of the process of the invention using marginal or active compounds from the first iteration and selecting additional chemistries to the nucleobase sequences thereof.

Thus, for example, an oligonucleotide chemistry different from that of the first set of oligonucleotides is assigned at general procedure step **400.** The nucleobase sequences of marginal compounds are used to direct the synthesis at general procedure step **500** of a second set of oligonucleotides having the second assigned chemistry. The resulting second set of oligonucleotide compounds is assayed in the same manner as the first set at procedure process step **700** and the results are examined to determine if compounds having sufficient activity have been generated at decision step **800.**

### 21. Identification of Sites Amenable to Antisense Technologies.

**In** a related process, a second oligonucleotide chemistry is assigned at procedure step **400** to the nucleobase sequences of all of the oligonucleotides (or, at least, all of the active and marginal compounds) and a second set of oligonucleotides is synthesized at procedure step **500** having the same nucleobase sequences as the first set of compounds. The resulting second set of oligonucleotide compounds is assayed in the same manner as the first set at procedure step **700** and active and marginal compounds are identified at procedure steps **800** and **1000.**

In order to identify sites on the target nucleic acid that are amenable to a variety of antisense technologies, the following mathematically simple steps are taken. The sequences of active and marginal compounds from two or more such automated syntheses/assays are compared and a set of nucleobase sequences that are active, or marginally so, in both sets of compounds is identified. The reverse complements of these nucleobase sequences corresponds to sequences of the target nucleic acid that are tractable to a variety of antisense and other sequence-based technologies. These antisense-sensitive sites are assembled into contiguous sequences (contigs) using the procedures described for assembling target nucleotide sequences (at procedure step **200).**

### 22. Systems for Executing Preferred Methods of the Invention.

An embodiment of computer, network and instrument resources for effecting the methods of the invention is shown in Figure 18. In this embodiment, four computer servers are provided. First, a large database server **2002** stores all chemical structure, sample tracking and genomic, assay, quality control, and program status data. Further, this database server serves as the platform for a document management system. Second, a compute engine **2004** runs computational programs including RNA folding, oligonucleotide walking, and genomic searching. Third, a file server **2006** allows raw instrument output storage and sharing of robot instructions. Fourth, a groupware server **2008** enhances staff communication and process scheduling.

A redundant high-speed network system is provided between the main servers and the bridges **2026, 2028** and **2030.** These bridges provide reliable network access to the many workstations and instruments deployed for this process. The instruments selected to support this embodiment are all designed to sample directly from standard 96 well microtiter plates, and include an optical density reader **2016,** a combined liquid chromatography and mass spectroscopy instrument **2018,** a gel fluorescence and scintillation imaging system **2032** and **2042,** a capillary gel electrophoreses system **2022** and a real-time PCR system **2034.**

Most liquid handling is accomplished automatically using robots with individually controllable robotic pipetters **2038** and **2020** as well as a 96-well pipette system **2040** for duplicating plates. Windows NT or Macintosh workstations **2044, 2024,** and **2036** are deployed for instrument control, analysis and productivity support.

### 23. Relational Database.

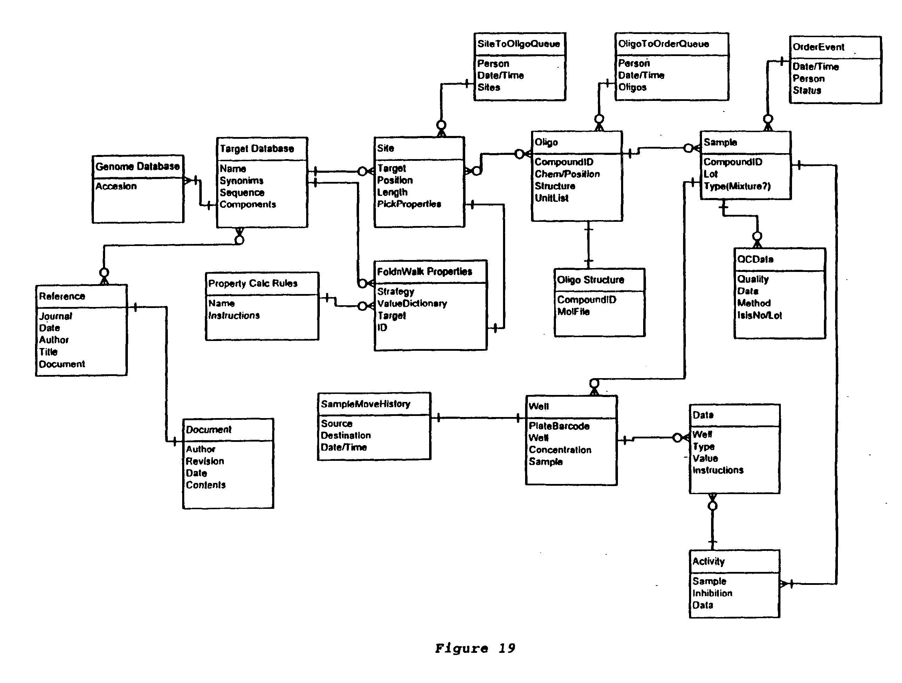

Data is stored in an appropriate database. For use with the methods of the invention, a relational database is preferred. Figure 19 illustrates the data structure of a sample relational database. Various elements of data are segregated among linked storage elements of the database.

### EXAMPLES

The following examples illustrate the invention and are not intended to limit the same. Those skilled in the art will recognize, or be able to ascertain through routine experimentation, numerous equivalents to the specific procedures, materials and devices described herein.

### EXAMPLE 1: Selection of CD40 as a Target

Cell-cell interactions are a feature of a variety of biological processes. In the activation of the immune response, for example, one of the earliest detectable events in a normal inflammatory response is adhesion of leukocytes to the vascular endothelium, followed by migration of leukocytes out of the vasculature to the site of infection or injury. The adhesion of leukocytes to vascular endothelium is an obligate step in their migration out of the vasculature (for a review, see Albelda *et al., FASEB J.,* 1994, *8*, 504). As is well known in the art, cell-cell interactions are also critical for propagation of both B-lymphocytes and T-lymphocytes resulting in enhanced humoral and cellular immune responses, respectively (for a reviews, see *Makgoba et al., Immunol. Today*, 1989, *10*, 417; Janeway, *Sci. Amer.,* 1993, *269,* 72).

CD40 was first characterized as a receptor expressed on B-lymphocytes. It was later found that engagement of B-cell CD40 with CD40L expressed on activated T-cells is essential for T-cell dependent B-cell activation (i.e. proliferation, immunoglobulin secretion, and class switching) (for a review, see *Gruss et al. Leuk. Lymphoma,* 1997*, 24,* 393). A full cDNA sequence for CD40 is available (GenBank accession number X60592, SEQ ID NO:85; SEQ ID NO:86 is protein).

As interest in CD40 mounted, it was subsequently revealed that functional CD40 is expressed on a variety of cell types other than B-cells, including macrophages, dendritic cells, thymic epithelial cells, Langerhans cells, and endothelial cells *(Ibid.).* These studies have led to the current belief that CD40 plays a much broader role in immune regulation by mediating interactions of T-cells with cell types other than B-cells. In support of this notion, it has been shown that stimulation of CD40 in macrophages and dendritic results is required for T-cell activation during antigen presentation (*Id*.). Recent evidence points to a role for CD40 in tissue inflammation as well. Production of the inflammatory mediators IL-12 and nitric oxide by macrophages has been shown to be CD40 dependent (Buhlmann *et al., J Clin. Immunol.,* 1996, *16,* 83). In endothelial cells, stimulation of CD40 by CD40L has been found to induce surface expression of E-selectin, ICAM-1, and VCAM-1, promoting adhesion of leukocytes to sites of inflammation (Buhlmann *et al., J. Clin. Immunol,* 1996, *16,* 83; Gruss *et al., Leuk Lymphoma*, 1997, *24*, 393). Finally, a number of reports have documented overexpression of CD40 in epithelial and hematopoietic tumors as well as tumor infiltrating endothelial cells, indicating that CD40 may play a role in tumor growth and/or angiogenesis as well *(Gruss et al., Leuk Lymphoma,* 1997, *24*, 393-422; Kluth *et al. Cancer Res,* 1997, *57*, 891).

Due to the pivotal role that CD40 plays in humoral immunity, the potential exists that therapeutic strategies aimed at downregulating CD40 may provide a novel class of agents useful in treating a number of immune associated disorders, including but not limited to graft versus host disease, graft rejection, and autoimmune diseases such as multiple sclerosis, systemic lupus erythematosus, and certain forms of arthritis. Inhibitors of CD40 may also prove useful as an anti-inflammatory compound, and could therefore be useful as treatment for a variety of diseases with an inflammatory component such as asthma, rheumatoid arthritis, allograft rejections, inflammatory bowel disease, and various dermatological conditions, including psoriasis. Finally, as more is learned about the association between CD40 overexpression and tumor growth, inhibitors of CD40 may prove useful as anti-tumor agents as well.

Currently, there are no known therapeutic agents which effectively inhibit the synthesis of CD40. To date, strategies aimed at inhibiting CD40 function have involved the use of a variety of agents that disrupt CD40/CD40L binding. These include monoclonal antibodies directed against either CD40 or CD40L, soluble forms of CD40, and synthetic peptides derived from a second CD40 binding protein, A20. The use of neutralizing antibodies against CD40 and/or CD40L in animal models has provided evidence that inhibition of CD40 stimulation would have therapeutic benefit for GVHD, allograft rejection, rheumatoid arthritis, SLE, MS, and B-cell lymphoma (Buhlmann *et al., J. Clin. Immunol*, 1996, *16,* 83). However, due to the expense, short half-life, and bioavailability problems associated with the use of large proteins as therapeutic agents, there is a long felt need for additional agents capable of effectively inhibiting CD40 function. Oligonucleotides compounds avoid many of the pitfalls of current agents used to block CD40/CD40L interactions and may therefore prove to be uniquely useful in a number of therapeutic applications.

### EXAMPLE 2: Generation of Virtual Oligonucleotides Targeted to CD40

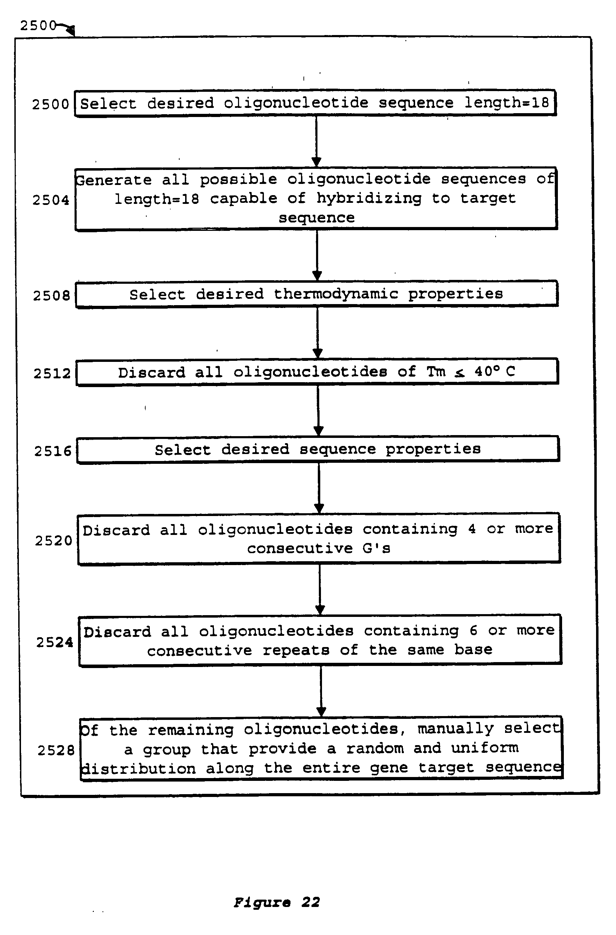

The process of the invention was used to select oligonucleotides targeted to CD40, generating the list of oligonucleotide sequences with desired properties as shown in Figure 22. From the assembled CD40 sequence, the process began with determining the desired oligonucleotide length to be eighteen nucleotides, as represented in step **2500.** All possible oligonucleotides of this length were generated by Oligo 5.0^{™}, as represented in step **2504.** Desired thermodynamic properties were selected in step **2508.** The single parameter used was oligonucleotides of melting temperature less than or equal to 40°C were discarded. In step **2512,** oligonucleotide melting temperatures were calculated by Oligo 5.0^{™}. Oligonucleotide sequences possessing an undesirable score were discarded. It is believed that oligonucleotides with melting temperatures near or below physiological and cell culture temperatures will bind poorly to target sequences. All oligonucleotide sequences remaining were exported into a spreadsheet. In step **2516,** desired sequence properties are selected. These include discarding oligonucleotides with at least one stretch of four guanosines in a row and stretches of six of any other nucleotide in a row. In step **2520,** a spreadsheet macro removed all oligonucleotides containing the text string "GGGG." In step **2524,** another spreadsheet macro removed all oligonucleotides containing the text strings "AAAAAA" or "CCCCCC" or "TTTTTT." From the remaining oligonucleotide sequences, 84 sequences were selected manually with the criteria of having an uniform distribution of oligonucleotide sequences throughout the target sequence, as represented in step **2528.** These oligonucleotide sequences were then passed to the next step in the process, assigning actual oligonucleotide chemistries to the sequences.

### EXAMPLE 3: Input Files For Automated Oligonucleotide Synthesis Command File (.cmd File)

Table 2 is a command file for synthesis of an oligonucleotide having regions of 2'-O-(2-methoxyethyl) nucleosides and a central region of 2'-deoxy nucleosides each linked by phosphorothioate internucleotide linkages.

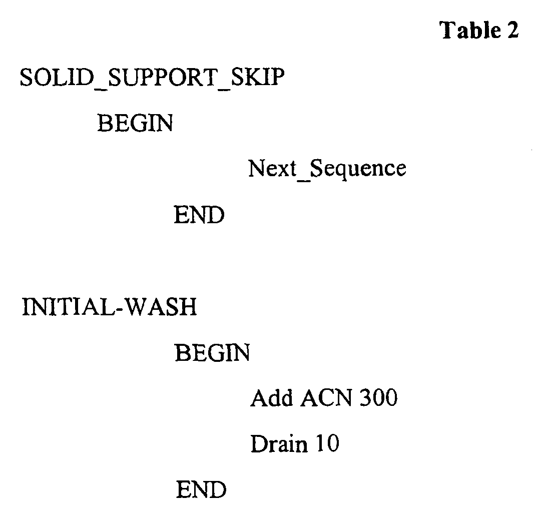

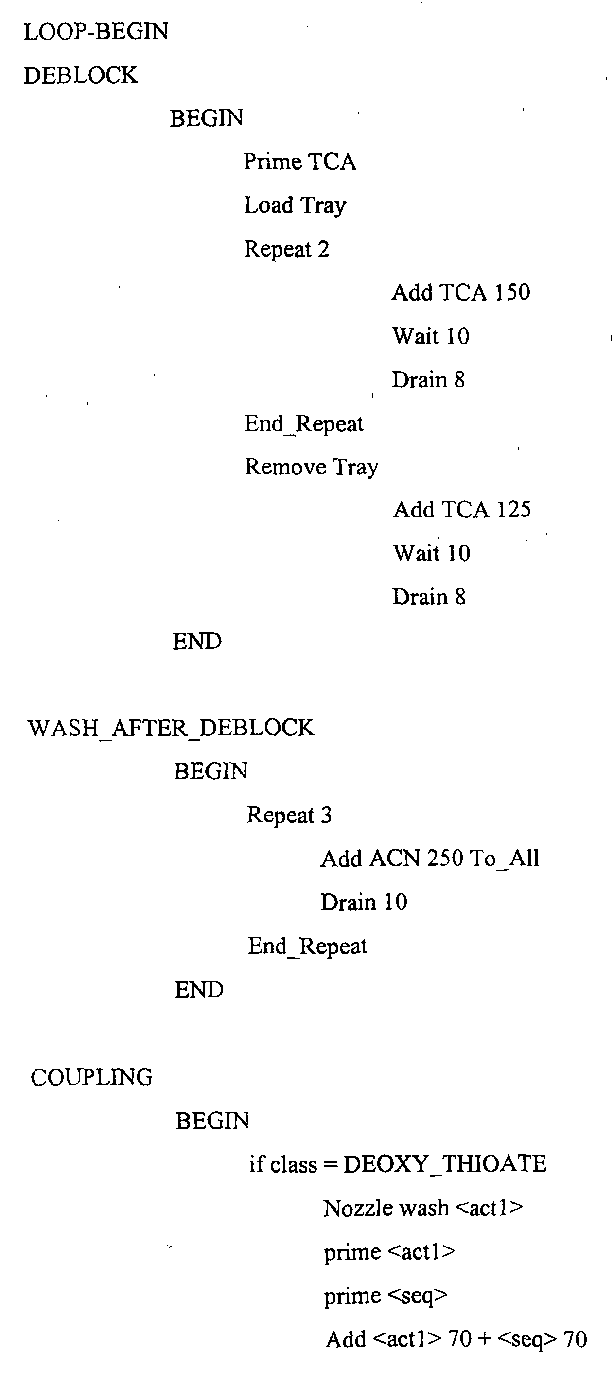

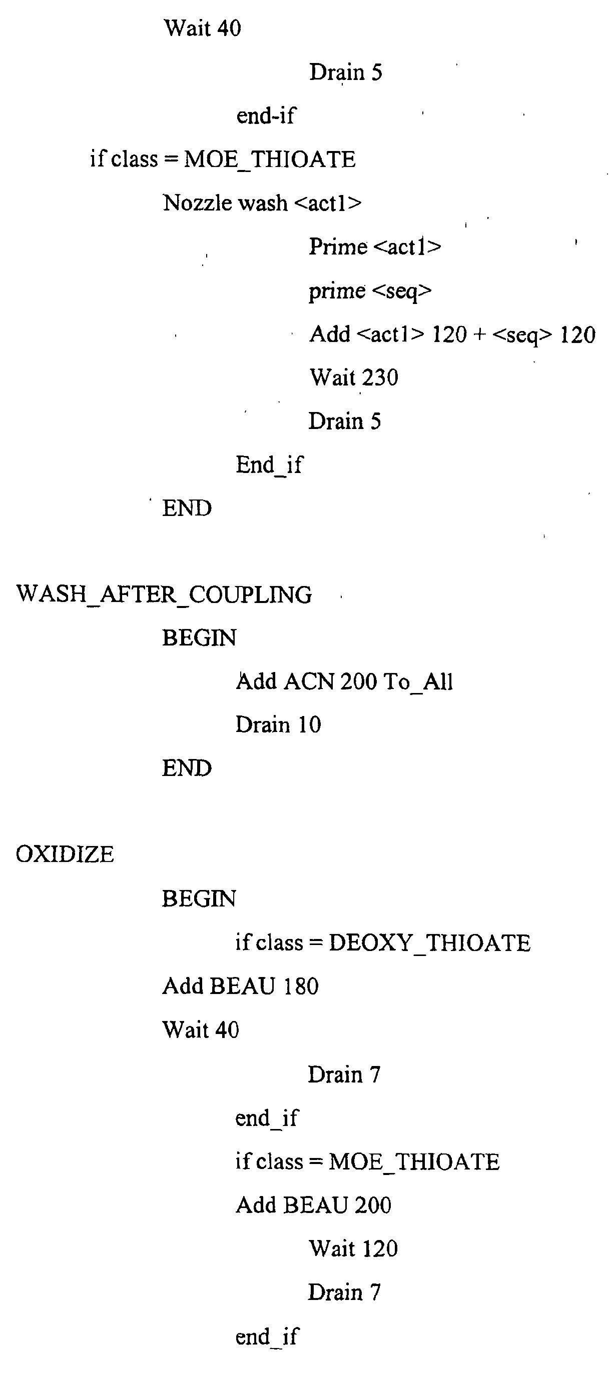

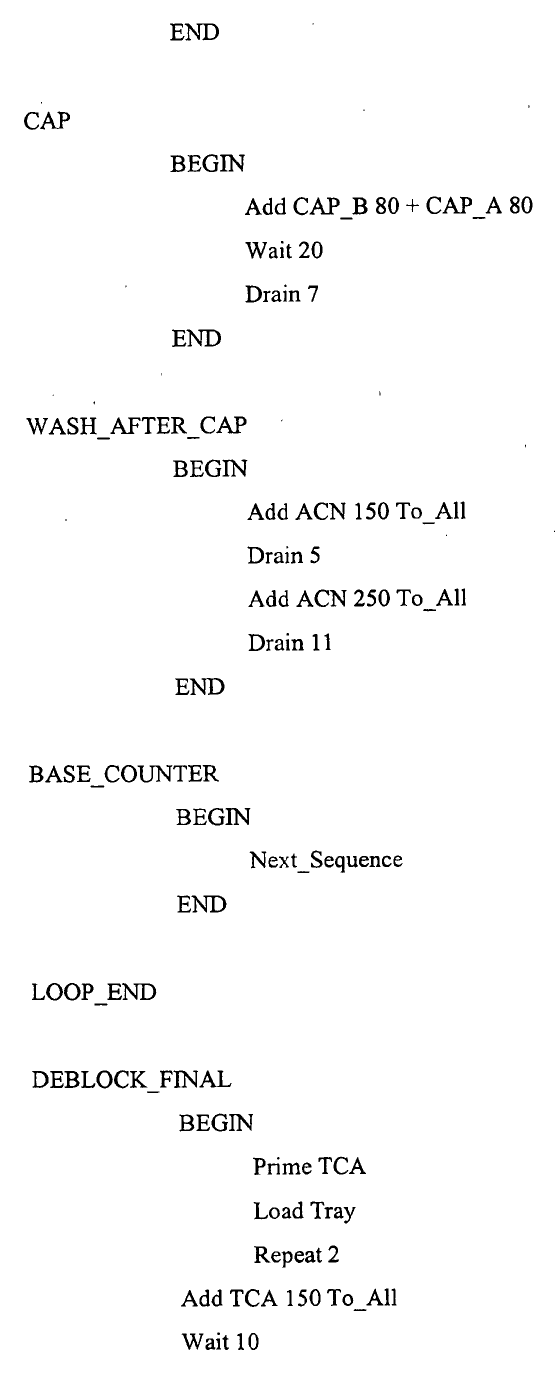

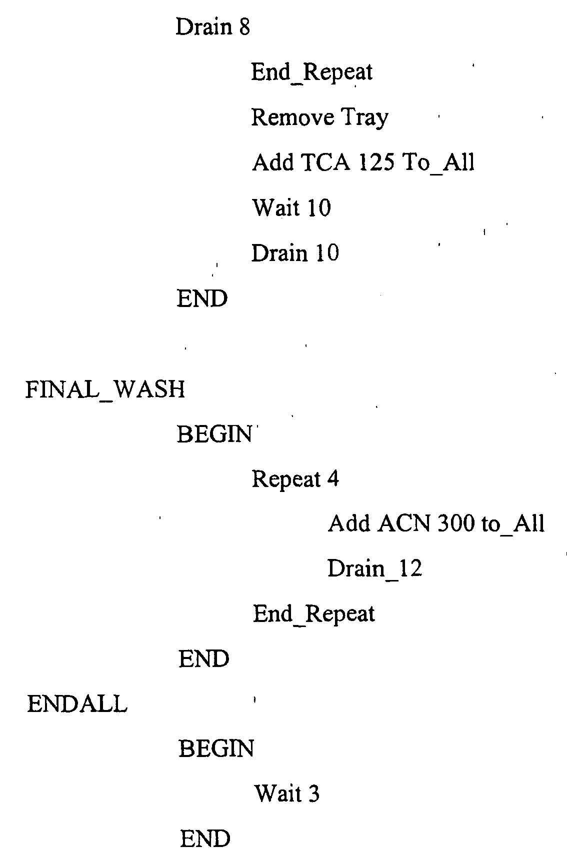

### Sequence files (.seq Files)

Table 3 is a .seq file for oligonucleotides having 2'-deoxy nucleosides linked by phosphorothioate internucleotide linkages.

**Table 3**

| Identity of columns: **Syn #, Well, Scale, Nucleotide at particular position** (identified using base identifier followed by backbone identifier where "s" is phosphorothioate). Note the columns wrap around to next line when longer than one line. | | | | | | | | | | | |
|---|---|---|---|---|---|---|---|---|---|---|---|
| 1 | A01 | 200 | As | Cs | Cs | As | Gs | Gs | As | Cs | Gs |
| Gs | Cs | Gs | Gs | As | Cs | Cs | As | G | | | |
| 2 | A02 | 200 | As | Cs | Gs | Gs | Cs | Gs | Gs | As | Cs |
| Cs | As | Gs | As | Gs | Ts | Gs | Gs | A | | | |
| 3 | A03 | 200 | As | Cs | Cs | As | As | Gs | Cs | As | Gs |
| As | Cs | Gs | Gs | As | Gs | As | Cs | G | | | |
| 4 | A04 | 200 | As | Gs | Gs | As | Gs | As | Cs | Cs | Cs |
| Cs | Gs | As | Cs | Gs | As | As | Cs | G | | | |
| 5 | A05 | 200 | As | Cs | Cs | Cs | Cs | Gs | As | Cs | Gs |
| As | As | Cs | Gs | As | Cs | Ts | Gs | G | | | |
| 6 | A06 | 200 | As | Cs | Gs | As | As | Cs | Gs | As | Cs |
| Ts | Gs | Gs | Cs | Gs | As | Cs | As | G | | | |
| 7 | A07 | 200 | As | Cs | Gs | As | Cs | Ts | Gs | Gs | Cs |
| Gs | As | Cs | As | Gs | Gs | Ts | As | G | | | |
| 8 | A08 | 200 | As | Cs | As | Gs | Gs | Ts | As | Gs | Gs |
| Ts | Cs | Ts | Ts | Gs | Gs | Ts | Gs | G | | | |
| 9 | A09 | 200 | As | Gs | Gs | Ts | Cs | Ts | Ts | Gs | Gs |
| Ts | Gs | Gs | Gs | Ts | Gs | As | Cs | G | | | |
| 10 | A10 | 200 | As | Gs | Ts | Cs | As | Cs | Gs | As | Cs |
| As | As | Gs | As | As | As | Cs | As | C | | | |
| 11 | A11 | 200 | As | Cs | Gs | As | Cs | As | As | Gs | As |
| As | As | Cs | As | Cs | Gs | Gs | Ts | C | | | |
| 12 | A12 | 200 | As | Gs | As | As | As | Cs | As | Cs | Gs |
| Gs | Ts | Cs | Gs | Gs | Ts | Cs | Cs | T | | | |
| 13 | B01 | 200 | As | As | Cs | As | Cs | Gs | Gs | Ts | Cs |
| Gs | Gs | Ts | Cs | Cs | Ts | Gs | Ts | C | | | |
| 14 | B02 | 200 | As | Cs | Ts | Cs | As | Cs | Ts | Gs | As |
| Cs | Gs | Ts | Gs | Ts | Cs | Ts | Cs | A | | | |
| 15 | B03 | 200 | As | Cs | Gs | Gs | As | As | Gs | Gs | As |
| As | Cs | Gs | Cs | Cs | As | Cs | Ts | T | | | |
| 16 | B04 | 200 | As | Ts | Cs | Ts | Gs | Ts | Gs | Gs | As |
| Cs | Cs | Ts | Ts | Gs | Ts | Cs | Ts | C | | | |
| 17 | B05 | 200 | As | Cs | As | Cs | Ts | Ts | Cs | Ts | Ts |
| Cs | Cs | Gs | As | Cs | Cs | Gs | Ts | G | | | |
| 18 | B06 | 200 | As | Cs | Ts | Cs | Ts | Cs | Gs | As | Cs |
| As | Cs | As | Gs | Gs | As | Cs | Gs | T | | | |
| 19 | B07 | 200 | As | As | As | Cs | Cs | Cs | Cs | As | Gs |
| Ts | Ts | Cs | Gs | Ts | Cs | Ts | As | A | | | |
| 20 | B08 | 200 | As | Ts | Gs | Ts | Cs | Cs | Cs | Cs | As |
| As | As | Gs | As | Cs | Ts | As | Ts | G | | | |
| 21 | B09 | 200 | As | Cs | Gs | Cs | Ts | Cs | Gs | Gs | Gs |
| As | Cs | Gs | Gs | Gs | Ts | Cs | As | G | | | |
| 22 | B10 | 200 | As | Gs | Cs | Cs | Gs | As | As | Gs | As |
| As | Gs | As | Gs | Gs | Ts | Ts | As | C | | | |
| 23 | B11 | 200 | As | Cs | As | Cs | As | Gs | Ts | As | Gs |
| As | Cs | Gs | As | As | As | Gs | Cs | T | | | |
| 24 | B12 | 200 | As | Cs | As | Cs | Ts | Cs | Ts | Gs | Gs |
| Ts | Ts | Ts | Cs | Ts | Gs | Gs | As | C | | | |
| 25 | C01 | 200 | As | Cs | Gs | As | Cs | Cs | As | Gs | As |
| As | As | Ts | As | Gs | Ts | Ts | Ts | T | | | |
| 26 | C02 | 200 | As | Gs | Ts | Ts | As | As | As | As | Gs |
| Gs | Gs | Cs | Ts | Gs | Cs | Ts | As | G | | | |
| 27 | C03 | 200 | As | Gs | Gs | Ts | Ts | Gs | Ts | Gs | As |
| Cs | Gs | As | Cs | Gs | As | Gs | Gs | T | | | |
| 28 | C04 | 200 | As | As | Ts | Gs | Ts | As | Cs | Cs | Ts |
| As | Cs | Gs | Gs | Ts | Ts | Gs | Gs | C | | | |
| 29 | C05 | 200 | As | Gs | Ts | Cs | As | Cs | Gs | Ts | Cs |
| Cs | Ts | Cs | Ts | Cs | Ts | Gs | Ts | C | | | |
| 30 | C06 | 200 | Cs | Ts | Gs | Gs | Cs | Gs | As | Cs | As |
| Gs | Gs | Ts | As | Gs | Gs | Ts | Cs | T | | | |
| 31 | C07 | 200 | Cs | Ts | Cs | Ts | Gs | Ts | Gs | Ts | Gs |
| As | Cs | Gs | Gs | Ts | Gs | Gs | Ts | C | | | |
| 32 | C08 | 200 | Cs | As | Gs | Gs | Ts | Cs | Gs | Ts | Cs |
| Ts | Ts | Cs | Cs | Cs | Gs | Ts | Gs | G | | | |
| 33 | C09 | 200 | Cs | Ts | Gs | Ts | Gs | Gs | Ts | As | Gs |
| As | Cs | Gs | Ts | Gs | Gs | As | Cs | A | | | |
| 34 | C10 | 200 | Cs | Ts | As | As | Cs | Gs | As | Ts | Gs |
| Ts | Cs | Cs | Cs | Cs | As | As | As | G | | | |
| 35 | C11 | 200 | Cs | Ts | Gs | Ts | Ts | Cs | Gs | As | Cs |
| As | Cs | Ts | Cs | Ts | Gs | Gs | Ts | T | | | |
| 36 | C12 | 200 | Cs | Ts | Gs | Gs | As | Cs | Cs | As | As |
| Cs | As | Cs | Gs | Ts | Ts | Gs | Ts | C | | | |
| 37 | D01 | 200 | Cs | Cs | Gs | Ts | Cs | Cs | Gs | Ts | Gs |
| Ts | Ts | Ts | Gs | Ts | Ts | Cs | Ts | G | | | |
| 38 | D02 | 200 | Cs | Ts | Gs | As | Cs | Ts | As | Cs | As |
| As | Cs | As | Gs | As | Cs | As | Cs | C | | | |
| 39 | D03 | 200 | Cs | As | As | Cs | As | Gs | As | Cs | As |
| Cs | Cs | As | Gs | Gs | Gs | Gs | Ts | C | | | |
| 40 | D04 | 200 | Cs | As | Gs | Gs | Gs | Gs | Ts | Cs | Cs |
| Ts | As | Gs | Cs | Cs | Gs | As | Cs | T | | | |
| 41 | D05 | 200 | Cs | Ts | Cs | Ts | As | Gs | Ts | Ts | As |
| As | As | As | Gs | Gs | Gs | Cs | Ts | G | | | |
| 42 | D06 | 200 | Cs | Ts | Gs | Cs | Ts | As | Gs | As | As |
| Gs | Gs | As | Cs | Cs | Gs | As | Gs | G | | | |
| 43 | D07 | 200 | Cs | Ts | Gs | As | As | As | Ts | Gs | Ts |
| As | Cs | Cs | Ts | As | Cs | Gs | Gs | T | | | |
| 44 | D08 | 200 | Cs | As | Cs | Cs | Cs | Gs | Ts | Ts | Ts |
| Gs | Ts | Cs | Cs | Gs | Ts | Cs | As | A | | | |
| 45 | D09 | 200 | Cs | Ts | Cs | Gs | As | Ts | As | Cs | Gs |
| Gs | Gs | Ts | Cs | As | Gs | Ts | Cs | A | | | |
| 46 | D10 | 200 | Gs | Gs | Ts | As | Gs | Gs | Ts | Cs | Ts |
| Ts | Gs | Gs | Ts | Gs | Gs | Gs | Ts | G | | | |
| 47 | D11 | 200 | Gs | As | Cs | Ts | Ts | Ts | Gs | Cs | Cs |
| Ts | Ts | As | Cs | Gs | Gs | As | As | G | | | |
| 48 | D12 | 200 | Gs | Ts | Gs | Gs | As | Gs | Ts | Cs | Ts |
| Ts | Ts | Gs | Ts | Cs | Ts | Gs | Ts | G | | | |
| 49 | E01 | 200 | Gs | Gs | As | Gs | Ts | Cs | Ts | Ts | Ts |
| Gs | Ts | Cs | Ts | Gs | Ts | Gs | Gs | T | | | |
| 50 | E02 | 200 | Gs | Gs | As | Cs | As | Cs | Ts | Cs | Ts |
| Cs | Gs | As | Cs | As | Cs | As | Gs | G | | | |
| 51 | E03 | 200 | Gs | As | Cs | As | Cs | As | Gs | Gs | As |
| Cs | Gs | Ts | Gs | Gs | Cs | Gs | As | G | | | |
| 52 | E04 | 200 | Gs | As | Gs | Ts | As | Cs | Gs | As | Gs |
| Cs | Gs | Gs | Gs | Cs | Cs | Gs | As | A | | | |
| 53 | E05 | 200 | Gs | As | Cs | Ts | As | Ts | Gs | Gs | Ts |
| As | Gs | As | Cs | Gs | Cs | Ts | Cs | G | | | |
| 54 | E06 | 200 | Gs | As | As | Gs | As | Gs | Gs | Ts | Ts |
| As | Cs | As | Cs | As | Gs | Ts | As | G | | | |
| 55 | E07 | 200 | Gs | As | Gs | Gs | Ts | Ts | As | Cs | As |
| Cs | As | Gs | Ts | As | Gs | As | Cs | G | | | |
| 56 | E08 | 200 | Gs | Ts | Ts | Gs | Ts | Cs | Cs | Gs | Ts |
| Cs | Cs | Gs | Ts | Gs | Ts | Ts | Ts | G | | | |
| 57 | E09 | 200 | Gs | As | Cs | Ts | Cs | Ts | Cs | Gs | Gs |
| Gs | As | Cs | Cs | As | Cs | Cs | As | C | | | |
| 58 | E10 | 200 | Gs | Ts | As | Gs | Gs | As | Gs | As | As |
| Cs | Cs | As | Cs | Gs | As | Cs | Cs | A | | | |
| 59 | E11 | 200 | Gs | Gs | Ts | Ts | Cs | Ts | Ts | Cs | Gs |
| Gs | Ts | Ts | Gs | Gs | Ts | Ts | As | T | | | |
| 60 | E12 | 200 | Gs | Ts | Gs | Gs | Gs | Gs | Ts | Ts | Cs |
| Gs | Ts | Cs | Cs | Ts | Ts | Gs | Gs | G | | | |
| 61 | F01 | 200 | Gs | Ts | Cs | As | Cs | Gs | Ts | Cs | Cs |
| Ts | Cs | Ts | Gs | As | As | As | Ts | G | | | |
| 62 | F02 | 200 | Gs | Ts | Cs | Cs | Ts | Cs | Cs | Ts | As |
| Cs | Cs | Gs | Ts | Ts | Ts | Cs | Ts | C | | | |
| 63 | F03 | 200 | Gs | Ts | Cs | Cs | Cs | Cs | As | Cs | Gs |
| Ts | Cs | Cs | Gs | Ts | Cs | Ts | Ts | C | | | |
| 64 | F04 | 200 | Ts | Cs | As | Cs | Cs | As | Gs | Gs | As |
| Cs | Gs | Gs | Cs | Gs | Gs | As | Cs | C | | | |
| 65 | F05 | 200 | Ts | As | Cs | Cs | As | As | Gs | Cs | As |
| Gs | As | Cs | Gs | Gs | As | Gs | As | C | | | |
| 66 | F06 | 200 | Ts | Cs | Cs | Ts | Gs | Ts | Cs | Ts | Ts |
| Ts | Gs | As | Cs | Cs | As | Cs | Ts | C | | | |
| 67 | F07 | 200 | Ts | Gs | Ts | Cs | Ts | Ts | Ts | Gs | As |
| Cs | Cs | As | Cs | Ts | Cs | As | Cs | T | | | |
| 68 | F08 | 200 | Ts | Gs | As | Cs | Cs | As | Cs | Ts | Cs |
| As | Cs | Ts | Gs | As | Cs | Gs | Ts | G | | | |
| 69 | F09 | 200 | Ts | Gs | As | Cs | Gs | Ts | Gs | Ts | Cs |
| Ts | Cs | As | As | Gs | Ts | Gs | As | C | | | |
| 70 | F10 | 200 | Ts | Cs | As | As | Gs | Ts | Gs | As | Cs |
| Ts | Ts | Ts | Gs | Cs | Cs | Ts | Ts | A | | | |
| 71 | F11 | 200 | Ts | Gs | Ts | Ts | Ts | As | Ts | Gs | As |
| Cs | Gs | Cs | Ts | Gs | Gs | Gs | Gs | T | | | |
| 72 | F12 | 200 | Ts | Ts | As | Ts | Gs | As | Cs | Gs | Cs |
| Ts | Gs | Gs | Gs | Gs | Ts | Ts | Gs | G | | | |
| 73 | G01 | 200 | Ts | Gs | As | Cs | Gs | Cs | Ts | Gs | Gs |
| Gs | Gs | Ts | Ts | Gs | Gs | As | Ts | C | | | |
| 74 | G02 | 200 | Ts | Cs | Gs | Ts | Cs | Ts | Ts | Cs | Cs |
| Cs | Gs | Ts | Gs | Gs | As | Gs | Ts | C | | | |
| 75 | G03 | 200 | Ts | Gs | Gs | Ts | As | Gs | As | Cs | Gs |
| Ts | Gs | Gs | As | Cs | As | Cs | Ts | T | | | |
| 76 | G04 | 200 | Ts | Ts | Cs | Ts | Ts | Cs | Cs | Gs | As |
| Cs | Cs | Gs | Ts | Gs | As | Cs | As | T | | | |
| 77 | G05 | 200 | Ts | Gs | Gs | Ts | As | Gs | As | Cs | Gs |
| Cs | Ts | Cs | Gs | Gs | Gs | As | Cs | G | | | |
| 78 | G06 | 200 | Ts | As | Gs | As | Cs | Gs | Cs | Ts | Cs |
| Gs | Gs | Gs | As | Cs | Gs | Gs | Gs | T | | | |
| 79 | G07 | 200 | Ts | Ts | Ts | Ts | As | Cs | As | Gs | Ts |
| Gs | Gs | Gs | As | As | Cs | Cs | Ts | G | | | |
| 80 | G08 | 200 | Ts | Gs | Gs | Gs | As | As | Cs | Cs | Ts |
| Gs | Ts | Ts | Cs | Gs | As | Cs | As | C | | | |
| 81 | G09 | 200 | Ts | Cs | Gs | Gs | Gs | As | Cs | Cs | As |
| Cs | Cs | As | Cs | Ts | As | Gs | Gs | G | | | |
| 82 | G10 | 200 | Ts | As | Gs | Gs | As | Cs | As | As | As |
| Cs | Gs | Gs | Ts | As | Gs | Gs | As | G | | | |
| 83 | G11 | 200 | Ts | Gs | Cs | Ts | As | Gs | As | As | Gs |
| Gs | As | Cs | Cs | Gs | As | Gs | Gs | T | | | |
| 84 | G12 | 200 | Ts | Cs | Ts | Gs | Ts | Cs | As | Cs | Ts |
| Cs | Cs | Gs | As | Cs | Gs | Ts | Gs | G | | | |

Table 4 is a .seq file for oligonucleotides having regions of 2'-O-(2-methoxyethyl)-nucleosides and a central region of 2'-deoxy nucleosides each linked by phosphorothioate internucleotide linkages.

**Table 4**

| Identity of columns: **Syn #, Well, Scale, Nucleotide at particular position** (identified using base identifier followed by backbone identifier where "s" is phosphorothioate and "moe" indicated a 2'-O-(2-methoxyethyl) substituted nucleoside). The columns wrap around to next line when longer than one line. |
|---|
| 1 A01 200 moeAs moeCs moeCs moeAs Gs Gs As Cs Gs Gs Cs Gs Gs As moeCs moeCs moeAs moeG |
| 2 A02 200 moeAs moeCs moeGs moeGs Cs Gs Gs As Cs Cs As Gs As Gs moeTs moeGs moeGs moeA |
| 3 A03 200 moeAs moeCs moeCs moeAs As Gs Cs As Gs As Cs Gs Gs As moeGs moeAs moeCs moeG |
| 4 A04 200 moeAs moeGs moeGs moeAs Gs As Cs Cs Cs Cs Gs As Cs Gs moeAs moeAs moeCs moeG |
| 5 A05 200 moeAs moeCs moeCs moeCs Cs Gs As Cs Gs As As Cs Gs As |
| moeCs moeTs moeGs moeG |
| 6 A06 200 moeAs moeCs moeGs moeAs As Cs Gs As Cs Ts Gs Gs Cs Gs moeAs moeCs moeAs moeG |
| 7 A07 200 moeAs moeCs moeGs moeAs Cs Ts Gs Gs Cs Gs As Cs As Gs moeGs moeTs moeAs moeG |
| 8 A08 200 moeAs moeCs moeAs moeGs Gs Ts As Gs Gs Ts Cs Ts Ts Gs moeGs moeTs moeGs moeG |
| 9 A09 200 moeAs moeGs moeGs moeTs Cs Ts Ts Gs Gs Ts Gs Gs Gs Ts moeGs moeAs moeCs moeG |
| 10 A10 200 moeAs moeGs moeTs moeCs As Cs Gs As Cs As As Gs As As moeAs moeCs moeAs moeC |
| 11 A11 200 moeAs moeCs moeGs moeAs Cs As As Gs As As As Cs As Cs moeGs moeGs moeTs moeC |
| 12 A12 200 moeAs moeGs moeAs moeAs As Cs As Cs Gs Gs Ts Cs Gs Gs moeTs moeCs moeCs moeT |
| 13 B01 200 moeAs moeAs moeCs moeAs Cs Gs Gs Ts Cs Gs Gs Ts Cs Cs moeTs moeGs moeTs moeC |
| 14 B02 200 moeAs moeCs moeTs moeCs As Cs Ts Gs As Cs Gs Ts Gs Ts moeCs moeTs moeCs moeA |
| 15 B03 200 moeAs moeCs moeGs moeGs As As Gs Gs As As Cs Gs Cs Cs moeAs moeCs moeTs moeT |
| 16 B04 200 moeAs moeTs moeCs moeTs Gs Ts Gs Gs As Cs Cs Ts Ts Gs moeTs moeCs moeTs moeC |
| 17 B05 200 moeAs moeCs moeAs moeCs Ts Ts Cs Ts Ts Cs Cs Gs As Cs moeCs moeGs moeTs moeG |
| 18 B06 200 moeAs moeCs moeTs moeCs Ts Cs Gs As Cs As Cs As Gs Gs moeAs moeCs moeGs moeT |
| 19 B07 200 moeAs moeAs moeAs moeCs Cs Cs Cs As Gs Ts Ts Cs Gs Ts moeCs moeTs moeAs moeA |
| 20 B08 200 moeAs moeTs moeGs moeTs Cs Cs Cs Cs As As As Gs As Cs moeTs moeAs moeTs moeG |
| 21 B09 200 moeAs moeCs moeGs moeCs Ts Cs Gs Gs Gs As Cs Gs Gs Gs moeTs moeCs moeAs moeG |
| 22 B10 200 moeAs moeGs moeCs moeCs Gs As As Gs As As Gs As Gs Gs moeTs moeTs moeAs moeC |
| 23 B11 200 moeAs moeCs moeAs moeCs As Gs Ts As Gs As Cs Gs As As moeAs moeGs moeCs moeT |
| 24 B12 200 moeAs moeCs moeAs moeCs Ts Cs Ts Gs Gs Ts Ts Ts Cs Ts moeGs moeGs moeAs moeC |
| 25 C01 200 moeAs moeCs moeGs moeAs Cs Cs As Gs As As As Ts As Gs moeTs moeTs moeTs inoeT |
| 26 C02 200 moeAs moeGs moeTs moeTs As As As As Gs Gs Gs Cs Ts Gs moeCs moeTs moeAs moeG |
| 27 C03 200 moeAs moeGs moeGs moeTs Ts Gs Ts Gs As Cs Gs As Cs Gs moeAs moeGs moeGs moeT |
| 28 C04 200 moeAs moeAs moeTs moeGs Ts As Cs Cs Ts As Cs Gs Gs Ts moeTs moeGs moeGs moeC |
| 29 C05 200 moeAs moeGs moeTs moeCs As Cs Gs Ts Cs Cs Ts Cs Ts Cs moeTs moeGs moeTs moeC |
| 30 C06 200 moeCs moeTs moeGs moeGs Cs Gs As Cs As Gs Gs Ts As Gs moeGs moeTs moeCs moeT |
| 31 C07 200 moeCs moeTs moeCs moeTs Gs Ts Gs Ts Gs As Cs Gs Gs Ts moeGs moeGs moeTs moeC |
| 32 C08 200 moeCs moeAs moeGs moeGs Ts Cs Gs Ts Cs Ts Ts Cs Cs Cs moeGs moeTs moeGs moeG |
| 33 C09 200 moeCs moeTs moeGs moeTs Gs Gs Ts As Gs As Cs Gs Ts Gs moeGs moeAs moeCs moeA |
| 34 C10 200 moeCs moeTs moeAs moeAs Cs Gs As Ts Gs Ts Cs Cs Cs Cs moeAs moeAs moeAs moeG |
| 35 C11 200 moeCs moeTs moeGs moeTs Ts Cs Gs As Cs As Cs Ts Cs Ts moeGs moeGs moeTs moeT |
| 36 C12 200 moeCs moeTs moeGs moeGs As Cs Cs As As Cs As Cs Gs Ts |
| moeTs moeGs moeTs moeC |
| 37 D01 200 moeCs moeCs moeGs moeTs Cs Cs Gs Ts Gs Ts Ts Ts Gs Ts moeTs moeCs moeTs moeG |
| 38 D02 200 moeCs moeTs moeGs moeAs Cs Ts As Cs As As Cs As Gs As moeCs moeAs moeCs moeC |
| 39 D03 200 moeCs moeAs moeAs moeCs As Gs As Cs As Cs Cs As Gs Gs moeGs moeGs moeTs moeC |
| 40 D04 200 moeCs moeAs moeGs moeGs Gs Gs Ts Cs Cs Ts As Gs Cs Cs moeGs moeAs moeCs moeT |
| 41 D05 200 moeCs moeTs moeCs moeTs As Gs Ts Ts As As As As Gs Gs moeGs moeCs moeTs moeG |
| 42 D06 200 moeCs moeTs moeGs moeCs Ts As Gs As As Gs Gs As Cs Cs moeGs moeAs moeGs moeG |
| 43 D07 200 moeCs moeTs moeGs moeAs As As Ts Gs Ts As Cs Cs Ts As moeCs moeGs moeGs moeT |
| 44 D08 200 moeCs moeAs moeCs moeCs Cs Gs Ts Ts Ts Gs Ts Cs Cs Gs moeTs moeCs moeAs moeA |
| 45 D09 200 moeCs moeTs moeCs moeGs As Ts As Cs Gs Gs Gs Ts Cs As moeGs moeTs moeCs moeA |
| 46 D10 200 moeGs moeGs moeTs moeAs Gs Gs Ts Cs Ts Ts Gs Gs Ts Gs moeGs moeGs moeTs moeG |
| 47 D11 200 moeGs moeAs moeCs moeTs Ts Ts Gs Cs Cs Ts Ts As Cs Gs moeGs moeAs moeAs moeG |
| 48 D12 200 moeGs moeTs moeGs moeGs As Gs Ts Cs Ts Ts Ts Gs Ts Cs moeTs moeGs moeTs moeG |
| 49 E01 200 moeGs moeGs moeAs moeGs Ts Cs Ts Ts Ts Gs Ts Cs Ts Gs moeTs moeGs moeGs moeT |
| 50 E02 200 moeGs moeGs moeAs moeCs As Cs Ts Cs Ts Cs Gs As Cs As moeCs moeAs moeGs moeG |
| 51 E03 200 moeGs moeAs moeCs moeAs Cs As Gs Gs As Cs Gs Ts Gs Gs moeCs moeGs moeAs moeG |
| 52 E04 200 moeGs moeAs moeGs moeTs As Cs Gs As Gs Cs Gs Gs Gs Cs moeCs moeGs moeAs moeA |
| 53 E05 200 moeGs moeAs moeCs moeTs As Ts Gs Gs Ts As Gs As Cs Gs moeCs moeTs moeCs moeG |
| 54 E06 200 moeGs moeAs moeAs moeGs As Gs Gs Ts Ts As Cs As Cs As moeGs moeTs moeAs moeG |
| 55 E07 200 moeGs moeAs moeGs moeGs Ts Ts As Cs As Cs As Gs Ts As moeGs moeAs moeCs moeG |
| 56 E08 200 moeGs moeTs moeTs moeGs Ts Cs Cs Gs Ts Cs Cs Gs Ts Gs moeTs moeTs moeTs moeG |
| 57 E09 200 moeGs moeAs moeCs moeTs Cs Ts Cs Gs Gs Gs As Cs Cs As moeCs moeCs moeAs moeC |
| 58 E10 200 moeGs moeTs moeAs moeGs Gs As Gs As As Cs Cs As Cs Gs moeAs moeCs moeCs moeA |
| 59 E11 200 moeGs moeGs moeTs moeTs Cs Ts Ts Cs Gs Gs Ts Ts Gs Gs moeTs moeTs moeAs moeT |
| 60 E12 200 moeGs moeTs moeGs moeGs Gs Gs Ts Ts Cs Gs Ts Cs Cs Ts moeTs moeGs moeGs moeG |
| 61 F01 200 moeGs moeTs moeCs moeAs Cs Gs Ts Cs Cs Ts Cs Ts Gs As moeAs moeAs moeTs moeG |
| 62 F02 200 moeGs moeTs moeCs moeCs Ts Cs Cs Ts As Cs Cs Gs Ts Ts moeTs moeCs moeTs moeC |
| 63 F03 200 moeGs moeTs moeCs moeCs Cs Cs As Cs Gs Ts Cs Cs Gs Ts moeCs moeTs moeTs moeC |
| 64 F04 200 moeTs moeCs moeAs moeCs Cs As Gs Gs As Cs Gs Gs Cs Gs moeGs moeAs moeCs moeC |
| 65 F05 200 moeTs moeAs moeCs moeCs As As Gs Cs As Gs As Cs Gs Gs moeAs moeGs moeAs moeC |
| 66 F06 200 moeTs moeCs moeCs moeTs Gs Ts Cs Ts Ts Ts Gs As Cs Cs moeAs moeCs moeTs moeC |
| 67 F07 200 moeTs moeGs moeTs moeCs Ts Ts Ts Gs As Cs Cs As Cs Ts |
| moeCs moeAs moeCs moeT |
| 68 F08 200 moeTs moeGs moeAs moeCs Cs As Cs Ts Cs As Cs Ts Gs As moeCs moeGs moeTs moeG |
| 69 F09 200 moeTs moeGs moeAs moeCs Gs Ts Gs Ts Cs Ts Cs As As Gs moeTs moeGs moeAs moeC |
| 70 F10 200 moeTs moeCs moeAs moeAs Gs Ts Gs As Cs Ts Ts Ts Gs Cs moeCs moeTs moeTs moeA |
| 71 F11 200 moeTs moeGs moeTs moeTs Ts As Ts Gs As Cs Gs Cs Ts Gs moeGs moeGs moeGs moeT |
| 72 F12 200 moeTs moeTs moeAs moeTs Gs As Cs Gs Cs Ts Gs Gs Gs Gs moeTs moeTs moeGs moeG |
| 73 G01 200 moeTs moeGs moeAs moeCs Gs Cs Ts Gs Gs Gs Gs Ts Ts Gs moeGs moeAs moeTs moeC |
| 74 G02 200 moeTs moeCs moeGs moeTs Cs Ts Ts Cs Cs Cs Gs Ts Gs Gs moeAs moeGs moeTs moeC |
| 75 G03 200 moeTs moeGs moeGs moeTs As Gs As Cs Gs Ts Gs Gs As Cs moeAs moeCs moeTs moeT |
| 76 G04 200 moeTs moeTs moeCs moeTs Ts Cs Cs Gs As Cs Cs Gs Ts Gs moeAs moeCs moeAs moeT |
| 77 G05 200 moeTs moeGs moeGs moeTs As Gs As Cs Gs Cs Ts Cs Gs Gs moeGs moeAs moeCs moeG |
| 78 G06 200 moeTs moeAs moeGs moeAs Cs Gs Cs Ts Cs Gs Gs Gs As Cs moeGs moeGs moeGs moeT |
| 79 G07 200 moeTs moeTs moeTs moeTs As Cs As Gs Ts Gs Gs Gs As As moeCs moeCs moeTs moeG |
| 80 G08 200 moeTs moeGs moeGs moeGs As As Cs Cs Ts Gs Ts Ts Cs Gs moeAs moeCs moeAs moeC |
| 81 G09 200 moeTs moeCs moeGs moeGs Gs As Cs Cs As Cs Cs As Cs Ts moeAs moeGs moeGs moeG |
| 82 G10 200 moeTs moeAs moeGs moeGs As Cs As As As Cs Gs Gs Ts As moeGs moeGs moeAs moeG |
| 83 G11 200 moeTs moeGs moeCs moeTs As Gs As As Gs Gs As Cs Cs Gs moeAs moeGs moeGs moeT |
| 84 G12 200 moeTs moeCs moeTs moeGs Ts Cs As Cs Ts Cs Cs Gs As Cs moeGs moeTs moeGs moeG |

### Reagent file (.tab File)

Table 5 is a .tab file for reagents necessary for synthesizing an oligonucleotides having both 2'-O-(2-methoxyethyl)nucleosides and 2'-deoxy nucleosides located therein.

**Table 5**

| Identity of columns: **GroupName, Bottle ID, ReagentName, FlowRate, Concentration.** Wherein reagent name is identified using base identifier, "moe" indicated a 2'-O-(2-methoxyethyl) substituted nucleoside and "cpg" indicates a control pore glass solid support medium. The columns wrap around to next line when longer than one line. | | | | | | | |
|---|---|---|---|---|---|---|---|
| SUPPORT | | | | | | | |
| | BEGIN | | | | | | |
| | 0 | moeG | moeG | cpg | 100 | 1 | |
| | 0 | moe5meC | moe5meC | cpg | 100 | 1 | |
| | 0 | moeA | moeA | cpg | 100 | 1 | |
| | 0 | moeT | moeT | cpg | 100 | 1 | |
| | END | | | | | | |
| DEBLOCK | | | | | | | |
| | BEGIN | | | | | | |
| | 70 | TCA | TCA | | | 100 | 1 |
| | END | | | | | | |
| WASH | | | | | | | |
| | BEGIN | | | | | | |
| | 65 | ACN | ACN | | | 190 | 1 |
| | END | | | | | | |
| OXIDIZERS | | | | | | | |
| | BEGIN | | | | | | |
| | 68 | BEAU | | BEAUCAGE | | 320 | 1 |
| | END | | | | | | |
| CAPPING | | | | | | | |
| | BEGIN | | | | | | |
| | 66 | CAP_B | | CAP_B | | 220 | 1 |
| | 67 | CAP_A | | CAP_A | | 230 | 1 |
| | END | | | | | | |
| DEOXY THIOATE | | | | | | | |
| | BEGIN | | | | | | |
| | 31,32 | Gs | deoxyG | | 270 | 1 | |
| | 39,40 | 5meCs 5methyl | deoxyC | | 270 | 1 | |
| | 37,38 | As | deoxyA | | 270 | 1 | |
| | 29,30 | Ts | deoxyT | | 270 | 1 | |
| | END | | | | | | |
| MOE-THIOATE | | | | | | | |
| | BEGIN | | | | | | |
| | 15,16 | moeGs | methoxyethoxyG | | 240 | 1 | |
| | 23,24 | moe5meCs | methoxyethoxyC | | 240 | 1 | |
| | 21,22 | moeAs | methoxyethoxyA | | 240 | 1 | |
| | 13,14 | moeTs | methoxyethoxyT | | 240 | 1 | |
| | END | | | | | | |
| ACTIVATORS | | | | | | | |
| | BEGIN | | | | | | |
| | 5,6,7,8 | SET | | s-ethyl-tet | 280 | | |
| | | Activates | | | | | |
| | | DEOXY_THIOATE | | | | | |
| | | MOE_THIOATE | | | | | |
| | END | | | | | | |

### EXAMPLE 4: Oligonucleotide Synthesis - 96 Well Plate Format

Oligonucleotides were synthesized via solid phase P(III) phosphoramidite chemistry using a multi well automated synthesizer utilizing input files as described in EXAMPLE 3 above. The oligonucleotides were synthesized by assembling 96 sequences simultaneously in a standard 96 well format. Phosphodiester internucleotide linkages were afforded by oxidation with aqueous iodine. Phosphorothioate internucleotide linkages were generated by sulfurization utilizing 3,H-1,2 benzodithiole-3-one 1,1 dioxide (Beaucage Reagent) in anhydrous acetonitrile. Standard base-protected beta-cyanoethyldiisopropyl phosphoramidites were purchased from commercial vendors (e.g. PE/ABI, Pharmacia). Non-standard nucleosides are synthesized as per known literature or patented methods. They are utilized as base protected beta-cyanoethyldiisopropyl phosphoramidites.

Following synthesis, oligonucleotides were cleaved from support and deprotected with concentrated NH₄OH at elevated temperature (55-60°C) for 12-16 hours and the released product then dried *in vacuo*. The dried product was then re-suspended in sterile water to afford a master plate from which all analytical and test plate samples are then diluted utilizing robotic pipettors.

### EXAMPLE 5: Alternative Oligonucleotide Synthesis

Unsubstituted and substituted phosphodiester oligonucleotides are alternately synthesized on an automated DNA synthesizer (Applied Biosystems model 380B) using standard phosphoramidite chemistry with oxidation by iodine.

Phosphorothioates are synthesized as per the phosphodiester oligonucleotides except the standard oxidation bottle was replaced by 0.2 M solution of 3H-1,2-benzodithiole-3-one 1,1-dioxide in acetonitrile for the stepwise thiation of the phosphite linkages. The thiation wait step was increased to 68 sec and was followed by the capping step. After cleavage from the CPG column and deblocking in concentrated ammonium hydroxide at 55°C (18 hr), the oligonucleotides were purified by precipitating twice with 2.5 volumes of ethanol from a 0.5 M NaCl solution.

Phosphinate oligonucleotides are prepared as described in U.S. Patent 5,508,270.

Alkyl phosphonate oligonucleotides are prepared as described in U.S. Patent 4,469,863.

3'-Deoxy-3'-methylene phosphonate oligonucleotides are prepared as described in U.S. Patents 5,610,289 or 5,625,050.

Phosphoramidite oligonucleotides are prepared as described in U.S. Patent, 5,256,775 or U.S. Patent 5,366,878.

Alkylphosphonothioate oligonucleotides are prepared as described in published PCT applications PCT/US94/00902 and PCT/US93/06976 (published as WO 94/17093 and WO 94/02499, respectively).

3'-Deoxy-3'-amino phosphoramidate oligonucleotides are prepared as described in U.S. Patent 5,476,925.

Phosphotriester oligonucleotides are prepared as described in U.S. Patent 5,023,243.

Boranophosphate oligonucleotides are prepared as described in U.S. Patents 5,130,302 and 5,177,198.

Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone compounds having, for instance, alternating MMI and PO or PS linkages are prepared as described in U.S. Patents 5,378,825; 5,386,023; 5,489,677; 5,602,240 and 5,610,289.

Formacetal and thioformacetal linked oligonucleosides are prepared as described in U.S. Patents 5,264,562 and 5,264,564.

Ethylene oxide linked oligonucleosides are prepared as described in U.S. Patent 5,223,618.

### EXAMPLE 6: PNA Synthesis

Peptide nucleic acids (PNAs) are prepared in accordance with any of the various procedures referred to in Peptide Nucleic Acids (PNA): Synthesis, Properties and Potential Applications, *Bioorganic & Medicinal Chemistry*, 1996, *4*, 5. They may also be prepared in accordance with U.S. Patents 5,539,082; 5,700,922, and 5,719,262.

### EXAMPLE 7: Chimeric Oligonucleotide Synthesis

Chimeric oligonucleotides, oligonucleosides or mixed oligonucleotides/oligonucleosides of the invention can be of several different types. These include a first type wherein the "gap" segment of linked nucleosides is positioned between 5' and 3' "wing" segments of linked nucleosides and a second "open end" type wherein the "gap" segment is located at either the 3' or the 5' terminus of the oligomeric compound. Oligonucleotides of the first type are also known in the art as "gapmers" or gapped oligonucleotides. Oligonucleotides of the second type are also known in the art as "hemimers" or "wingmers."

### A. [2'-O-Me]--[2'-deoxy]--[2'-O-Me] Chimeric Phosphorothioate Oligonucleotides

Chimeric oligonucleotides having 2'-O-alkyl phosphorothioate and 2'-deoxy phosphorothioate oligonucleotide segments are synthesized using 2'-deoxy-5'-dimethoxytrityl-3'-O-phosphoramidites for the DNA portion and 5'-dimethoxytrityl-2'-O-methyl-3'-O-phosphoramidites for 5' and 3' wings. The standard synthesis cycle is modified by increasing the wait step after the delivery of tetrazole and base to 600 s repeated four times for DNA and twice for 2'-0-methyl. The fully protected oligonucleotide was cleaved from the support and the phosphate group is deprotected in 3:1 Ammonia/Ethanol at room temperature overnight then lyophilized to dryness. Treatment in methanolic ammonia for 24 hrs at room temperature is done to deprotect all bases and the samples are again lyophilized to dryness.

### B. [2'-O-(2-Methoxyethyl)]--[2'-deoxy]--[2'-O-(2-Methoxyethyl)] Chimeric Phosphorothioate Oligonucleotides

[2'-O-(2-methoxyethyl)]--[2'-deoxy]--[-2'-O-(2-methoxyethyl)] chimeric phosphorothioate oligonucleotides are prepared as per the procedure above for the 2'-O-methyl chimeric oligonucleotide, with the substitution of 2'-O-(2-methoxyethyl) amidites for the 2'-O-methyl amidites. ,

### C. [2'-O-(2-Methoxyethyl)Phosphodiester]--[2'-deoxy Phosphorothioate]-[2'-O-(2-Methoxyethyl) Phosphodiester] Chimeric Oligonucleotide

[2'-O-(2-methoxyethyl phosphodiester]--[2'-deoxy phosphorothioate]--[2'-O-(2-methoxyethyl) phosphodiester] chimeric oligonucleotides are prepared as per the above procedure for the 2'-O-methyl chimeric oligonucleotide with the substitution of 2'-O-(2-methoxyethyl) amidites for the 2'-O-methyl amidites in the wing portions. Sulfurization utilizing 3,H-1,2 benzodithiole-3-one 1,1 dioxide (Beaucage Reagent) is used to generate the phosphorothioate internucleotide linkages within the wing portions of the chimeric structures. Oxidization with iodine is used to generate the phosphodiester internucleotide linkages for the center gap.

Other chimeric oligonucleotides, chimeric oligonucleosides and mixed chimeric oligonucleotides/oligonucleosides are synthesized according to United States Patent 5,623,065.

### EXAMPLE 8: Output Oligonucleotides From Automated Oligonucleotide Synthesis

Using the .seq files, the .cmd files and .tab file of Example 3, oligonucleotides were prepared as per the protocol of the 96 well format of Example 4. The oligonucleotides were prepared utilizing phosphorothioate chemistry to give in one instance a first library of phosphorothioate oligodeoxynucleotides. The oligonucleotides were prepared in a second instance as a second library of hybrid oligonucleotides having phosphorothioate backbones with a first and third "wing" region of 2'-O-(2-methoxyethyl)nucleotides on either side of a center gap region of 2'-deoxy nucleotides. The two libraries contained the same set of oligonucleotide sequences. Thus the two libraries are redundant with respect to sequence but are unique with respect to the combination of sequence and chemistry. Because the sequences of the second library of compounds is the same as the first (however the chemistry is different), for brevity sake, the second library is not shown.

For illustrative purposes Tables 6-a and 6-b show the sequences of an initial first library, i.e., a library of phosphorothioate oligonucleotides targeted to a CD40 target. The compounds of Table 6-a shows the members of this library listed in compliance with the established rule for listing SEQ ID NO:, i.e., in numerical SEQ ID NO: order.

**Table 6-a**

| **Sequences of Oligonucleotides Targeted to CD40 by SEQ ID NO.:** | |
|---|---|
| NUCLEOBASE SEQUENCE | SEQ ID NO. |
| CCAGGCGGCAGGACCACT | 1 |
| GACCAGGCGGCAGGACCA | 2 |
| AGGTGAGACCAGGCGGCA | 3 |
| CAGAGGCAGACGAACCAT | 4 |
| GCAGAGGCAGACGAACCA | 5 |
| GCAAGCAGCCCCAGAGGA | 6 |
| GGTCAGCAAGCAGCCCCA | 7 |
| GACAGCGGTCAGCAAGCA | 8 |
| GATGGACAGCGGTCAGCA | 9 |
| TCTGGATGGACAGCGGTC | 10 |
| GGTGGTTCTGGATGGACA | 11 |
| GTGGGTGGTTCTGGATGG | 12 |
| GCAGTGGGTGGTTCTGGA | 13 |
| CACAAAGAACAGCACTGA | 14 |
| CTGGCACAAAGAACAGCA | 15 |
| TCCTGGCTGGCACAAAGA | 16 |
| CTGTCCTGGCTGGCACAA | 17 |
| CTCACCAGTTTCTGTCCT | 18 |
| TCACTCACCAGTTTCTGT | 19 |
| GTGCAGTCACTCACCAGT | 20 |
| ACTCTGTGCAGTCACTCA | 21 |
| CAGTGAACTCTGTGCAGT | 22 |
| ATTCCGTTTCAGTGAACT | 23 |
| GAAGGCATTCCGTTTCAG | 24 |
| TTCACCGCAAGGAAGGCA | 25 |
| CTCTGTTCCAGGTGTCTA | 26 |
| CTGGTGGCAGTGTGTCTC | 27 |
| TGGGGTCGCAGTATTTGT | 28 |
| GGTTGGGGTCGCAGTATT | 29 |
| CTAGGTTGGGGTCGCAGT | 30 |
| GGTGCCCTTCTGCTGGAC | 31 |
| CTGAGGTGCCCTTCTGCT | 32 |
| GTGTCTGTTTCTGAGGTG | 33 |
| TGGTGTCTGTTTCTGAGG | 34 |
| ACAGGTGCAGATGGTGTC | 35 |
| TTCACAGGTGCAGATGGT | 36 |
| GTGCCAGCCTTCTTCACA | 37 |
| TACAGTGCCAGCCTTCTT | 38 |
| GGACACAGCTCTCACAGG | 39 |
| TGCAGGACACAGCTCTCA | 40 |
| GAGCGGTGCAGGACACAG | 41 |
| AAGCCGGGCGAGCATGAG | 42 |
| AATCTGCTTGACCCCAAA | 43 |
| GAAACCCCTGTAGCAATC | 44 |
| GTATCAGAAACCCCTGTA | 45 |
| GCTCGCAGATGGTATCAG | 46 |
| GCAGGGCTCGCAGATGGT | 47 |
| TGGGCAGGGCTCGCAGAT | 48 |
| GACTGGGCAGGGCTCGCA | 49 |
| CATTGGAGAAGAAGCCGA | 50 |
| GATGACACATTGGAGAAG | 51 |
| GCAGATGACACATTGGAG | 52 |
| TCGAAAGCAGATGACACA | 53 |
| GTCCAAGGGTGACATTTT | 54 |
| CACAGCTTGTCCAAGGGT | 55 |
| TTGGTCTCACAGCTTGTC | 56 |
| CAGGTCTTTGGTCTCACA | 57 |
| CTGTTGCACAACCAGGTC | 58 |
| GTTTGTGCCTGCCTGTTG | 59 |
| GTCTTGTTTGTGCCTGCC | 60 |
| CCACAGACAACATCAGTC | 61 |
| CTGGGGACCACAGACAAC | 62 |
| TCAGCCGATCCTGGGGAC | 63 |
| CACCACCAGGGCTCTCAG | 64 |
| GGGATCACCACCAGGGCT | 65 |
| GAGGATGGCAAACAGGAT | 66 |
| ACCAGCACCAAGAGGATG | 67 |
| TTTTGATAAAGACCAGCA | 68 |
| TATTGGTTGGCTTCTTGG | 69 |
| GGGTTCCTGCTTGGGGTG | 70 |
| GTCGGGAAAATTGATCTC | 71 |
| GATCGTCGGGAAAATTGA | 72 |
| GGAGCCAGGAAGATCGTC | 73 |
| TGGAGCCAGGAAGATCGT | 74 |
| TGGAGCAGCAGTGTTGGA | 75 |
| GTAAAGTCTCCTGCACTG | 76 |
| TGGCATCCATGTAAAGTC | 77 |
| CGGTTGGCATCCATGTAA | 78 |
| CTCTTTGCCATCCTCCTG | 79 |
| CTGTCTCTCCTGCACTGA | 80 |
| GGTGCAGCCTCACTGTCT | 81 |
| AACTGCCTGTTTGCCCAC | 82 |
| CTTCTGCCTGCACCCCTG | 83 |
| ACTGACTGGGCATAGCTC | 84 |

The sequences shown in Table 6-a, above, and Table 6-b, below, are in a 5' to 3' direction. This is reversed with respect to 3' to 5' direction shown in the .seq files of Example 3. For synthesis purposes, the .seq files are generated reading from 3' to 5'. This allows for aligning all of the 3' most "A" nucleosides together, all of the 3' most "G" nucleosides together, all of the 3' most "C" nucleosides together and all of the 3' most "T" nucleosides together. Thus when the first nucleoside of each particular oligonucleotide (attached to the solid support) is added to the wells on the plates, machine movement is reduced since an automatic pipette can move in a linear manner down one row and up another on the 96 well plate.

The location of the well holding each particular oligonucleotides is indicated by row and column. There are eight rows designated A to G and twelve columns designated 1 to 12 in a typical 96 well format plate. Any particular well location is indicated by its "Well No." which is indicated by the combination of the row and the column, e.g. A08 is the well at row A, column 8.

In Table 6-b below, the oligonucleotides of Table 6-a are shown reordered according to the Well No. on their synthesis plate. The order shown in Table 6-b is the actually order as synthesized on an automated synthesizer taking advantage of the preferred placement of the first nucleoside according to the above alignment criteria.

**Table 6-b:**

| **Sequences of Oligonucleotides Targeted to CD40 Order by Synthesis Well No.** | | |
|---|---|---|
| Well No. | | SEQ ID NO: |
| A01 | GACCAGGCGGCAGGACCA | 2 |
| A02 | AGGTGAGACCAGGCGGCA | 3 |
| A03 | GCAGAGGCAGACGAACCA | 5 |
| A04 | GCAAGCAGCCCCAGAGGA | 6 |
| A05 | GGTCAGCAAGCAGCCCCA | 7 |
| A06 | GACAGCGGTCAGCAAGCA | 8 |
| A07 | GATGGACAGCGGTCAGCA | 9 |
| A08 | GGTGGTTCTGGATGGACA | 11 |
| A09 | GCAGTGGGTGGTTCTGGA | 13 |
| A10 | CACAAAGAACAGCACTGA | 14 |
| A11 | CTGGCACAAAGAACAGCA | 15 |
| A12 | TCCTGGCTGGCACAAAGA | 16 |
| B01 | CTGTCCTGGCTGGCACAA | 17 |
| B02 | ACTCTGTGCAGTCACTCA | 21 |
| B03 | TTCACCGCAAGGAAGGCA | 25 |
| B04 | CTCTGTTCCAGGTGTCTA | 26 |
| B05 | GTGCCAGCCTTCTTCACA | 37 |
| B06 | TGCAGGACACAGCTCTCA | 40 |
| B07 | AATCTGCTTGACCCCAAA | 43 |
| B08 | GTATCAGAAACCCCTGTA | 45 |
| B09 | GACTGGGCAGGGCTCGCA | 49 |
| B10 | CATTGGAGAAGAAGCCGA | 50 |
| B11 | TCGAAAGCAGATGACACA | 53 |
| B12 | CAGGTCTTTGGTCTCACA | 57 |
| C01 | TTTTGATAAAGACCAGCA | 68 |
| C02 | GATCGTCGGGAAAATTGA | 72 |
| C03 | TGGAGCAGCAGTGTTGGA | 75 |
| C04 | CGGTTGGCATCCATGTAA | 78 |
| C05 | CTGTCTCTCCTGCACTGA | 80 |
| C06 | TCTGGATGGACAGCGGTC | 10 |
| C07 | CTGGTGGCAGTGTGTCTC | 27 |
| C08 | GGTGCCCTTCTGCTGGAC | 31 |
| C09 | ACAGGTGCAGATGGTGTC | 35 |
| C10 | GAAACCCCTGTAGCAATC | 44 |
| C11 | TTGGTCTCACAGCTTGTC | 56 |
| C12 | CTGTTGCACAACCAGGTC | 58 |
| D01 | GTCTTGTTTGTGCCTGCC | 60 |
| D02 | CCACAGACAACATCAGTC | 61 |
| D03 | CTGGGGACCACAGACAAC | 62 |
| D04 | TCAGCCGATCCTGGGGAC | 63 |
| D05 | GTCGGGAAAATTGATCTC | 71 |
| D06 | GGAGCCAGGAAGATCGTC | 73 |
| D07 | TGGCATCCATGTAAAGTC | 77 |
| D08 | AACTGCCTGTTTGCCCAC | 82 |
| D09 | ACTGACTGGGCATAGCTC | 84 |
| D10 | GTGGGTGGTTCTGGATGG | 12 |
| D11 | GAAGGCATTCCGTTTCAG | 24 |
| D12 | GTGTCTGTTTCTGAGGTG | 33 |
| E01 | TGGTGTCTGTTTCTGAGG | 34 |
| E02 | GGACACAGCTCTCACAGG | 39 |
| E03 | GAGCGGTGCAGGACACAG | 41 |
| E04 | AAGCCGGGCGAGCATGAG | 42 |
| E05 | GCTCGCAGATGGTATCAG | 46 |
| E06 | GATGACACATTGGAGAAG | 51 |
| E07 | GCAGATGACACATTGGAG | 52 |
| E08 | GTTTGTGCCTGCCTGTTG | 59 |
| E09 | CACCACCAGGGCTCTCAG | 64 |
| E10 | ACCAGCACCAAGAGGATG | 67 |
| E11 | TATTGGTTGGCTTCTTGG | 69 |
| E12 | GGGTTCCTGCTTGGGGTG | 70 |
| F01 | GTAAAGTCTCCTGCACTG | 76 |
| F02 | CTCTTTGCCATCCTCCTG | 79 |
| F03 | CTTCTGCCTGCACCCCTG | 83 |
| F04 | CCAGGCGGCAGGACCACT | 1 |
| F05 | CAGAGGCAGACGAACCAT | 4 |
| F06 | CTCACCAGTTTCTGTCCT | 18 |
| F07 | TCACTCACCAGTTTCTGT | 19 |
| F08 | GTGCAGTCACTCACCAGT | 20 |
| F09 | CAGTGAACTCTGTGCAGT | 22 |
| F10 | ATTCCGTTTCAGTGAACT | 23 |
| F11 | TGGGGTCGCAGTATTTGT | 28 |
| F12 | GGTTGGGGTCGCAGTATT | 29 |
| G01 | CTAGGTTGGGGTCGCAGT | 30 |
| G02 | CTGAGGTGCCCTTCTGCT | 32 |
| G03 | TTCACAGGTGCAGATGGT | 36 |
| G04 | TACAGTGCCAGCCTTCTT | 38 |
| G05 | GCAGGGCTCGCAGATGGT | 47 |
| G06 | TGGGCAGGGCTCGCAGAT | 48 |
| G07 | GTCCAAGGGTGACATTTT | 54 |
| G08 | CACAGCTTGTCCAAGGGT | 55 |
| G09 | GGGATCACCACCAGGGCT | 65 |
| G10 | GAGGATGGCAAACAGGAT | 66 |
| G11 | TGGAGCCAGGAAGATCGT | 74 |
| G12 | GGTGCAGCCTCACTGTCT | 81 |

### EXAMPLE 9: Oligonucleotide Analysis

### A. Oligonucleotide Analysis - 96 Well Plate Format

The concentration of oligonucleotide in each well was assessed by dilution of samples and UV absorption spectroscopy. The full-length integrity of the individual products was evaluated by capillary electrophoresis (CE) in either the 96 well format (Beckman MDQ) or, for individually prepared samples, on a commercial CE apparatus (e.g., Beckman 5000, ABI 270). Base and backbone composition was confirmed by mass analysis of the compounds utilizing electrospray-mass spectroscopy. All assay test plates were diluted from the master plate using single and multi-channel robotic pipettors.

### B. Alternative Oligonucleotide Analysis

After cleavage from the controlled pore glass support (Applied Biosystems) and deblocking in concentrated ammonium hydroxide at 55°C for 18 hours, the oligonucleotides or oligonucleosides are purified by precipitation twice out of 0.5 M NaCl with 2.5 volumes ethanol. Synthesized oligonucleotides are analyzed by polyacrylamide gel electrophoresis on denaturing gels. Oligonucleotide purity is checked by ³¹P nuclear magnetic resonance spectroscopy, and/or by HPLC, as described by Chiang *et al., J. Biol. Chem*. 1991, *266*, 18162.

### EXAMPLE 10: Automated Assay of CD40 Oligonucleotide Activity

### A. Poly(A)+ mRNA isolation.

Poly(A)+ mRNA was isolated according to Miura *et al.* (*Clin. Chem*., 1996, *42*, 1758). Briefly, for cells grown on 96-well plates, growth medium was removed from the cells and each well was washed with 200 µl cold PBS. 60 µl lysis buffer (10 mM Tris-HCl, pH 7.6, 1 mM EDTA, 0.5 M NaCl, 0.5% NP-40, 20 mM vanadyl-ribonucleoside complex) was added to each well, the plate was gently agitated and then incubated at room temperature for five minutes. 55 µl of lysate was transferred to Oligo d(T) coated 96 well plates (AGCT Inc., Irvine, CA). Plates were incubated for 60 minutes at room temperature, washed 3 times with 200 ml of wash buffer (10 mM Tris-HCl pH 7.6, 1 mM EDTA, 0.3 M NaCl). After the final wash, the plate was blotted on paper towels to remove excess wash buffer and then air-dried for 5 minutes. 60 ml of elution buffer (5 mM Tris-HCl pH 7.6), preheated to 70°C was added to each well, the plate was incubated on a 90°C plate for 5 minutes, and the eluate then transferred to a fresh 96-well plate. Cells grown on 100 mm or other standard plates may be treated similarly, using appropriate volumes of all solutions.

### B. Total RNA isolation

Total mRNA was isolated using an RNEASY 96™ kit and buffers purchased from Qiagen Inc. (Valencia CA) following the manufacturer's recommended procedures. Briefly, for cells grown on 96-well plates, growth medium was removed from the cells and each well was washed with 200 mL cold PBS. 100 mL Buffer RLT was added to each well and the plate vigorously agitated for 20 seconds. 100 mL of 70% ethanol was then added to each well and the contents mixed by pipetting three times up and down. The samples were then transferred to the RNEASY 96^{™} well plate attached to a QIAVAC^{™} manifold fitted with a waste collection tray and attached to a vacuum source. Vacuum was applied for 15 seconds. 1 mL of Buffer RW1 was added to each well of the RNEASY 96^{™} plate and the vacuum again applied for 15 seconds. 1 mL of Buffer RPE was then added to each well of the RNEASY 96^{™} plate and the vacuum applied for a period of 15 seconds. The Buffer RPE wash was then repeated and the vacuum was applied for an additional 10 minutes. The plate was then removed from the QIAVAC^{™} manifold and blotted dry on paper towels. The plate was then re-attached to the QIAVAC^{™} manifold fitted with a collection tube rack containing 1.2 mL collection tubes. RNA was then eluted by pipetting 60 mL water into each well, incubating 1 minute, and then applying the vacuum for 30 seconds. The elution step was repeated with an additional 60 mL water.

### C. RT-PCR Analysis of CD40 mRNA Levels

Quantitation of CD40 mRNA levels was determined by reverse transcriptase polymerase chain reaction (RT-PCR) using the ABI PRISM^{™}_7700 Sequence Detection System (PE-Applied Biosystems, Foster City, CA) according to manufacturer's instructions. This is a closed-tube, non-gel-based, fluorescence detection system which allows high-throughput quantitation of polymerase chain reaction (PCR) products in real-time.

As opposed to standard PCR, in which amplification products are quantitated after the PCR is completed, products in RT-PCR are quantitated as they accumulate. This is accomplished by including in the PCR reaction an oligonucleotide probe that anneals specifically between the forward and reverse PCR primers, and contains two fluorescent dyes. A reporter dye (e.g., JOE or FAM, PE-Applied Biosystems, Foster City, CA) is attached to the 5' end of the probe and a quencher dye (e.g., TAMRA, PE-Applied Biosystems, Foster City, CA) is attached to the 3' end of the probe. When the probe and dyes are intact, reporter dye emission is quenched by the proximity of the 3' quencher dye. During amplification, annealing of the probe to the target sequence creates a substrate that can be cleaved by the 5'-exonuclease activity of Taq polymerase. During the extension phase of the PCR amplification cycle, cleavage of the probe by Taq polymerase releases the reporter dye from the remainder of the probe (and hence from the quencher moiety) and a sequence-specific fluorescent signal is generated.

With each cycle, additional reporter dye molecules are cleaved from their respective probes, and the fluorescence intensity is monitored at regular (six-second) intervals by laser optics built into the ABI PRISM^{™} 7700 Sequence Detection System. In each assay, a series of parallel reactions containing serial dilutions of mRNA from untreated control samples generates a standard curve that is used to quantitate the percent inhibition after antisense oligonucleotide treatment of test samples.

RT-PCR reagents were obtained from PE-Applied Biosystems, Foster City, CA. RT-PCR reactions were carried out by adding 25 ml PCR cocktail (1x TAQMAN^{™} buffer A, 5.5 mM MgCl₂, 300 mM each of dATP, dCTP and dGTP, 600 mM ofdUTP, 100 nM each of forward primer, reverse primer, and probe, 20 U RNAse inhibitor, 1.25 units AMPLITAQ GOLD^{™}, and 12.5 U MuLV reverse transcriptase) to 96 well plates containing 25 ml poly(A) mRNA solution. The RT reaction was carried out by incubation for 30 minutes at 48°C. following a 10 minute incubation at 95 °C to activate the AMPLITAQ GOLD^{™}, 40 cycles of a two-step PCR protocol were carried out: 95 °C for 15 seconds (denaturation) followed by 60°C for 1.5 minutes (annealing/extension).

For CD40, the PCR primers were:
forward: 5' CAGAGTTCACTGAAACGGAATGC 3' (SEQ ID NO:87)
reverse: 5' GGTGGCAGTGTGTCTCTCTGTTC 3' (SEQ ID NO:88), and
PCR probe: 5' *FAM*-TTCCTTGCGGTGAAAGCGAATTCCT-*TAMRA* 3' (SEQ ID NO:89) where *FAM* (PE-Applied Biosystems, Foster City, CA) is the fluorescent reporter dye and *TAMRA* (PE-Applied Biosystems, Foster City, CA) is the quencher dye.

For GAPDH, the PCR primers were:
forward: 5' GAAGGTGAAGGTCGGAGTC 3' (SEQ ID NO:90)
reverse: 5' GAAGATGGTGATGGGATTTC 3' (SEQ ID NO:91), and
PCR probe: 5' *JOE*-CAAGCTTCCCGTTCTCAGCC-*TAMRA* 3' (SEQ ID NO:92)
where *JOE* (PE-Applied Biosystems, Foster City, CA) is the fluorescent reporter dye and *TAMRA* (PE-Applied Biosystems, Foster City, CA) is the quencher dye.

### EXAMPLE 11: Inhibition of CD40 Expression by Phosphorothioate Oligodeoxynucleotides

In accordance with the present invention, a series of oligonucleotides complementary to mRNA were designed to target different regions of the human CD40 mRNA, using published sequences (GenBank accession number X60592 SEQ ID NO: 85). The oligonucleotides are shown in Table 7. Target sites are indicated by the beginning nucleotide numbers, as given in the sequence source reference (X60592), to which the oligonucleotide binds. All compounds in Table 7 are oligodeoxynucleotides with phosphorothioate backbones (internucleoside linkages) throughout. Data are averages from three experiments.

**Table 7:**

| **Inhibition of CD40 mRNA Levels by Phosphorothioate Oligodeoxynucleotides** | | | | |
|---|---|---|---|---|
| **ISIS#** | **TARGET SITE** | **SEQUENCE** | **% INHIB.** | **SEQ ID NO.** |
| 18623 | 18 | CCAGGCGGCAGGACCACT | 30.71 | 1 |
| 18624 | 20 | GACCAGGCGGCAGGACCA | 28.09 | 2 |
| 18625 | 26 | AGGTGAGACCAGGCGGCA | 21.89 | 3 |
| 18626 | 48 | CAGAGGCAGACGAACCAT | 0.00 | 4 |
| 18627 | 49 | GCAGAGGCAGACGAACCA | 0.00 | 5 |
| 18628 | 73 | GCAAGCAGCCCCAGAGGA | 0.00 | 6 |
| 18629 | 78 | GGTCAGCAAGCAGCCCCA | 29.96 | 7 |
| 18630 | 84 | GACAGCGGTCAGCAAGCA | 0.00 | 8 |
| 18631 | 88 | GATGGACAGCGGTCAGCA | 0.00 | 9 |
| 18632 | 92 | TCTGGATGGACAGCGGTC | 0.00 | 10 |
| 18633 | 98 | GGTGGTTCTGGATGGACA | 0.00 | 11 |
| 18634 | 101 | GTGGGTGGTTCTGGATGG | 0.00 | 12 |
| 18635 | 104 | GCAGTGGGTGGTTCTGGA | 0.00 | 13 |
| 18636 | 152 | CACAAAGAACAGCACTGA | 0.00 | 14 |
| 18637 | 156 | CTGGCACAAAGAACAGCA | 0.00 | 15 |
| 18638 | 162 | TCCTGGCTGGCACAAAGA | 0.00 | 16 |
| 18639 | 165 | CTGTCCTGGCTGGCACAA | 4.99 | 17 |
| 18640 | 176 | CTCACCAGTTTCTGTCCT | 0.00 | 18 |
| 18641 | 179 | TCACTCACCAGTTTCTGT | 0.00 | 19 |
| 18642 | 185 | GTGCAGTCACTCACCAGT | 0.00 | 20 |
| 18643 | 190 | ACTCTGTGCAGTCACTCA | 0.00 | 21 |
| 18644 | 196 | CAGTGAACTCTGTGCAGT | 5.30 | 22 |
| 18645 | 205 | ATTCCGTTTCAGTGAACT | 0.00 | 23 |
| 18646 | 211 | GAAGGCATTCCGTTTCAG | 9.00 | 24 |
| 18647 | 222 | TTCACCGCAAGGAAGGCA | 0.00 | 25 |
| 18648 | 250 | CTCTGTTCCAGGTGTCTA | 0.00 | 26 |
| 18649 | 267 | CTGGTGGCAGTGTGTCTC | 0.00 | 27 |
| 18650 | 286 | TGGGGTCGCAGTATTTGT | 0.00 | 28 |
| 18651 | 289 | GGTTGGGGTCGCAGTATT | 0.00 | 29 |
| 18652 | 292 | CTAGGTTGGGGTCGCAGT | 0.00 | 30 |
| 18653 | 318 | GGTGCCCTTCTGCTGGAC | 19.67 | 31 |
| 18654 | 322 | CTGAGGTGCCCTTCTGCT | 15.63 | 32 |
| 18655 | 332 | GTGTCTGTTTCTGAGGTG | 0.00 | 33 |
| 18656 | 334 | TGGTGTCTGTTTCTGAGG | 0.00 | 34 |
| 18657 | 345 | ACAGGTGCAGATGGTGTC | 0.00 | 35 |
| 18658 | 348 | TTCACAGGTGCAGATGGT | 0.00 | 36 |
| 18659 | 360 | GTGCCAGCCTTCTTCACA | 5.67 | 37 |
| 18660 | 364 | TACAGTGCCAGCCTTCTT | 7.80 | 38 |
| 18661 | 391 | GGACACAGCTCTCACAGG | 0.00 | 39 |
| 18662 | 395 | TGCAGGACACAGCTCTCA | 0.00 | 40 |
| 18663 | 401 | GAGCGGTGCAGGACACAG | 0.00 | 41 |
| 18664 | 416 | AAGCCGGGCGAGCATGAG | 0.00 | 42 |
| 18665 | 432 | AATCTGCTTGACCCCAAA | 5.59 | 43 |
| 18666 | 446 | GAAACCCCTGTAGCAATC | 0.10 | 44 |
| 18667 | 452 | GTATCAGAAACCCCTGTA | 0.00 | 45 |
| 18668 | 463 | GCTCGCAGATGGTATCAG | 0.00 | 46 |
| 18669 | 468 | GCAGGGCTCGCAGATGGT | 34.05 | 47 |
| 18670 | 471 | TGGGCAGGGCTCGCAGAT | 0.00 | 48 |
| 18671 | 474 | GACTGGGCAGGGCTCGCA | 2.71 | 49 |
| 18672 | 490 | CATTGGAGAAGAAGCCGA | 0.00 | 50 |
| 18673 | 497 | GATGACACATTGGAGAAG | 0.00 | 51 |
| 18674 | 500 | GCAGATGACACATTGGAG | 0.00 | 52 |
| 18675 | 506 | TCGAAAGCAGATGACACA | 0.00 | 53 |
| 18676 | 524 | GTCCAAGGGTGACATTTT | 8.01 | 54 |
| 18677 | 532 | CACAGCTTGTCCAAGGGT | 0.00 | 55 |
| 18678 | 539 | TTGGTCTCACAGCTTGTC | 0.00 | 56 |
| 18679 | 546 | CAGGTCTTTGGTCTCACA | 6.98 | 57 |
| 18680 | 558 | CTGTTGCACAACCAGGTC | 18.76 | 58 |
| 18681 | 570 | GTTTGTGCCTGCCTGTTG | 2.43 | 59 |
| 18682 | 575 | GTCTTGTTTGTGCCTGCC | 0.00 | 60 |
| 18683 | 590 | CCACAGACAACATCAGTC | 0.00 | 61 |
| 18684 | 597 | CTGGGGACCACAGACAAC | 0.00 | 62 |
| 18685 | 607 | TCAGCCGATCCTGGGGAC | 0.00 | 63 |
| 18686 | 621 | CACCACCAGGGCTCTCAG | 23.31 | 64 |
| 18687 | 626 | GGGATCACCACCAGGGCT | 0.00 | 65 |
| 18688 | 657 | GAGGATGGCAAACAGGAT | 0.00 | 66 |
| 18689 | 668 | ACCAGCACCAAGAGGATG | 0.00 | 67 |
| 18690 | 679 | TTTTGATAAAGACCAGCA | 0.00 | 68 |
| 18691 | 703 | TATTGGTTGGCTTCTTGG | 0.00 | 69 |
| 18692 | 729 | GGGTTCCTGCTTGGGGTG | 0.00 | 70 |
| 18693 | 750 | GTCGGGAAAATTGATCTC | 0.00 | 71 |
| 18694 | 754 | GATCGTCGGGAAAATTGA | 0.00 | 72 |
| 18695 | 765 | GGAGCCAGGAAGATCGTC | 0.00 | 73 |
| 18696 | 766 | TGGAGCCAGGAAGATCGT | 0.00 | 74 |
| 18697 | 780 | TGGAGCAGCAGTGTTGGA | 0.00 | 75 |
| 18698 | 796 | GTAAAGTCTCCTGCACTG | 0.00 | 76 |
| 18699 | 806 | TGGCATCCATGTAAAGTC | 0.00 | 77 |
| 18700 | 810 | CGGTTGGCATCCATGTAA | 0.00 | 78 |
| 18701 | 834 | CTCTTTGCCATCCTCCTG | 4.38 | 79 |
| 18702 | 861 | CTGTCTCTCCTGCACTGA | 0.00 | 80 |
| 18703 | 873 | GGTGCAGCCTCACTGTCT | 0.00 | 81 |
| 18704 | 910 | AACTGCCTGTTTGCCCAC | 33.89 | 82 |
| 18705 | 954 | CTTCTGCCTGCACCCCTG | 0.00 | 83 |
| 18706 | 976 | ACTGACTGGGCATAGCTC | 0.00 | 84 |

As shown in Table 7, SEQ ID NOS: 1, 2, 7, 47 and 82 demonstrated at least 25% inhibition of CD40 expression and are therefore preferred compounds of the invention.

### EXAMPLE 12: Inhibition of CD40 Expression by Phosphorothioate 2'-MOE Gapmer Oligonucleotides

In accordance with the present invention, a second series of oligonucleotides complementary to mRNA were designed to target different regions of the human CD40 mRNA, using published sequence X60592. The oligonucleotides are shown in Table 8. Target sites are indicated by the beginning or initial nucleotide numbers, as given in the sequence source reference (X60592), to which the oligonucleotide binds.

All compounds in Table 8 are chimeric oligonucleotides ("gapmers") 18 nucleotides in length, composed of a central "gap" region consisting of ten 2'-deoxynucleotides, which is flanked on both sides (5' and 3' directions) by four-nucleotide "wings." The wings are composed of 2'-O-(2-methoxyethyl) (2'-MOE) nucleotides. The intersugar (backbone) linkages are phosphorothioate (P=S) throughout the oligonucleotide. Cytidine residues in the 2'-MOE wings are 5-methylcytidines.
Data are averaged from three experiments.

**Table 8:**

| **Inhibition of CD40 mRNA Levels by Chimeric Phosphorothioate Oligonucleotides** | | | | |
|---|---|---|---|---|
| **ISIS#** | **TARGET** | **SEQUENCE** | **% Inhibition** | **SEQ ID** |
| 19211 | 18 | CCAGGCGGCAGGACCACT | 75.71 | 1 |
| 19212 | 20 | GACCAGGCGGCAGGACCA | 77.23 | 2 |
| 19213 | 26 | AGGTGAGACCAGGCGGCA | 80.82 | 3 |
| 19214 | 48 | CAGAGGCAGACGAACCAT | 23.68 | 4 |
| 19215 | 49 | GCAGAGGCAGACGAACCA | 45.97 | 5 |
| 19216 | 73 | GCAAGCAGCCCCAGAGGA | 65.80 | 6 |
| 19217 | 78 | GGTCAGCAAGCAGCCCCA | 74.73 | 7 |
| 19218 | 84 | GACAGCGGTCAGCAAGCA | 67.21 | 8 |
| 19219 | 88 | GATGGACAGCGGTCAGCA | 65.14 | 9 |
| 19220 | 92 | TCTGGATGGACAGCGGTC | 78.71 | 10 |
| 19221 | 98 | GGTGGTTCTGGATGGACA | 81.33 | 11 |
| 19222 | 101 | GTGGGTGGTTCTGGATGG | 57.79 | 12 |
| 19223 | 104 | GCAGTGGGTGGTTCTGGA | 73.70 | 13 |
| 19224 | 152 | CACAAAGAACAGCACTGA | 40.25 | 14 |
| 19225 | 156 | CTGGCACAAAGAACAGCA | 60.11 | 15 |
| 19226 | 162 | TCCTGGCTGGCACAAAGA | 10.18 | 16 |
| 19227 | 165 | CTGTCCTGGCTGGCACAA | 24.37 | 17 |
| 19228 | 176 | CTCACCAGTTTCTGTCCT | 22.30 | 18 |
| 19229 | 179 | TCACTCACCAGTTTCTGT | 40.64 | 19 |
| 19230 | 185 | GTGCAGTCACTCACCAGT | 82.04 | 20 |
| 19231 | 190 | ACTCTGTGCAGTCACTCA | 37.59 | 21 |
| 19232 | 196 | CAGTGAACTCTGTGCAGT | 40.26 | 22 |
| 19233 | 205 | ATTCCGTTTCAGTGAACT | 56.03 | 23 |
| 19234 | 211 | GAAGGCATTCCGTTTCAG | 32.21 | 24 |
| 19235 | 222 | TTCACCGCAAGGAAGGCA | 61.03 | 25 |
| 19236 | 250 | CTCTGTTCCAGGTGTCTA | 62.19 | 26 |
| 19237 | 267 | CTGGTGGCAGTGTGTCTC | 70.32 | 27 |
| 19238 | 286 | TGGGGTCGCAGTATTTGT | 0.00 | 28 |
| 19239 | 289 | GGTTGGGGTCGCAGTATT | 19.40 | 29 |
| 19240 | 292 | CTAGGTTGGGGTCGCAGT | 36.32 | 30 |
| 19241 | 318 | GGTGCCCTTCTGCTGGAC | 78.91 | 31 |
| 19242 | 322 | CTGAGGTGCCCTTCTGCT | 69.84 | 32 |
| 19243 | 332 | GTGTCTGTTTCTGAGGTG | 63.32 | 33 |
| 19244 | 334 | TGGTGTCTGTTTCTGAGG | 42.83 | 34 |
| 19245 | 345 | ACAGGTGCAGATGGTGTC | 73.31 | 35 |
| 19246 | 348 | TTCACAGGTGCAGATGGT | 47.72 | 36 |
| 19247 | 360 | GTGCCAGCCTTCTTCACA | 61.32 | 37 |
| 19248 | 364 | TACAGTGCCAGCCTTCTT | 46.82 | 38 |
| 19249 | 391 | GGACACAGCTCTCACAGG | 0.00 | 39 |
| 19250 | 395 | TGCAGGACACAGCTCTCA | 52.05 | 40 |
| 19251 | 401 | GAGCGGTGCAGGACACAG | 50.15 | 41 |
| 19252 | 416 | AAGCCGGGCGAGCATGAG | 32.36 | 42 |
| 19253 | 432 | AATCTGCTTGACCCCAAA | 0.00 | 43 |
| 19254 | 446 | GAAACCCCTGTAGCAATC | 0.00 | 44 |
| 19255 | 452 | GTATCAGAAACCCCTGTA | 36.13 | 45 |
| 19256 | 463 | GCTCGCAGATGGTATCAG | 64.65 | 46 |
| 19257 | 468 | GCAGGGCTCGCAGATGGT | 74.95 | 47 |
| 19258 | 471 | TGGGCAGGGCTCGCAGAT | 0.00 | 48 |
| 19259 | 474 | GACTGGGCAGGGCTCGCA | 82.00 | 49 |
| 19260 | 490 | CATTGGAGAAGAAGCCGA | 41.31 | 50 |
| 19261 | 497 | GATGACACATTGGAGAAG | 13.81 | 51 |
| 19262 | 500 | GCAGATGACACATTGGAG | 78.48 | 52 |
| 19263 | 506 | TCGAAAGCAGATGACACA | 59.28 | 53 |
| 19264 | 524 | GTCCAAGGGTGACATTTT | 70.99 | 54 |
| 19265 | 532 | CACAGCTTGTCCAAGGGT | 0.00 | 55 |
| 19266 | 539 | TTGGTCTCACAGCTTGTC | 45.92 | 56 |
| 19267 | 546 | CAGGTCTTTGGTCTCACA | 63.95 | 57 |
| 19268 | 558 | CTGTTGCACAACCAGGTC | 82.32 | 58 |
| 19269 | 570 | GTTTGTGCCTGCCTGTTG | 70.10 | 59 |
| 19270 | 575 | GTCTTGTTTGTGCCTGCC | 68.95 | 60 |
| 19271 | 590 | CCACAGACAACATCAGTC | 11.22 | 61 |
| 19272 | 597 | CTGGGGACCACAGACAAC | 9.04 | 62 |
| 19273 | 607 | TCAGCCGATCCTGGGGAC | 0.00 | 63 |
| 19274 | 621 | CACCACCAGGGCTCTCAG | 23.08 | 64 |
| 19275 | 626 | GGGATCACCACCAGGGCT | 57.94 | 65 |
| 19276 | 657 | GAGGATGGCAAACAGGAT | 49.14 | 66 |
| 19277 | 668 | ACCAGCACCAAGAGGATG | 3.48 | 67 |
| 19278 | 679 | TTTTGATAAAGACCAGCA | 30.58 | 68 |
| 19279 | 703 | TATTGGTTGGCTTCTTGG | 49.26 | 69 |
| 19280 | 729 | GGGTTCCTGCTTGGGGTG | 13.95 | 70 |
| 19281 | 750 | GTCGGGAAAATTGATCTC | 54.78 | 71 |
| 19282 | 754 | GATCGTCGGGAAAATTGA | 0.00 | 72 |
| 19283 | 765 | GGAGCCAGGAAGATCGTC | 69.47 | 73 |
| 19284 | 766 | TGGAGCCAGGAAGATCGT | 54.48 | 74 |
| 19285 | 780 | TGGAGCAGCAGTGTTGGA | 15.17 | 75 |
| 19286 | 796 | GTAAAGTCTCCTGCACTG | 30.62 | 76 |
| 19287 | 806 | TGGCATCCATGTAAAGTC | 65.03 | 77 |
| 19288 | 810 | CGGTTGGCATCCATGTAA | 34.49 | 78 |
| 19289 | 834 | CTCTTTGCCATCCTCCTG | 41.84 | 79 |
| 19290 | 861 | CTGTCTCTCCTGCACTGA | 25.68 | 80 |
| 19291 | 873 | GGTGCAGCCTCACTGTCT | 76.27 | 81 |
| 19292 | 910 | AACTGCCTGTTTGCCCAC | 63.34 | 82 |
| 19293 | 954 | CTTCTGCCTGCACCCCTG | 0.00 | 83 |
| 19294 | 976 | ACTGACTGGGCATAGCTC | 11.55 | 84 |

As shown in Table 8, SEQ ID NOS: 1, 2, 3, 6, 7, 8, 9, 10, 11, 12, 13, 15, 20, 23, 25, 26, 27, 31, 32, 33, 35, 37, 40, 41, 46, 47, 49, 52, 53, 54, 57, 58, 59, 60, 65, 71, 73, 74, 77, 81 and 82 demonstrated at least 50% inhibition of CD40 expression and are therefore preferred compounds of the invention.

### EXAMPLE 13: Oligonucleotide-Sensitive Sites of the CD40 Target Nucleic Acid

As the data presented in the preceding two Examples shows, several sequences were present in preferred compounds of two distinct oligonucleotide chemistries. Specifically, compounds having SEQ ID NOS: 1, 2, 7, 47 and 82 are preferred in both instances. These compounds map to different regions of the CD40 transcript but nevertheless define accessible sites of the target nucleic acid.

For example, SEQ ID NOS: 1 and 2 overlap each other and both map to the 5-untranslated region (5'-UTR) of CD40. Accordingly, this region of CD40 is particularly preferred for modulation via sequence-based technologies. Similarly, SEQ ID NOS: 7 and 47 map to the open reading frame of CD40, whereas SEQ ID NO: 82 maps to the 3'-untranslated region (3'-UTR). Thus, the ORF and 3'-UTR of CD40 may be targeted by sequence-based technologies as well.

The reverse complements of the active CD40 compounds are easily determined by those skilled in the art and may be assembled to yield nucleotide sequences corresponding to accessible sites on the target nucleic acid. For example, the assembled reverse complement of SEQ ID NOS: 1 and 2 is represented below as SEQ ID NO:93:

| | |
|---|---|
| 5'- AGTGGTCCTGCCGCCTGGTC -3' | SEQ ID NO:93 |
| TCACCAGGACGGCGGACC -5' | SEQ ID NO:1 |
| ACCAGGACGGCGGACCAG -5' | SEQ ID NO:2 |

Through multiple iterations of the process of the invention, more extensive "footprints" are generated. A library of this information is compiled and may be used by those skilled in the art in a variety of sequence-based technologies to study the molecular and biological functions of CD40 and to investigate or confirm its role in various diseases and disorders.

### EXAMPLE 14: Site Selection Program

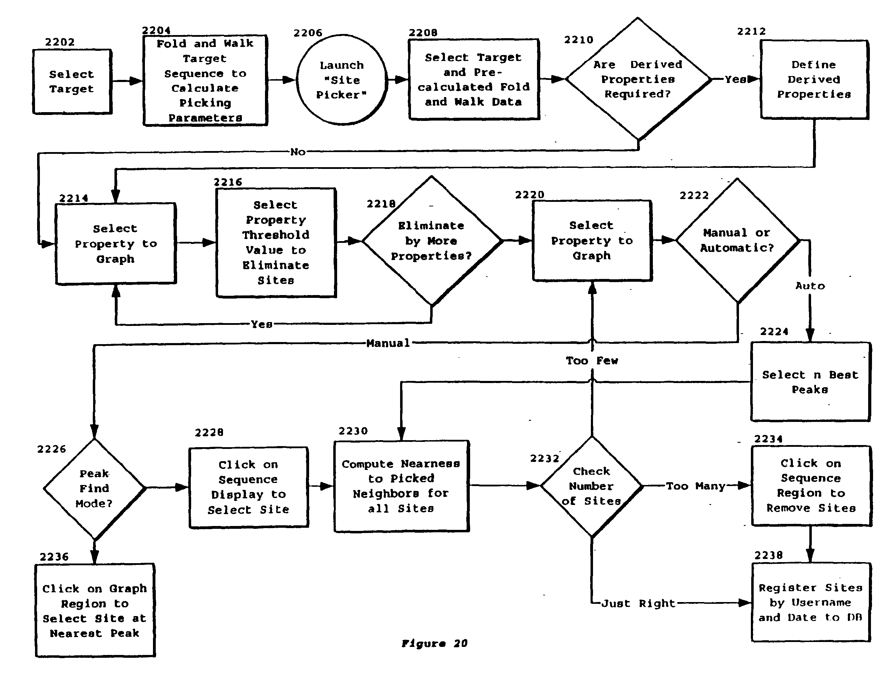

In a preferred embodiment of the invention, illustrated in Figure 20, an application is deployed which facilitates the selection process for determining the target positions of the oligos to be synthesized, or "sites." This program is written using a three-tiered object-oriented approach. All aspects of the software described, therefore, are tightly integrated with the relational database. For this reason, explicit database read and write steps are not shown. It should be assumed that each step described includes database access. The description below illustrates one way the program can be used. The actual interface allows users to skip from process to process at will, in any order.

Before running the site picking program, the target must have all relevant properties computed as described previously and indicated in process step **2204.** When the site picking program is launched at process step **2206** the user is presented with a panel showing targets which have previously been selected and had their properties calculated. The user selects one target to work with at process step **2208** and proceeds to decide if any derived properties will be needed at process step **2210.** Derived properties are calculated by performing mathematical operations on combinations of pre-calculated properties as defined by the user at process step **2212.**

The derived properties are made available as peers with all the pre-calculated properties. The user selects one of the properties to view plotted versus target position at process step **2214.** This graph is shown above a linear representation of the target. The horizontal or position axis of both the graph and target are linked and scalable by the user. The zoom range goes from showing the full target length to showing individual target bases as letters and individual property points. The user next selects a threshold value below or above which all sites will be eliminated from future consideration at process step **2216**. The user decides whether to eliminate more sites based on any other properties at process step **2218.** If they choose to eliminate more, they return to pick another property to display at process step **2214** and threshold at process step **2216.**

After eliminating sites, the user selects from the remaining list by choosing any property at process step **2220** and then choosing a manual or automatic selection technique at process step **2222.** In the automatic technique, the user decides whether they want to pick from maxima or minima and the number of maxima or minima to be selected as sites at process step **2224.** The software automatically finds and picks the points. When picking manually the user must decide if they wish to use automatic peak finding at process step **2226.** If the user selects automatic peak finding, then user must click on the graphed property with the mouse at process step **2236.** The nearest maxima or minima, depending on the modifier key held down, to the selected point will be picked as the site. Without the peak finding option, the user must pick a site at process step **2238** by clicking on its position on the linear representation of target.

Each time a site, or group of sites, is picked, a dynamic property is calculated for all possible sites (not yet eliminated) at process step **2230.** This property indicates the nearness of the site to a picked site allowing the user to pick sites in subsequent iterations based on target coverage. After new sites are picked, the user determines if the desired number of sites has been picked. If too few sites have been picked the user returns to pick more **2220.** If too many sites have been picked, the user may eliminate them by selecting and deleting them on the target display at process step **2234.** If the correct number of sites is picked, and the user is satisfied with the set of picked sites, the user registers these sites to the database along with their name, notebook number, and page number at process step **2238.** The database time stamps this registration event.

### EXAMPLE 15: Site Selection Program

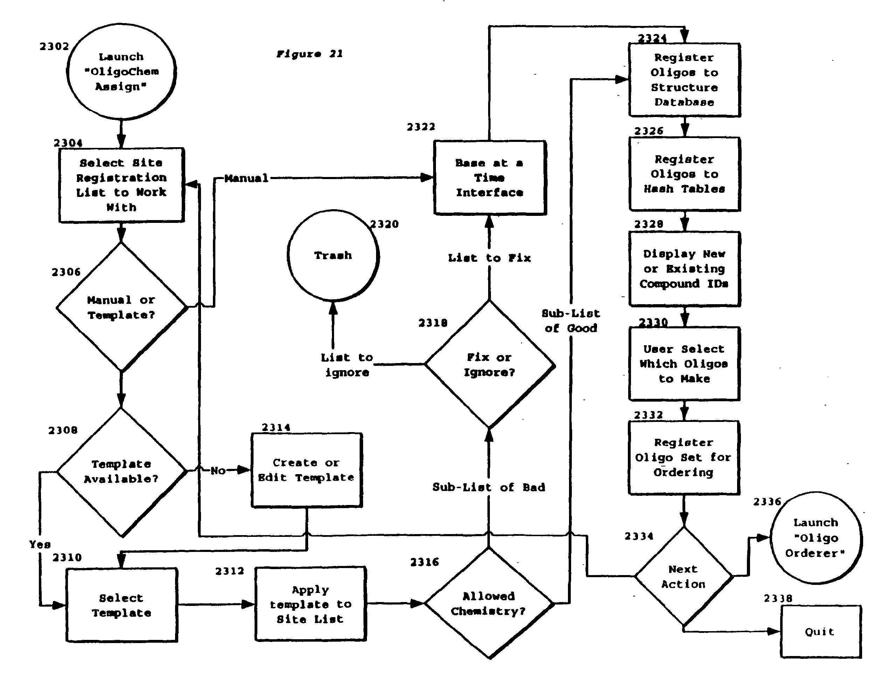

In a preferred embodiment of the invention, illustrated in Figure 21, an application is deployed which facilitates the assignment of specific chemical structure to the complement of the sequence of the sites previously picked and facilitates the registration and ordering of these now fully defined antisense compounds. This program is written using a three-tiered object-oriented approach. All aspects of the software described, therefore, are tightly integrated with the relational database. For this reason, explicit database read and write steps are not shown, it being understood that each step described also includes appropriate database read/write access.

To begin using the oligonucleotide chemistry assignment program, the user launches it at process step **2302.** The user then selects from the previously selected sets of oligonucleotides at process step **2304,** registered to the database in site picker's process step **2238.** Next, the user must decide whether to manually assign the chemistry a base at a time, or run the sites through a template at process step **2306.** If the user chooses to use a template, they must determine if a desired template is available at process step **2308.** If a template is not available with the desired chemistry modifications and the correct length, the user can define one at process step **2314.**

To define a template, the user must select the length of the oligonucleotide the template is to define. This oligonucleotide is then represented as a bar with selectable regions. The user sets the number of regions on the oligonucleotide, and the positions and lengths of these regions by dragging them back and forth on the bar. Each region is represented by a different color.

For each region, the user defines the chemistry modifications for the sugars, the linkers, and the heterocycles at each base position in the region. At least four heterocycle chemistries must be given, one for each of the four possible base types (A, G, C or T or U) in the site sequence the template will be applied to. A user interface is provided to select these chemistries which show the molecular structure of each component selected and its modification name. By pushing on a pop-up list next to each of the pictures, the user may choose from a list of structures and names, those possible to put in this place. For example, the heterocycle that represents the base type G is shown as a two dimensional structure diagram. If the user clicks on the pop-up list, a row of other possible structures and names is shown. The user drags the mouse to the desired chemistry and releases the mouse. Now the newly selected molecule is displayed as the choice for G type heterocycle modifications.

Once the user has created a template, or selected an existing one, the software applies the template at process step **2312** to each of the complements of the sites in the list. When the templates are applied, it is possible that chemistries will be defined which are impossible to make with the chemical precursors presently used on the automatic synthesizer. To check this, a database is maintained of all precursors previously designed, and their availability for automated synthesis. When the templates are applied, the resulting molecules are tested at process step **2316** against this database to see if they are readily synthesized.

If a molecule is not readily synthesized, it is added to a list that the user inspects. At process step **2318,** the user decides whether to modify the chemistry to make it compatible with the currently recognized list of available chemistries or to ignore it. To modify a chemistry, the user must use the base at a time interface at process step **2322.** The user can also choose to go directly to this step, bypassing templates all together at process step **2306.**

The base at a time interface at process step **2322** is very similar to the template editor at process step **2314** except that instead of specifying chemistries for regions, they are defined one base at a time. This interface also differs in that it dynamically checks to see if the design is readily synthesized as the user makes selections. In other words, each choice made limits the choices the software makes available on the pop-up selection lists. To accommodate this function, an additional choice is made available on each pop-up of "not defined." For example, this allows the user to inhibit linker choice from restricting the sugar choices by first setting the linker to "not defined." The user would then pick the sugar, and then pick from the remaining linker choices available.

Once all of the sites on the list are assigned chemistries or dropped, they are registered at process step **2324** to a commercial chemical structure database. Registering to this database makes sure the structure is unique, assigns it a new identifier if it is unique, and allows future structure and substructure searching by creating various hash-tables. The compound definition is also stored at process step **2326** to various hash tables referred to as chemistry/position tables. These allow antisense compound searching and categorization based on oligonucleotide chemistry modification sequences and equivalent base sequences.

The results of the registration are displayed at process step **2328** with the new IDs if they are new compounds and with the old IDs if they have been previously registered. The user next selects which of the compounds processed they wish to order for synthesis at process step **2330** and registers an order list at process step **2332** by including scientist name, notebook number and page number. The database time-stamps this entry. The user may then choose at process step **2334,** to quit the program at process step **2338,** go back to the beginning and choose a new site list to work with process step **2304,** or start the oligonucleotide ordering interface at process step **2336.**

### EXAMPLE 16: Gene Walk to Optimize Oligonucleotide Sequence

A gene walk is executed using a CD40 antisense oligonucleotide having SEQ ID NO:15 (5'-CTGGCACAAAGAACAGCA-3'). In effecting this gene walk, the following parameters are used:

| **Gene Walk Parameter** | **Entered value** |
|---|---|
| Oligonucleotide Sequence ID: | 15 |
| Name of Gene Target: | CD40 |
| Scope of Gene Walk: | 20 |
| Sequence Shift Increment: | 1 |

Entering these values and effecting the gene walk centered on SEQ ID NO: 15 automatically generates the following new oligonucleotides:

**Table 9:**

| **Oligonucleotide Generated By Gene Walk** | |
|---|---|
| **SEQ ID** | **Sequence** |
| 94 | GAACAGCACTGACTGTTT |
| 95 | AGAACAGCACTGACTGTT |
| 96 | AAGAACAGCACTGACTGT |
| 97 | AAAGAACAGCACTGACTG |
| 98 | CAAAGAACAGCACTGACT |
| 99 | ACAAAGAACAGCACTGAC |
| 100 | CACAAAGAACAGCACTGA |
| 101 | GCACAAAGAACAGCACTG |
| 102 | GGCACAAAGAACAGCACT |
| 103 | TGGCACAAAGAACAGCAC |
| 15 | CTGGCACAAAGAACAGCA |
| 104 | GCTGGCACAAAGAACAGC |
| 105 | GGCTGGCACAAAGAACAG |
| 106 | TGGCTGGCACAAAGAACA |
| 107 | CTGGCTGGCACAAAGAAC |
| 108 | CCTGGCTGGCACAAAGAA |
| 109 | TCCTGGCTGGCACAAAGA |
| 110 | GTCCTGGCTGGCACAAAG |
| 111 | TGTCCTGGCTGGCACAAA |
| 112 | CTGTCCTGGCTGGCACAA |
| 113 | TCTGTCCTGGCTGCCACA |

The list shown above contains 20 new oligonucleotide sequences directed against the CD40 nucleic acid sequence. They are ordered by the position along the CD40 sequence at which the 5' terminus of each oligonucleotide hybridizes. Thus, the first ten oligonucleotides are single-base frame shift sequences directed against the CD40 sequence upstream of compound SEQ ID NO: 15 and the latter ten are single-base frame shift sequences directed against the CD40 sequence downstream of compound SEQ ID NO: 15.

### EXAMPLE 17: Automated Assay of RhoC Oligonucleotide Activity

RhoC, a member of the Rho subfamily of small GTPases, is a protein that has been shown to be involved in a diverse set of signaling pathways including the ultimate regulation of the dynamic organization of the cytoskeleton.

Oligonucleotides were designed as described in Example 2, synthesized as described in Examples 3 through 8, analyzed as described in Examples 9 and assayed as described in Example 10 except for target specific primer and probes. RhoC probes and primers were designed to hybridize to the human RhoC sequence, using published sequence information (GenBank accession number L25081 SEQ ID NO:114; SEQ ID NO:115 is protein).

For RhoC the PCR primers were:
forward primer: TGATGTCATCCTCATGTGCTTCT (SEQ ID NO: 116)
reverse primer: CCAGGATGATGGGCACGTT (SEQ ID NO: 117) and the PCR probe
was: FAM-CGACAGCCCTGACAGCCTGGAAA-TAMRA (SEQ ID NO:118) where FAM (PE-Applied Biosystems, Foster City, CA) is the fluorescent reporter dye) and TAMRA (PE-Applied Biosystems, Foster City, CA) is the quencher dye.

### EXAMPLE 18: Antisense inhibition of RhoC expression- phosphorothioate oligodeoxynucleotides

In accordance with the present invention, a series of oligonucleotides were designed to target different regions of the human RhoC RNA, using published sequences (GenBank accession number L25081 SEQ ID NO: 113). The oligonucleotides are shown in Table 9. Target sites are indicated by nucleotide numbers, as given in the sequence source reference (Genbank accession no. L25081), to which the oligonucleotide binds. All compounds in Table 10 are oligodeoxynucleotides with phosphorothioate backbones (internucleoside linkages) throughout. The compounds were analyzed for effect on RhoC mRNA levels by quantitative real-time PCR as described in other examples herein. Data are averages from three experiments. If present, "N.D." indicates "no data".

**Table 10**

| **Inhibition of RhoC mRNA levels by phosphorothioate oligodeoxynucleotides** | | | | | |
|---|---|---|---|---|---|
| **ISIS#** | **REGION** | **TARGET SITE** | **SEQUENCE** | **% Inhibition** | **SEQ ID NO.** |
| 25304 | 5' UTR | 4 | gagctgagatgaagtcaa | 29 | 119 |
| 25305 | 5' UTR | 44 | gctgaagttcccaggctg | 25 | 120 |
| 25306 | 5' UTR | 47 | ccggctgaagttcccagg | 42 | 121 |
| 25307 | Coding | 104 | ggcaccatccccaacgat | 81 | 122 |
| 25308 | Coding | 105 | aggcaccatccccaacga | 81 | 123 |
| 25309 | Coding | 111 | tcccacaggcaccatccc | 70 | 124 |
| 25310 | Coding | 117 | aggtcttcccacaggcac | 40 | 125 |
| 25311 | Coding | 127 | atgaggaggcaggtcttc | 41 | 126 |
| 25312 | Coding | 139 | ttgctgaagacgatgagg | 23 | 127 |
| 25313 | Coding | 178 | tcaaagacagtagggacg | . 0 | 128 |
| 25314 | Coding | 181 | ttctcaaagacagtaggg | 2 | 129 |
| 25315 | Coding | 183 | agttctcaaagacagtag | 38 | 130 |
| 25316 | Coding | 342 | tgttttccaggctgtcag | 59 | 131 |
| 25317 | Coding | 433 | tcgtcttgcctcaggtcc | 79 | 132 |
| 25318 | Coding | 439 | gtgtgctcgtcttgcctc | 67 | 133 |
| 25319 | Coding | 445 | ctcctggtgtgctcgtct | 67 | 134 |
| 25320 | Coding | 483 | cagaccgaacgggctcct | 65 | 135 |
| 25321 | Coding | 488 | ttcctcagaccgaacggg | 57 | 136 |
| 25322 | Coding | 534 | actcaaggtagccaaagg | 33 | 137 |
| 25323 | Coding | 566 | ctcccgcactccctcctt | 91 | 138 |
| 25324 | Coding | 575 | ctcaaacacctcccgcac | 34 | 139 |
| 25325 | Coding | 581 | ggccatctcaaacacctc | 64 | 140 |
| 25326 | Coding | 614 | cttgttcttgcggacctg | 72 | 141 |
| 25327 | Coding | 625 | cccctccgacgcttgttc | 66 | 142 |
| 25328 | 3' UTR | 737 | gtatggagccctcaggag | 60 | 143 |
| 25329 | 3' UTR | 746 | gagccttcagtatggagc | 63 | 144 |
| 25330 | 3' UTR | 753 | gaaaatggagccttcagt | 24 | 145 |
| 25331 | 3' UTR | 759 | ggaactgaaaatggagcc | 2 | 146 |
| 25332 | 3' UTR | 763 | ggagggaactgaaaatgg | 13 | 147 |
| 25333 | 3' UTR | 766 | gcaggagggaactgaaaa | 27 | 148 |
| 25334 | 3' UTR | 851 | agggcagggcataggcgt | 31 | 149 |
| 25335 | 3' UTR | 854 | ggaagggcagggcatagg | 21 | 150 |
| 25336 | 3' UTR | 859 | catgaggaagggcagggc | 0 | 151 |
| 25337 | 3' UTR | 920 | taaagtgctggtgtgtga | 39 | 152 |
| 25338 | 3' UTR | 939 | cctgtgagccagaagtgt | 69 | 153 |
| 25339 | 3' UTR | 941 | ttcctgtgagccagaagt | 69 | 154 |
| 25340 | 3' UTR | 945 | cactttcctgtgagccag | 82 | 155 |
| 25341 | 3' UTR | 948 | agacactttcctgtgagc | 69 | 156 |
| 25342 | 3' UTR | 966 | actctgggtccctactgc | 20 | 157 |
| 25343 | 3' UTR | 992 | tgcagaaacaactccagg | 0 | 158 |

### Example 19: Antisense inhibition of RhoC expression- phosphorothioate 2'-MOE gapmer oligonucleotides

In accordance with the present invention, a second series of oligonucleotides targeted to human RhoC were synthesized. The oligonucleotide sequences are shown in Table 11. Target sites are indicated by nucleotide numbers, as given in the sequence source reference (Genbank accession No. L25081), to which the oligonucleotide binds.

All compounds in Table 2 are chimeric oligonucleotides ("gapmers") 18 nucleotides in length, composed of a central "gap" region consisting of ten 2'-deoxynucleotides, which is flanked on both sides (5' and 3' directions) by four-nucleotide "wings". The wings are composed of 2'-methoxyethyl (2'-MOE) nucleotides. The internucleoside (backbone) linkages are phosphorothioate (P=S) throughout the oligonucleotide. Cytidine residues in the 2'-MOE wings are 5-methylcytidines.

Data were obtained by real-time quantitative PCR as described in other examples herein and are averaged from three experiments. If present, "N.D." indicates "no data".

**Table 11**

| **Inhibition of RhoC mRNA levels by chimeric phosphorothioate oligonucleotides having 2'-MOE wings and a deoxy gap** | | | | | |
|---|---|---|---|---|---|
| **ISIS#** | **REGION** | **TARGET SITE** | **SEQUENCE** | **% Inhibition** | **SEQ ID NO.** |
| 25344 | 5' UTR | 4 | gagctgagatgaagtcaa | 0 | 119 |
| 25345 | 5' UTR | 44 | gctgaagttcccaggctg | 35 | 120 |
| 25346 | 5' UTR | 47 | ccggctgaagttcccagg | 53 | 121 |
| 25347 | Coding | 104 | ggcaccatccccaacgat | 50 | 122 |
| 25348 | Coding | 105 | aggcaccatccccaacga | 56 | 123 |
| 25349 | Coding | 111 | tcccacaggcaccatccc | 4 | 124 |
| 25350 | Coding | 117 | aggtcttcccacaggcac | 11 | 125 |
| 25351 | Coding | 127 | atgaggaggcaggtcttc | 6 | 126 |
| 25352 | Coding | 139 | ttgctgaagacgatgagg | 15 | 127 |
| 25353 | Coding | 178 | tcaaagacagtagggacg | 32 | 128 |
| 25354 | Coding | 181 | ttctcaaagacagtaggg | 7 | 129 |
| 25355 | Coding | 183 | agttctcaaagacagtag | 39 | 130 |
| 25356 | Coding | 342 | tgttttccaggctgtcag | 59 | 131 |
| 25357 | Coding | 433 | tcgtcttgcctcaggtcc | 48 | 132 |
| 25358 | Coding | 439 | gtgtgctcgtcttgcctc | 36 | 133 |
| 25359 | Coding | 445 | ctcctggtgtgctcgtct | 61 | 134 |
| 25360 | Coding | 483 | cagaccgaacgggctcct | 50 | 135 |
| 25361 | Coding | 488 | ttcctcagaccgaacggg | 14 | 136 |
| 25362 | Coding | 534 | actcaaggtagccaaagg | 32 | 137 |
| 25363 | Coding | 566 | ctcccgcactccctcctt | 21 | 138 |
| 25364 | Coding | 575 | ctcaaacacctcccgcac | 9 | 139 |
| 25365 | Coding | 581 | ggccatctcaaacacctc | 66 | 140 |
| 25366 | Coding | 614 | cttgttcttgcggacctg | 61 | 141 |
| 25367 | Coding | 625 | cccctccgacgcttgttc | 0 | 142 |
| 25368 | 3' UTR | 737 | gtatggagccctcaggag | 28 | 143 |
| 25369 | 3' UTR | 746 | gagccttcagtatggagc | 32 | 144 |
| 25370 | 3' UTR | 753 | gaaaatggagccttcagt | 0 | 145 |
| 25371 | 3' UTR | 759 | ggaactgaaaatggagcc | 40 | 146 |
| 25372 | 3' UTR | 763 | ggagggaactgaaaatgg | 45 | 147 |
| 25373 | 3' UTR | 766 | gcaggagggaactgaaaa | 35 | 148 |
| 25374 | 3' UTR | 851 | agggcagggcataggcgt | 5 | 149 |
| 25375 | 3' UTR | 854 | ggaagggcagggcatagg | 0 | 150 |
| 25376 | 3' UTR | 859 | catgaggaagggcagggc | 0 | 151 |
| 25377 | 3' UTR | 920 | taaagtgctggtgtgtga | 20 | 152 |
| 25378 | 3' UTR | 939 | cctgtgagccagaagtgt | 67 | 153 |
| 25379 | 3' UTR | 941 | ttcctgtgagccagaagt | 61 | 154 |
| 25380 | 3' UTR | 945 | cactttcctgtgagccag | 80 | 155 |
| 25381 | 3' UTR | 948 | agacactttcctgtgagc | 0 | 156 |
| 25382 | 3' UTR | 966 | actctgggtccctactgc | 0 | 157 |
| 25383 | 3' UTR | 992 | tgcagaaacaactccagg | 0 | 158 |

### EXAMPLE 20: Automated Assay of Cellular Inhibitor of Apoptosis-2 Expression Oligonucleotide Activity

Cellular Inhibitor of Apoptosis-2 (also known as c-IAP-2, apoptosis inhibitor 2, API-2, hIAP-1, and MIHC) is a member of the inhibitor of apoptosis (IAP) family of anti-apoptotic proteins which interfere with the transmission of intracellular death signals.

Oligonucleotides were designed as described in Example 2, synthesized as described in Examples 3 through 8, analyzed as described in Examples 9 and assayed as described in Example 10 except for target specific primer and probes. Cellular Inhibitor of Apoptosis-2 probes and primers were designed to hybridize to the human Cellular Inhibitor of Apoptosis-2 sequence, using published sequence information (GenBank accession number U37546, SEQ ID NO:159; SEQ ID NO:160 is protein).

For Cellular Inhibitor of Apoptosis-2 the PCR primers were:
forward primer: GGACTCAGGTGTTGGGAATCTG (SEQ ID NO:161)
reverse primer: CAAGTACTCACACCTTGGAAACCA (SEQ ID NO:162) and the PCR probe was: FAM-AGATGATCCATGGGTTCAACATGCCAA-TAMRA (SEQ ID NO: 163) where FAM (PE-Applied Biosystems, Foster City, CA) is the fluorescent reporter dye) and TAMRA (PE-Applied Biosystems, Foster City, CA) is the quencher dye.

### EXAMPLE 21: Antisense inhibition of Cellular Inhibitor of Apoptosis-2 expression-phosphorothioate oligodeoxynucleotides

In accordance with the present invention, a series of oligonucleotides were designed to target different regions of the human Cellular Inhibitor of Apoptosis-2 RNA, using published sequences (GenBank accession number U37546, SEQ ID NO: 159). The oligonucleotides are shown in Table 12. Target sites are indicated by nucleotide numbers, as given in the sequence source reference (Genbank accession no. U37546), to which the oligonucleotide binds. All compounds in Table 12 are oligodeoxynucleotides with phosphorothioate backbones (internucleoside linkages) throughout. The compounds were analyzed for effect on Cellular Inhibitor of Apoptosis-2 mRNA levels by quantitative real-time PCR as described in other examples herein. Data are averages from three experiments. If present, "N.D." indicates "no data".

**Table 12**

| **Inhibition of Cellular Inhibitor of Apoptosis-2 mRNA levels by phosphorothioate oligodeoxynucleotides** | | | | | |
|---|---|---|---|---|---|
| **ISIS#** | **REGION** | **TARGET SITE** | **SEQUENCE** | **% Inhibition** | **SEQ ID NO.** |
| 23412 | 5' UTR | 3 | actgaagacattttgaat | 62 | 164 |
| 23413 | 5' UTR | 37 | cttagaggtacgtaaaat | 29 | 165 |
| 23414 | 5' UTR | 49 | gcacttttatttcttaga | 70 | 166 |
| 23415 | 5' UTR | 62 | attttaattagaagcact | 0 | 167 |
| 23416 | 5' UTR | 139 | accatatttcactgattc | 70 | 168 |
| 23417 | 5' UTR | 167 | ctaactcaaaggaggaaa | 0 | 169 |
| 23418 | 5' UTR | 175 | cacaagacctaactcaaa | 27 | 170 |
| 23419 | 5' UTR | 268 | gctctgctgtcaagtgtt | 57 | 171 |
| 23420 | 5' UTR | 303 | tgtgtgactcatgaagct | 23 | 172 |
| 23421 | 5' UTR | 335 | ttcagtggcattcaatca | 23 | 173 |
| 23422 | 5' UTR | 357 | cttctccaggctactaga | 50 | 174 |
| 23423 | 5' UTR | 363 | ggtcaacttctccaggct | 65 | 175 |
| 23424 | 5' UTR | 437 | taaaacccttcacagaag | 0 | 176 |
| 23425 | 5' LTTR | 525 | ttaaggaagaaatacaca | 0 | 177 |
| 23426 | 5' UTR | 651 | gcatggctttgcttttat | 0 | 178 |
| 23427 | Coding | 768 | caaacgtgttggcgcttt | 35 | 179 |
| 23428 | Coding | 830 | agcaggaaaagtggaata | 0 | 180 |
| 23429 | Coding | 1015 | ttaacggaatttagactc | 0 | 181 |
| 23430 | Coding | 1064 | atttgttactgaagaagg | 0 | 182 |
| 23431 | Coding | 1118 | agagccacggaaatatcc | 9 | 183 |
| 23432 | Coding | 1168 | aaatcttgatttgctctg | 7 | 184 |
| 23433 | Coding | 1231 | gtaagtaatctggcattt | 0 | 185 |
| 23434 | Coding | 1323 | agcaagccactctgtctc | 50 | 186 |
| 23435 | Coding | 1436 | tgaagtgtcttgaagctg | 0 | 187 |
| 23436 | Coding | 1580 | tttgacatcatcactgtt | 0 | 188 |
| 23437 | Coding | 1716 | tggcttgaacttgacgga | 0 | 189 |
| 23438 | Coding | 1771 | tcatctcctgggctgtct | 40 | 190 |
| 23439 | Coding | 1861 | gcagcattaatcacagga | 0 | 191 |
| 23440 | Coding | 2007 | tttctctctcctcttccc | 10 | 192 |
| 23441 | Coding | 2150 | aacatcatgttcttgttc | 9 | 193 |
| 23442 | Coding | 2273 | atataacacagcttcagc | 0 | 194 |
| 23443 | Coding | 2350 | aattgttcttccactggt | 0 | 195 |
| 23444 | Coding | 2460 | aagaaggagcacaatctt | 70 | 196 |
| 23445 | 3' UTR | 2604 | gaaaccaaattaggataa | 12 | 197 |
| 23446 | 3' UTR | 2753 | tgtagtgctacctctttt | 69 | 198 |
| 23447 | 3' UTR | 2779 | ctgaaattttgattgaat | 14 | 199 |
| 23448 | 3' UTR | 2795 | tacaatttcaataatgct | 38 | 200 |
| 23449 | 3' UTR | 2920 | gggtctcagtatgctgcc | 21 | 201 |
| 23450 | 3' UTR | 3005 | ccttcgatgtataggaca | 0 | 202 |
| 23451 | 3' UTR | 3040 | catgtccctaaaatgtca | 0 | 203 |

### EXAMPLE 22: Antisense inhibition of Cellular Inhibitor of Apoptosis-2 expression-phosphorothioate 2'-MOE gapmer oligonucleotides

In accordance with the present invention, a second series of oligonucleotides targeted to human Cellular Inhibitor of Apoptosis-2 were synthesized. The oligonucleotide sequences are shown in Table 13. Target sites are indicated by nucleotide numbers, as given in the sequence source reference (Genbank accession no. U37546), to which the oligonucleotide binds.

All compounds in Table 13 are chimeric oligonucleotides ("gapmers") 18 nucleotides in length, composed of a central "gap" region consisting of ten 2'-deoxynucleotides, which is flanked on both sides (5' and 3' directions) by four-nucleotide "wings". The wings are composed of 2'-methoxyethyl (2'-MOE)nucleotides. The internucleoside (backbone) linkages are phosphorothioate (P=S) throughout the oligonucleotide. Cytidine residues in the 2'-MOE wings are 5-methylcytidines.

Data were obtained by real-time quantitative PCR as described in other examples herein and are averaged from three experiments. If present, "N.D." indicates "no data".

**Table 13**

| **Inhibition of Cellular Inhibitor of Apoptosis-2 mRNA levels by chimeric phosphorothioate oligonucleotides having 2'-MOE wings and a deoxy gap** | | | | | |
|---|---|---|---|---|---|
| **ISIS#** | **REGION** | **TARGET SITE** | **SEQUENCE** | **% Inhibition** | **SEQ ID NO.** |
| 23452 | 5'UTR | 3 | actgaagacattttgaat | 35 | 164 |
| 23453 | 5' UTR | 37 | cttagaggtacgtaaaat | 26 | 165 |
| 23454 | 5' UTR | 49 | gcacttttatttcttaga | 76 | 166 |
| 23455 | 5' UTR | 62 | attttaattagaagcact | 0 | 167 |
| 23456 | 5' UTR | 139 | accatatttcactgattc | 0 | 168 |
| 23457 | 5' UTR | 167 | ctaactcaaaggaggaaa | 5 | 169 |
| 23458 | 5' UTR | 175 | cacaagacctaactcaaa | 0 | 170 |
| 23459 | 5' UTR | 268 | gctctgctgtcaagtgtt | 57 | 171 |
| 23460 | 5' UTR | 303 | tgtgtgactcatgaagct | 67 | 172 |
| 23461 | 5' UTR | 335 | ttcagtggcattcaatca | 59 | 173 |
| 23462 | 5' UTR | 357 | cttctccaggctactaga | 0 | 174 |
| 23463 | 5' UTR | 363 | ggtcaacttctccaggct | 75 | 175 |
| 23464 | 5' UTR | 437 | taaaacccttcacagaag | 11 | 176 |
| 23465 | 5' UTR | 525 | ttaaggaagaaatacaca | 0 | 177 |
| 23466 | 5' UTR | 651 | gcatggctttgcttttat | 46 | 178 |
| 23467 | Coding | 768 | caaacgtgttggcgcttt | 47 | 179 |
| 23468 | Coding | 830 | agcaggaaaagtggaata | 39 | 180 |
| 23469 | Coding | 1015 | ttaacggaatttagactc | 12 | 181 |
| 23470 | Coding | 1064 | atttgttactgaagaagg | 34 | 182 |
| 23471 | Coding | 1118 | agagccacggaaatatcc | 54 | 183 |
| 23472 | Coding | 1168 | aaatcttgatttgctctg | 34 | 184 |
| 23473 | Coding | 1231 | gtaagtaatctggcattt | 0 | 185 |
| 23474 | Coding | 1323 | agcaagccactctgtctc | 42 | 186 |
| 23475 | Coding | 1436 | tgaagtgtcttgaagctg | 0 | 187 |
| 23476 | Coding | 1580 | tttgacatcatcactgtt | 57 | 188 |
| 23477 | Coding | 1716 | tggcttgaacttgacgga | 23 | 189 |
| 23478 | Coding | 1771 | tcatctcctgggctgtct | 66 | 190 |
| 23479 | Coding | 1861 | gcagcattaatcacagga | 65 | 191 |
| 23480 | Coding | 2007 | tttctctctcctcttccc | 0 | 192 |
| 23481 | Coding | 2150 | aacatcatgttcttgttc | 13 | 193 |
| 23482 | Coding | 2273 | atataacacagcttcagc | 0 | 194 |
| 23483 | Coding | 2350 | aattgttcttccactggt | 60 | 195 |
| 23484 | Coding | 2460 | aagaaggagcacaatctt | 65 | 196 |
| 23485 | 3' UTR | 2604 | gaaaccaaattaggataa | 0 | 197 |
| 23486 | 3' UTR | 2753 | tgtagtgctacctctttt | 73 | 198 |
| 23487 | 3' UTR | 2779 | ctgaaattttgattgaat | 4 | 199 |
| 23488 | 3'UTR | 2795 | tacaatttcaataatgct | 0 | 200 |
| 23489 | 3' UTR | 2920 | gggtctcagtatgctgcc | 42 | 201 |
| 23490 | 3' UTR | 3005 | ccttcgatgtataggaca | 71 | 202 |
| 23491 | 3' UTR | 3040 | catgtccctaaaatgtca | 45 | 203 |

### EXAMPLE 23: Automated Assay of ELK-1 Oligonucleotide Activity

ELK-1 (also known as p62TCF) is a member of the ternary complex factor (TCF) subfamily of Ets domain proteins and utilizes a bipartite recognition mechanism mediated by both protein-DNA and protein-protein interactions. This results in gene regulation not only by direct DNA binding but also by indirect DNA binding through recruitment by other factors (Rao et al., *Science,* **1989**, *244*, 66-70). The formation of ternary complexes with an array of proteins allows the differential regulation of many genes. The mechanism by which ELK-1 controls various signal transduction pathways involves regulating the activity of the Egr-1, pip92, nur77 and c-fos promoters by binding to the serum response element (SRE) in these promoters in response to extracellular stimuli such as growth factors, mitogens and oncogene products (Sharrocks et al., *Int. J. Biochem. Cell Biol*., **1997,** *29*, 1371-1387). ELK-1 has also been shown to mediate other functions within the cell including apoptosis.

Oligonucleotides were designed as described in Example 2, synthesized as described in Examples 3 through 8, analyzed as described in Examples 9 and assayed as described in Example 10 except for target specific primer and probes. ELK-1 probes and primers were designed to hybridize to the human ELK-1 sequence, using published sequence information (GenBank accession number M25269 SEQ ID NO:204; SEQ ID NO:205 is protein).

For ELK-1 the PCR primers were:
forward primer: GCAAGGCAATGGCCACAT (SEQ ID NO: 206)
reverse primer: CTCCTCTGCATCCACCAGCTT (SEQ ID NO: 207) and the PCR probe was: FAM-TCTCCTGGACTTCACGGGATGGTGGT-TAMRA (SEQ ID NO: 208) where FAM (PE-Applied Biosystems, Foster City, CA) is the fluorescent reporter dye) and TAMRA (PE-Applied Biosystems, Foster City, CA) is the quencher dye.

### EXAMPLE 24: Antisense inhibition of ELK-1 expression-phosphorothioate oligodeoxynucleotides

In accordance with the present invention, a series of oligonucleotides were designed to target different regions of the human ELK-1 RNA, using published sequences (GenBank accession number M25269 SEQ ID NO: 204). The oligonucleotides are shown in Table 14. Target sites are indicated by nucleotide numbers, as given in the sequence source reference (Genbank accession no. M25269), to which the oligonucleotide binds. All compounds in Table 14 are oligodeoxynucleotides with phosphorothioate backbones (internucleoside linkages) throughout. The compounds were analyzed for effect on ELK-1 mRNA levels by quantitative real-time PCR as described in other examples herein. Data are averages from three experiments. If present, "N.D." indicates "no data".

**Table 14**

| **Inhibition of ELK-1 mRNA levels by phosphorothioate oligodeoxynucleotides** | | | | | |
|---|---|---|---|---|---|
| **ISIS#** | **REGION** | **TARGET SITE** | **SEQUENCE** | **% Inhibition** | **SEQ ID NO.** |
| 24752 | 5' UTR | 11 | cccctgcgtttccctaca | 15 | 209 |
| 24753 | 5' UTR | 50 | ggtggtggtggcggtggc | 29 | 210 |
| 24754 | 5' UTR | 139 | ggcgttggcaatgttggc | 82 | 211 |
| 24755 | 5' UTR | 167 | aagttgaggctgtgtgta | 0 | 212 |
| 24756 | 5' UTR | 189 | aggccacggacgggtctc | 92 | 213 |
| 24757 | 5' UTR | 229 | gattgattcgctacgatg | 71 | 214 |
| 24758 | 5' UTR | 255 | gggatgcggaggagtgcg | 74 | 215 |
| 24759 | 5'UTR | 289 | agtgctcacgccatccca | 22 | 216 |
| 24760 | Coding | 328 | aaactgccacagcgtcac | 64 | 217 |
| 24761 | Coding | 381 | gaagtccaggagatgatg | 62 | 218 |
| 24762 | Coding | 395 | caccaccatcccgtgaag | 88 | 219 |
| 24763 | Coding | 455 | tcttgttcttgcgtagtc | 62 | 220 |
| 24764 | Coding | 512 | tgttcttgtcatagtagt | 52 | 221 |
| 24765 | Coding | 527 | tcaccttgcggatgatgt | 57 | 222 |
| 24766 | Coding | 582 | gagcaccctgcgacctca | 72 | 223 |
| 24767 | Coding | 600 | ggcgggcagtcctcagtg | 82 | 224 |
| 24768 | Coding | 787 | ggtgaaggtggaatagag | 58 | 225 |
| 24769 | Coding | 993 | tccgatttcaggtttggg | 55 | 226 |
| 24770 | Coding | 1110 | ttggtggtttctggcaca | 67 | 227 |
| 24771 | Coding | 1132 | tggagggacttctggctc | 69 | 228 |
| 24772 | Coding | 1376 | gcgtaggaagcagggatg | 34 | 229 |
| 24773 | Coding | 1440 | gtgctccagaagtgaatg | 64 | 230 |
| 24774 | Coding | 1498 | actggatggaaactggaa | 34 | 231 |
| 24775 | Coding | 1541 | ggccatccacgctgatag | 74 | 232 |
| 24776 | 3' UTR | 1701 | ccaccacaatcagagcat | 74 | 233 |
| 24777 | 3' UTR | 1711 | gatccccaccccaccaca | 16 | 234 |
| 24778 | 3' UTR | 1765 | tgttttctgtggaggaga | 48 | 235 |
| 24779 | 3' UTR | 1790 | aaacagagaagttgtgga | 11 | 236 |
| 24780 | 3' UTR | 1802 | gggactgacagaaaacag | 0 | 237 |
| 24781 | 3' UTR | 1860 | ataaataaataaaccgcc | 18 | 238 |
| 24782 | 3' UTR | 1894 | gttaggtcaggctcatcc | 56 | 239 |
| 24783 | 3' UTR | 1974 | gttctcaagccagacctc | 52 | 240 |
| 24784 | 3' UTR | 1992 | aataaagaaagaaaggtc | 41 | 241 |
| 24785 | 3' UTR | 2006 | agggcaggctgagaaata | 29 | 242 |
| 24786 | 3' UTR | 2053 | cttctactcacatccaaa | 54 | 243 |
| 24787 | 3' UTR | 2068 | caaaacaaactaactctt | 24 | 244 |
| 24788 | 3' UTR | 2080 | ggaataataaaacaaaac | 40 | 245 |
| 24789 | 3' UTR | 2107 | ttcttcctggacccctga | 93 | 246 |
| 24790 | 3' UTR | 2161 | ccaagggtgtgattcttc | 81 | 247 |
| 24791 | 3' UTR | 2200 | tgtctgagagaaaggttg | 55 | 248 |

### EXAMPLE 25: Antisense inhibition of ELK-1 expression- phosphorothioate 2'-MOE gapmer oligonucleotides

In accordance with the present invention, a second series of oligonucleotides targeted to human ELK-1 were synthesized. The oligonucleotide sequences are shown in Table 15. Target sites are indicated by nucleotide numbers, as given in the sequence source reference (Genbank accession no. M25269), to which the oligonucleotide binds.

All compounds in Table 15 are chimeric oligonucleotides ("gapmers") 18 nucleotides in length, composed of a central "gap" region consisting of ten 2'-deoxynucleotides, which is flanked on both sides (5' and 3' directions) by four-nucleotide "wings". The wings are composed of 2'-methoxyethyl (2'-MOE)nucleotides. The internucleoside (backbone) linkages are phosphorothioate (P=S) throughout the oligonucleotide. Cytidine residues in the 2'-MOE wings are 5-methylcytidines.

Data were obtained by real-time quantitative PCR as described in other examples herein and are averaged from three experiments. If present, "N.D." indicates "no data".

**Table 15**

| **Inhibition of ELK-1 mRNA levels by chimeric phosphorothioate oligonucleotides having 2'-MOE wings and a deoxy gap** | | | | | |
|---|---|---|---|---|---|
| **ISIS#** | **REGION** | **TARGET SITE** | **SEQUENCE** | **% Inhibition** | **SEQ ID NO.** |
| 24792 | 5' UTR | 11 | cccctgcgtttccctaca | 23 | 209 |
| 24793 | 5' UTR | 50 | ggtggtggtggcggtggc | 80 | 210 |
| 24794 | 5' UTR | 139 | ggcgttggcaatgttggc | 91 | 211 |
| 24795 | 5' UTR | 167 | aagttgaggctgtgtgta | 27 | 212 |
| 24796 | 5' UTR | 189 | aggccacggacgggtctc | 79 | 213 |
| 24797 | 5' UTR | 229 | gattgattcgctacgatg | 69 | 214 |
| 24798 | 5' UTR | 255 | gggatgcggaggagtgcg | 42 | 215 |
| 24799 | 5'UTR | 289 | agtgctcacgccatccca | 45 | 216 |
| 24800 | Coding | 328 | aaactgccacagcgtcac | 57 | 217 |
| 24801 | Coding | 381 | gaagtccaggagatgatg | 55 | 218 |
| 24802 | Coding | 395 | caccaccatcccgtgaag | 41 | 219 |
| 24803 | Coding | 455 | tcttgttcttgcgtagtc | 80 | 220 |
| 24804 | Coding | 512 | tgttcttgtcatagtagt | 65 | 221 |
| 24805 | Coding | 527 | tcaccttgcggatgatgt | 70 | 222 |
| 24806 | Coding | 582 | gagcaccctgcgacctca | 64 | 223 |
| 24807 | Coding | 600 | ggcgggcagtcctcagtg | 67 | 224 |
| 24808 | Coding | 787 | ggtgaaggtggaatagag | 45 | 225 |
| 24809 | Coding | 993 | tccgatttcaggtttggg | 75 | 226 |
| 24810 | Coding | 1110 | ttggtggtttctggcaca | 82 | 227 |
| 24811 | Coding | 1132 | tggagggacttctggctc | 60 | 228 |
| 24812 | Coding | 1376 | gcgtaggaagcagggatg | 49 | 229 |
| 24813 | Coding | 1440 | gtgctccagaagtgaatg | 71 | 230 |
| 24814 | Coding | 1498 | actggatggaaactggaa | 62 | 231 |
| 24815 | Coding | 1541 | ggccatccacgctgatag | 78 | 232 |
| 24816 | 3' UTR | 1701 | ccaccacaatcagagcat | 54 | 233 |
| 24817 | 3' UTR | 1711 | gatccccaccccaccaca | 44 | 234 |
| 24818 | 3'UTR | 1765 | tgttttctgtggaggaga | 74 | 235 |
| 24819 | 3' UTR | 1790 | aaacagagaagttgtgga | 64 | 236 |
| 24820 | 3' UTR | 1802 | gggactgacagaaaacag | 16 | 237 |
| 24821 | 3' UTR | 1860 | ataaataaataaaccgcc | 38 | 238 |
| 24822 | 3' UTR | 1894 | gttaggtcaggctcatcc | 59 | 239 |
| 24823 | 3' UTR | 1974 | gttctcaagccagacctc | 62 | 240 |
| 24824 | 3' UTR | 1992 | aataaagaaagaaaggtc | 35 | 241 |
| 24825 | 3' UTR | 2006 | agggcaggctgagaaata | 0 | 242 |
| 24826 | 3' UTR | 2053 | cttctactcacatccaaa | 46 | 243 |
| 24827 | 3' UTR | 2068 | caaaacaaactaactctt | 38 | 244 |
| 24828 | 3' UTR | 2080 | ggaataataaaacaaaac | 37 | 245 |
| 24829 | 3' UTR | 2107 | ttcttcctggacccctga | 71 | 246 |
| 24830 | 3' UTR | 2161 | ccaagggtgtgattcttc | 88 | 247 |
| 24831 | 3' UTR | 2200 | tgtctgagagaaaggttg | 65 | 248 |

### EXAMPLE 26: Automated Assay of Gi alpha proteins Oligonucleotide Activity

G-alpha-11 is a member of the Gq subfamily of G proteins whose primary function is to activate PLC-b isoforms producing second messengers and affecting intracellular calcium stores.

Oligonucleotides were designed as described in Example 2, synthesized as described in Examples 3 through 8, analyzed as described in Examples 9 and assayed as described in Example 10 except for target specific primer and probes. G-alpha-11 probes and primers were designed to hybridize to the human G-alpha-11 sequence, using published sequence information (GenBank accession number AF011497, SEQ ID NO:249; SEQ ID NO:250 is protein). For G-alpha-11 the PCR primers were: forward primer: TGACCACCTTCGAGCATCAG (SEQ ID NO: 251) reverse primer: CGGTCGTAGCATTCCTGGAT (SEQ ID NO: 252) and the PCR probe was: FAM-TCAGTGCCATCAAGACCCTGTGGGAG-TAMRA (SEQ ID NO: 253) where FAM (PE-Applied Biosystems, Foster City, CA) is the fluorescent reporter dye) and TAMRA (PE-Applied Biosystems, Foster City, CA) is the quencher dye.

### EXAMPLE 27: Antisense inhibition of G-alpha-11 expression- phosphorothioate oligodeoxynucleotides

In accordance with the present invention, a series of oligonucleotides were designed to target different regions of the human G-alpha-11 RNA, using published sequences (GenBank accession number AF011497 SEQ ID NO: 249). The oligonucleotides are shown in Table 16. Target sites are indicated by nucleotide numbers, as given in the sequence source reference (Genbank accession no. AF011497), to which the oligonucleotide binds. All compounds in Table 16 are oligodeoxynucleotides with phosphorothioate backbones (internucleoside linkages) throughout. The compounds were analyzed for effect on G-alpha-11 mRNA levels by quantitative real-time PCR as described in other examples herein. Data are averages from three experiments. If present, "N.D." indicates "no data".

**Table 16**

| **Inhibition of G-alpha-11 mRNA levels by phosphorothioate oligodeoxynucleotides** | | | | | |
|---|---|---|---|---|---|
| **ISIS#** | **REGION** | **TARGET SITE** | **SEQUENCE** | **% Inhibition** | **SEQ ID NO.** |
| 20576 | Coding | 1 | gatggactccagagtcat | 0 | 254 |
| 20577 | Coding | 6 | gccatgatggactccaga | 75 | 255 |
| 20578 | Coding | 9 | cacgccatgatggactcc | 0 | 256 |
| 20579 | Coding | 25 | ctcatcgctcaggcaaca | 61 | 257 |
| 20580 | Coding | 31 | cttcacctcatcgctcag | 20 | 258 |
| 20581 | Coding | 36 | gactccttcacctcatcg | 15 | 259 |
| 20582 | Coding | 45 | atccgcttggactccttc | 17 | 260 |
| 20583 | Coding | 50 | cgttgatccgcttggact | 0 | 261 |
| 20584 | Coding | 61 | ctcgatctcggcgttgat | 0 | 262 |
| 20585 | Coding | 77 | cccgccgcagctgcttct | 58 | 263 |
| 20586 | Coding | 106 | cttgagctcgcgccgggc | 31 | 264 |
| 20587 | Coding | 116 | gcagcagcagcttgagct | 0 | 265 |
| 20588 | Coding | 127 | gcccgtgccgagcagcag | 0 | 266 |
| 20589 | Coding | 146 | acgtgctcttcccgctct | 28 | 267 |
| 20590 | Coding | 159 | atctgcttgatgaacgtg | 0 | 268 |
| 20591 | Coding | 162 | cgcatctgcttgatgaac | 0 | 269 |
| 20592 | Coding | 184 | gtagccggcgccgtggat | 1 | 270 |
| 20593 | Coding | 197 | tgtcctcctccgagtagc | 0 | 271 |
| 20594 | Coding | 199 | cttgtcctcctccgagta | 79 | 272 |
| 20595 | Coding | 207 | aagccgcgcttgtcctcc | 56 | 273 |
| 20596 | Coding | 222 | tagacgagcttggtgaag | 0 | 274 |
| 20597 | Coding | 230 | tgttctggtagacgagct | 0 | 275 |
| 20598 | Coding | 242 | tggcggtgaagatgttct | 0 | 276 |
| 20599 | Coding | 258 | cggatcatggcctgcatg | 1 | 277 |
| 20600 | Coding | 271 | cgtctccatggcccggat | 49 | 278 |
| 20601 | Coding | 285 | tagaggatcttgagcgtc | 0 | 279 |
| 20602 | Coding | 287 | tgtagaggatcttgagcg | 0 | 280 |
| 20603 | Coding | 297 | tgctcgtacttgtagagg | 7 | 281 |
| 20604 | Coding | 306 | gccttgttctgctcgtac | 25 | 282 |
| 20605 | Coding | 309 | ttggccttgttctgctcg | 0 | 283 |
| 20606 | Coding | 319 | caggagcgcattggcctt | 0 | 284 |
| 20607 | Coding | 340 | ctccacgtccacctcccg | 69 | 285 |
| 20608 | Coding | 349 | ggtcaccttctccacgtc | 27 | 286 |
| 20609 | Coding | 362 | gatgctcgaaggtggtca | 33 | 287 |
| 20610 | Coding | 373 | actgacgtactgatgctc | 36 | 288 |
| 20611 | Coding | 382 | cttgatggcactgacgta | 78 | 289 |
| 20612 | Coding | 388 | cagggtcttgatggcact | 0 | 290 |
| 20613 | Coding | 409 | ctggatgcccgggtcctc | 0 | 291 |
| 20614 | Coding | 411 | tcctggatgcccgggtcc | 30 | 292 |
| 20615 | Coding | 429 | cgcctgcggtcgtagcat | 0 | 293 |
| 20616 | Coding | 440 | gctggtactcgcgcctgc | 41 | 294 |
| 20617 | Coding | 459 | tacttggcagagtcggag | 34 | 295 |
| 20618 | Coding | 468 | gtcaggtagtacttggca | 76 | 296 |
| 20619 | Coding | 479 | ggtcaacgtcggtcaggt | 18 | 297 |
| 20620 | Coding | 489 | gtggcgatgcggtcaacg | 1 | 298 |
| 20621 | Coding | 503 | gcaggtagcccaaggtgg | 20 | 299 |
| 20622 | Coding | 518 | cgtcctgctgggtgggca | 40 | 300 |
| 20623 | Coding | 544 | ggtggtgggcacgcggac | 0 | 301 |
| 20624 | Coding | 555 | tcgatgatgccggtggtg | 0 | 302 |
| 20625 | Coding | 572 | ccaggtcgaaagggtact | 0 | 303 |
| 20626 | Coding | 578 | tgttctccaggtcgaaag | 33 | 304 |
| 20627 | Coding | 584 | agatgatgttctccaggt | 0 | 305 |
| 20628 | Coding | 591 | atccggaagatgatgttc | 0 | 306 |
| 20629 | Coding | 624 | ctccgctccgaccgctgg | 56 | 307 |
| 20630 | Coding | 634 | gatccacttcctccgctc | 59 | 308 |
| 20631 | Coding | 655 | tgtcacgttctcaaagca | 0 | 309 |
| 20632 | Coding | 663 | atgatggatgtcacgttc | 0 | 310 |
| 20633 | Coding | 671 | cgagaaacatgatggatg | 0 | 311 |
| 20634 | Coding | 682 | gctgagggcgacgagaaa | 75 | 312 |
| 20635 | Coding | 709 | cgactccaccaggacttg | 40 | 313 |
| 20636 | Coding | 726 | atccggttctcgttgtcc | 22 | 314 |
| 20637 | Coding | 728 | ccatccggttctcgttgt | 19 | 315 |
| 20638 | Coding | 744 | agggctttgctctcctcc | 77 | 316 |
| 20639 | Coding | 754 | ggtccggaacagggcttt | 26 | 317 |
| 20640 | Coding | 766 | gtaggtgatgatggtccg | 0 | 318 |
| 20641 | Coding | 787 | ggaggagttctggaacca | 64 | 319 |
| 20642 | Coding | 803 | tgaggaagaggatgacgg | 0 | 320 |
| 20643 | Coding | 818 | gcaggtccttcttgttga | 6 | 321 |
| 20644 | Coding | 831 | atcttgtcctccagcagg | 4 | 322 |
| 20645 | Coding | 842 | gcgagtacaggatcttgt | 17 | 323 |
| 20646 | Coding | 858 | aagtagtccaccaggtgc | 0 | 324 |
| 20647 | Coding | 910 | gatgaactcccgcgccgc | 52 | 325 |
| 20648 | Coding | 935 | ggttcaggtccacgaaca | 71 | 326 |
| 20649 | Coding | 958 | gtagatgatcttgtcgct | 0 | 327 |
| 20650 | Coding | 972 | cacgtgaagtgtgagtag | 0 | 328 |
| 20651 | Coding | 993 | atgttctccgtgtcggtg | 0 | 329 |
| 20652 | Coding | 1414 | acggccgcgaacacgaag | 6 | 330 |
| 20653 | Coding | 1027 | gatggtgtccttcacggc | 0 | 331 1 |
| 20654 | Coding | 1043 | tcaggttcagctgcagga | 3 | 332 |
| 20655 | Coding | 1059 | accagattgtactccttc | 0 | 333 |

### EXAMPLE 28: Antisense inhibition of G-alpha-11 expression- phosphorothioate 2'-MOE gapmer oligonucleotides

In accordance with the present invention, a second series of oligonucleotides targeted to human G-alpha-11 were synthesized. The oligonucleotide sequences are shown in Table 17. Target sites are indicated by nucleotide numbers, as given in the sequence source reference (Genbank accession no. AF011497), to which the oligonucleotide binds.

All compounds in Table 17 are chimeric oligonucleotides ("gapmers") 18 nucleotides in length, composed of a central "gap" region consisting of ten 2'-deoxynucleotides, which is flanked on both sides (5' and 3' directions) by four-nucleotide "wings". The wings are composed of 2'-methoxyethyl (2'-MOE)nucleotides. The internucleoside (backbone) linkages are phosphorothioate (P=S) throughout the oligonucleotide. Cytidine residues in the 2'-MOE wings are 5-methylcytidines.

Data were obtained by real-time quantitative PCR as described in other examples herein and are averaged from three experiments. If present, "N.D." indicates "no data".

**Table 17**

| **Inhibition of G-alpha-11 mRNA levels by chimeric phosphorothioate oligonucleotides having 2'-MOE wings and a deoxy gap** | | | | | |
|---|---|---|---|---|---|
| **ISIS#** | **REGION** | **TARGET SITE** | **SEQUENCE** | **% Inhibition** | **SEQ ID NO.** |
| 20981 | Coding | 1 | gatggactccagagtcat | 0 | 254 |
| 20982 | Coding | 6 | gccatgatggactccaga | 0 | 255 |
| 20983 | Coding | 9 | cacgccatgatggactcc | 0 | 256 |
| 20984 | Coding | 25 | ctcatcgctcaggcaaca | 0 | 257 |
| 20985 | Coding | 31 | cttcacctcatcgctcag | 2 | 258 |
| 20986 | Coding | 36 | gactccttcacctcatcg | 0 | 259 |
| 20987 | Coding | 45 | atccgcttggactccttc | 19 | 260 |
| 20988 | Coding | 50 | cgttgatccgcttggact | 15 | 261 |
| 20989 | Coding | 61 | ctcgatctcggcgttgat | 0 | 262 |
| 20990 | Coding | 77 | cccgccgcagctgcttct | 41 | 263 |
| 20991 | Coding | 106 | cttgagctcgcgccgggc | 19 | 264 |
| 20992 | Coding | 116 | gcagcagcagcttgagct | 23 | 265 |
| 20993 | Coding | 127 | gcccgtgccgagcagcag | 38 | 266 |
| 20994 | Coding | 146 | acgtgctcttcccgctct | 34 | 267 |
| 20995 | Coding | 159 | atctgcttgatgaacgtg | 56 | 268 |
| 20996 | Coding | 162 | cgcatctgcttgatgaac | 31 | 269 |
| 20997 | Coding | 184 | gtagccggcgccgtggat | 0 | 270 |
| 20998 | Coding | 197 | tgtcctcctccgagtagc | 42 | 271 |
| 20999 | Coding | 199 | cttgtcctcctccgagta | 0 | 272 |
| 21000 | Coding | 207 | aagccgcgcttgtcctcc | 73 | 273 |
| 21001 | Coding | 222 | tagacgagcttggtgaag | 0 | 274 |
| 21002 | Coding | 230 | tgttctggtagacgagct | 61 | 275 |
| 21003 | Coding | 242 | tggcggtgaagatgttct | 14 | 276 |
| 21004 | Coding | 258 | cggatcatggcctgcatg | 84 | 277 |
| 21005 | Coding | 271 | cgtctccatggcccggat | 70 | 278 |
| 21006 | Coding | 285 | tagaggatcttgagcgtc | 39 | 279 |
| 21007 | Coding | 287 | tgtagaggatcttgagcg | 28 | 280 |
| 21008 | Coding | 297 | tgctcgtacttgtagagg | 70 | 281 |
| 21009 | Coding | 306 | gccttgttctgctcgtac | 76 | 282 |
| 21010 | Coding | 309 | ttggccttgttctgctcg | 0 | 283 |
| 21011 | Coding | 319 | caggagcgcattggcctt | 87 | 284 |
| 21012 | Coding | 340 | ctccacgtccacctcccg | 0 | 285 |
| 21013 | Coding | 349 | ggtcaccttctccacgtc | 69 | 286 |
| 21014 | Coding | 362 | gatgctcgaaggtggtca | 0 | 287 |
| 21015 | Coding | 373 | actgacgtactgatgctc | 69 | 288 |
| 21016 | Coding | 382 | cttgatggcactgacgta | 32 | 289 |
| 21017 | Coding | 388 | cagggtcttgatggcact | 19 | 290 |
| 21018 | Coding | 409 | ctggatgcccgggtcctc | 63 | 291 |
| 21019 | Coding | 411 | tcctggatgcccgggtcc | 56 | 292 |
| 21020 | Coding | 429 | cgcctgcggtcgtagcat | 73 | 293 |
| 21021 | Coding | 440 | gctggtactcgcgcctgc | 68 | 294 |
| 21022 | Coding | 459 | tacttggcagagtcggag | 50 | 295 |
| 21023 | Coding | 468 | gtcaggtagtacttggca | 13 | 296 |
| 21024 | Coding | 479 | ggtcaacgtcggtcaggt | 64 | 297 |
| 21025 | Coding | 489 | gtggcgatgcggtcaacg | 52 | 298 |
| 21026 | Coding | 503 | gcaggtagcccaaggtgg | 52 | 299 |
| 21027 | Coding | 518 | cgtcctgctgggtgggca | 0 | 300 |
| 21028 | Coding | 544 | ggtggtgggcacgcggac | 81 | 301 |
| 21029 | Coding | 555 | tcgatgatgccggtggtg | 48 | 302 |
| 21030 | Coding | 572 | ccaggtcgaaagggtact | 61 | 303 |
| 21031 | Coding | 578 | tgttctccaggtcgaaag | 0 | 304 |
| 21032 | Coding | 584 | agatgatgttctccaggt | 0 | 305 |
| 21033 | Coding | 591 | atccggaagatgatgttc | 0 | 306 |
| 21034 | Coding | 624 | ctccgctccgaccgctgg | 59 | 307 |
| 21035 | Coding | 634 | gatccacttcctccgctc | 17 | 308 |
| 21036 | Coding | 655 | tgtcacgttctcaaagca | 9 | 309 |
| 21037 | Coding | 663 | atgatggatgtcacgttc | 41 | 310 |
| 21038 | Coding | 671 | cgagaaacatgatggatg | 0 | 311 |
| 21039 | Coding | 682 | gctgagggcgacgagaaa | 11 | 312 |
| 21040 | Coding | 709 | cgactccaccaggacttg | 0 | 313 |
| 21041 | Coding | 726 | atccggttctcgttgtcc | 67 | 314 |
| 21042 | Coding | 728 | ccatccggttctcgttgt | 30 | 315 |
| 21043 | Coding | 744 | agggctttgctctcctcc | 61 | 316 |
| 21044 | Coding | 754 | ggtccggaacagggcttt | 72 | 317 |
| 21045 | Coding | 766 | gtaggtgatgatggtccg | 68 | 318 |
| 21046 | Coding | 787 | ggaggagttctggaacca | 54 | 319 |
| 21047 | Coding | 803 | tgaggaagaggatgacgg | 23 | 320 |
| 21048 | Coding | 818 | gcaggtccttcttgttga | 0 | 321 |
| 21049 | Coding | 831 | atcttgtcctccagcagg | 39 | 322 |
| 21050 | Coding | 842 | gcgagtacaggatcttgt | 74 | 323 |
| 21051 | Coding | 858 | aagtagtccaccaggtgc | 36 | 324 |
| 21052 | Coding | 910 | gatgaactcccgcgccgc | 67 | 325 |
| 21053 | Coding | 935 | ggttcaggtccacgaaca | 37 | 326 |
| 21054 | Coding | 958 | gtagatgatcttgtcgct | 64 | 327 |
| 21055 | Coding | 972 | cacgtgaagtgtgagtag | 37 | 328 |
| 21056 | Coding | 993 | atgttctccgtgtcggtg | 0 | 329 |
| 21057 | Coding | 1014 | acggccgcgaacacgaag | 0 | 330 |
| 21058 | Coding | 1027 | gatggtgtccttcacggc | 69 | 331 |
| 21059 | Coding | 1043 | tcaggttcagctgcagga | 0 | 332 |
| 21060 | Coding | 1059 | accagattgtactccttc | 0 | 333 |

### EXAMPLE 29: Automated Assay of AKT-1 Oligonucleotide Activity

Akt-1 (also known as PKB alpha and RAC-PK alpha) is a member of the AKT/PKB family of serine/threonine kinases and has been shown to be involved in a diverse set of signaling pathways.

Oligonucleotides were designed as described in Example 2, synthesized as described in Examples 3 through 8, analyzed as described in Examples 9 and assayed as described in Example 10 except for target specific primer and probes. AKT-1 probes and primers were designed to hybridize to the human AKT-1 sequence, using published sequence information (GenBank accession number M63167 SEQ ID NO:334; SEQ ID NO:335 is protein). For Akt-1 the PCR primers were: forward primer: CGTGACCATGAACGAGTTTGA (SEQ ID NO: 336) reverse primer: CAGGATCACCTTGCCGAAA (SEQ ID NO: 337) and the PCR probe was: FAM-CTGAAGCTGCTGGGCAAGGGCA-TAMRA (SEQ ID NO: 338) where FAM (PE-Applied Biosystems, Foster City, CA) is the fluorescent reporter dye) and TAMRA (PE-Applied Biosystems, Foster City, CA) is the quencher dye.

### EXMAMPLE 30: Antisense inhibition of Akt-1 expression- phosphorothioate oligodeoxynucleotides

In accordance with the present invention, a series of oligonucleotides were designed to target different regions of the human Akt-1 RNA, using published sequences (GenBank accession number M63167, SEQ ID NO: 334). The oligonucleotides are shown in Table 18. Target sites are indicated by nucleotide numbers, as given in the sequence source reference (Genbank accession no. M63167), to which the oligonucleotide binds. All compounds in Table 18 are oligodeoxynucleotides with phosphorothioate backbones (internucleoside linkages) throughout. The compounds were analyzed for effect on Akt-1 mRNA levels by quantitative real-time PCR as described in other examples herein. Data are averages from three experiments. If present, "N.D." indicates "no data".

**Table 18**

| **Inhibition of Akt-1 mRNA levels by phosphorothioate oligodeoxynucleotides** | | | | | |
|---|---|---|---|---|---|
| **ISIS#** | **REGION** | **TARGET SITE** | **SEQUENCE** | **% Inhibition** | **SEQ ID NO.** |
| 28880 | 5' UTR | 4 | ccctgtgccctgtcccag | 55 | 339 |
| 28881 | 5' UTR | 27 | cctaagcccctggtgaca | 15 | 340 |
| 28882 | 5' UTR | 62 | ctttgacttctttgaccc | 68 | 341 |
| 28883 | 5' UTR | 70 | ggcagcccctttgacttc | 53 | 342 |
| 28884 | Coding | 213 | caaccctccttcacaata | 24 | 343 |
| 28885 | Coding | 234 | tactcccctcgtttgtgc | 0 | 344 |
| 28886 | Coding | 281 | tgccatcattcttgagga | 65 | 345 |
| 28887 | Coding | 293 | agccaatgaaggtgccat | 67 | 346 |
| 28888 | Coding | 352 | cacagagaagttgttgag | 22 | 347 |
| 28889 | Coding | 496 | agtctggatggcggttgt | 49 | 348 |
| 28890 | Coding | 531 | tcctcctcctcctgcttc | 9 | 349 |
| 28891 | Coding | 570 | cctgagttgtcactgggt | 49 | 350 |
| 28892 | Coding | 666 | ccgaaagtgcccttgccc | 56 | 351 |
| 28893 | Coding | 744 | gccacgatgacttccttc | 60 | 352 |
| 28894 | Coding | 927 | cggtcctcggagaacaca | 0 | 353 |
| 28895 | Coding | 990 | acgttcttctccgagtgc | 30 | 354 |
| 28896 | Coding | 1116 | gtgccgcaaaaggtcttc | 66 | 355 |
| 28897 | Coding | 1125 | tactcaggtgtgccgcaa | 66 | 356 |
| 28898 | Coding | 1461 | ggcttgaagggtgggctg | 41 | 357 |
| 28899 | Coding | 1497 | tcaaaatacctggtgtca | 51 | 358 |
| 28900 | Coding | 1512 | gccgtgaactcctcatca | 56 | 359 |
| 28901 | Coding | 1541 | ggtcaggtggtgtgatgg | 0 | 360 |
| 28902 | Coding | 1573 | ctcgctgtccacacactc | 61 | 361 |
| 28903 | 3' UTR | 1671 | gcctctccatccctccaa | 76 | 362 |
| 28904 | 3' UTR | 1739 | acagcgtggcttctctca | 12 | 363 |
| 28905 | 3' UTR | 1814 | ttttcttccctaccccgc | 64 | 364 |
| 28906 | 3' UTR | 1819 | gatagttttcttccctac | 0 | 365 |
| 28907 | 3' UTR | 1831 | taaaacccgcaggatagt | 74 | 366 |
| 28908 | 3' UTR | 1856 | ggagaacaaactggatga | 0 | 367 |
| 28909 | 3' UTR | 1987 | ctggctgacagagtgagg | 59 | 368 |
| 28910 | 3' UTR | 1991 | gcggctggctgacagagt | 61 | 369 |
| 28911 | 3'UTR | 2031 | cccagagagatgacagat | 46 | 370 |
| 28912 | 3'UTR | 2127 | gctgctgtgtgcctgcca | 38 | 371 |
| 28913 | 3'UTR | 2264 | cataatacacaataacaa | 39 | 372 |
| 28914 | 3' UTR | 2274 | atttgaacaacataatac | 11 | 373 |
| 28915 | 3'UTR | 2397 | aagtgctaccgtggagag | 57 | 374 |
| 28916 | 3' UTR | 2407 | cgaaaaggtcaagtgcta | 41 | 375 |
| 28917 | 3'UTR | 2453 | cagggagtcagggagggc | 13 | 376 |
| 28918 | 3' UTR | 2545 | aaagttgaatgttgtaaa | 10 | 377 |
| 28919 | 3' UTR | 2553 | aaaatactaaagttgaat | 25 | 378 |

### EXAMPLE 31: Antisense inhibition of Akt-1 expression- phosphorothioate 2'-MOE gapmer oligonucleotides

In accordance with the present invention, a second series of oligonucleotides targeted to human Akt-1 were synthesized. The oligonucleotide sequences are shown in Table 19. Target sites are indicated by nucleotide numbers, as given in the sequence source reference (Genbank accession no. M63167), to which the oligonucleotide binds.

All compounds in Table 19 are chimeric oligonucleotides ("gapmers") 18 nucleotides in length, composed of a central "gap" region consisting of ten 2'-deoxynucleotides, which is flanked on both sides (5' and 3' directions) by four-nucleotide "wings". The wings are composed of 2'-methoxyethyl (2'-MOE)nucleotides. The internucleoside (backbone) linkages are phosphorothioate (P=S) throughout the oligonucleotide. Cytidine residues in the 2'-MOE wings are 5-methylcytidines.

Data were obtained by real-time quantitative PCR as described in other examples herein and are averaged from three experiments. If present, "N.D." indicates "no data".

**Table 19**

| **Inhibition of Akt-1 mRNA levels by chimeric phosphorothioate oligonucleotides having 2'-MOE wings and a deoxy gap** | | | | | |
|---|---|---|---|---|---|
| **ISIS#** | **REGION** | **TARGET SITE** | **SEQUENCE** | **% Inhibition** | **SEQ ID NO.** |
| 28920 | 5' UTR | 4 | ccctgtgccctgtcccag | 88 | 339 |
| 28921 | 5' UTR | 27 | cctaagcccctggtgaca | 44 | 340 |
| 28922 | 5' UTR | 62 | ctttgacttctttgaccc | 61 | 341 |
| 28923 | 5' UTR | 70 | ggcagcccctttgacttc | 79 | 342 |
| 28924 | Coding | 213 | caaccctccttcacaata | 72 | 343 |
| 28925 | Coding | 234 | tactcccctcgtttgtgc | 39 | 344 |
| 28926 | Coding | 281 | tgccatcattcttgagga | 73 | 345 |
| 28927 | Coding | 293 | agccaatgaaggtgccat | 62 | 346 |
| 28928 | Coding | 352 | cacagagaagttgttgag | 48 | 347 |
| 28929 | Coding | 496 | agtctggatggcggttgt | 43 | 348 |
| 28930 | Coding | 531 | tcctcctccfcctgcttc | 49 | 349 |
| 28931 | Coding | 570 | cctgagttgtcactgggt | 71 | 350 |
| 28932 | Coding | 666 | ccgaaagtgcccttgccc | 64 | 351 |
| 28933 | Coding | 744 | gccacgatgacttccttc | 66 | 352 |
| 28934 | Coding | 927 | cggtcctcggagaacaca | 77 | 353 |
| 28935 | Coding | 990 | acgttcttctccgagtgc | 89 | 354 |
| 28936 | Coding | 1116 | gtgccgcaaaaggtcttc | 61 | 355 |
| 28937 | Coding | 1125 | tactcaggtgtgccgcaa | 74 | 356 |
| 28938 | Coding | 1461 | ggcttgaagggtgggctg | 54 | 357 |
| 28939 | Coding | 1497 | tcaaaatacctggtgtca | 78 | 358 |
| 28940 | Coding | 1512 | gccgtgaactcctcatca | 88 | 359 |
| 28941 | Coding | 1541 | ggtcaggtggtgtgatgg | 71 | 360 |
| 28942 | Coding | 1573 | ctcgctgtccacacactc | 83 | 361 |
| 28943 | 3' UTR | 1671 | gcctctccatccctccaa | 86 | 362 |
| 28944 | 3' UTR | 1739 | acagcgtggcttctctca | 73 | 363 |
| 28945 | 3' UTR | 1814 | ttttcttccctaccccgc | 77 | 364 |
| 28946 | 3' UTR | 1819 | gatagttttcttccctac | 43 | 365 |
| 28947 | 3' UTR | 1831 | taaaacccgcaggatagt | 64 | 366 |
| 28948 | 3' UTR | 1856 | ggagaacaaactggatga | 70 | 367 |
| 28949 | 3' UTR | 1987 | ctggctgacagagtgagg | 90 | 368 |
| 28950 | 3' UTR | 1991 | gcggctggctgacagagt | 82 | 369 |
| 28951 | 3' UTR | 2031 | cccagagagatgacagat | 53 | 370 |
| 28952 | 3' UTR | 2127 | gctgctgtgtgcctgcca | 80 | 371 |
| 28953 | 3' UTR | 2264 | cataatacacaataacaa | 48 | 372 |
| 28954 | 3' UTR | 2274 | atttgaacaacataatac | 39 | 373 |
| 28955 | 3' UTR | 2397 | aagtgctaccgtggagag | 38 | 374 |
| 28956 | 3' UTR | 2407 | cgaaaaggtcaagtgcta | 83 | 375 |
| 28957 | 3' UTR | 2453 | cagggagtcagggagggc | 59 | 376 |
| 28958 | 3' UTR | 2545 | aaagttgaatgttgtaaa | 25 | 377 |
| 28959 | 3' UTR | 2553 | aaaatactaaagttgaat | 45 | 378 |

## Claims

1. An automated method of generating a set of oligonucleotides that modulate the expression of a target nucleic acid sequence via binding of said oligonucleotides with said target nucleic acid sequence, comprising the steps of:
(a) generating a library of nucleobase sequences *in silico* according to defined criteria, wherein said defined criteria are a thermodynamic property and at least one other criterion selected from accessibility of a target nucleic acid sequence, targeting to functional regions of said target nucleic acid sequence and uniform distribution across said target nucleic acid sequence, and registering said library of nucleobase sequences as data in a database;
(b) using said data to robotically synthesize a plurality of synthetic oligonucleotides having at least some of said nucleobase sequences, and registering criteria related to said synthetic oligonucleotides as additional data in said database;
(c) robotically assaying said plurality of synthetic oligonucleotides for modulation of expression of said target nucleic acid sequence, and registering criteria related to said assay as further additional data in said database; and
(d) using the results of step (c) as input for step (a) in at least one further iteration of steps (a), (b) and (c).

2. The method of claim 1, further comprising the step of choosing an oligonucleotide chemistry between steps (a) and (b), and wherein said synthesizing step (b) comprises robotically synthesizing a set of synthetic oligonucleotides having said nucleobase sequences of step (a) and the chosen oligonucleotide chemistry.

3. The method of claim 1 or claim 2, wherein said target nucleic acid sequence is that of a genomic DNA, a cDNA, a product of a polymerase chain reaction, an expressed sequence tag, an mRNA or a structural RNA.

4. The method of any one of claims 1-3, wherein said target nucleic acid sequence is a human nucleic acid sequence.

5. The method of any one of claims 1-4, comprising the further step of selecting a subset of said set of synthetic oligonucleotides having a desired level of modulation of expression of said target nucleic acid sequence.

6. The method of any one of claims 1-5 wherein in step (c) said plurality of synthetic oligonucleotides is robotically assayed by computer-controlled polymerase chain reaction or by computer-controlled enzyme-linked immunosorbent assay.

7. Use of the method of any one of claims 1-6 to identify nucleic acid sequences amenable to antisense binding of oligonucleotides.

## Patentansprüche

1. Ein automatisiertes Verfahren zur Erzeugung einer Reihe von Oligonukleotiden, welche die Expression einer Ziel-Nukleinsäuresequenz durch Bindung von genannten Oligonukleotiden an die genannte Ziel-Nukleinsäuresequenz modulieren, umfassend die folgenden Schritte:
(a) Erzeugung einer Sammlung von Nukleinbasesequenzen *in silico* gemäß definierter Kriterien, wobei die genannten definierten Kriterien eine thermodynamische Eigenschaft und mindestens ein weiteres Kriterium sind, welches von verfügbaren für eine Ziel-Nukleinsäuresequenz ausgewählt ist, wobei es auf funktionale Bereiche der genannten Ziel-Nukleinsäuresequenz und Gleichverteilung über die genannte Ziel-Nukleinsäuresequenz gerichtet ist, und genannte Sammlung von Nukleinbasesequenzen als Daten in einer Datenbank gespeichert sind;
(b) Verwendung genannter Daten zur automatisierten Herstellung einer Vielzahl synthetischer Oligonukleotide, welche mindestens einige der genannten Nukleinbasensequenzen aufweisen, und die Speicherung von auf die genannten synthetischen Oligonukleotide bezogenen Kriterien als zusätzliche Daten in der genannten Datenbank;
(c) automatisierte Testung der genannten synthetischen Oligonukleotide zur Modulierung der Expression der genannten Ziel-Nukleinsäuresequenz, und die Speicherung von auf den genannten Test bezogenen Kriterien als weitere zusätzliche Daten in der genannten Datenbank; und
(d) Verwendung von Resultaten von Schritt (c) als Eingabe für Schritt (a) in mindestens einer weiteren Iteration der Schritte (a), (b) und (c).

2. Das Verfahren nach Anspruch 1, das weiterhin den Verfahrensschritt der Auswahl einer Oligonukleotidchemie zwischen den Schritten (a) und (b) aufweist, und wobei der genannte Syntheseschritt (b) eine automatisierte Synthese einer Reihe von synthetischen Oligonukleotiden umfasst, welche die genannten Nukleinbasensequenzen von Schritt (a) und die ausgewählte Oligonukleotidchemie aufweisen.

3. Das Verfahren nach Anspruch 1 oder Anspruch 2, wobei die genannte Ziel-Nukleinsäuresequenz die einer genomischen DNA, einer cDNA, eines Produkts einer Polymerase-Kettenreaktion, eines exprimierten Sequenz-Tags, einer mRNA oder einer strukturellen RNA ist.

4. Das Verfahren nach einem der Ansprüche 1-3, wobei die genannte Ziel-Nukleinsäuresequenz eine humane Nukleinsäuresequenz ist.

5. Das Verfahren nach einem der Ansprüche 1-4, welches den zusätzlichen Schritt der Auswahl einer Untermenge der genannten Reihe von synthetischen Oligonukleotiden aufweist, welche einen erwünschten Grad an Modulierung der Expression der genannten Ziel-Nukleinsäuresequenz haben.

6. Das Verfahren nach einem der Ansprüche 1-5, wobei im Schritt (c) die genannte Vielzahl der synthetischen Oligonukleotide durch eine computergesteuerte Polymerase-Kettenreaktion oder durch einen computergesteuerten Enzyme-linked Immunosorbent Assay getestet wird.

7. Das Verwendung des Verfahrens nach einem der Ansprüche 1-6 zur Identifizierung von Nukleinsäuresequenzen, die für Antisense-Bindung von Oligonukleotiden geeignet sind.

## Revendications

1. Procédé automatisé de formation d'un ensemble d'oligonucléotides qui modulent l'expression d'une séquence cible d'acide nucléique par l'intermédiaire de la fixation des oligonucléotides à la séquence cible d'acide nucléique, comprenant les stades dans lesquels :
(a) on forme une banque de séquences de nucléobase in silico suivant des critères définis, les critères définis étant une propriété thermodynamique et au moins un autre critère choisi parmi l'accessibilité d'une séquence cible d'acide nucléique, le ciblage à des régions fonctionnelles de la séquence cible d'acide nucléique et une répartition uniforme dans la séquence cible d'acide nucléique et on enregistre la banque de séquences de nucléobase sous forme de données dans une base de données;
(b) on utilise les données pour synthétiser robotiquement une pluralité d'oligonucléotides synthétiques ayant au moins certaines des séquences de nucléobase et on enregistre des critères reliés aux oligonucléotides synthétiques en tant que données supplémentaires dans la base de données ;
(c) on soumet robotiquement la pluralité d'oligonucléotides synthétiques à un essai de modulation d'expression de la séquence cible d'acide nucléique et on enregistre des critères reliés à l'essai comme autres données supplémentaires dans la base de données ; et
(d) on utilise les résultats du stade (c) comme entrée pour la stade (a) dans au moins une autre itération des stades (a), (b) et (c).

2. Procédé suivant la revendication 1 comprenant, en outre, le stade de choisir une chimie d'oligonucléotide entre les stades (a) et (b) et dans lequel, dans le stade (b) de synthèse, on synthétise robotiquement un jeu d'oligonucléotides synthétiques ayant les séquences de nucléobase du stade (a) et la chimie d'oligonucléotide qui a été choisie.

3. Procédé suivant la revendication 1 ou la revendication 2, dans lequel la séquence cible d'acide nucléique est celle d'un ADN génomique, d'un ADNc, d'un produit d'une réaction en chaîne de la polymérase, une étiquette de séquence exprimée, un ARNm ou un ARN structural.

4. Procédé suivant l'une quelconque des revendications 1 à 3, dans lequel la séquence cible d'acide nucléique est une séquence humaine d'acide nucléique.

5. Procédé suivant l'une quelconque des revendications 1 à 4, comprenant le stade supplémentaire de sélection d'un sous-ensemble de l'ensemble d'oligonucléotides synthétiques ayant un niveau souhaité de modulation d'expression de la séquence cible d'acide nucléique.

6. Procédé suivant l'une quelconque des revendications 1 à 5, dans lequel au stade (c) on soumet robotiquement la pluralité d'oligonucléotides synthétiques à un essai par réaction en chaîne de la polymérase commandée par ordinateur ou par un essai d'immunosorbant lié à un enzyme commandé par ordinateur.

7. Utilisation du procédé suivant l'une quelconque des revendications 1 à 6 pour identifier des séquences d'acide nucléique pouvant être amenées à une fixation antisense d'oligonucléotides.


### SEQUENCE LISTING

<110> ISIS Pharmaceuticals, Inc.
   Cowsert, Lex M.
   Baker, Brenda F.
   McNeil, John
   Freier, Susan M.
   Sasmor, Henri M.
   Brooks, Douglas G.
   Ohashi, Cara
   Wyatt, Jacqueline R.
   Borchers, Alexander
   Vickers, Timothy A. ,
<120> Identification of Genetic Targets for Modulation by Oligonucleotides and Generation of Oligonucleotides for Gene Modulation
<130> ISIS-3456
<140>
   <141>
<150> US 09/067,638
   <151> 1998-04-28
<150> US 60/081,483
   <151> 1998-04-13
<160> 372
<210> 1
   <211> 18
   <212> DNA
   <213> Artificial Sequence
<223> Antisense Oligonucleotide
<400> 1
   ccaggcggca ggaccact 18
<210> 2
   <211> 18
   <212> DNA
   <213> Artificial Sequence
<223> Antisense Oligonucleotide
<400> 2
   gaccaggcgg caggacca 18
<210> 3
   <211> 18
   <212> DNA
   <213> Artificial Sequence
<223> Antisense Oligonucleotide
<400> 3
   aggtgagacc aggcggca 18
<210> 4
   <211> 18
   <212> DNA
   <213> Artificial Sequence
<223> Antisense Oligonucleotide
<400> 4
   cagaggcaga cgaaccat 18
<210> 5
   <211> 18
   <212> DNA
   <213> Artificial Sequence
<223> Antisense Oligonucleotide
<400> 5
   gcagaggcag acgaacca 18
<210> 6
   <211> 18
   <212> DNA
   <213> Artificial Sequence
<223> Antisense Oligonucleotide
<400> 6
   gcaagcagcc ccagagga 18
<210> 7
   <211> 18
   <212> DNA
   <213> Artificial Sequence
<223> Antisense Oligonucleotide
<400> 7
   ggtcagcaag cagcccca 18
<210> 8
   <211> 18
   <212> DNA
   <213> Artificial Sequence
<223> Antisense Oligonucleotide
<400> 8
   gacagcggtc agcaagca 18
<210> 9
   <211> 18
   <212> DNA
   <213> Artificial Sequence
<223> Antisense Oligonucleotide
<400> 9
   gatggacagc ggtcagca 18
<210> 10
   <211> 18
   <212> DNA
   <213> Artificial Sequence
<223> Antisense Oligonucleotide
<400> 10
   tctggatgga cagcggtc 18
<210> 11
   <211> 18
   <212> DNA
   <213> Artificial Sequence
<223> Antisense Oligonucleotide
<400> 11
   ggtggttctg gatggaca 18
<210> 12
   <211> 18
   <212> DNA
   <213> Artificial Sequence
<223> Antisense Oligonucleotide
<400> 12
   gtgggtggtt ctggatgg 18
<210> 13
   <211> 18
   <212> DNA
   <213> Artificial Sequence
<223> Antisense Oligonucleotide
<400> 13
   gcagtgggtg gttctgga 18
<210> 14
   <211> 18
   <212> DNA
   <213> Artificial Sequence
<223> Antisense Oligonucleotide
<400> 14
   cacaaagaac agcactga 18
<210> 15
   <211> 18
   <212> DNA
   <213> Artificial Sequence
<223> Antisense Oligonucleotide
<400> 15
   ctggcacaaa gaacagca 18
<210> 16
   <211> 18
   <212> DNA
   <213> Artificial Sequence
<223> Antisense Oligonucleotide
<400> 16
   tcctggctgg cacaaaga 18
<210> 17
   <211> 18
   <212> DNA
   <213> Artificial Sequence
<223> Antisense Oligonucleotide
<400> 17
   ctgtcctggc tggcacaa 18
<210> 18
   <211> 18
   <212> DNA
   <213> Artificial Sequence
<223> Antisense Oligonucleotide
<400> 18
   ctcaccagtt tctgtcct 18
<210> 19
   <211> 18
   <212> DNA
   <213> Artificial Sequence
<223> Antisense Oligonucleotide
<400> 19
   tcactcacca gtttctgt 18
<210> 20
   <211> 18
   <212> DNA
   <213> Artificial Sequence
<223> Antisense Oligonucleotide
<400> 20
   gtgcagtcac tcaccagt 18
<210> 21
   <211> 18
   <212> DNA
   <213> Artificial Sequence
<223> Antisense Oligonucleotide
<400> 21
   actctgtgca gtcactca 18
<210> 22
   <211> 18
   <212> DNA
   <213> Artificial Sequence
<223> Antisense Oligonucleotide
<400> 22
   cagtgaactc tgtgcagt 18
<210> 23
   <211> 18
   <212> DNA
   <213> Artificial Sequence
<223> Antisense Oligonucleotide
<400> 23
   attccgtttc agtgaact 18
<210> 24
   <211> 18
   <212> DNA
   <213> Artificial Sequence
<223> Antisense Oligonucleotide
<400> 24
   gaaggcattc cgtttcag 18
<210> 25
   <211> 18
   <212> DNA
   <213> Artificial Sequence
<223> Antisense Oligonucleotide
<400> 25
   ttcaccgcaa ggaaggca 18
<210> 26
   <211> 18
   <212> DNA
   <213> Artificial Sequence
<223> Antisense Oligonucleotide
<400> 26
   ctctgttcca ggtgtcta 18
<210> 27
   <211> 18
   <212> DNA
   <213> Artificial Sequence
<223> Antisense Oligonucleotide
<400> 27
   ctggtggcag tgtgtctc 18
<210> 28
   <211> 18
   <212> DNA
   <213> Artificial Sequence
<223> Antisense Oligonucleotide
<400> 28
   tggggtcgca gtatttgt 18
<210> 29
   <211> 18
   <212> DNA
   <213> Artificial Sequence
<223> Antisense Oligonucleotide
<400> 29
   ggttggggtc gcagtatt 18
<210> 30
   <211> 18
   <212> DNA
   <213> Artificial Sequence
<223> Antisense Oligonucleotide
<400> 30
   ctaggttggg gtcgcagt 18
<210> 31
   <211> 18
   <212> DNA
   <213> Artificial Sequence
<223> Antisense Oligonucleotide
<400> 31
   ggtgcccttc tgctggac 18
<210> 32
   <211> 18
   <212> DNA
   <213> Artificial Sequence
<223> Antisense Oligonucleotide
<400> 32
   ctgaggtgcc cttctgct 18
<210> 33
   <211> 18
   <212> DNA
   <213> Artificial Sequence
<223> Antisense Oligonucleotide
<400> 33
   gtgtctgttt ctgaggtg 18
<210> 34
   <211> 18
   <212> DNA
   <213> Artificial Sequence
<223> Antisense Oligonucleotide
<400> 34
   tggtgtctgt ttctgagg 18
<210> 35
   <211> 18
   <212> DNA
   <213> Artificial Sequence
<223> Antisense Oligonucleotide
<400> 35
   acaggtgcag atggtgtc 18
<210> 36
   <211> 18
   <212> DNA
   <213> Artificial Sequence
<223> Antisense Oligonucleotide
<400> 36
   ttcacaggtg cagatggt 18
<210> 37
   <211> 18
   <212> DNA
   <213> Artificial Sequence
<223> Antisense Oligonucleotide
<400> 37
   gtgccagcct tcttcaca 18
<210> 38
   <211> 18
   <212> DNA
   <213> Artificial Sequence
<223> Antisense Oligonucleotide
<400> 38
   tacagtgcca gccttctt 18
<210> 39
   <211> 18
   <212> DNA
   <213> Artificial Sequence
<223> Antisense Oligonucleotide
<400> 39
   ggacacagct ctcacagg 18
<210> 40
   <211> 18
   <212> DNA
   <213> Artificial Sequence
<223> Antisense Oligonucleotide
<400> 40
   tgcaggacac agctctca 18
<210> 41
   <211> 18
   <212> DNA
   <213> Artificial Sequence
<223> Antisense Oligonucleotide
<400> 41
   gagcggtgca ggacacag 18
<210> 42
   <211> 18
   <212> DNA
   <213> Artificial Sequence
<223> Antisense Oligonucleotide
<400> 42
   aagccgggcg agcatgag 18
<210> 43
   <211> 18
   <212> DNA
   <213> Artificial Sequence
<223> Antisense Oligonucleotide
<400> 43
   aatctgcttg accccaaa 18
<210> 44
   <211> 18
   <212> DNA
   <213> Artificial Sequence
<223> Antisense Oligonucleotide
<400> 44
   gaaacccctg tagcaatc 18
<210> 45
   <211> 18
   <212> DNA
   <213> Artificial Sequence
<223> Antisense Oligonucleotide
<400> 45
   gtatcagaaa cccctgta 18
<210> 46
   <211> 18
   <212> DNA
   <213> Artificial Sequence
<223> Antisense Oligonucleotide
<400> 46
   gctcgcagat ggtatcag 18
<210> 47
   <211> 18
   <212> DNA
   <213> Artificial Sequence
<223> Antisense Oligonucleotide
<400> 47
   gcagggctcg cagatggt 18
<210> 48
   <211> 18
   <212> DNA
   <213> Artificial Sequence
<223> Antisense Oligonucleotide
<400> 48
   tgggcagggc tcgcagat 18
<210> 49
   <211> 18
   <212> DNA
   <213> Artificial Sequence
<223> Antisense Oligonucleotide
<400> 49
   gactgggcag ggctcgca 18
<210> 50
   <211> 18
   <212> DNA
   <213> Artificial Sequence
<223> Antisense Oligonucleotide
<400> 50
   cattggagaa gaagccga 18
<210> 51
   <211> 18
   <212> DNA
   <213> Artificial Sequence
<223> Antisense Oligonucleotide
<400> 51
   gatgacacat tggagaag 18
<210> 52
   <211> 18
   <212> DNA
   <213> Artificial Sequence
<223> Antisense Oligonucleotide
<400> 52
   gcagatgaca cattggag 18
<210> 53
   <211> 18
   <212> DNA
   <213> Artificial Sequence
<223> Antisense Oligonucleotide
<400> 53
   tcgaaagcag atgacaca 18
<210> 54
   <211> 18
   <212> DNA
   <213> Artificial Sequence
<223> Antisense Oligonucleotide
<400> 54
   gtccaagggt gacatttt 18
<210> 55
   <211> 18
   <212> DNA
   <213> Artificial Sequence
<223> Antisense Oligonucleotide
<400> 55
   cacagcttgt ccaagggt 18
<210> 56
   <211> 18
   <212> DNA
   <213> Artificial Sequence
<223> Antisense Oligonucleotide
<400> 56
   ttggtctcac agcttgtc 18
<210> 57
   <211> 18
   <212> DNA
   <213> Artificial Sequence
<223> Antisense Oligonucleotide
<400> 57
   caggtctttg gtctcaca 18
<210> 58
   <211> 18
   <212> DNA
   <213> Artificial Sequence
<223> Antisense Oligonucleotide
<400> 58
   ctgttgcaca accaggtc 18
<210> 59
   <211> 18
   <212> DNA
   <213> Artificial Sequence
<223> Antisense Oligonucleotide
<400> 59
   gtttgtgcct gcctgttg 18
<210> 60
   <211> 18
   <212> DNA
   <213> Artificial Sequence
<223> Antisense Oligonucleotide
<400> 60
   gtcttgtttg tgcctgcc 18
<210> 61
   <211> 18
   <212> DNA
   <213> Artificial Sequence
<223> Antisense Oligonucleotide
<400> 61
   ccacagacaa catcagtc 18
<210> 62
   <211> 18
   <212> DNA
   <213> Artificial Sequence
<223> Antisense Oligonucleotide
<400> 62
   ctggggacca cagacaac 18
<210> 63
   <211> 18
   <212> DNA
   <213> Artificial Sequence
<223> Antisense Oligonucleotide
<400> 63
   tcagccgatc ctggggac 18
<210> 64
   <211> 18
   <212> DNA
   <213> Artificial Sequence
<223> Antisense Oligonucleotide
<400> 64
   caccaccagg gctctcag 18
<210> 65
   <211> 18
   <212> DNA
   <213> Artificial Sequence
<223> Antisense Oligonucleotide
<400> 65
   gggatcacca ccagggct 18
<210> 66
   <211> 18
   <212> DNA
   <213> Artificial Sequence
<223> Antisense Oligonucleotide
<400> 66
   gaggatggca aacaggat 18
<210> 67
   <211> 18
   <212> DNA
   <213> Artificial Sequence
<223> Antisense Oligonucleotide
<400> 67
   accagcacca agaggatg 18
<210> 68
   <211> 18
   <212> DNA
   <213> Artificial Sequence
<223> Antisense Oligonucleotide
<400> 68
   ttttgataaa gaccagca 18
<210> 69
   <211> 18
   <212> DNA
   <213> Artificial Sequence
<223> Antisense Oligonucleotide
<400> 69
   tattggttgg cttcttgg 18
<210> 70
   <211> 18
   <212> DNA
   <213> Artificial Sequence
<223> Antisense Oligonucleotide
<400> 70
   gggttcctgc ttggggtg 18
<210> 71
   <211> 18
   <212> DNA
   <213> Artificial Sequence
<223> Antisense Oligonucleotide
<400> 71
   gtcgggaaaa ttgatctc 18
<210> 72
   <211> 18
   <212> DNA
   <213> Artificial Sequence
<223> Antisense Oligonucleotide
<400> 72
   gatcgtcggg aaaattga 18
<210> 73
   <211> 18
   <212> DNA
   <213> Artificial Sequence
<223> Antisense Oligonucleotide
<400> 73
   ggagccagga agatcgtc 18
<210> 74
   <211> 18
   <212> DNA
   <213> Artificial Sequence
<223> Antisense Oligonucleotide
<400> 74
   tggagccagg aagatcgt 18
<210> 75
   <211> 18
   <212> DNA
   <213> Artificial Sequence
<223> Antisense Oligonucleotide
<400> 75
   tggagcagca gtgttgga 18
<210> 76
   <211> 18
   <212> DNA
   <213> Artificial Sequence
<223> Antisense Oligonucleotide
<400> 76
   gtaaagtctc ctgcactg 18
<210> 77
   <211> 18
   <212> DNA
   <213> Artificial Sequence
<223> Antisense Oligonucleotide
<400> 77
   tggcatccat gtaaagtc 18
<210> 78
   <211> 18
   <212> DNA
   <213> Artificial Sequence
<223> Antisense Oligonucleotide
<400> 78
   cggttggcat ccatgtaa 18
<210> 79
   <211> 18
   <212> DNA
   <213> Artificial Sequence
<223> Antisense Oligonucleotide
<400> 79
   ctctttgcca tcctcctg 18
<210> 80
   <211> 18
   <212> DNA
   <213> Artificial Sequence
<223> Antisense Oligonucleotide
<400> 80
   ctgtctctcc tgcactga 18
<210> 81
   <211> 18
   <212> DNA
   <213> Artificial Sequence
<223> Antisense Oligonucleotide
<400> 81
   ggtgcagcct cactgtct 18
<210> 82
   <211> 18
   <212> DNA
   <213> Artificial Sequence
<223> Antisense Oligonucleotide
<400> 82
   aactgcctgt ttgcccac 18
<210> 83
   <211> 18
   <212> DNA
   <213> Artificial Sequence
<223> Antisense Oligonucleotide
<400> 83
   cttctgcctg cacccctg 18
<210> 84
   <211> 18
   <212> DNA
   <213> Artificial Sequence
<223> Antisense Oligonucleotide
<400> 84
   actgactggg catagctc 18
<210> 85
   <211> 1004
   <212> DNA
   <213> Homo sapiens
<220>
   <221> CDS
   <222> (48)..(881)
<400> 85
   gcctcgctcg ggcgcccagt ggtcctgccg cctggtctca cctcgcc atg gtt cgt      56
                                                       Met Val Arg
                                                        1
   ctg cct ctg cag tgc gtc ctc tgg ggc tgc ttg ctg acc gct gtc cat     104
   Leu Pro Leu Gln Cys Val Leu Trp Gly Cys Leu Leu Thr Ala Val His
   5 10 15
   cca gaa cca ccc act gca tgc aga gaa aaa cag tac cta ata aac agt 152
   Pro Glu Pro Pro Thr Ala Cys Arg Glu Lys Gln Tyr Leu Ile Asn Ser
   20 25 30 35
   cag tgc tgt tct ttg tgc cag cca gga cag aaa ctg gtg agt gac tgc 200
   Gln Cys Cys Ser Leu Cys Gln Pro Gly Gln Lys Leu Val Ser Asp Cys
   40 45 50
   aca gag ttc act gaa acg gaa tgc ctt cct tgc ggt gaa agc gaa ttc 248
   Thr Glu Phe Thr Glu Thr Glu Cys Leu Pro Cys Gly Glu Ser Glu Phe
   55 60 65
   cta gac acc tgg aac aga gag aca cac tgc cac cag cac aaa tac tgc 296
   Leu Asp Thr Trp Asn Arg Glu Thr His Cys His Gln His Lys Tyr Cys
   70 75 80
   gac ccc aac cta ggg ctt cgg gtc cag cag aag ggc acc tca gaa aca 344
   Asp Pro Asn Leu Gly Leu Arg Val Gln Gln Lys Gly Thr Ser Glu Thr
   85 90 95
   gac acc atc tgc acc tgt gaa gaa ggc tgg cac tgt acg agt gag gcc 392
   Asp Thr Ile Cys Thr Cys Glu Glu Gly Trp His Cys Thr Ser Glu Ala
   100 105 110 115
   tgt gag agc tgt gtc ctg cac cgc tca tgc tcg ccc ggc ttt ggg gtc 440
   Cys Glu Ser Cys Val Leu His Arg Ser Cys Ser Pro Gly Phe Gly Val
   120 125 130
   aag cag att gct aca ggg gtt tct gat acc atc tgc gag ccc tgc cca 488
   Lys Gln Ile Ala Thr Gly Val Ser Asp Thr Ile Cys Glu Pro Cys Pro
   135 140 145
   gtc ggc ttc ttc tcc aat gtg tca tct gct ttc gaa aaa tgt cac cct 536
   Val Gly Phe Phe Ser Asn Val Ser Ser Ala Phe Glu Lys Cys His Pro
   150 155 160
   tgg aca agc tgt gag acc aaa gac ctg gtt gtg caa cag gca ggc aca 584
   Trp Thr Ser Cys Glu Thr Lys Asp Leu Val Val Gln Gln Ala Gly Thr
   165 170 175
   aac aag act gat gtt gtc tgt ggt ccc cag gat cgg ctg aga gcc ctg 632
   Asn Lys Thr Asp Val Val Cys Gly Pro Gln Asp Arg Leu Arg Ala Leu
   180 185 190 195
   gtg gtg atc ccc atc atc ttc ggg atc ctg ttt gcc atc ctc ttg gtg 680
   Val Val Ile Pro Ile Ile Phe Gly Ile Leu Phe Ala Ile Leu Leu Val
   200 205 210
   ctg gtc ttt atc aaa aag gtg gcc aag aag cca acc aat aag gcc ccc 728
   Leu Val Phe Ile Lys Lys Val Ala Lys Lys Pro Thr Asn Lys Ala Pro
   215 220 225
   cac ccc aag cag gaa ccc cag gag atc aat ttt ccc gac gat ctt cct 776
   His Pro Lys Gln Glu Pro Gln Glu Ile Asn Phe Pro Asp Asp Leu Pro
   230 235 240
   ggc tcc aac act gct gct cca gtg cag gag act tta cat gga tgc caa 824
   Gly Ser Asn Thr Ala Ala Pro Val Gln Glu Thr Leu His Gly Cys Gln
   245 250 255
   ccg gtc acc cag gag gat ggc aaa gag agt cgc atc tca gtg cag gag 872
   Pro Val Thr Gln Glu Asp Gly Lys Glu Ser Arg Ile Ser Val Gln Glu
   260 265 270 275
   aga cag tga ggctgcaccc acccaggagt gtggccacgt gggcaaacag 921
   Arg Gln
   gcagttggcc agagagcctg gtgctgctgc tgcaggggtg caggcagaag cggggagcta 981
   tgcccagtca gtgccagccc ctc 1004
<210> 86
   <211> 23
   <212> DNA
   <213> Artificial Sequence
<223> PCR Primer
<400> 86
   cagagttcac tgaaacggaa tgc 23
<210> 87
   <211> 23
   <212> DNA
   <213> Artificial Sequence
<223> PCR Primer
<400> 87
   ggtggcagtg tgtctctctg ttc 23
<210> 88
   <211> 25
   <212> DNA
   <213> Artificial Sequence
<223> PCR Probe
<400> 88
   ttccttgcgg tgaaagcgaa ttcct 25
<210> 89
   <211> 19
   <212> DNA
   <213> Artificial Sequence
<223> PCR Primer
<400> 89
   gaaggtgaag gtcggagtc 19
<210> 90
   <211> 20
   <212> DNA
   <213> Artificial Sequence
<223> PCR Primer
<400> 90
   gaagatggtg atgggatttc 20
<210> 91
   <211> 20
   <212> DNA
   <213> Artificial Sequence
<223> PCR Probe
<400> 91
   caagcttccc gttctcagcc 20
<210> 92
   <211> 20
   <212> DNA
   <213> Artificial Sequence
<223> Assembled Target Region
<400> 92
   agtggtcctg ccgcctggtc 20
<210> 93
   <211> 18
   <212> DNA
   <213> Artificial Sequence
<223> Antisense Oligonucleotide
<400> 93
   gaacagcact gactgttt 18
<210> 94
   <211> 18
   <212> DNA
   <213> Artificial Sequence
<223> Antisense Oligonucleotide
<400> 94
   agaacagcac tgactgtt 18
<210> 95
   <211> 18
   <212> DNA
   <213> Artificial Sequence
<223> Antisense Oligonucleotide
<400> 95
   aagaacagca ctgactgt 18
<210> 96
   <211> 18
   <212> DNA
   <213> Artificial Sequence
<223> Antisense Oligonucleotide
<400> 96
   aaagaacagc actgactg 18
<210> 97
   <211> 18
   <212> DNA
   <213> Artificial Sequence
<223> Antisense Oligonucleotide
<400> 97
   caaagaacag cactgact 18
<210> 98
   <211> 18
   <212> DNA
   <213> Artificial Sequence
<223> Antisense Oligonucleotide
<400> 98
   acaaagaaca gcactgac 18
<210> 99
<400> 99
   000
<210> 100
   <211> 18
   <212> DNA
   <213> Artificial Sequence
<223> Antisense Oligonucleotide
<400> 100
   gcacaaagaa cagcactg 18
<210> 101
   <211> 18
   <212> DNA
   <213> Artificial Sequence
<223> Antisense Oligonucleotide
<400> 101
   ggcacaaaga acagcact 18
<210> 102
   <211> 18
   <212> DNA
   <213> Artificial Sequence
<223> Antisense Oligonucleotide
<400> 102
   tggcacaaag aacagcac 18
<210> 103
   <211> 18
   <212> DNA
   <213> Artificial Sequence
<223> Antisense Oligonucleotide
<400> 103
   gctggcacaa agaacagc 18
<210> 104
   <211> 18
   <212> DNA
   <213> Artificial Sequence
<223> Antisense Oligonucleotide
<400> 104
   ggctggcaca aagaacag 18
<210> 105
   <211> 18
   <212> DNA
   <213> Artificial Sequence
<223> Antisense Oligonucleotide
<400> 105
   tggctggcac aaagaaca 18
<210> 106
   <211> 18
   <212> DNA
   <213> Artificial Sequence
<223> Antisense Oligonucleotide
<400> 106
   ctggctggca caaagaac 18
<210> 107
   <211> 18
   <212> DNA
   <213> Artificial Sequence
<223> Antisense Oligonucleotide
<400> 107
   cctggctggc acaaagaa 18
<210> 108
<400> 108
   000
<210> 109
   <211> 18
   <212> DNA
   <213> Artificial Sequence
<223> Antisense Oligonucleotide
<400> 109
   gtcctggctg gcacaaag 18
<210> 110
   <211> 18
   <212> DNA
   <213> Artificial Sequence
<223> Antisense Oligonucleotide
<400> 110
   tgtcctggct ggcacaaa 18
<210> 111
<400> 111
   000
<210> 112
   <211> 18
   <212> DNA
   <213> Artificial Sequence
<223> Antisense Oligonucleotide
<400> 112
   tctgtcctgg ctggcaca 18
<210> 113
   <211> 1058
   <212> DNA
   <213> Homo sapiens
<220>
   <221> CDS
   <222> (77)..(658)
<400> 113
   gccttgactt catctcagct ccagagcccg ccctctcttc ctgcagcctg ggaacttcag 60
   ccggctggag cccacc atg gct gca atc cga aag aag ctg gtg atc gtt 109
   Met Ala Ala Ile Arg Lys Lys Leu Val Ile Val
   1 5 10
   ggg gat ggt gcc tgt ggg aag acc tgc ctc ctc atc gtc ttc agc aag 157
   Gly Asp Gly Ala Cys Gly Lys Thr Cys Leu Leu Ile Val Phe Ser Lys
   15 20 25
   gat cag ttt ccg gag gtc tac gtc cct act gtc ttt gag aac tat att 205
   Asp Gln Phe Pro Glu Val Tyr Val Pro Thr Val Phe Glu Asn Tyr Ile
   30 35 40
   gcg gac att gag gtg gac ggc aag cag gtg gag ctg gct ctg tgg gac 253
   Ala Asp Ile Glu Val Asp Gly Lys Gln Val Glu Leu Ala Leu Trp Asp
   45 50 55
   aca gca ggg cag gaa gac tat gat cga ctg cgg cct ctc tcc tac ccg 301
   Thr Ala Gly Gln Glu Asp Tyr Asp Arg Leu Arg Pro Leu Ser Tyr Pro
   60 65 70 75
   gac act gat gtc atc ctc atg tgc ttc tcc atc gac agc cct gac agc 349
   Asp Thr Asp Val Ile Leu Met Cys Phe Ser Ile Asp Ser Pro Asp Ser
   80 85 90
   ctg gaa aac att cct gag aag tgg acc cca gag gtg aag cac ttc tgc 397
   Leu Glu Asn Ile Pro Glu Lys Trp Thr Pro Glu Val Lys His Phe Cys
   95 100 105
   ccc aac gtg ccc atc atc ctg gtg ggg aat aag aag gac ctg agg caa 445
   Pro Asn Val Pro Ile Ile Leu Val Gly Asn Lys Lys Asp Leu Arg Gln
   110 115 120
   gac gag cac acc agg aga gag ctg gcc aag atg aag cag gag ccc gtt 493
   Asp Glu His Thr Arg Arg Glu Leu Ala Lys Met Lys Gln Glu Pro Val
   125 130 135
   cgg tct gag gaa ggc cgg gac atg gcg aac cgg atc agt gcc ttt ggc 541
   Arg Ser Glu Glu Gly Arg Asp Met Ala Asn Arg Ile Ser Ala Phe Gly
   140 145 150 155
   tac ctt gag tgc tca gcc aag acc aag gag gga gtg cgg gag gtg ttt 589
   Tyr Leu Glu Cys Ser Ala Lys Thr Lys Glu Gly Val Arg Glu Val Phe
   160 165 170
   gag atg gcc act cgg gct ggc ctc cag gtc cgc aag aac aag cgt cgg 637
   Glu Met Ala Thr Arg Ala Gly Leu Gln Val Arg Lys Asn Lys Arg Arg
   175 180 185
   agg ggc tgt ccc att ctc tga gatcccccca aagggcccct ttcctacatg 688
   Arg Gly Cys Pro Ile Leu
   190
   ccccctccct tcacaggggt acagaaatta tccccctaca accccagcct cctgagggct 748
   ccatactgaa ggctccattt tcagttccct cctgcccagg actgcattgt tttctagccc 808
   cgaggtgtgg cacgggccct ccctcccagc gctctgggag ccacgcctat gccctgccct 868
   tcctcatggg cccctgggga tcttgcccct ttgaccttcc ccaaaggatg gtcacacacc 928
   agcactttat acacttctgg ctcacaggaa agtgtctgca gtagggaccc agagtcccag 988
   gcccctggag ttgtttctgc aggggccttg tctctcactg catttggtca ggggggcatg 1048
   aataaaggct 1058
<210> 114
   <211> 23
   <212> DNA
   <213> Artificial Sequence
<223> PCR Primer
<400> 114
   tgatgtcatc ctcatgtgct tct 23
<210> 115
   <211> 19
   <212> DNA
   <213> Artificial Sequence
<223> PCR Primer
<400> 115
   ccaggatgat gggcacgtt 19
<210> 116
   <211> 23
   <212> DNA
   <213> Artificial Sequence
<223> PCR Probe
<400> 116
   cgacagccct gacagcctgg aaa 23
<210> 117
   <211> 18
   <212> DNA
   <213> Artificial Sequence
<223> Antisense Oligonucleotide
<400> 117
   gagctgagat gaagtcaa 18
<210> 118
   <211> 18
   <212> DNA
   <213> Artificial Sequence
<223> Antisense Oligonucleotide
<400> 118
   gctgaagttc ccaggctg 18
<210> 119
   <211> 18
   <212> DNA
   <213> Artificial Sequence
<223> Antisense Oligonucleotide
<400> 119
   ccggctgaag ttcccagg 18
<210> 120
   <211> 18
   <212> DNA
   <213> Artificial Sequence
<223> Antisense Oligonucleotide
<400> 120
   ggcaccatcc ccaacgat 18
<210> 121
   <211> 18
   <212> DNA
   <213> Artificial Sequence
<223> Antisense Oligonucleotide
<400> 121
   aggcaccatc cccaacga 18
<210> 122
   <211> 18
   <212> DNA
   <213> Artificial Sequence
<223> Antisense Oligonucleotide
<400> 122
   tcccacaggc accatccc 18
<210> 123
   <211> 18
   <212> DNA
   <213> Artificial Sequence
<223> Antisense Oligonucleotide
<400> 123
   aggtcttccc acaggcac 18
<210> 124
   <211> 18
   <212> DNA
   <213> Artificial Sequence
<223> Antisense Oligonucleotide
<400> 124
   atgaggaggc aggtcttc 18
<210> 125
   <211> 18
   <212> DNA
   <213> Artificial Sequence
<223> Antisense Oligonucleotide
<400> 125
   ttgctgaaga cgatgagg 18
<210> 126
   <211> 18
   <212> DNA
   <213> Artificial Sequence
<223> Antisense Oligonucleotide
<400> 126
   tcaaagacag tagggacg 18
<210> 127
   <211> 18
   <212> DNA
   <213> Artificial Sequence
<223> Antisense Oligonucleotide
<400> 127
   ttctcaaaga cagtaggg 18
<210> 128
   <211> 18
   <212> DNA
   <213> Artificial Sequence
<223> Antisense Oligonucleotide
<400> 128
   agttctcaaa gacagtag 18
<210> 129
   <211> 18
   <212> DNA
   <213> Artificial Sequence
<223> Antisense Oligonucleotide
<400> 129
   tgttttccag gctgtcag 18
<210> 130
   <211> 18
   <212> DNA
   <213> Artificial Sequence
<223> Antisense Oligonucleotide
<400> 130
   tcgtcttgcc tcaggtcc 18
<210> 131
   <211> 18
   <212> DNA
   <213> Artificial Sequence
<223> Antisense Oligonucleotide
<400> 131
   gtgtgctcgt cttgcctc 18
<210> 132
   <211> 18
   <212> DNA
   <213> Artificial Sequence
<223> Antisense Oligonucleotide
<400> 132
   ctcctggtgt gctcgtct 18
<210> 133
   <211> 18
   <212> DNA
   <213> Artificial Sequence
<223> Antisense Oligonucleotide
<400> 133
   cagaccgaac gggctcct 18
<210> 134
   <211> 18
   <212> DNA
   <213> Artificial Sequence
<223> Antisense Oligonucleotide
<400> 134
   ttcctcagac cgaacggg 18
<210> 135
   <211> 18
   <212> DNA
   <213> Artificial Sequence
<223> Antisense Oligonucleotide
<400> 135
   actcaaggta gccaaagg 18
<210> 136
   <211> 18
   <212> DNA
   <213> Artificial Sequence
<223> Antisense Oligonucleotide
<400> 136
   ctcccgcact ccctcctt 18
<210> 137
   <211> 18
   <212> DNA
   <213> Artificial Sequence
<223> Antisense Oligonucleotide
<400> 137
   ctcaaacacc tcccgcac 18
<210> 138
   <211> 18
   <212> DNA
   <213> Artificial Sequence
<223> Antisense Oligonucleotide
<400> 138
   ggccatctca aacacctc 18
<210> 139
   <211> 18
   <212> DNA
   <213> Artificial Sequence
<223> Antisense Oligonucleotide
<400> 139
   cttgttcttg cggacctg 18
<210> 140
   <211> 18
   <212> DNA
   <213> Artificial Sequence
<223> Antisense Oligonucleotide
<400> 140
   cccctccgac gcttgttc 18
<210> 141
   <211> 18
   <212> DNA
   <213> Artificial Sequence
<223> Antisense Oligonucleotide
<400> 141
   gtatggagcc ctcaggag 18
<210> 142
   <211> 18
   <212> DNA
   <213> Artificial Sequence
<223> Antisense Oligonucleotide
<400> 142
   gagccttcag tatggagc 18
<210> 143
   <211> 18
   <212> DNA
   <213> Artificial Sequence
<223> Antisense Oligonucleotide
<400> 143
   gaaaatggag ccttcagt 18
<210> 144
   <211> 18
   <212> DNA
   <213> Artificial Sequence
<223> Antisense Oligonucleotide
<400> 144
   ggaactgaaa atggagcc 18
<210> 145
   <211> 18
   <212> DNA
   <213> Artificial Sequence
<223> Antisense Oligonucleotide
<400> 145
   ggagggaact gaaaatgg 18
<210> 146
   <211> 18
   <212> DNA
   <213> Artificial Sequence
<223> Antisense Oligonucleotide
<400> 146
   gcaggaggga actgaaaa 18
<210> 147
   <211> 18
   <212> DNA
   <213> Artificial Sequence
<223> Antisense Oligonucleotide
<400> 147
   agggcagggc ataggcgt 18
<210> 148
   <211> 18
   <212> DNA
   <213> Artificial Sequence
<223> Antisense Oligonucleotide
<400> 148
   ggaagggcag ggcatagg 18
<210> 149
   <211> 18
   <212> DNA
   <213> Artificial Sequence
<223> Antisense Oligonucleotide
<400> 149
   catgaggaag ggcagggc 18
   <210> 150
   <211> 18
   <212> DNA
   <213> Artificial Sequence
<223> Antisense Oligonucleotide
<400> 150
   taaagtgctg gtgtgtga 18
<210> 151
   <211> 18
   <212> DNA
   <213> Artificial Sequence
<223> Antisense Oligonucleotide
<400> 151
   cctgtgagcc agaagtgt 18
<210> 152
   <211> 18
   <212> DNA
   <213> Artificial Sequence
<223> Antisense Oligonucleotide
<400> 152
   ttcctgtgag ccagaagt 18
<210> 153
   <211> 18
   <212> DNA
   <213> Artificial Sequence
<223> Antisense Oligonucleotide
<400> 153
   cactttcctg tgagccag 18
<210> 154
   <211> 18
   <212> DNA
   <213> Artificial Sequence
<223> Antisense Oligonucleotide
<400> 154
   agacactttc ctgtgagc 18
<210> 155
   <211> 18
   <212> DNA
   <213> Artificial Sequence
<223> Antisense Oligonucleotide
<400> 155
   actctgggtc cctactgc 18
<210> 156
   <211> 18
   <212> DNA
   <213> Artificial Sequence
<223> Antisense Oligonucleotide
<400> 156
   tgcagaaaca actccagg 18
<210> 157
   <211> 3076
   <212> DNA
   <213> Homo sapiens
<220>
   <221> CDS
   <222> (725)..(2539)
<400> 157
   gaattcaaaa tgtcttcagt tgtaaatctt accattattt tacgtacctc taagaaataa 60
   aagtgcttct aattaaaata tgatgtcatt aattatgaaa tacttcttga taacagaagt 120
   tttaaaatag ccatcttaga atcagtgaaa tatggtaatg tattattttc ctcctttgag 180
   ttaggtcttg tgcttttttt tcctggccac taaatttcac aatttccaaa aagcaaaata 240
   aacatattct gaatattttt gctgtgaaac acttgacagc agagctttcc accatgaaaa 300
   gaagcttcat gagtcacaca ttacatcttt gggttgattg aatgccactg aaacattcta 360
   gtagcctgga gaagttgacc tacctgtgga gatgcctgcc attaaatggc atcctgatgg 420
   cttaatacac atcactcttc tgtgaagggt tttaattttc aacacagctt actctgtagc 480
   atcatgttta cattgtatgt ataaagatta tacaaaggtg caattgtgta tttcttcctt 540
   aaaatgtatc agtataggat ttagaatctc catgttgaaa ctctaaatgc atagaaataa 600
   aaataataaa aaatttttca ttttggcttt tcagcctagt attaaaactg ataaaagcaa 660
   agccatgcac aaaactacct ccctagagaa aggctagtcc cttttcttcc ccattcattt 720
   catt atg aac ata gta gaa aac agc ata ttc tta tca aat ttg atg 766
   Met Asn Ile Val Glu Asn Ser Ile Phe Leu Ser Asn Leu Met
   1 5 10
   aaa agc gcc aac acg ttt gaa ctg aaa tac gac ttg tca tgt gaa ctg 814
   Lys Ser Ala Asn Thr Phe Glu Leu Lys Tyr Asp Leu Ser Cys Glu Leu
   15 20 25 30
   tac cga atg tct acg tat tcc act ttt cct gct ggg gtt cct gtc tca 862
   Tyr Arg Met Ser Thr Tyr Ser Thr Phe Pro Ala Gly Val Pro Val Ser
   35 40 45
   gaa agg agt ctt gct cgt gct ggt ttc tat tac act ggt gtg aat gac 910
   Glu Arg Ser Leu Ala Arg Ala Gly Phe Tyr Tyr Thr Gly Val Asn Asp
   50 55 60
   aag gtc aaa tgc ttc tgt tgt ggc ctg atg ctg gat aac tgg aaa aga 958
   Lys Val Lys Cys Phe Cys Cys Gly Leu Met Leu Asp Asn Trp Lys Arg
   65 70 75
   gga gac agt cct act gaa aag cat aaa aag ttg tat cct agc tgc aga 1006
   Gly Asp Ser Pro Thr Glu Lys His Lys Lys Leu Tyr Pro Ser Cys Arg
   80 85 90
   ttc gtt cag agt cta aat tcc gtt aac aac ttg gaa gct acc tct cag 1054
   Phe Val Gln Ser Leu Asn Ser Val Asn Asn Leu Glu Ala Thr Ser Gln
   95 100 105 110
   cct act ttt cct tct tca gta aca aat tcc aca cac tca tta ctt ccg 1102
   Pro Thr Phe Pro Ser Ser Val Thr Asn Ser Thr His Ser Leu Leu Pro
   115 120 125
   ggt aca gaa aac agt gga tat ttc cgt ggc tct tat tca aac tct cca 1150
   Gly Thr Glu Asn Ser Gly Tyr Phe Arg Gly Ser Tyr Ser Asn Ser Pro
   130 135 140
   tca aat cct gta aac tcc aga gca aat caa gat ttt tct gcc ttg atg 1198
   Ser Asn Pro Val Asn Ser Arg Ala Asn Gln Asp Phe Ser Ala Leu Met
   145 150 155
   aga agt tcc tac cac tgt gca atg aat aac gaa aat gcc aga tta ctt 1246
   Arg Ser Ser Tyr His Cys Ala Met Asn Asn Glu Asn Ala Arg Leu Leu
   160 165 170
   act ttt cag aca tgg cca ttg act ttt ctg tcg cca aca gat ctg gca 1294
   Thr Phe Gln Thr Trp Pro Leu Thr Phe Leu Ser Pro Thr Asp Leu Ala
   175 180 185 190
   aaa gca ggc ttt tac tac ata gga cct gga gac aga gtg gct tgc ttt 1342
   Lys Ala Gly Phe Tyr Tyr Ile Gly Pro Gly Asp Arg Val Ala Cys Phe
   195 200 205
   gcc tgt ggt gga aaa ttg agc aat tgg gaa ccg aag gat aat gct atg 1390
   Ala Cys Gly Gly Lys Leu Ser Asn Trp Glu Pro Lys Asp Asn Ala Met
   210 215 220
   tca gaa cac ctg aga cat ttt ccc aaa tgc cca ttt ata gaa aat cag 1438
   Ser Glu His Leu Arg His Phe Pro Lys Cys Pro Phe Ile Glu Asn Gln
   225 230 235
   ctt caa gac act tca aga tac aca gtt tct aat ctg agc atg cag aca 1486
   Leu Gln Asp Thr Ser Arg Tyr Thr Val Ser Asn Leu Ser Met Gln Thr
   240 245 250
   cat gca gcc cgc ttt aaa aca ttc ttt aac tgg ccc tct agt gtt cta 1534
   His Ala Ala Arg Phe Lys Thr Phe Phe Asn Trp Pro Ser Ser Val Leu
   255 260 265 270
   gtt aat cct gag cag ctt gca agt gcg ggt ttt tat tat gtg ggt aac 1582
   Val Asn Pro Glu Gln Leu Ala Ser Ala Gly Phe Tyr Tyr Val Gly Asn
   275 280 285
   agt gat gat gtc aaa tgc ttt tgc tgt gat ggt gga ctc agg tgt tgg 1630
   Ser Asp Asp Val Lys Cys Phe Cys Cys Asp Gly Gly Leu Arg Cys Trp
   290 295 300
   gaa tct gga gat gat cca tgg gtt caa cat gcc aag tgg ttt cca agg 1678
   Glu Ser Gly Asp Asp Pro Trp Val Gln His Ala Lys Trp Phe Pro Arg
   305 310 315
   tgt gag tac ttg ata aga att aaa gga cag gag ttc atc cgt caa gtt 1726
   Cys Glu Tyr Leu Ile Arg Ile Lys Gly Gln Glu Phe Ile Arg Gln Val
   320 325 330
   caa gcc agt tac cct cat cta ctt gaa cag ctg cta tcc aca tca gac 1774
   Gln Ala Ser Tyr Pro His Leu Leu Glu Gln Leu Leu Ser Thr Ser Asp
   335 340 345 350
   agc cca gga gat gaa aat gca gag tca tca att atc cat ttt gaa cct 1822
   Ser Pro Gly Asp Glu Asn Ala Glu Ser Ser Ile Ile His Phe Glu Pro
   355 360 365
   gga gaa gac cat tca gaa gat gca atc atg atg aat act cct gtg att 1870
   Gly Glu Asp His Ser Glu Asp Ala Ile Met Met Asn Thr Pro Val Ile
   370 375 380
   aat gct gcc gtg gaa atg ggc ttt agt aga agc ctg gta aaa cag aca 1918
   Asn Ala Ala Val Glu Met Gly Phe Ser Arg Ser Leu Val Lys Gln Thr
   385 390 395
   gtt caa aga aaa atc cta gca act gga gag aat tat aga cta gtc aat 1966
   Val Gln Arg Lys Ile Leu Ala Thr Gly Glu Asn Tyr Arg Leu Val Asn
   400 405 410
   gat ctt gtg tta gac tta ctc aat gca gaa gat gaa ata agg gaa gag 2014
   Asp Leu Val Leu Asp Leu Leu Asn Ala Glu Asp Glu Ile Arg Glu Glu
   415 420 425 430
   gag aga gaa aga gca act gag gaa aaa gaa tca aat gat tta tta tta 2062
   Glu Arg Glu Arg Ala Thr Glu Glu Lys Glu Ser Asn Asp Leu Leu Leu
   435 440 445
   atc cgg aag aat aga atg gca ctt ttt caa cat ttg act tgt gta att 2110
   Ile Arg Lys Asn Arg Met Ala Leu Phe Gln His Leu Thr Cys Val Ile
   450 455 460
   cca atc ctg gat agt cta cta act gcc gga att att aat gaa caa gaa 2158
   Pro Ile Leu Asp Ser Leu Leu Thr Ala Gly Ile Ile Asn Glu Gln Glu
   465 470 475
   cat gat gtt att aaa cag aag aca cag acg tct tta caa gca aga gaa 2206
   His Asp Val Ile Lys Gln Lys Thr Gln Thr Ser Leu Gln Ala Arg Glu
   480 485 490
   ctg att gat acg att tta gta aaa gga aat att gca gcc act gta ttc 2254
   Leu Ile Asp Thr Ile Leu Val Lys Gly Asn Ile Ala Ala Thr Val Phe
   495 500 505 510
   aga aac tct ctg caa gaa gct gaa gct gtg tta tat gag cat tta ttt 2302
   Arg Asn Ser Leu Gln Glu Ala Glu Ala Val Leu Tyr Glu His Leu Phe
   515 520 525
   gtg caa cag gac ata aaa tat att ccc aca gaa gat gtt tca gat cta 2350
   Val Gln Gln Asp Ile Lys Tyr Ile Pro Thr Glu Asp Val Ser Asp Leu
   530 535 540
   cca gtg gaa gaa caa ttg cgg aga cta caa gaa gaa aga aca tgt aaa 2398
   Pro Val Glu Glu Gln Leu Arg Arg Leu Gln Glu Glu Arg Thr Cys Lys
   545 550 555
   gtg tgt atg gac aaa gaa gtg tcc ata gtg ttt att cct tgt ggt cat 2446
   Val Cys Met Asp Lys Glu Val Ser Ile Val Phe Ile Pro Cys Gly His
   560 565 570
   cta gta gta tgc aaa gat tgt gct cct tct tta aga aag tgt cct att 2494
   Leu Val Val Cys Lys Asp Cys Ala Pro Ser Leu Arg Lys Cys Pro Ile
   575 580 585 590
   tgt agg agt aca atc aag ggt aca gtt cgt aca ttt ctt tca tga 2539
   Cys Arg Ser Thr Ile Lys Gly Thr Val Arg Thr Phe Leu Ser
   595 600 605
   agaagaacca aaacatcatc taaactttag aattaattta ttaaatgtat tataacttta 2599
   acttttatcc taatttggtt tccttaaaat ttttatttat ttacaactca aaaaacattg 2659
   ttttgtgtaa catatttata tatgtatcta aaccatatga acatatattt tttagaaact 2719
   aagagaatga taggcttttg ttcttatgaa cgaaaaagag gtagcactac aaacacaata 2779
   ttcaatcaaa atttcagcat tattgaaatt gtaagtgaag taaaacttaa gatatttgag 2839
   ttaaccttta agaattttaa atattttggc attgtactaa tacctggttt tttttttgtt 2899
   ttgttttttt gtacagacag ggcagcatac tgagaccctg cctttaaaaa caaacagaac 2959
   aaaaacaaaa caccagggac acatttctct gtcttttttg atcagtgtcc tatacatcga 3019
   aggtgtgcat atatgttgaa tgacatttta gggacatggt gtttttataa agaattc 3076
<210> 158
   <211> 22
   <212> DNA
   <213> Artificial Sequence
<223> PCR Primer
<400> 158
   ggactcaggt gttgggaatc tg 22
<210> 159
   <211> 24
   <212> DNA
   <213> Artificial Sequence
<223> PCR Primer
<400> 159
   caagtactca caccttggaa acca 24
<210> 160
   <211> 27
   <212> DNA
   <213> Artificial Sequence
<223> PCR Probe
<400> 160
   agatgatcca tgggttcaac atgccaa 27
<210> 161
   <211> 18
   <212> DNA
   <213> Artificial Sequence
<223> Antisense Oligonucleotide
<400> 161
   actgaagaca ttttgaat 18
<210> 162
   <211> 18
   <212> DNA
   <213> Artificial Sequence
<223> Antisense Oligonucleotide
<400> 162
   cttagaggta cgtaaaat 18
<210> 163
   <211> 18
   <212> DNA
   <213> Artificial Sequence
<223> Antisense Oligonucleotide
<400> 163
   gcacttttat ttcttaga 18
<210> 164
   <211> 18
   <212> DNA
   <213> Artificial Sequence
<223> Antisense Oligonucleotide
<400> 164
   attttaatta gaagcact 18
<210> 165
   <211> 18
   <212> DNA
   <213> Artificial Sequence
<223> Antisense Oligonucleotide
<400> 165
   accatatttc actgattc 18
<210> 166
   <211> 18
   <212> DNA
   <213> Artificial Sequence
<223> Antisense Oligonucleotide
<400> 166
   ctaactcaaa ggaggaaa 18
<210> 167
   <211> 18
   <212> DNA
   <213> Artificial Sequence
<223> Antisense Oligonucleotide
<400> 167
   cacaagacct aactcaaa 18
<210> 168
   <211> 18
   <212> DNA
   <213> Artificial Sequence
<223> Antisense Oligonucleotide
<400> 168
   gctctgctgt caagtgtt 18
<210> 169
   <211> 18
   <212> DNA
   <213> Artificial Sequence
<223> Antisense Oligonucleotide
<400> 169
   tgtgtgactc atgaagct 18
<210> 170
   <211> 18
   <212> DNA
   <213> Artificial Sequence
<223> Antisense Oligonucleotide
<400> 170
   ttcagtggca ttcaatca 18
<210> 171
   <211> 18
   <212> DNA
   <213> Artificial Sequence
<223> Antisense Oligonucleotide
<400> 171
   cttctccagg ctactaga 18
<210> 172
   <211> 18
   <212> DNA
   <213> Artificial Sequence
<223> Antisense Oligonucleotide
<400> 172
   ggtcaacttc tccaggct 18
<210> 173
   <211> 18
   <212> DNA
   <213> Artificial Sequence
<223> Antisense Oligonucleotide
<400> 173
   taaaaccctt cacagaag 18
<210> 174
   <211> 18
   <212> DNA
   <213> Artificial Sequence
<223> Antisense Oligonucleotide
<400> 174
   ttaaggaaga aatacaca 18
<210> 175
   <211> 18
   <212> DNA
   <213> Artificial Sequence
<223> Antisense Oligonucleotide
<400> 175
   gcatggcttt gcttttat 18
<210> 176
   <211> 18
   <212> DNA
   <213> Artificial Sequence
<223> Antisense Oligonucleotide
<400> 176
   caaacgtgtt ggcgcttt 18
<210> 177
   <211> 18
   <212> DNA
   <213> Artificial Sequence
<223> Antisense Oligonucleotide
<400> 177
   agcaggaaaa gtggaata 18
<210> 178
   <211> 18
   <212> DNA
   <213> Artificial Sequence
<223> Antisense Oligonucleotide
<400> 178
   ttaacggaat ttagactc 18
<210> 179
   <211> 18
   <212> DNA
   <213> Artificial Sequence
<223> Antisense Oligonucleotide
<400> 179
   atttgttact gaagaagg 18
<210> 180
   <211> 18
   <212> DNA
   <213> Artificial Sequence
<223> Antisense Oligonucleotide
<400> 180
   agagccacgg aaatatcc 18
<210> 181
   <211> 18
   <212> DNA
   <213> Artificial Sequence
<223> Antisense Oligonucleotide
<400> 181
   aaatcttgat ttgctctg 18
<210> 182
   <211> 18
   <212> DNA
   <213> Artificial Sequence
<223> Antisense Oligonucleotide
<400> 182
   gtaagtaatc tggcattt 18
<210> 183
   <211> 18
   <212> DNA
   <213> Artificial Sequence
<223> Antisense Oligonucleotide
<400> 183
   agcaagccac tctgtctc 18
<210> 184
   <211> 18
   <212> DNA
   <213> Artificial Sequence
<223> Antisense Oligonucleotide
<400> 184
   tgaagtgtct tgaagctg 18
<210> 185
   <211> 18
   <212> DNA
   <213> Artificial Sequence
<223> Antisense Oligonucleotide
<400> 185
   tttgacatca tcactgtt 18
<210> 186
   <211> 18
   <212> DNA
   <213> Artificial Sequence
<223> Antisense Oligonucleotide
<400> 186
   tggcttgaac ttgacgga 18
<210> 187
   <211> 18
   <212> DNA
   <213> Artificial Sequence
<223> Antisense Oligonucleotide
<400> 187
   tcatctcctg ggctgtct 18
<210> 188
   <211> 18
   <212> DNA
   <213> Artificial Sequence
<223> Antisense Oligonucleotide
<400> 188
   gcagcattaa tcacagga 18
<210> 189
   <211> 18
   <212> DNA
   <213> Artificial Sequence
<223> Antisense Oligonucleotide
<400> 189
   tttctctctc ctcttccc 18
<210> 190
   <211> 18
   <212> DNA
   <213> Artificial Sequence
<223> Antisense Oligonucleotide
<400> 190
   aacatcatgt tcttgttc 18
<210> 191
   <211> 18
   <212> DNA
   <213> Artificial Sequence
<223> Antisense Oligonucleotide
<400> 191
   atataacaca gcttcagc 18
<210> 192
   <211> 18
   <212> DNA
   <213> Artificial Sequence
<223> Antisense Oligonucleotide
<400> 192
   aattgttctt ccactggt 18
<210> 193
   <211> 18
   <212> DNA
   <213> Artificial Sequence
<223> Antisense Oligonucleotide
<400> 193
   aagaaggagc acaatctt 18
<210> 194
   <211> 18
   <212> DNA
   <213> Artificial Sequence
<223> Antisense Oligonucleotide
<400> 194
   gaaaccaaat taggataa 18
<210> 195
   <211> 18
   <212> DNA
   <213> Artificial Sequence
<223> Antisense Oligonucleotide
<400> 195
   tgtagtgcta cctctttt 18
<210> 196
   <211> 18
   <212> DNA
   <213> Artificial Sequence
<223> Antisense Oligonucleotide
<400> 196
   ctgaaatttt gattgaat 18
<210> 197
   <211> 18
   <212> DNA
   <213> Artificial Sequence
<223> Antisense Oligonucleotide
<400> 197
   tacaatttca ataatgct 18
<210> 198
   <211> 18
   <212> DNA
   <213> Artificial Sequence
<223> Antisense Oligonucleotide
<400> 198
   gggtctcagt atgctgcc 18
<210> 199
   <211> 18
   <212> DNA
   <213> Artificial Sequence
<223> Antisense Oligonucleotide
<400> 199
   ccttcgatgt ataggaca 18
<210> 200
   <211> 18
   <212> DNA
   <213> Artificial Sequence
<223> Antisense Oligonucleotide
<400> 200
   catgtcccta aaatgtca 18
<210> 201
   <211> 2266
   <212> DNA
   <213> Homo sapiens
<220>
   <221> CDS
   <222> (316)..(1602)
<400> 201
   aattccgagc tgtagggaaa cgcaggggcg gcttctaggt gctgccgccg ccaccgccac 60
   caccacctcc accgccgcct cggaacccag gcctgggggg cggtggggcc gcgtatggag 120
   cccccgcccc ccggagctgc caacattgcc aacgccaccg ccacgctaca cacagcctca 180
   actttcagga gacccgtccg tggccttatt tattccaccc ttcctgtaca tcgtagcgaa 240
   tcaatccgtg gcgccgcact cctccgcatc cctctttaac agtacccctg ggatggcgtg 300
   agcactcccc cagcg atg gac cca tct gtg acg ctg tgg cag ttt ctg 348
   Met Asp Pro Ser Val Thr Leu Trp Gln Phe Leu
   1 5 10
   ctg cag ctg ctg aga gag caa ggc aat ggc cac atc atc tcc tgg act 396
   Leu Gln Leu Leu Arg Glu Gln Gly Asn Gly His Ile Ile Ser Trp Thr
   15 20 25
   tca cgg gat ggt ggt gaa ttc aag ctg gtg gat gca gag gag gtg gcc 444
   Ser Arg Asp Gly Gly Glu Phe Lys Leu Val Asp Ala Glu Glu Val Ala
   30 35 40
   cgg ctg tgg gga cta cgc aag aac aag acc aac atg aat tac gac aag 492
   Arg Leu Trp Gly Leu Arg Lys Asn Lys Thr Asn Met Asn Tyr Asp Lys
   45 50 55
   ctc agc cgg gcc ttg cgg tac tac tat gac aag aac atc atc cgc aag 540
   Leu Ser Arg Ala Leu Arg Tyr Tyr Tyr Asp Lys Asn Ile Ile Arg Lys
   60 65 70 75
   gtg agc ggc cag aag ttc gtc tac aag ttt gtg tcc tac cct gag gtc 588
   Val Ser Gly Gln Lys Phe Val Tyr Lys Phe Val Ser Tyr Pro Glu Val
   80 85 90
   gca ggg tgc tcc act gag gac tgc ccg ccc cag cca gag gtg tct gtt 636
   Ala Gly Cys Ser Thr Glu Asp Cys Pro Pro Gln Pro Glu Val Ser Val
   95 100 105
   acc tcc acc atg cca aat gtg gcc cct gct gct ata cat gcc gcc cca 684
   Thr Ser Thr Met Pro Asn Val Ala Pro Ala Ala Ile His Ala Ala Pro
   110 115 120
   ggg gac act gtc tct gga aag cca ggc aca ccc aag ggt gca gga atg 732
   Gly Asp Thr Val Ser Gly Lys Pro Gly Thr Pro Lys Gly Ala Gly Met
   125 130 135
   gca ggc cca ggc ggt ttg gca cgc agc agc cgg aac gag tac atg cgc 780
   Ala Gly Pro Gly Gly Leu Ala Arg Ser Ser Arg Asn Glu Tyr Met Arg
   140 145 150 155
   tcg ggc ctc tat tcc acc ttc acc atc cag tct ctg cag ccg cag cca 828
   Ser Gly Leu Tyr Ser Thr Phe Thr Ile Gln Ser Leu Gln Pro Gln Pro
   160 165 170
   ccc cct cat cct cgg cct gct gtg gtg ctc ccc aat gca gct cct gca 876
   Pro Pro His Pro Arg Pro Ala Val Val Leu Pro Asn Ala Ala Pro Ala
   175 180 185
   ggg gca gca gcg ccc ccc tcg ggg agc agg agc acc agt cca agc ccc 924
   Gly Ala Ala Ala Pro Pro Ser Gly Ser Arg Ser Thr Ser Pro Ser Pro
   190 195 200
   ttg gag gcc tgt ctg gag gct gaa gag gcc ggc ttg cct ctg cag gtc 972
   Leu Glu Ala Cys Leu Glu Ala Glu Glu Ala Gly Leu Pro Leu Gln Val
   205 210 215
   atc ctg acc ccg ccc gag gcc cca aac ctg aaa tcg gaa gag ctt aat 1020
   Ile Leu Thr Pro Pro Glu Ala Pro Asn Leu Lys Ser Glu Glu Leu Asn
   220 225 230 235
   gtg gag ccg ggt ttg ggc cgg gct ttg ccc cca gaa gtg aaa gta gaa 1068
   Val Glu Pro Gly Leu Gly Arg Ala Leu Pro Pro Glu Val Lys Val Glu
   240 245 250
   ggg ccc aag gaa gag ttg gaa gtt gcg ggg gag aga ggg ttt gtg cca 1116
   Gly Pro Lys Glu Glu Leu Glu Val Ala Gly Glu Arg Gly Phe Val Pro
   255 260 265
   gaa acc acc aag gcc gag cca gaa gtc cct cca cag gag ggc gtg cca 1164
   Glu Thr Thr Lys Ala Glu Pro Glu Val Pro Pro Gln Glu Gly Val Pro
   270 275 280
   gcc cgg ctg ccc gcg gtt gtt atg gac acc gca ggg cag gcg ggc ggc 1212
   Ala Arg Leu Pro Ala Val Val Met Asp Thr Ala Gly Gln Ala Gly Gly
   285 290 295
   cat gcg gct tcc agc cct gag atc tcc cag ccg cag aag ggc cgg aag 1260
   His Ala Ala Ser Ser Pro Glu Ile Ser Gln Pro Gln Lys Gly Arg Lys
   300 305 310 315
   ccc cgg gac cta gag ctt cca ctc agc ccg agc ctg cta ggt ggg ccg 1308
   Pro Arg Asp Leu Glu Leu Pro Leu Ser Pro Ser Leu Leu Gly Gly Pro
   320 325 330
   gga ccc gaa cgg acc cca gga tcg gga agt ggc tcc ggc ctc cag gct 1356
   Gly Pro Glu Arg Thr Pro Gly Ser Gly Ser Gly Ser Gly Leu Gln Ala
   335 340 345
   ccg ggg ccg gcg ctg acc cca tcc ctg ctt cct acg cat aca ttg acc 1404
   Pro Gly Pro Ala Leu Thr Pro Ser Leu Leu Pro Thr His Thr Leu Thr
   350 355 360
   ccg gtg ctg ctg aca ccc agc tcg ctg cct cct agc att cac ttc tgg 1452
   Pro Val Leu Leu Thr Pro Ser Ser Leu Pro Pro Ser Ile His Phe Trp
   365 370 375
   agc acc ctg agt ccc att gcg ccc cgt agc ccg gcc aag ctc tcc ttc 1500
   Ser Thr Leu Ser Pro Ile Ala Pro Arg Ser Pro Ala Lys Leu Ser Phe
   380 385 390 395
   cag ttt cca tcc agt ggc agc gcc cag gtg cac atc cct tct atc agc 1548
   Gln Phe Pro Ser Ser Gly Ser Ala Gln Val His Ile Pro Ser Ile Ser
   400 405 410
   gtg gat ggc ctc tcg acc ccc gtg gtg ctc tcc cca ggg ccc cag aag 1596
   Val Asp Gly Leu Ser Thr Pro Val Val Leu Ser Pro Gly Pro Gln Lys
   415 420 425
   cca tga ctactaccac caccaccacc accccttctg gggtcactcc atccatgctc 1652
   Pro
   tctccagcca gccatctcaa ggagaaacat agttcaactg aaagactcat gctctgattg 1712
   tggtggggtg gggatccttg ggaagaatta ctcccaagag taactctcat tatctcctcc 1772
   acagaaaaca cacagcttcc acaacttctc tgttttctgt cagtccccca gtggccgccc 1832
   ttacacgtct cctacttcaa tggtaggggc ggtttattta tttatttttt gaaggccact 1892
   gggatgagcc tgacctaacc ttttagggtg gttaggacat ctcccccacc tccccacttt 1952
   tttccccaag acaagacaat cgaggtctgg cttgagaacg acctttcttt ctttatttct 2012
   cagcctgccc ttggggagat gagggagccc tgtctgcgtt tttggatgtg agtagaagag 2072
   ttagtttgtt ttgttttatt attcctggcc atactcaggg gtccaggaag aatttgtacc 2132
   atttaatggg ttgggagtct tggccaagga agaatcacac ccttggaata gaaatttcca 2192
   cctcccccaa cctttctctc agacagctta tcctttttca accaactttt tggccaggga 2252
   ggaatgtccc tttt 2266
<210> 202
   <211> 18
   <212> DNA
   <213> Artificial Sequence
<223> PCR Primer
<400> 202
   gcaaggcaat ggccacat 18
<210> 203
   <211> 21
   <212> DNA
   <213> Artificial Sequence
<223> PCR Primer
<400> 203
   ctcctctgca tccaccagct t 21
<210> 204
   <211> 26
   <212> DNA
   <213> Artificial Sequence
<223> PCR Probe
<400> 204
   tctcctggac ttcacgggat ggtggt 26
<210> 205
   <211> 18
   <212> DNA
   <213> Artificial Sequence
<223> Antisense Oligonucleotide
<400> 205
   cccctgcgtt tccctaca 18
<210> 206
   <211> 18
   <212> DNA
   <213> Artificial Sequence
<223> Antisense Oligonucleotide
<400> 206
   ggtggtggtg gcggtggc 18
<210> 207
   <211> 18
   <212> DNA
   <213> Artificial Sequence
<223> Antisense Oligonucleotide
<400> 207
   ggcgttggca atgttggc 18
<210> 208
   <211> 18
   <212> DNA
   <213> Artificial Sequence
<223> Antisense Oligonucleotide
<400> 208
   aagttgaggc tgtgtgta 18
<210> 209
   <211> 18
   <212> DNA
   <213> Artificial Sequence
<223> Antisense Oligonucleotide
<400> 209
   aggccacgga cgggtctc 18
<210> 210
   <211> 18
   <212> DNA
   <213> Artificial Sequence
<223> Antisense Oligonucleotide
<400> 210
   gattgattcg ctacgatg 18
<210> 211
   <211> 18
   <212> DNA
   <213> Artificial Sequence
<223> Antisense Oligonucleotide
<400> 211
   gggatgcgga ggagtgcg 18
<210> 212
   <211> 18
   <212> DNA
   <213> Artificial Sequence
<223> Antisense Oligonucleotide
<400> 212
   agtgctcacg ccatccca 18
<210> 213
   <211> 18
   <212> DNA
   <213> Artificial Sequence
<223> Antisense Oligonucleotide
<400> 213
   aaactgccac agcgtcac 18
<210> 214
   <211> 18
   <212> DNA
   <213> Artificial Sequence
<223> Antisense Oligonucleotide
<400> 214
   gaagtccagg agatgatg 18
<210> 215
   <211> 18
   <212> DNA
   <213> Artificial Sequence
<223> Antisense Oligonucleotide
<400> 215
   caccaccatc ccgtgaag 18
<210> 216
   <211> 18
   <212> DNA
   <213> Artificial Sequence
<223> Antisense Oligonucleotide
<400> 216
   tcttgttctt gcgtagtc 18
<210> 217
   <211> 18
   <212> DNA
   <213> Artificial Sequence
<223> Antisense Oligonucleotide
<400> 217
   tgttcttgtc atagtagt 18
<210> 218
   <211> 18
   <212> DNA
   <213> Artificial Sequence
<223> Antisense Oligonucleotide
<400> 218
   tcaccttgcg gatgatgt 18
<210> 219
   <211> 18
   <212> DNA
   <213> Artificial Sequence
<223> Antisense Oligonucleotide
<400> 219
   gagcaccctg cgacctca 18
<210> 220
   <211> 18
   <212> DNA
   <213> Artificial Sequence
<223> Antisense Oligonucleotide
<400> 220
   ggcgggcagt cctcagtg 18
<210> 221
   <211> 18
   <212> DNA
   <213> Artificial Sequence
<223> Antisense Oligonucleotide
<400> 221
   ggtgaaggtg gaatagag 18
<210> 222
   <211> 18
   <212> DNA
   <213> Artificial Sequence
<223> Antisense Oligonucleotide
<400> 222
   tccgatttca ggtttggg 18
<210> 223
   <211> 18
   <212> DNA
   <213> Artificial Sequence
<223> Antisense Oligonucleotide
<400> 223
   ttggtggttt ctggcaca 18
<210> 224
   <211> 18
   <212> DNA
   <213> Artificial Sequence
<223> Antisense Oligonucleotide
<400> 224
   tggagggact tctggctc 18
<210> 225
   <211> 18
   <212> DNA
   <213> Artificial Sequence
<223> Antisense Oligonucleotide
<400> 225
   gcgtaggaag cagggatg 18
<210> 226
   <211> 18
   <212> DNA
   <213> Artificial Sequence
<223> Antisense Oligonucleotide
<400> 226
   gtgctccaga agtgaatg 18
<210> 227
   <211> 18
   <212> DNA
   <213> Artificial Sequence
<223> Antisense Oligonucleotide
<400> 227
   actggatgga aactggaa 18
<210> 228
   <211> 18
   <212> DNA
   <213> Artificial Sequence
<223> Antisense Oligonucleotide
<400> 228
   ggccatccac gctgatag 18
<210> 229
   <211> 18
   <212> DNA
   <213> Artificial Sequence
<223> Antisense Oligonucleotide
<400> 229
   ccaccacaat cagagcat 18
<210> 230
   <211> 18
   <212> DNA
   <213> Artificial Sequence
<223> Antisense Oligonucleotide
<400> 230
   gatccccacc ccaccaca 18
<210> 231
   <211> 18
   <212> DNA
   <213> Artificial Sequence
<223> Antisense Oligonucleotide
<400> 231
   tgttttctgt ggaggaga 18
<210> 232
   <211> 18
   <212> DNA
   <213> Artificial Sequence
<223> Antisense Oligonucleotide
<400> 232
   aaacagagaa gttgtgga 18
<210> 233
   <211> 18
   <212> DNA
   <213> Artificial Sequence
<223> Antisense Oligonucleotide
<400> 233
   gggactgaca gaaaacag 18
<210> 234
   <211> 18
   <212> DNA
   <213> Artificial Sequence
<223> Antisense Oligonucleotide
<400> 234
   ataaataaat aaaccgcc 18
<210> 235
   <211> 18
   <212> DNA
   <213> Artificial Sequence
<223> Antisense Oligonucleotide
<400> 235
   gttaggtcag gctcatcc 18
<210> 236
   <211> 18
   <212> DNA
   <213> Artificial Sequence
<223> Antisense Oligonucleotide
<400> 236
   gttctcaagc cagacctc 18
<210> 237
   <211> 18
   <212> DNA
   <213> Artificial Sequence
<223> Antisense Oligonucleotide
<400> 237
   aataaagaaa gaaaggtc 18
<210> 238
   <211> 18
   <212> DNA
   <213> Artificial Sequence
<223> Antisense Oligonucleotide
<400> 238
   agggcaggct gagaaata 18
<210> 239
   <211> 18
   <212> DNA
   <213> Artificial Sequence
<223> Antisense Oligonucleotide
<400> 239
   cttctactca catccaaa 18
<210> 240
   <211> 18
   <212> DNA
   <213> Artificial Sequence
<223> Antisense Oligonucleotide
<400> 240
   caaaacaaac taactctt 18
<210> 241
   <211> 18
   <212> DNA
   <213> Artificial Sequence
<223> Antisense Oligonucleotide
<400> 241
   ggaataataa aacaaaac 18
<210> 242
   <211> 18
   <212> DNA
   <213> Artificial Sequence
<223> Antisense Oligonucleotide
<400> 242
   ttcttcctgg acccctga 18
<210> 243
   <211> 18
   <212> DNA
   <213> Artificial Sequence
<223> Antisense Oligonucleotide
<400> 243
   ccaagggtgt gattcttc 18
<210> 244
   <211> 18
   <212> DNA
   <213> Artificial Sequence
<223> Antisense Oligonucleotide
<400> 244
   tgtctgagag aaaggttg 18
<210> 245
   <211> 1080
   <212> DNA
   <213> Homo sapiens
<220>
   <221> CDS
   <222> (1)..(1080)
<400> 245
   atg act ctg gag tcc atc atg gcg tgt tgc ctg agc gat gag gtg aag 48
   gag tcc aag cgg atc aac gcc gag atc gag aag cag ctg cgg cgg gac 96
   aag cgc gac gcc cgg cgc gag ctc aag ctg ctg ctg ctc ggc acg ggc 144
   gag agc ggg aag agc acg ttc atc aag cag atg cgc atc atc cac ggc 192
   gcc ggc tac tcg gag gag gac aag cgc ggc ttc acc aag ctc gtc tac 240
   cag aac atc ttc acc gcc atg cag gcc atg atc cgg gcc atg gag acg 288
   ctc aag atc ctc tac aag tac gag cag aac aag gcc aat gcg ctc ctg 336
   atc cgg gag gtg gac gtg gag aag gtg acc acc ttc gag cat cag tac 384
   gtc agt gcc atc aag acc ctg tgg gag gac ccg ggc atc cag gaa tgc 432
   tac gac cgc agg cgc gag tac cag ctc tcc gac tct gcc aag tac tac 480
   ctg acc gac gtt gac cgc atc gcc acc ttg ggc tac ctg ccc acc cag 528
   cag gac gtg ctg cgg gtc cgc gtg ccc acc acc ggc atc atc gag tac 576
   cct ttc gac ctg gag aac atc atc ttc cgg atg gtg gat gtg ggg ggc 624
   cag cgg tcg gag cgg agg aag tgg atc cac tgc ttt gag aac gtg aca 672
   tcc atc atg ttt ctc gtc gcc ctc agc gaa tac gac caa gtc ctg gtg 720
   gag tcg gac aac gag aac cgg atg gag gag agc aaa gcc ctg ttc cgg 768
   acc atc atc acc tac ccc tgg ttc cag aac tcc tcc gtc atc ctc ttc 816
   ctc aac aag aag gac ctg ctg gag gac aag atc ctg tac tcg cac ctg 864
   gtg gac tac ttc ccc gag ttc gat ggt ccc cag cgg gac gcc cag gcg 912
   gcg cgg gag ttc atc ccg aag atg ttc gtg gac ctg aac ccc gac agc 960
   gac aag atc atc tac tca cac ttc acg tgt gcc acc gac acg gag aac 1008
   atc cgc ttc gtg ttc gcg gcc gtg aag gac acc atc ctg cag ctg aac 1056
   ctg aag gag tac aat ctg gtc taa 1080
<210> 246
   <211> 20
   <212> DNA
   <213> Artificial Sequence
<223> PCR Primer
<400> 246
   tgaccacctt cgagcatcag 20
<210> 247
   <211> 20
   <212> DNA
   <213> Artificial Sequence
<223> PCR Primer
<400> 247
   cggtcgtagc attcctggat 20
<210> 248
   <211> 26
   <212> DNA
   <213> Artificial Sequence
<223> PCR Probe
<400> 248
   tcagtgccat caagaccctg tgggag 26
<210> 249
<211> 18
   <212> DNA
   <213> Artificial Sequence
<223> Antisense Oligonucleotide
<400> 249
   gatggactcc agagtcat 18
<210> 250
   <211> 18
   <212> DNA
   <213> Artificial Sequence
<223> Antisense Oligonucleotide
<400> 250
   gccatgatgg actccaga 18
<210> 251
   <211> 18
   <212> DNA
   <213> Artificial Sequence
<223> Antisense Oligonucleotide
<400> 251
   cacgccatga tggactcc 18
<210> 252
   <211> 18
   <212> DNA
   <213> Artificial Sequence
<223> Antisense Oligonucleotide
<400> 252
   ctcatcgctc aggcaaca 18
<210> 253
   <211> 18
   <212> DNA
   <213> Artificial Sequence
<223> Antisense Oligonucleotide
<400> 253
   cttcacctca tcgctcag 18
<210> 254
   <211> 18
   <212> DNA
   <213> Artificial Sequence
<223> Antisense Oligonucleotide
<400> 254
   gactccttca cctcatcg 18
<210> 255
   <211> 18
   <212> DNA
   <213> Artificial Sequence
<223> Antisense Oligonucleotide
<400> 255
   atccgcttgg actccttc 18
<210> 256
   <211> 18
   <212> DNA
   <213> Artificial Sequence
<223> Antisense Oligonucleotide
<400> 256
   cgttgatccg cttggact 18
<210> 257
   <211> 18
   <212> DNA
   <213> Artificial Sequence
<223> Antisense Oligonucleotide
<400> 257
   ctcgatctcg gcgttgat 18
<210> 258
   <211> 18
   <212> DNA
   <213> Artificial Sequence
<223> Antisense Oligonucleotide
<400> 258
   cccgccgcag ctgcttct 18
<210> 259
   <211> 18
   <212> DNA
   <213> Artificial Sequence
<223> Antisense Oligonucleotide
<400> 259
   cttgagctcg cgccgggc 18
<210> 260
   <211> 18
   <212> DNA
   <213> Artificial Sequence
<223> Antisense Oligonucleotide
<400> 260
   gcagcagcag cttgagct 18
<210> 261
   <211> 18
   <212> DNA
   <213> Artificial Sequence
<223> Antisense Oligonucleotide
<400> 261
   gcccgtgccg agcagcag 18
<210> 262
   <211> 18
   <212> DNA
   <213> Artificial Sequence
<223> Antisense Oligonucleotide
<400> 262
   acgtgctctt cccgctct 18
<210> 263
   <211> 18
   <212> DNA
   <213> Artificial Sequence
<223> Antisense Oligonucleotide
<400> 263
   atctgcttga tgaacgtg 18
<210> 264
   <211> 18
   <212> DNA
   <213> Artificial Sequence
<223> Antisense Oligonucleotide
<400> 264
   cgcatctgct tgatgaac 18
<210> 265
   <211> 18
   <212> DNA
   <213> Artificial Sequence
<223> Antisense Oligonucleotide
<400> 265
   gtagccggcg ccgtggat 18
<210> 266
   <211> 18
   <212> DNA
   <213> Artificial Sequence
<223> Antisense Oligonucleotide
<400> 266
   tgtcctcctc cgagtagc 18
<210> 267
   <211> 18
   <212> DNA
   <213> Artificial Sequence
<223> Antisense Oligonucleotide
<400> 267
   cttgtcctcc tccgagta 18
<210> 268
   <211> 18
   <212> DNA
   <213> Artificial Sequence
<223> Antisense Oligonucleotide
<400> 268
   aagccgcgct tgtcctcc 18
<210> 269
   <211> 18
   <212> DNA
   <213> Artificial Sequence
<223> Antisense Oligonucleotide
<400> 269
   tagacgagct tggtgaag 18
<210> 270
   <211> 18
   <212> DNA
   <213> Artificial Sequence
<223> Antisense Oligonucleotide
<400> 270
   tgttctggta gacgagct 18
<210> 271
   <211> 18
   <212> DNA
   <213> Artificial Sequence
<223> Antisense Oligonucleotide
<400> 271
   tggcggtgaa gatgttct 18
<210> 272
   <211> 18
   <212> DNA
   <213> Artificial Sequence
<223> Antisense Oligonucleotide
<400> 272
   cggatcatgg cctgcatg 18
<210> 273
   <211> 18
   <212> DNA
   <213> Artificial Sequence
<223> Antisense Oligonucleotide
<400> 273
   cgtctccatg gcccggat 18
<210> 274
   <211> 18
   <212> DNA
   <213> Artificial Sequence
<223> Antisense Oligonucleotide
<400> 274
   tagaggatct tgagcgtc 18
<210> 275
   <211> 18
   <212> DNA
   <213> Artificial Sequence
<223> Antisense Oligonucleotide
<400> 275
   tgtagaggat cttgagcg 18
<210> 276
   <211> 18
   <212> DNA
   <213> Artificial Sequence
<223> Antisense Oligonucleotide
<400> 276
   tgctcgtact tgtagagg 18
<210> 277
   <211> 18
   <212> DNA
   <213> Artificial Sequence
<223> Antisense Oligonucleotide
<400> 277
   gccttgttct gctcgtac 18
<210> 278
   <211> 18
   <212> DNA
   <213> Artificial Sequence
<223> Antisense Oligonucleotide
<400> 278
   ttggccttgt tctgctcg 18
<210> 279
   <211> 18
   <212> DNA
   <213> Artificial Sequence
<223> Antisense Oligonucleotide
<400> 279
   caggagcgca ttggcctt 18
<210> 280
   <211> 18
   <212> DNA
   <213> Artificial Sequence
<223> Antisense Oligonucleotide
<400> 280
   ctccacgtcc acctcccg 18
<210> 281
   <211> 18
   <212> DNA
   <213> Artificial Sequence
<223> Antisense Oligonucleotide
<400> 281
   ggtcaccttc tccacgtc 18
<210> 282
   <211> 18
   <212> DNA
   <213> Artificial Sequence
<223> Antisense Oligonucleotide
<400> 282
   gatgctcgaa ggtggtca 18
<210> 283
   <211> 18
   <212> DNA
   <213> Artificial Sequence
<223> Antisense Oligonucleotide
<400> 283
   actgacgtac tgatgctc 18
<210> 284
   <211> 18
   <212> DNA
   <213> Artificial Sequence
<223> Antisense Oligonucleotide
<400> 284
   cttgatggca ctgacgta 18
<210> 285
   <211> 18
   <212> DNA
   <213> Artificial Sequence
<223> Antisense Oligonucleotide
<400> 285
   cagggtcttg atggcact 18
<210> 286
   <211> 18
   <212> DNA
   <213> Artificial Sequence
<223> Antisense Oligonucleotide
<400> 286
   ctggatgccc gggtcctc 18
<210> 287
   <211> 18
   <212> DNA
   <213> Artificial Sequence
<223> Antisense Oligonucleotide
<400> 287
   tcctggatgc ccgggtcc 18
<210> 288
   <211> 18
   <212> DNA
   <213> Artificial Sequence
<223> Antisense Oligonucleotide
<400> 288
   cgcctgcggt cgtagcat 18
<210> 289
   <211> 18
   <212> DNA
   <213> Artificial Sequence
<223> Antisense Oligonucleotide
<400> 289
   gctggtactc gcgcctgc 18
<210> 290
   <211> 18
   <212> DNA
   <213> Artificial Sequence
<223> Antisense Oligonucleotide
<400> 290
   tacttggcag agtcggag 18
<210> 291
   <211> 18
   <212> DNA
   <213> Artificial Sequence
<223> Antisense Oligonucleotide
<400> 291
   gtcaggtagt acttggca 18
<210> 292
   <211> 18
   <212> DNA
   <213> Artificial Sequence
<223> Antisense Oligonucleotide
<400> 292
   ggtcaacgtc ggtcaggt 18
<210> 293
   <211> 18
   <212> DNA
   <213> Artificial Sequence
<223> Antisense Oligonucleotide
<400> 293
   gtggcgatgc ggtcaacg 18
<210> 294
   <211> 18
   <212> DNA
   <213> Artificial Sequence
<223> Antisense Oligonucleotide
<400> 294
   gcaggtagcc caaggtgg 18
<210> 295
   <211> 18
   <212> DNA
   <213> Artificial Sequence
<223> Antisense Oligonucleotide
<400> 295
   cgtcctgctg ggtgggca 18
<210> 296
   <211> 18
   <212> DNA
   <213> Artificial Sequence
<223> Antisense Oligonucleotide
<400> 296
   ggtggtgggc acgcggac 18
<210> 297
   <211> 18
   <212> DNA
   <213> Artificial Sequence
<223> Antisense Oligonucleotide
<400> 297
   tcgatgatgc cggtggtg 18
<210> 298
   <211> 18
   <212> DNA
   <213> Artificial Sequence
<223> Antisense Oligonucleotide
<400> 298
   ccaggtcgaa agggtact 18
<210> 299
   <211> 18
   <212> DNA
   <213> Artificial Sequence
<223> Antisense Oligonucleotide
<400> 299
   tgttctccag gtcgaaag 18
<210> 300
   <211> 18
   <212> DNA
   <213> Artificial Sequence
<223> Antisense Oligonucleotide
<400> 300
   agatgatgtt ctccaggt 18
<210> 301
   <211> 18
   <212> DNA
   <213> Artificial Sequence
<223> Antisense Oligonucleotide
<400> 301
   atccggaaga tgatgttc 18
<210> 302
   <211> 18
   <212> DNA
   <213> Artificial Sequence
<223> Antisense Oligonucleotide
<400> 302
   ctccgctccg accgctgg 18
<210> 303
   <211> 18
   <212> DNA
   <213> Artificial Sequence
<223> Antisense Oligonucleotide
<400> 303
   gatccacttc ctccgctc 18
<210> 304
   <211> 18
   <212> DNA
   <213> Artificial Sequence
<223> Antisense Oligonucleotide
<400> 304
   tgtcacgttc tcaaagca 18
<210> 305
   <211> 18
   <212> DNA
   <213> Artificial Sequence
<223> Antisense Oligonucleotide
<400> 305
   atgatggatg tcacgttc 18
<210> 306
   <211> 18
   <212> DNA
   <213> Artificial Sequence
<223> Antisense Oligonucleotide
<400> 306
   cgagaaacat gatggatg 18
<210> 307
   <211> 18
   <212> DNA
   <213> Artificial Sequence
<223> Antisense Oligonucleotide
<400> 307
   gctgagggcg acgagaaa 18
<210> 308
   <211> 18
   <212> DNA
   <213> Artificial Sequence
<223> Antisense Oligonucleotide
<400> 308
   cgactccacc aggacttg 18
<210> 309
   <211> 18
   <212> DNA
   <213> Artificial Sequence
<223> Antisense Oligonucleotide
<400> 309
   atccggttct cgttgtcc 18
<210> 310
   <211> 18
   <212> DNA
   <213> Artificial Sequence
<223> Antisense Oligonucleotide
<400> 310
   ccatccggtt ctcgttgt 18
<210> 311
   <211> 18
   <212> DNA
   <213> Artificial Sequence
<223> Antisense Oligonucleotide
<400> 311
   agggctttgc tctcctcc 18
<210> 312
   <211> 18
   <212> DNA
   <213> Artificial Sequence
<223> Antisense Oligonucleotide
<400> 312
   ggtccggaac agggcttt 18
<210> 313
   <211> 18
   <212> DNA
   <213> Artificial Sequence
<223> Antisense Oligonucleotide
<400> 313
   gtaggtgatg atggtccg 18
<210> 314
   <211> 18
   <212> DNA
   <213> Artificial Sequence
<223> Antisense Oligonucleotide
<400> 314
   ggaggagttc tggaacca 18
<210> 315
   <211> 18
   <212> DNA
   <213> Artificial Sequence
<223> Antisense Oligonucleotide
<400> 315
   tgaggaagag gatgacgg 18
<210> 316
   <211> 18
   <212> DNA
   <213> Artificial Sequence
   <223> Antisense Oligonucleotide
<400> 316
   gcaggtcctt cttgttga 18
<210> 317
   <211> 18
   <212> DNA
   <213> Artificial Sequence
<223> Antisense Oligonucleotide
<400> 317
   atcttgtcct ccagcagg 18
<210> 318
   <211> 18
   <212> DNA
   <213> Artificial Sequence
<223> Antisense Oligonucleotide
<400> 318
   gcgagtacag gatcttgt 18
<210> 319
   <211> 18
   <212> DNA
   <213> Artificial Sequence
<223> Antisense Oligonucleotide
<400> 319
   aagtagtcca ccaggtgc 18
<210> 320
   <211> 18
   <212> DNA
   <213> Artificial Sequence
<223> Antisense Oligonucleotide
<400> 320
   gatgaactcc cgcgccgc 18
<210> 321
   <211> 18
   <212> DNA
   <213> Artificial Sequence
<223> Antisense Oligonucleotide
<400> 321
   ggttcaggtc cacgaaca 18
<210> 322
   <211> 18
   <212> DNA
   <213> Artificial Sequence
<223> Antisense Oligonucleotide
<400> 322
   gtagatgatc ttgtcgct 18
<210> 323
   <211> 18
   <212> DNA
   <213> Artificial Sequence
<223> Antisense Oligonucleotide
<400> 323
   cacgtgaagt gtgagtag 18
<210> 324
   <211> 18
   <212> DNA
   <213> Artificial Sequence
<223> Antisense Oligonucleotide
<400> 324
   atgttctccg tgtcggtg 18
<210> 325
   <211> 18
   <212> DNA
   <213> Artificial Sequence
<223> Antisense Oligonucleotide
<400> 325
   acggccgcga acacgaag 18
<210> 326
   <211> 18
   <212> DNA
   <213> Artificial Sequence
<223> Antisense Oligonucleotide
<400> 326
   gatggtgtcc ttcacggc 18
<210> 327
   <211> 18
   <212> DNA
   <213> Artificial Sequence
<223> Antisense Oligonucleotide
<400> 327
   tcaggttcag ctgcagga 18
<210> 328
   <211> 18
   <212> DNA
   <213> Artificial Sequence
<223> Antisense Oligonucleotide
<400> 328
   accagattgt actccttc 18
<210> 329
   <211> 2610
   <212> DNA
   <213> Homo sapiens
<220>
   <221> CDS
   <222> (199)..(1641)
<400> 329
   atcctgggac agggcacagg gccatctgtc accaggggct tagggaaggc cgagccagcc 60
   tgggtcaaag aagtcaaagg ggctgcctgg aggaggcagc ctgtcagctg gtgcatcaga 120
   ggctgtggcc aggccagctg ggctcgggga gcgccagcct gagaggagcg cgtgagcgtc 180
   gcgggagcct cgggcacc atg agc gac gtg gct att gtg aag gag ggt tgg 231
   Met Ser Asp Val Ala Ile Val Lys Glu Gly Trp
   1 5 10
   ctg cac aaa cga ggg gag tac atc aag acc tgg cgg cca cgc tac ttc 279
   Leu His Lys Arg Gly Glu Tyr Ile Lys Thr Trp Arg Pro Arg Tyr Phe
   15 20 25
   ctc ctc aag aat gat ggc acc ttc att ggc tac aag gag cgg ccg cag 327
   Leu Leu Lys Asn Asp Gly Thr Phe Ile Gly Tyr Lys Glu Arg Pro Gln
   30 35 40
   gat gtg gac caa cgt gag gct ccc ctc aac aac ttc tct gtg gcg cag 375
   Asp Val Asp Gln Arg Glu Ala Pro Leu Asn Asn Phe Ser Val Ala Gln
   45 50 55
   tgc cag ctg atg aag acg gag cgg ccc cgg ccc aac acc ttc atc atc 423
   Cys Gln Leu Met Lys Thr Glu Arg Pro Arg Pro Asn Thr Phe Ile Ile
   60 65 70 75
   cgc tgc ctg cag tgg acc act gtc atc gaa cgc acc ttc cat gtg gag 471
   Arg Cys Leu Gln Trp Thr Thr Val Ile Glu Arg Thr Phe His Val Glu
   80 85 90
   act cct gag gag cgg gag gag tgg aca acc gcc atc cag act gtg gct 519
   Thr Pro Glu Glu Arg Glu Glu Trp Thr Thr Ala Ile Gln Thr Val Ala
   95 100 105
   gac ggc ctc aag aag cag gag gag gag gag atg gac ttc cgg tcg ggc 567
   Asp Gly Leu Lys Lys Gln Glu Glu Glu Glu Met Asp Phe Arg Ser Gly
   110 115 120
   tca ccc agt gac aac tca ggg gct gaa gag atg gag gtg tcc ctg gcc 615
   Ser Pro Ser Asp Asn Ser Gly Ala Glu Glu Met Glu Val Ser Leu Ala
   125 130 135
   aag ccc aag cac cgc gtg acc atg aac gag ttt gag tac ctg aag ctg 663
   Lys Pro Lys His Arg Val Thr Met Asn Glu Phe Glu Tyr Leu Lys Leu
   140 145 150 155
   ctg ggc aag ggc act ttc ggc aag gtg atc ctg gtg aag gag aag gcc 711
   Leu Gly Lys Gly Thr Phe Gly Lys Val Ile Leu Val Lys Glu Lys Ala
   160 165 170
   aca ggc cgc tac tac gcc atg aag atc ctc aag aag gaa gtc atc gtg 759
   Thr Gly Arg Tyr Tyr Ala Met Lys Ile Leu Lys Lys Glu Val Ile Val
   175 180 185
   gcc aag gac gag gtg gcc cac aca ctc acc gag aac cgc gtc ctg cag 807
   Ala Lys Asp Glu Val Ala His Thr Leu Thr Glu Asn Arg Val Leu Gln
   190 195 200
   aac tcc agg cac ccc ttc ctc aca gcc ctg aag tac tct ttc cag acc 855
   Asn Ser Arg His Pro Phe Leu Thr Ala Leu Lys Tyr Ser Phe Gln Thr
   205 210 215
   cac gac cgc ctc tgc ttt gtc atg gag tac gcc aac ggg ggc gag ctg 903
   His Asp Arg Leu Cys Phe Val Met Glu Tyr Ala Asn Gly Gly Glu Leu
   220 225 230 235
   ttc ttc cac ctg tcc cgg gaa cgt gtg ttc tcc gag gac cgg gcc cgc 951
   Phe Phe His Leu Ser Arg Glu Arg Val Phe Ser Glu Asp Arg Ala Arg
   240 245 250
   ttc tat ggc gct gag att gtg tca gcc ctg gac tac ctg cac tcg gag 999
   Phe Tyr Gly Ala Glu Ile Val Ser Ala Leu Asp Tyr Leu His Ser Glu
   255 260 265
   aag aac gtg gtg tac cgg gac ctc aag ctg gag aac ctc atg ctg gac 1047
   Lys Asn Val Val Tyr Arg Asp Leu Lys Leu Glu Asn Leu Met Leu Asp
   270 275 280
   aag gac ggg cac att aag atc aca gac ttc ggg ctg tgc aag gag ggg 1095
   Lys Asp Gly His Ile Lys Ile Thr Asp Phe Gly Leu Cys Lys Glu Gly
   285 290 295
   atc aag gac ggt gcc acc atg aag acc ttt tgc ggc aca cct gag tac 1143
   Ile Lys Asp Gly Ala Thr Met Lys Thr Phe Cys Gly Thr Pro Glu Tyr
   300 305 310 315
   ctg gcc ccc gag gtg ctg gag gac aat gac tac ggc cgt gca gtg gac 1191
   Leu Ala Pro Glu Val Leu Glu Asp Asn Asp Tyr Gly Arg Ala Val Asp
   320 325 330
   tgg tgg ggg ctg ggc gtg gtc atg tac gag atg atg tgc ggt cgc ctg 1239
   Trp Trp Gly Leu Gly Val Val Met Tyr Glu Met Met Cys Gly Arg Leu
   335 340 345
   ccc ttc tac aac cag gac cat gag aag ctt ttt gag ctc atc ctc atg 1287
   Pro Phe Tyr Asn Gln Asp His Glu Lys Leu Phe Glu Leu Ile Leu Met
   350 355 360
   gag gag atc cgc ttc ccg cgc acg ctt ggt ccc gag gcc aag tcc ttg 1335
   Glu Glu Ile Arg Phe Pro Arg Thr Leu Gly Pro Glu Ala Lys Ser Leu
   365 370 375
   ctt tca ggg ctg ctc aag aag gac ccc aag cag agg ctt ggc ggg ggc 1383
   Leu Ser Gly Leu Leu Lys Lys Asp Pro Lys Gln Arg Leu Gly Gly Gly
   380 385 390 395
   tcc gag gac gcc aag gag atc atg cag cat cgc ttc ttt gcc ggt atc 1431
   Ser Glu Asp Ala Lys Glu Ile Met Gln His Arg Phe Phe Ala Gly Ile
   400 405 410
   gtg tgg cag cac gtg tac gag aag aag ctc agc cca ccc ttc aag ccc 1479
   Val Trp Gln His Val Tyr Glu Lys Lys Leu Ser Pro Pro Phe Lys Pro
   415 420 425
   cag gtc acg tcg gag act gac acc agg tat ttt gat gag gag ttc acg 1527
   Gln Val Thr Ser Glu Thr Asp Thr Arg Tyr Phe Asp Glu Glu Phe Thr
   430 435 440
   gcc cag atg atc acc atc aca cca cct gac caa gat gac agc atg gag 1575
   Ala Gln Met Ile Thr Ile Thr Pro Pro Asp Gln Asp Asp Ser Met Glu
   445 450 455
   tgt gtg gac agc gag cgc agg ccc cac ttc ccc cag ttc tcc tac tcg 1623
   Cys Val Asp Ser Glu Arg Arg Pro His Phe Pro Gln Phe Ser Tyr Ser
   460 465 470 475
   gcc agc agc acg gcc tga ggcggcggtg gactgcgctg gacgatagct 1671
   Ala Ser Ser Thr Ala
   480
   tggagggatg gagaggcggc ctcgtgccat gatctgtatt taatggtttt tatttctcgg 1731
   gtgcatttga gagaagccac gctgtcctct cgagcccaga tggaaagacg tttttgtgct 1791
   gtgggcagca ccctcccccg cagcggggta gggaagaaaa ctatcctgcg ggttttaatt 1851
   tatttcatcc agtttgttct ccgggtgtgg cctcagccct cagaacaatc cgattcacgt 1911
   agggaaatgt taaggacttc tacagctatg cgcaatgtgg cattgggggg ccgggcaggt 1971
   cctgcccatg tgtcccctca ctctgtcagc cagccgccct gggctgtctg tcaccagcta 2031
   tctgtcatct ctctggggcc ctgggcctca gttcaacctg gtggcaccag atgcaacctc 2091
   actatggtat gctggccagc accctctcct gggggtggca ggcacacagc agccccccag 2151
   cactaaggcc gtgtctctga ggacgtcatc ggaggctggg cccctgggat gggaccaggg 2211
   atgggggatg ggccagggtt tacccagtgg gacagaggag caaggtttaa atttgttatt 2271
   gtgtattatg ttgttcaaat gcattttggg ggtttttaat ctttgtgaca ggaaagccct 2331
   cccccttccc cttctgtgtc acagttcttg gtgactgtcc caccggagcc tccccctcag 2391
   atgatctctc cacggtagca cttgaccttt tcgacgctta acctttccgc tgtcgcccca 2451
   ggccctccct gactccctgt gggggtggcc atccctgggc ccctccacgc ctcctggcca 2511
   gacgctgccg ctgccgctgc accacggcgt ttttttacaa cattcaactt tagtattttt 2571
   actattataa tataatatgg aaccttccct ccaaattct 2610
<210> 330
   <211> 21
   <212> DNA
   <213> Artificial Sequence
<223> PCR Primer
<400> 330
   cgtgaccatg aacgagtttg a 21
<210> 331
   <211> 19
   <212> DNA
   <213> Artificial Sequence
<223> PCR Primer
<400> 331
   caggatcacc ttgccgaaa 19
<210> 332
   <211> 22
   <212> DNA
   <213> Artificial Sequence
<223> PCR Probe
<400> 332
   ctgaagctgc tgggcaaggg ca 22
<210> 333
   <211> 18
   <212> DNA
   <213> Artificial Sequence
<223> Antisense Oligonucleotide
<400> 333
   ccctgtgccc tgtcccag 18
<210> 334
   <211> 18
   <212> DNA
   <213> Artificial Sequence
<223> Antisense Oligonucleotide
<400> 334
   cctaagcccc tggtgaca 18
<210> 335
   <211> 18
   <212> DNA
   <213> Artificial Sequence
<223> Antisense Oligonucleotide
<400> 335
   ctttgacttc tttgaccc 18
<210> 336
   <211> 18
   <212> DNA
   <213> Artificial Sequence
<223> Antisense Oligonucleotide
<400> 336
   ggcagcccct ttgacttc 18
<210> 337
   <211> 18
   <212> DNA
   <213> Artificial Sequence
<223> Antisense Oligonucleotide
<400> 337
   caaccctcct tcacaata 18
<210> 338
   <211> 18
   <212> DNA
   <213> Artificial Sequence
<223> Antisense Oligonucleotide
<400> 338
   tactcccctc gtttgtgc 18
<210> 339
   <211> 18
   <212> DNA
   <213> Artificial Sequence
<223> Antisense Oligonucleotide
<400> 339
   tgccatcatt cttgagga 18
<210> 340
   <211> 18
   <212> DNA
   <213> Artificial Sequence
<223> Antisense Oligonucleotide
<400> 340
   agccaatgaa ggtgccat 18
<210> 341
   <211> 18
   <212> DNA
   <213> Artificial Sequence
<223> Antisense Oligonucleotide
<400> 341
   cacagagaag ttgttgag 18
<210> 342
   <211> 18
   <212> DNA
   <213> Artificial Sequence
<223> Antisense Oligonucleotide
<400> 342
   agtctggatg gcggttgt 18
<210> 343
   <211> 18
   <212> DNA
   <213> Artificial Sequence
<223> Antisense Oligonucleotide
<400> 343
   tcctcctcct cctgcttc 18
<210> 344
   <211> 18
   <212> DNA
   <213> Artificial Sequence
<223> Antisense Oligonucleotide
<400> 344
   cctgagttgt cactgggt 18
<210> 345
   <211> 18
   <212> DNA
   <213> Artificial Sequence
<223> Antisense Oligonucleotide
<400> 345
   ccgaaagtgc ccttgccc 18
<210> 346
   <211> 18
   <212> DNA
   <213> Artificial Sequence
<223> Antisense Oligonucleotide
<400> 346
   gccacgatga cttccttc 18
<210> 347
   <211> 18
   <212> DNA
   <213> Artificial Sequence
<223> Antisense Oligonucleotide
<400> 347
   cggtcctcgg agaacaca 18
<210> 348
   <211> 18
   <212> DNA
   <213> Artificial Sequence
<223> Antisense Oligonucleotide
<400> 348
   acgttcttct ccgagtgc 18
<210> 349
   <211> 18
   <212> DNA
   <213> Artificial Sequence
<223> Antisense Oligonucleotide
<400> 349
   gtgccgcaaa aggtcttc 18
<210> 350
   <211> 18
   <212> DNA
   <213> Artificial Sequence
<223> Antisense Oligonucleotide
<400> 350
   tactcaggtg tgccgcaa 18
<210> 351
   <211> 18
   <212> DNA
   <213> Artificial Sequence
<223> Antisense Oligonucleotide
<400> 351
   ggcttgaagg gtgggctg 18
<210> 352
   <211> 18
   <212> DNA
   <213> Artificial Sequence
<223> Antisense Oligonucleotide
<400> 352
   tcaaaatacc tggtgtca 18
<210> 353
   <211> 18
   <212> DNA
   <213> Artificial Sequence
<223> Antisense Oligonucleotide
<400> 353
   gccgtgaact cctcatca 18
<210> 354
   <211> 18
   <212> DNA
   <213> Artificial Sequence
<223> Antisense Oligonucleotide
<400> 354
   ggtcaggtgg tgtgatgg 18
<210> 355
   <211> 18
   <212> DNA
   <213> Artificial Sequence
<223> Antisense Oligonucleotide
<400> 355
   ctcgctgtcc acacactc 18
<210> 356
   <211> 18
   <212> DNA
   <213> Artificial Sequence
<223> Antisense Oligonucleotide
<400> 356
   gcctctccat ccctccaa 18
<210> 357
   <211> 18
   <212> DNA
   <213> Artificial Sequence
<223> Antisense Oligonucleotide
<400> 357
   acagcgtggc ttctctca 18
<210> 358
   <211> 18
   <212> DNA
   <213> Artificial Sequence
<223> Antisense Oligonucleotide
<400> 358
   ttttcttccc taccccgc 18
<210> 359
   <211> 18
   <212> DNA
   <213> Artificial Sequence
<223> Antisense Oligonucleotide
<400> 359
   gatagttttc ttccctac 18
<210> 360
   <211> 18
   <212> DNA
   <213> Artificial Sequence
   <223> Antisense Oligonucleotide
<400> 360
   taaaacccgc aggatagt 18
<210> 361
   <211> 18
   <212> DNA
   <213> Artificial Sequence
<223> Antisense Oligonucleotide
<400> 361
   ggagaacaaa ctggatga 18
<210> 362
   <211> 18
   <212> DNA
   <213> Artificial Sequence
<223> Antisense Oligonucleotide
<400> 362
   ctggctgaca gagtgagg 18
<210> 363
   <211> 18
   <212> DNA
   <213> Artificial Sequence
<223> Antisense Oligonucleotide
<400> 363
   gcggctggct gacagagt 18
<210> 364
   <211> 18
   <212> DNA
   <213> Artificial Sequence
<223> Antisense Oligonucleotide
<400> 364
   cccagagaga tgacagat 18
<210> 365
   <211> 18
   <212> DNA
   <213> Artificial Sequence
<223> Antisense Oligonucleotide
<400> 365
   gctgctgtgt gcctgcca 18
<210> 366
   <211> 18
   <212> DNA
   <213> Artificial Sequence
<223> Antisense Oligonucleotide
<400> 366
   cataatacac aataacaa 18
<210> 367
   <211> 18
   <212> DNA
   <213> Artificial Sequence
<223> Antisense Oligonucleotide
<400> 367
   atttgaacaa cataatac 18
<210> 368
   <211> 18
   <212> DNA
   <213> Artificial Sequence
<223> Antisense Oligonucleotide
<400> 368
   aagtgctacc gtggagag 18
<210> 369
   <211> 18
   <212> DNA
   <213> Artificial Sequence
<223> Antisense Oligonucleotide
<400> 369
   cgaaaaggtc aagtgcta 18
<210> 370
   <211> 18
   <212> DNA
   <213> Artificial Sequence
<223> Antisense Oligonucleotide
<400> 370
   cagggagtca gggagggc 18
<210> 371
   <211> 18
   <212> DNA
   <213> Artificial Sequence
<223> Antisense Oligonucleotide
<400> 371
   aaagttgaat gttgtaaa 18
<210> 372
   <211> 18
   <212> DNA
   <213> Artificial Sequence
<223> Antisense Oligonucleotide
<400> 372
   aaaatactaa agttgaat 18

### SEQUENCE LISTING

<110> ISIS Pharmaceuticals, Inc.
   Cowsert, Lex M.
   Baker, Brenda F.
   McNeil, John
   Freier, Susan M.
   Sasmor, Henri M.
   Brooks, Douglas G.
   Ohashi, Cara
   Wyatt, Jacqueline R.
   Borchers, Alexander H.
   Vickers, Timothy A.
<120> Identification of Genetic Targets for Modulation by Oligonucleotides and Generation of Oligonucleotides for Gene Modulation
<130> P025649EP
<140> EP99922713.5
   <141> 1999-04-13
<150> 09/067,638
   <151> 1998-04-28
<150> 60/081,483
   <151> 1998-04-13
<160> 378
<170> Microsoft Word 97
<210> 1
   <211> 18
   <212> DNA
   <213> Artificial Sequence
<220>
   <221> misc_feature
   <223> Antisense Oligonucleotide
<400> 1
   ccaggcggca ggaccact 18
<210> 2
   <211> 18
   <212> DNA
   <213> Artificial Sequence
<220>
   <221> misc_feature
   <223> Antisense Oligonucleotide
<400> 2
   gaccaggcgg caggacca 18
<210> 3
   <211> 18
   <212> DNA
   <213> Artificial Sequence
<220>
   <221> misc_feature
   <223> Antisense Oligonucleotide
<400> 3
   aggtgagacc aggcggca 18
<210> 4
   <211> 18
   <212> DNA
   <213> Artificial Sequence
<220>
   <221> misc_feature
   <223> Antisense Oligonucleotide
<400> 4
   cagaggcaga cgaaccat 18
<210> 5
   <211> 18
   <212> DNA
   <213> Artificial Sequence
<220>
   <221> misc_feature
   <223> Antisense Oligonucleotide
<400> 5
   gcagaggcag acgaacca 18
<210> 6
   <211> 18
   <212> DNA
   <213> Artificial Sequence
<220>
   <221> misc_feature
   <223> Antisense Oligonucleotide
<400> 6
   gcaagcagcc ccagagga 18
<210> 7
   <211> 18
   <212> DNA
   <213> Artificial Sequence
<220>
   <221> misc_feature
   <223> Antisense Oligonucleotide
<400> 7
   ggtcagcaag cagcccca 18
<210> 8
   <211> 18
   <212> DNA
   <213> Artificial Sequence
<220>
   <221> misc_feature
   <223> Antisense Oligonucleotide
<400> 8
   gacagcggtc agcaagca 18
<210> 9
   <211> 18
   <212> DNA
   <213> Artificial Sequence
<220>
   <221> misc_feature
   <223> Antisense Oligonucleotide
<400> 9
   gatggacagc ggtcagca 18
<210> 10
   <211> 18
   <212> DNA
   <213> Artificial Sequence
<220>
   <221> misc_feature
   <223> Antisense Oligonucleotide
<400> 10
   tctggatgga cagcggtc 18
<210> 11
   <211> 18
   <212> DNA
   <213> Artificial Sequence
<220>
   <221> misc_feature
   <223> Antisense Oligonucleotide
<400> 11
   ggtggttctg gatggaca 18
<210> 12
   <211> 18
   <212> DNA
   <213> Artificial Sequence
<220>
   <221> misc_feature
   <223> Antisense Oligonucleotide
<400> 12
   gtgggtggtt ctggatgg 18
<210> 13
   <211> 18
   <212> DNA
   <213> Artificial Sequence
<220>
   <221> misc_feature
   <223> Antisense Oligonucleotide
<400> 13
   gcagtgggtg gttctgga 18
<210> 14
   <211> 18
   <212> DNA
   <213> Artificial Sequence
<220>
   <221> misc_feature
   <223> Antisense Oligonucleotide
<400> 14
   cacaaagaac agcactga 18
<210> 15
   <211> 18
   <212> DNA
   <213> Artificial Sequence
<220>
   <221> misc_feature
   <223> Antisense Oligonucleotide
<400> 15
   ctggcacaaa gaacagca 18
<210> 16
   <211> 18
   <212> DNA
   <213> Artificial Sequence
<220>
   <221> misc_feature
   <223> Antisense Oligonucleotide
<400> 16
   tcctggctgg cacaaaga 18
<210> 17
   <211> 18
   <212> DNA
   <213> Artificial Sequence
<220>
   <221> misc_feature
   <223> Antisense Oligonucleotide
<400> 17
   ctgtcctggc tggcacaa 18
<210> 18
   <211> 18
   <212> DNA
   <213> Artificial Sequence
<220>
   <221> misc_feature
   <223> Antisense Oligonucleotide
<400> 18
   ctcaccagtt tctgtcct 18
<210> 19
   <211> 18
   <212> DNA
   <213> Artificial Sequence
<220>
   <221> misc_feature
   <223> Antisense Oligonucleotide
<400> 19
   tcactcacca gtttctgt 18
<210> 20
   <211> 18
   <212> DNA
   <213> Artificial Sequence
<220>
   <221> misc_feature
   <223> Antisense Oligonucleotide
<400> 20
   gtgcagtcac tcaccagt 18
<210> 21
   <211> 18
   <212> DNA
   <213> Artificial Sequence
<220>
   <221> misc_feature
   <223> Antisense Oligonucleotide
<400> 21
   actctgtgca gtcactca 18
<210> 22
   <211> 18
   <212> DNA
   <213> Artificial Sequence
<220>
   <221> misc_feature
   <223> Antisense Oligonucleotide
<400> 22
   cagtgaactc tgtgcagt 18
<210> 23
   <211> 18
   <212> DNA
   <213> Artificial Sequence
<220>
   <221> misc_feature
   <223> Antisense Oligonucleotide
<400> 23
   attccgtttc agtgaact 18
<210> 24
   <211> 18
   <212> DNA
   <213> Artificial Sequence
<220>
   <221> misc_feature
   <223> Antisense Oligonucleotide
<400> 24
   gaaggcattc cgtttcag 18
<210> 25
   <211> 18
   <212> DNA
   <213> Artificial Sequence
<220>
   <221> misc_feature
   <223> Antisense Oligonucleotide
<400> 25
   ttcaccgcaa ggaaggca 18
<210> 26
   <211> 18
   <212> DNA
   <213> Artificial Sequence
<220>
   <221> misc_feature
   <223> Antisense Oligonucleotide
<400> 26
   ctctgttcca ggtgtcta 18
<210> 27
   <211> 18
   <212> DNA
   <213> Artificial Sequence
<220>
   <221> misc_feature
   <223> Antisense Oligonucleotide
<400> 27
   ctggtggcag tgtgtctc 18
<210> 28
   <211> 18
   <212> DNA
   <213> Artificial Sequence
<220>
   <221> misc_feature
   <223> Antisense Oligonucleotide
<400> 28
   tggggtcgca gtatttgt 18
<210> 29
   <211> 18
   <212> DNA
   <213> Artificial Sequence
<220>
   <221> misc_feature
   <223> Antisense Oligonucleotide
<400> 29
   ggttggggtc gcagtatt 18
<210> 30
   <211> 18
   <212> DNA
   <213> Artificial Sequence
<220>
   <221> misc_feature
   <223> Antisense Oligonucleotide
<400> 30
   ctaggttggg gtcgcagt 18
<210> 31
   <211> 18
   <212> DNA
   <213> Artificial Sequence
<220>
   <221> misc_feature
   <223> Antisense Oligonucleotide
<400> 31
   ggtgcccttc tgctggac 18
<210> 32
   <211> 18
   <212> DNA
   <213> Artificial Sequence
<220>
   <221> misc_feature
   <223> Antisense Oligonucleotide
<400> 32
   ctgaggtgcc cttctgct 18
<210> 33
   <211> 18
   <212> DNA
   <213> Artificial Sequence
<220>
   <221> misc_feature
   <223> Antisense Oligonucleotide
   <400> 33
   gtgtctgttt ctgaggtg 18
<210> 34
   <211> 18
   <212> DNA
   <213> Artificial Sequence
<220>
   <221> misc_feature
   <223> Antisense Oligonucleotide
<400> 34
   tggtgtctgt ttctgagg 18
<210> 35
   <211> 18
   <212> DNA
   <213> Artificial Sequence
<220>
   <221> misc_feature
   <223> Antisense Oligonucleotide
<400> 35
   acaggtgcag atggtgtc 18
<210> 36
   <211> 18
   <212> DNA
   <213> Artificial Sequence
<220>
   <221> misc_feature
   <223> Antisense Oligonucleotide
<400> 36
   ttcacaggtg cagatggt 18
<210> 37
   <211> 18
   <212> DNA
   <213> Artificial Sequence
<220>
   <221> misc_feature
   <223> Antisense Oligonucleotide
<400> 37
   gtgccagcct tcttcaca 18
<210> 38
   <211> 18
   <212> DNA
   <213> Artificial Sequence
<220>
   <221> misc_feature
   <223> Antisense Oligonucleotide
<400> 38
   tacagtgcca gccttctt 18
<210> 39
   <211> 18
   <212> DNA
   <213> Artificial Sequence
<220>
   <221> misc_feature
   <223> Antisense Oligonucleotide
<400> 39
   ggacacagct ctcacagg 18
<210> 40
   <211> 18
   <212> DNA
   <213> Artificial Sequence
<220>
   <221> misc_feature
   <223> Antisense Oligonucleotide
<400> 40
   tgcaggacac agctctca 18
<210> 41
   <211> 18
   <212> DNA
   <213> Artificial Sequence
<220>
   <221> misc_feature
   <223> Antisense Oligonucleotide
<400> 41
   gagcggtgca ggacacag 18
<210> 42
   <211> 18
   <212> DNA
   <213> Artificial Sequence
<220>
   <221> misc_feature
   <223> Antisense Oligonucleotide
<400> 42
   aagccgggcg agcatgag 18
<210> 43
   <211> 18
   <212> DNA
   <213> Artificial Sequence
<220>
   <221> misc_feature
   <223> Antisense Oligonucleotide
<400> 43
   aatctgcttg accccaaa 18
<210> 44
   <211> 18
   <212> DNA
   <213> Artificial Sequence
<220>
   <221> misc_feature
   <223> Antisense Oligonucleotide
<400> 44
   gaaacccctg tagcaatc 18
<210> 45
   <211> 18
   <212> DNA
   <213> Artificial Sequence
<220>
   <221> misc_feature
   <223> Antisense Oligonucleotide
<400> 45
   gtatcagaaa cccctgta 18
<210> 46
   <211> 18
   <212> DNA
   <213> Artificial Sequence
<220>
   <221> misc_feature
   <223> Antisense Oligonucleotide
   <400> 46
   gctcgcagat ggtatcag 18
<210> 47
   <211> 18
   <212> DNA
   <213> Artificial Sequence
<220>
   <221> misc_feature
   <223> Antisense Oligonucleotide
<400> 47
   gcagggctcg cagatggt 18
<210> 48
   <211> 18
   <212> DNA
   <213> Artificial Sequence
<220>
   <221> misc_feature
   <223> Antisense Oligonucleotide
<400> 48
   tgggcagggc tcgcagat 18
<210> 49
   <211> 18
   <212> DNA
   <213> Artificial Sequence
<220>
   <221> misc_feature
   <223> Antisense Oligonucleotide
<400> 49
   gactgggcag ggctcgca 18
<210> 50
   <211> 18
   <212> DNA
   <213> Artificial Sequence
<220>
   <221> misc_feature
   <223> Antisense Oligonucleotide
<400> 50
   cattggagaa gaagccga 18
<210> 51
   <211> 18
   <212> DNA
   <213> Artificial Sequence
<220>
   <221> misc_feature
   <223> Antisense Oligonucleotide
<400> 51
   gatgacacat tggagaag 18
<210> 52
   <211> 18
   <212> DNA
   <213> Artificial Sequence
<220>
   <221> misc_feature
   <223> Antisense Oligonucleotide
<400> 52
   gcagatgaca cattggag 18
<210> 53
   <211> 18
   <212> DNA
   <213> Artificial Sequence
<220>
   <221> misc_feature
   <223> Antisense Oligonucleotide
<400> 53
   tcgaaagcag atgacaca 18
<210> 54
   <211> 18
   <212> DNA
   <213> Artificial Sequence
<220>
   <221> misc_feature
   <223> Antisense Oligonucleotide
<400> 54
   gtccaagggt gacatttt 18
<210> 55
   <211> 18
   <212> DNA
   <213> Artificial Sequence
<220>
   <221> misc_feature
   <223> Antisense Oligonucleotide
<400> 55
   cacagcttgt ccaagggt 18
<210> 56
   <211> 18
   <212> DNA
   <213> Artificial Sequence
<220>
   <221> misc_feature
   <223> Antisense Oligonucleotide
<400> 56
   ttggtctcac agcttgtc 18
<210> 57
   <211> 18
   <212> DNA
   <213> Artificial Sequence
<220>
   <221> misc_feature
   <223> Antisense Oligonucleotide
<400> 57
   caggtctttg gtctcaca 18
<210> 58
   <211> 18
   <212> DNA
   <213> Artificial Sequence
<220>
   <221> misc_feature
   <223> Antisense Oligonucleotide
<400> 58
   ctgttgcaca accaggtc 18
<210> 59
   <211> 18
   <212> DNA
   <213> Artificial Sequence
<220>
   <221> misc_feature
   <223> Antisense Oligonucleotide
<400> 59
   gtttgtgcct gcctgttg 18
<210> 60
   <211> 18
   <212> DNA
   <213> Artificial Sequence
<220>
   <221> misc_feature
   <223> Antisense Oligonucleotide
<400> 60
   gtcttgtttg tgcctgcc 18
<210> 61
   <211> 18
   <212> DNA
   <213> Artificial Sequence
<220>
   <221> misc_feature
   <223> Antisense Oligonucleotide
<400> 61
   ccacagacaa catcagtc 18
<210> 62
   <211> 18
   <212> DNA
   <213> Artificial Sequence
<220>
   <221> misc_feature
   <223> Antisense Oligonucleotide
<400> 62
   ctggggacca cagacaac 18
<210> 63
   <211> 18
   <212> DNA
   <213> Artificial Sequence
<220>
   <221> misc_feature
   <223> Antisense Oligonucleotide
<400> 63
   tcagccgatc ctggggac 18
<210> 64
   <211> 18
   <212> DNA
   <213> Artificial Sequence
<220>
   <221> misc_feature
   <223> Antisense Oligonucleotide
<400> 64
   caccaccagg gctctcag 18
<210> 65
   <211> 18
   <212> DNA
   <213> Artificial Sequence
<220>
   <221> misc_feature
   <223> Antisense Oligonucleotide
<400> 65
   gggatcacca ccagggct 18
<210> 66
   <211> 18
   <212> DNA
   <213> Artificial Sequence
<220>
   <221> misc_feature
   <223> Antisense Oligonucleotide
<400> 66
   gaggatggca aacaggat 18
<210> 67
   <211> 18
   <212> DNA
   <213> Artificial Sequence
<220>
   <221> misc_feature
   <223> Antisense Oligonucleotide
<400> 67
   accagcacca agaggatg 18
<210> 68
   <211> 18
   <212> DNA
   <213> Artificial Sequence
<220>
   <221> misc_feature
   <223> Antisense Oligonucleotide
<400> 68
   ttttgataaa gaccagca 18
<210> 69
   <211> 18
   <212> DNA
   <213> Artificial Sequence
<220>
   <221> misc_feature
   <223> Antisense Oligonucleotide
<400> 69
   tattggttgg cttcttgg 18
<210> 70
   <211> 18
   <212> DNA
   <213> Artificial Sequence
<220>
   <221> misc_feature
   <223> Antisense Oligonucleotide
<400> 70
   gggttcctgc ttggggtg 18
<210> 71
   <211> 18
   <212> DNA
   <213> Artificial Sequence
<220>
   <221> misc_feature
   <223> Antisense Oligonucleotide
<400> 71
   gtcgggaaaa ttgatctc 18
<210> 72
   <211> 18
   <212> DNA
   <213> Artificial Sequence
<220>
   <221> misc_feature
   <223> Antisense Oligonucleotide
<400> 72
   gatcgtcggg aaaattga 18
<210> 73
   <211> 18
   <212> DNA
   <213> Artificial Sequence
<220>
   <221> misc_feature
   <223> Antisense Oligonucleotide
<400> 73
   ggagccagga agatcgtc 18
<210> 74
   <211> 18
   <212> DNA
   <213> Artificial Sequence
<220>
   <221> misc_feature
   <223> Antisense Oligonucleotide
<400> 74
   tggagccagg aagatcgt 18
<210> 75
   <211> 18
   <212> DNA
   <213> Artificial Sequence
<220>
   <221> misc_feature
   <223> Antisense Oligonucleotide
<400> 75
   tggagcagca gtgttgga 18
<210> 76
   <211> 18
   <212> DNA
   <213> Artificial Sequence
<220>
   <221> misc_feature
   <223> Antisense Oligonucleotide
<400> 76
   gtaaagtctc ctgcactg 18
<210> 77
   <211> 18
   <212> DNA
   <213> Artificial Sequence
<220>
   <221> misc_feature
   <223> Antisense Oligonucleotide
<400> 77
   tggcatccat gtaaagtc 18
<210> 78
   <211> 18
   <212> DNA
   <213> Artificial Sequence
<220>
   <221> misc_feature
   <223> Antisense Oligonucleotide
<400> 78
   cggttggcat ccatgtaa 18
<210> 79
   <211> 18
   <212> DNA
   <213> Artificial Sequence
<220>
   <221> misc_feature
   <223> Antisense Oligonucleotide
<400> 79
   ctctttgcca tcctcctg 18
<210> 80
   <211> 18
   <212> DNA
   <213> Artificial Sequence
<220>
   <221> misc_feature
   <223> Antisense Oligonucleotide
<400> 80
   ctgtctctcc tgcactga 18
<210> 81
   <211> 18
   <212> DNA
   <213> Artificial Sequence
<220>
   <221> misc_feature
   <223> Antisense Oligonucleotide
<400> 81
   ggtgcagcct cactgtct 18
<210> 82
   <211> 18
   <212> DNA
   <213> Artificial Sequence
<220>
   <221> misc_feature
   <223> Antisense Oligonucleotide
<400> 82
   aactgcctgt ttgcccac 18
<210> 83
   <211> 18
   <212> DNA
   <213> Artificial Sequence
<220>
   <221> misc_feature
   <223> Antissense Oligonucleotide
<400> 83
   cttctgcctg cacccctg 18
<210> 84
   <211> 18
   <212> DNA
   <213> Artificial Sequence
<220>
   <221> misc_feature
   <223> Antisense Oligonucleotide
<400> 84
   actgactggg catagctc 18
<210> 85
   <211> 1004
   <212> DNA
   <213> Homo sapiens
<220>
   <221> CDS
   <222> (48)..(881)
<400> 85
   gcctcgctcg ggcgcccagt ggtcctgccg cctggtctca cctcgcc atg gtt cgt 56
                                                       Met Val Arg
                                                       1
   ctg cct ctg cag tgc gtc ctc tgg ggc tgc ttg ctg acc gct gtc cat        104
   Leu Pro Leu Gln Cys Val Leu Trp Gly Cys Leu Leu Thr Ala Val His
       5                   10                  15
   cca gaa cca ccc act gca tgc aga gaa aaa cag tac cta ata aac agt        152
   Pro Glu Pro Pro Thr Ala Cys Arg Glu Lys Gln Tyr Leu Ile Asn Ser
   20                  25                  30                  35
   cag tgc tgt tct ttg tgc cag cca gga cag aaa ctg gtg agt gac tgc        200
   Gln Cys Cys Ser Leu Cys Gln Pro Gly Gln Lys Leu Val Ser Asp Cys
                   40                  45                  50
   aca gag ttc act gaa acg gaa tgc ctt cct tgc ggt gaa agc gaa ttc        248
   Thr Glu Phe Thr Glu Thr Glu Cys Leu Pro Cys Gly Glu Ser Glu Phe
               55                  60                  65
   cta gac acc tgg aac aga gag aca cac tgc cac cag cac aaa tac tgc        296
   Leu Asp Thr Trp Asn Arg Glu Thr His Cys His Gln His Lys Tyr Cys
           70                  75                  80
   gac ccc aac cta ggg ctt cgg gtc cag cag aag ggc acc tca gaa aca        344
   Asp Pro Asn Leu Gly Leu Arg Val Gln Gln Lys Gly Thr Ser Glu Thr
       85                  90                  95
   gac acc atc tgc acc tgt gaa gaa ggc tgg cac tgt acg agt gag gcc        392
   Asp Thr Ile Cys Thr Cys Glu Glu Gly Trp His Cys Thr Ser Glu Ala
   100                 105                 110                 115
   tgt gag agc tgt gtc ctg cac cgc tca tgc tcg ccc ggc ttt ggg gtc        440
   Cys Glu Ser Cys Val Leu His Arg Ser Cys Ser Pro Gly Phe Gly Val
                   120                 125                 130
   aag cag att gct aca ggg gtt tct gat acc atc tgc gag ccc tgc cca        488
   Lys Gln Ile Ala Thr Gly Val Ser Asp Thr Ile Cys Glu Pro Cys Pro
               135                 140                 145
   gtc ggc ttc ttc tcc aat gtg tca tct gct ttc gaa aaa tgt cac cct        536
   Val Gly Phe Phe Ser Asn Val Ser Ser Ala Phe Glu Lys Cys His Pro
           150                 155                 160
   tgg aca agc tgt gag acc aaa gac ctg gtt gtg caa cag gca ggc aca        584
   Trp Thr Ser Cys Glu Thr Lys Asp Leu Val Val Gln Gln Ala Gly Thr
       165                 170                 175
   aac aag act gat gtt gtc tgt ggt ccc cag gat cgg ctg aga gcc ctg        632
   Asn Lys Thr Asp Val Val Cys Gly Pro Gln Asp Arg Leu Arg Ala Leu
   180                 185                 190                 195
   gtg gtg atc ccc atc atc ttc ggg atc ctg ttt gcc atc ctc ttg gtg        680
   Val Val Ile Pro Ile Ile Phe Gly Ile Leu Phe Ala Ile Leu Leu Val
                   200                 205                 210
   ctg gtc ttt atc aaa aag gtg gcc aag aag cca acc aat aag gcc ccc        728
   Leu Val Phe Ile Lys Lys Val Ala Lys Lys Pro Thr Asn Lys Ala Pro
               215                 220                 225
   cac ccc aag cag gaa ccc cag gag atc aat ttt ccc gac gat ctt cct        776
   His Pro Lys Gln Glu Pro Gln Glu Ile Asn Phe Pro Asp Asp Leu Pro
                   230                 235                 240
   ggc tcc aac act gct gct cca gtg cag gag act tta cat gga tgc caa      824
   Gly Ser Asn Thr Ala Ala Pro Val Gln Glu Thr Leu His Gly Cys Gln
       245                 250                 255
   ccg gtc acc cag gag gat ggc aaa gag agt cgc atc tca gtg cag gag      872
   Pro Val Thr Gln Glu Asp Gly Lys Glu Ser Arg Ile Ser Val Gln Glu
   260                 265                 270                 275
   aga cag tga ggctgcaccc acccaggagt gtggccacgt gggcaaacag              921
   Arg Gln
   gcagttggcc agagagcctg gtgctgctgc tgcaggggtg caggcagaag cggggagcta    981
   tgcccagtca gtgccagccc ctc                                           1004
<210> 86
   <211> 277
   <212> PRT
   <213> Homo sapiens
<400> 86
   Met Val Arg Leu Pro Leu Gln Cys Val Leu Trp Gly Cys Leu Leu Thr
   1               5                   10                  15
   Ala Val His Pro Glu Pro Pro Thr Ala Cys Arg Glu Lys Gln Tyr Leu
               20                  25                  30
   Ile Asn Ser Gln Cys Cys Ser Leu Cys Gln Pro Gly Gln Lys Leu Val
           35                  40                  45
   Ser Asp Cys Thr Glu Phe Thr Glu Thr Glu Cys Leu Pro Cys Gly Glu
       50                  55                  60
   Ser Glu Phe Leu Asp Thr Trp Asn Arg Glu Thr His Cys His Gln His
   65                  70                  75                  80
   Lys Tyr Cys Asp Pro Asn Leu Gly Leu Arg Val Gln Gln Lys Gly Thr
                   85                  90                  95
   Ser Glu Thr Asp Thr Ile Cys Thr Cys Glu Glu Gly Trp His Cys Thr
               100                 105                 110
   Ser Glu Ala Cys Glu Ser Cys Val Leu His Arg Ser Cys Ser Pro Gly
           115                 120                 125
   Phe Gly Val Lys Gln Ile Ala Thr Gly Val Ser Asp Thr Ile Cys Glu
       130                 135                 140
   Pro Cys Pro Val Gly Phe Phe Ser Asn Val Ser Ser Ala Phe Glu Lys
   145                 150                 155                 160
   Cys His Pro Trp Thr Ser Cys Glu Thr Lys Asp Leu Val Val Gln Gln
                   165                 170                 175
   Ala Gly Thr Asn Lys Thr Asp Val Val Cys Gly Pro Gln Asp Arg Leu
               180                 185                 190
   Arg Ala Leu Val Val Ile Pro Ile Ile Phe Gly Ile Leu Phe Ala Ile
           195                 200                 205
   Leu Leu Val Leu Val Phe Ile Lys Lys Val Ala Lys Lys Pro Thr Asn
       210                 215                 220
   Lys Ala Pro His Pro Lys Gln Glu Pro Gln Glu Ile Asn Phe Pro Asp
   225                 230                 235                 240
   Asp Leu Pro Gly Ser Asn Thr Ala Ala Pro Val Gln Glu Thr Leu His
                   245                 250                 255
   Gly Cys Gln Pro Val Thr Gln Glu Asp Gly Lys Glu Ser Arg Ile Ser
               260                 265                 270
   Val Gln Glu Arg Gln
           275
<210> 87
   <211> 23
   <212> DNA
   <213> Artificial Sequence
<220>
   <221> misc_feature
   <223> PCR Primer
<400> 87
   cagagttcac tgaaacggaa tgc 23
<210> 88
   <211> 23
   <212> DNA
   <213> Artificial Sequence
<220>
   <221> misc_feature
   <223> PCR Primer
<400> 88
   ggtggcagtg tgtctctctg ttc 23
<210> 89
   <211> 25
   <212> DNA
   <213> Artificial Sequence
<220>
   <221> misc_feature
   <223> PCR Probe
<400> 89
   ttccttgcgg tgaaagcgaa ttcct 25
<210> 90
   <211> 19
   <212> DNA
   <213> Artificial Sequence
<220>
   <221> misc_feature
   <223> PCR Primer
<400> 90
   gaaggtgaag gtcggagtc 19
<210> 91
   <211> 20
   <212> DNA
   <213> Artificial Sequence
<220>
   <221> misc_feature
   <223> PCR Primer
<400> 91
   gaagatggtg atgggatttc 20
<210> 92
   <211> 20
   <212> DNA
   <213> Artificial Sequence
<220>
   <221> misc_feature
   <223> PCR Probe
<400> 92
   caagcttccc gttctcagcc 20
<210> 93
   <211> 20
   <212> DNA
   <213> Artificial Sequence
<220>
   <221> misc_feature
   <223> Assembled Target Region
<400> 93
   agtggtcctg ccgcctggtc 20
<210> 94
   <211> 18
   <212> DNA
   <213> Artificial Sequence
<220>
   <221> misc_feature
   <223> Antisense Oligonucleotide
<400> 94
   gaacagcact gactgttt 18
<210> 95
   <211> 18
   <212> DNA
   <213> Artificial Sequence
<220>
   <221> misc_feature
   <223> Antisense Oligonucleotide
<400> 95
   agaacagcac tgactgtt 18
<210> 96
   <211> 18
   <212> DNA
   <213> Artificial Sequence
<220>
   <221> misc_feature
   <223> Antisense Oligonucleotide
<400> 96
   aagaacagca ctgactgt 18
<210> 97
   <211> 18
   <212> DNA
   <213> Artificial Sequence
<220>
   <221> misc_feature
   <223> Antisense Oligonucleotide
<400> 97
   aaagaacagc actgactg 18
<210> 98
   <211> 18
   <212> DNA
   <213> Artificial Sequence
<220>
   <221> misc_feature
   <223> Antisense Oligonucleotide
<400> 98
   caaagaacag cactgact 18
<210> 99
   <211> 18
   <212> DNA
   <213> Artificial Sequence
<220>
   <221> misc_feature
   <223> Antisense Oligonucleotide
<400> 99
   acaaagaaca gcactgac 18
<210> 100
   <211> 0
   <212> DNA
   <213> Artificial Sequence
<220>
   <221> misc_feature
   <223> Antisense Oligonulceotide
<400> 100
   000
<210> 101
   <211> 18
   <212> DNA
   <213> Artificial Sequence
<220>
   <221> misc_feature
   <223> Antisense Oligonucleotide
<400> 101
   gcacaaagaa cagcactg 18
<210> 102
   <211> 18
   <212> DNA
   <213> Artificial Sequence
<220>
   <221> misc_feature
   <223> Antisense Oligonucleotide
<400> 102
   ggcacaaaga acagcact 18
<210> 103
   <211> 18
   <212> DNA
   <213> Artificial Sequence
<220>
   <221> misc_feature
   <223> Antisense Oligonucleotide
<400> 103
   tggcacaaag aacagcac 18
<210> 104
   <211> 18
   <212> DNA
   <213> Artificial Sequence
<220>
   <221> misc_feature
   <223> Antisense Oligonucleotide
<400> 104
   gctggcacaa agaacagc 18
<210> 105
   <211> 18
   <212> DNA
   <213> Artificial Sequence
<220>
   <221> misc_feature
   <223> Antisense Oligonucleotide
<400> 105
   ggctggcaca aagaacag 18
<210> 106
   <211> 18
   <212> DNA
   <213> Artificial Sequence
<220>
   <221> misc_feature
   <223> Antisense Oligonucleotide
<400> 106
   tggctggcac aaagaaca 18
<210> 107
   <211> 18
   <212> DNA
   <213> Artificial Sequence
<220>
   <221> misc_feature
   <223> Antisense Oligonucleotide
<400> 107
   ctggctggca caaagaac 18
<210> 108
   <211> 18
   <212> DNA
   <213> Artificial Sequence
<220>
   <221> misc_feature
   <223> Antisense Oligonucleotide
<400> 108
   cctggctggc acaaagaa 18
<210> 109
   <211> 0
   <212> DNA
   <213> Artificial Sequence
<220>
   <221> misc_feature
   <223> Antisense Oligonucleotide
<400> 109
   000
<210> 110
   <211> 18
   <212> DNA
   <213> Artificial Sequence
<220>
   <221> misc_feature
   <223> Antisense Oligonucleotide
<400> 110
   gtcctggctg gcacaaag 18
<210> 111
   <211> 18
   <212> DNA
   <213> Artificial Sequence
<220>
   <221> misc_feature
   <223> Antisense Oligonucleotide
<400> 111
   tgtcctggct ggcacaaa 18
<210> 112
   <211> 0
   <212> DNA
   <213> Artificial Sequence
<220>
   <221> misc_feature
   <223> Antisense Oligonucleotide
<400> 112
   000
<210> 113
   <211> 18
   <212> DNA
   <213> Artificial Sequence
<220>
   <221> misc_feature
   <223> Antisense Oligonucleotide
<400> 113
   tctgtcctgg ctggcaca 18
<210> 114
   <211> 1058
   <212> DNA
   <213> Homo sapiens
<220>
   <221> CDS
   <222> (77)..(658)
<400> 114
   gccttgactt catctcagct ccagagcccg ccctctcttc ctgcagcctg ggaacttcag      60
   ccggctggag cccacc atg gct gca atc cga aag aag ctg gtg atc gtt ggg     112
                     Met Ala Ala Ile Arg Lys Lys Leu Val Ile Val Gly
                     1               5                   10
   gat ggt gcc tgt ggg aag acc tgc ctc ctc atc gtc ttc agc aag gat     160
   Asp Gly Ala Cys Gly Lys Thr Cys Leu Leu Ile Val Phe Ser Lys Asp
           15                  20                  25
   cag ttt ccg gag gtc tac gtc cct act gtc ttt gag aac tat att gcg     208
   Gln Phe Pro Glu Val Tyr Val Pro Thr Val Phe Glu Asn Tyr Ile Ala
       30                  35                  40
   gac att gag gtg gac ggc aag cag gtg gag ctg gct ctg tgg gac aca     256
   Asp Ile Glu Val Asp Gly Lys Gln Val Glu Leu Ala Leu Trp Asp Thr
   45                  50                  55                  60
   gca ggg cag gaa gac tat gat cga ctg cgg cct ctc tcc tac ccg gac     304
   Ala Gly Gln Glu Asp Tyr Asp Arg Leu Arg Pro Leu Ser Tyr Pro Asp
                   65                  70                  75
   act gat gtc atc ctc atg tgc ttc tcc atc gac agc cct gac agc ctg     352
   Thr Asp Val Ile Leu Met Cys Phe Ser Ile Asp Ser Pro Asp Ser Leu
               80                  85                  90
   gaa aac att cct gag aag tgg acc cca gag gtg aag cac ttc tgc ccc     400
   Glu Asn Ile Pro Glu Lys Trp Thr Pro Glu Val Lys His Phe Cys Pro
           95                  100                 105
   aac gtg ccc atc atc ctg gtg ggg aat aag aag gac ctg agg caa gac     448
   Asn Val Pro Ile Ile Leu Val Gly Asn Lys Lys Asp Leu Arg Gln Asp
       110                 115                 120
   gag cac acc agg aga gag ctg gcc aag atg aag cag gag ccc gtt cgg     496
   Glu His Thr Arg Arg Glu Leu Ala Lys Met Lys Gln Glu Pro Val Arg
   125                 130                 135                 140
   tct gag gaa ggc cgg gac atg gcg aac cgg atc agt gcc ttt ggc tac     544
   Ser Glu Glu Gly Arg Asp Met Ala Asn Arg Ile Ser Ala Phe Gly Tyr
                   145                 150                 155
   ctt gag tgc tca gcc aag acc aag gag gga gtg cgg gag gtg ttt gag     592
   Leu Glu Cys Ser Ala Lys Thr Lys Glu Gly Val Arg Glu Val Phe Glu
               160                 165                 170
   atg gcc act cgg gct ggc ctc cag gtc cgc aag aac aag cgt cgg agg     640
   Met Ala Thr Arg Ala Gly Leu Gln Val Arg Lys Asn Lys Arg Arg Arg
           175                 180                 185
   ggc tgt ccc att ctc tga gatcccccca aagggccctt ttcctacatg            688
   Gly Cys Pro Ile Leu
       190
   ccccctccct tcacaggggt acagaaatta tccccctaca accccagcct cctgagggct   748
   ccatactgaa ggctccattt tcagttccct cctgcccagg actgcattgt tttctagccc    808
   cgaggtgtgg cacgggccct ccctcccagc gctctgggag ccacgcctat gccctgccct    868
   tcctcatggg cccctgggga tcttgcccct ttgaccttcc ccaaaggatg gtcacacacc    928
   agcactttat acacttctgg ctcacaggaa agtgtctgca gtagggaccc agagtcccag    988
   gcccctggag ttgtttctgc aggggccttg tctctcactg catttggtca ggggggcatg    1048
   aataaaggct 1058
<210> 115
   <211> 193
   <212> PRT
   <213> Homo sapiens
<400> 115
   Met Ala Ala Ile Arg Lys Lys Leu Val Ile Val Gly Asp Gly Ala Cys
   1               5                   10                  15
   Gly Lys Thr Cys Leu Leu Ile Val Phe Ser Lys Asp Gln Phe Pro Glu
               20                  25                  30
   Val Tyr Val Pro Thr Val Phe Glu Asn Tyr Ile Ala Asp Ile Glu Val
           35                  40                  45
   Asp Gly Lys Gln Val Glu Leu Ala Leu Trp Asp Thr Ala Gly Gln Glu
       50                  55                  60
   Asp Tyr Asp Arg Leu Arg Pro Leu Ser Tyr Pro Asp Thr Asp Val Ile
   65                  70                  75                  80
   Leu Met Cys Phe Ser Ile Asp Ser Pro Asp Ser Leu Glu Asn Ile Pro
                   85                  90                  95
   Glu Lys Trp Thr Pro Glu Val Lys His Phe Cys Pro Asn Val Pro Ile
               100                 105                 110
   Ile Leu Val Gly Asn Lys Lys Asp Leu Arg Gln Asp Glu His Thr Arg
           115                 120                 125
   Arg Glu Leu Ala Lys Met Lys Gln Glu Pro Val Arg Ser Glu Glu Gly
       130                 135                 140
   Arg Asp Met Ala Asn Arg Ile Ser Ala Phe Gly Tyr Leu Glu Cys Ser
   145                 150                 155                 160
   Ala Lys Thr Lys Glu Gly Val Arg Glu Val Phe Glu Met Ala Thr Arg
                   165                 170                 175
   Ala Gly Leu Gln Val Arg Lys Asn Lys Arg Arg Arg Gly Cys Pro Ile
               180                 185                 190
   Leu
<210> 116
   <211> 23
   <212> DNA
   <213> Artificial Sequence
<220>
   <221> misc_feature
   <223> PCR Primer
<400> 116
   tgatgtcatc ctcatgtgct tct 23
<210> 117
   <211> 19
   <212> DNA
   <213> Artificial Sequence
<220>
   <221> misc_feature
   <223> PCR Primer
<400> 117
   ccaggatgat gggcacgtt 19
<210> 118
   <211> 23
   <212> DNA
   <213> Artificial Sequence
<220>
   <221> misc_feature
   <223> PCR Probe
<400> 118
   cgacagccct gacagcctgg aaa 23
<210> 119
   <211> 18
   <212> DNA
   <213> Artificial Sequence
<220>
   <221> misc_feature
   <223> Antisense Oligonucleotide
<400> 119
   gagctgagat gaagtcaa 18
<210> 120
   <211> 18
   <212> DNA
   <213> Artificial Sequence
<220>
   <221> misc_feature
   <223> Antisense Oligonucleotide
<400> 120
   gctgaagttc ccaggctg 18
<210> 121
   <211> 18
   <212> DNA
   <213> Artificial Sequence
<220>
   <221> misc_feature
   <223> Antisense Oligonucleotide
<400> 121
   ccggctgaag ttcccagg 18
<210> 122
   <211> 18
   <212> DNA
   <213> Artificial Sequence
<220>
   <221> misc_feature
   <223> Antisense Oligonucleotide
<400> 122
   ggcaccatcc ccaacgat 18
<210> 123
   <211> 18
   <212> DNA
   <213> Artificial Sequence
<220>
   <221> misc_feature
   <223> Antisense Oligonucleotide
<400> 123
   aggcaccatc cccaacga 18
<210> 124
   <211> 18
   <212> DNA
   <213> Artificial Sequence
<220>
   <221> misc_feature
   <223> Antisense Oligonucleotide
<400> 124
   tcccacaggc accatccc 18
<210> 125
   <211> 18
   <212> DNA
   <213> Artificial Sequence
<220>
   <221> misc_feature
   <223> Antisense Oligonucleotide
<400> 125
   aggtcttccc acaggcac 18
<210> 126
   <211> 18
   <212> DNA
   <213> Artificial Sequence
<220>
   <221> misc_feature
   <223> Antisense Oligonucleotide
<400> 126
   atgaggaggc aggtcttc 18
<210> 127
   <211> 18
   <212> DNA
   <213> Artificial Sequence
<220>
   <221> misc_feature
   <223> Antisense Oligonucleotide
<400> 127
   ttgctgaaga cgatgagg 18
<210> 128
   <211> 18
   <212> DNA
   <213> Artificial Sequence
<220>
   <221> misc_feature
   <223> Antisense Oligonucleotide
<400> 128
   tcaaagacag tagggacg 18
<210> 129
   <211> 18
   <212> DNA
   <213> Artificial Sequence
<220>
   <221> misc_feature
   <223> Antisense Oligonucleotide
<400> 129
   ttctcaaaga cagtaggg 18
<210> 130
   <211> 18
   <212> DNA
   <213> Artificial Sequence
<220>
   <221> misc_feature
   <223> Antisense Oligonucleotide
<400> 130
   agttctcaaa gacagtag 18
<210> 131
   <211> 18
   <212> DNA
   <213> Artificial Sequence
<220>
   <221> misc_feature
   <223> Antisense Oligonucleotide
<400> 131
   tgttttccag gctgtcag 18
<210> 132
   <211> 18
   <212> DNA
   <213> Artificial Sequence
<220>
   <221> misc_feature
   <223> Antisense Oligonucleotide
<400> 132
   tcgtcttgcc tcaggtcc 18
<210> 133
   <211> 18
   <212> DNA
   <213> Artificial Sequence
<220>
   <221> misc_feature
   <223> Antisense Oligonucleotide
<400> 133
   gtgtgctcgt cttgcctc 18
<210> 134
   <211> 18
   <212> DNA
   <213> Artificial Sequence
<220>
   <221> misc_feature
   <223> Antisense Oligonucleotide
<400> 134
   ctcctggtgt gctcgtct 18
<210> 135
   <211> 18
   <212> DNA
   <213> Artificial Sequence
<220>
   <221> misc_feature
   <223> Antisense Oligonucleotide
<400> 135
   cagaccgaac gggctcct 18
<210> 136
   <211> 18
   <212> DNA
   <213> Artificial Sequence
<220>
   <221> misc_feature
   <223> Antisense Oligonucleotide
<400> 136
   ttcctcagac cgaacggg 18
<210> 137
   <211> 18
   <212> DNA
   <213> Artificial Sequence
<220>
   <221> misc_feature
   <223> Antisense Oligonucleotide
<400> 137
   actcaaggta gccaaagg 18
<210> 138
   <211> 18
   <212> DNA
   <213> Artificial Sequence
<220>
   <221> misc_feature
   <223> Antisense Oligonucleotide
<400> 138
   ctcccgcact ccctcctt 18
<210> 139
   <211> 18
   <212> DNA
   <213> Artificial Sequence
<220>
   <221> misc_feature
   <223> Antisense Oligonucleotide
<400> 139
   ctcaaacacc tcccgcac 18
<210> 140
   <211> 18
   <212> DNA
   <213> Artificial Sequence
<220>
   <221> misc_feature
   <223> Antisense Oligonucleotide
<400> 140
   ggccatctca aacacctc 18
<210> 141
   <211> 18
   <212> DNA
   <213> Artificial Sequence
<220>
   <221> misc_feature
   <223> Antisense Oligonucleotide
<400> 141
   cttgttcttg cggacctg 18
<210> 142
   <211> 18
   <212> DNA
   <213> Artificial Sequence
<220>
   <221> misc_feature
   <223> Antisense Oligonucleotide
<400> 142
   cccctccgac gcttgttc 18
<210> 143
   <211> 18
   <212> DNA
   <213> Artificial Sequence
<220>
   <221> misc_feature
   <223> Antisense Oligonucleotide
<400> 143
   gtatggagcc ctcaggag 18
<210> 144
   <211> 18
   <212> DNA
   <213> Artificial Sequence
<220>
   <221> misc_feature
   <223> Antisense Oligonucleotide
<400> 144
   gagccttcag tatggagc 18
<210> 145
   <211> 18
   <212> DNA
   <213> Artificial Sequence
<220>
   <221> misc_feature
   <223> Antisense Oligonucleotide
<400> 145
   gaaaatggag ccttcagt 18
<210> 146
   <211> 18
   <212> DNA
   <213> Artificial Sequence
<220>
   <221> misc_feature
   <223> Antisense Oligonucleotide
<400> 146
   ggaactgaaa atggagcc 18
<210> 147
   <211> 18
   <212> DNA
   <213> Artificial Sequence
<220>
   <221> misc_feature
   <223> Antisense Oligonucleotide
<400> 147
   ggagggaact gaaaatgg 18
<210> 148
   <211> 18
   <212> DNA
   <213> Artificial Sequence
<220>
   <221> misc_feature
   <223> Antisense Oligonucleotide
<400> 148
   gcaggaggga actgaaaa 18
<210> 149
   <211> 18
   <212> DNA
   <213> Artificial Sequence
<220>
   <221> misc_feature
   <223> Antisense Oligonucleotide
<400> 149
   agggcagggc ataggcgt 18
<210> 150
   <211> 18
   <212> DNA
   <213> Artificial Sequence
<220>
   <221> misc_feature
   <223> Antisense Oligonucleotide
<400> 150
   ggaagggcag ggcatagg 18
<210> 151
   <211> 18
   <212> DNA
   <213> Artificial Sequence
<220>
   <221> misc_feature
   <223> Antisense Oligonucleotide
<400> 151
   catgaggaag ggcagggc 18
<210> 152
   <211> 18
   <212> DNA
   <213> Artificial Sequence
<220>
   <221> misc_feature
   <223> Antisense Oligonucleotide
<400> 152
   taaagtgctg gtgtgtga 18
<210> 153
   <211> 18
   <212> DNA
   <213> Artificial Sequence
<220>
   <221> misc_feature
   <223> Antisense Oligonucleotide
<400> 153
   cctgtgagcc agaagtgt 18
<210> 154
   <211> 18
   <212> DNA
   <213> Artificial Sequence
<220>
   <221> misc_feature
   <223> Antisense Oligonucleotide
<400> 154
   ttcctgtgag ccagaagt 18
<210> 155
   <211> 18
   <212> DNA
   <213> Artificial Sequence
<220>
   <221> misc_feature
   <223> Antisense Oligonucleotide
<400> 155
   cactttcctg tgagccag 18
<210> 156
   <211> 18
   <212> DNA
   <213> Artificial Sequence
<220>
   <221> misc_feature
   <223> Antisense Oligonucleotide
<400> 156
   agacactttc ctgtgagc 18
<210> 157
   <211> 18
   <212> DNA
   <213> Artificial Sequence
<220>
   <221> misc_feature
   <223> Antisense Oligonucleotide
<400> 157
   actctgggtc cctactgc 18
<210> 158
   <211> 18
   <212> DNA
   <213> Artificial Sequence
<220>
   <221> misc_feature
   <223> Antisense Oligonucleotide
<400> 158
   tgcagaaaca actccagg 18
<210> 159
   <211> 3076
   <212> DNA
   <213> Homo sapiens
<220>
   <221> CDS
   <222> (725)..(2539)
<400> 159
   gaattcaaaa tgtcttcagt tgtaaatctt accattattt tacgtacctc taagaaataa     60
   aagtgcttct aattaaaata tgatgtcatt aattatgaaa tacttcttga taacagaagt    120
   tttaaaatag ccatcttaga atcagtgaaa tatggtaatg tattattttc ctcctttgag    180
   ttaggtcttg tgcttttttt tcctggccac taaatttcac aatttccaaa aagcaaaata    240
   aacatattct gaatattttt gctgtgaaac acttgacagc agagctttcc accatgaaaa    300
   gaagcttcat gagtcacaca ttacatcttt gggttgattg aatgccactg aaacattcta    360
   gtagcctgga gaagttgacc tacctgtgga gatgcctgcc attaaatggc atcctgatgg    420
   cttaatacac atcactcttc tgtgaagggt tttaattttc aacacagctt actctgtagc    480
   atcatgttta cattgtatgt ataaagatta tacaaaggtg caattgtgta tttcttcctt    540
   aaaatgtatc agtataggat ttagaatctc catgttgaaa ctctaaatgc atagaaataa    600
   aaataataaa aaatttttca ttttggcttt tcagcctagt attaaaactg ataaaagcaa    660
   agccatgcac aaaactacct ccctagagaa aggctagtcc cttttcttcc ccattcattt    720
   catt atg aac ata gta gaa aac agc ata ttc tta tca aat ttg atg aaa      769
        Met Asn Ile Val Glu Asn Ser Ile Phe Leu Ser Asn Leu Met Lys
        1               5                   10                  15
   agc gcc aac acg ttt gaa ctg aaa tac gac ttg tca tgt gaa ctg tac      817
   Ser Ala Asn Thr Phe Glu Leu Lys Tyr Asp Leu Ser Cys Glu Leu Tyr
                   20                  25                  30
   cga atg tct acg tat tcc act ttt cct gct ggg gtt cct gtc tca gaa      865
   Arg Met Ser Thr Tyr Ser Thr Phe Pro Ala Gly Val Pro Val Ser Glu
               35                  40                  45
   agg agt ctt gct cgt gct ggt ttc tat tac act ggt gtg aat gac aag      913
   Arg Ser Leu Ala Arg Ala Gly Phe Tyr Tyr Thr Gly Val Asn Asp Lys
           50                  55                  60
   gtc aaa tgc ttc tgt tgt ggc ctg atg ctg gat aac tgg aaa aga gga      961
   Val Lys Cys Phe Cys Cys Gly Leu Met Leu Asp Asn Trp Lys Arg Gly
       65                  70                  75
   gac agt cct act gaa aag cat aaa aag ttg tat cct agc tgc aga ttc     1009
   Asp Ser Pro Thr Glu Lys His Lys Lys Leu Tyr Pro Ser Cys Arg Phe
   80                  85                  90                  95
   gtt cag agt cta aat tcc gtt aac aac ttg gaa gct acc tct cag cct     1057
   Val Gln Ser Leu Asn Ser Val Asn Asn Leu Glu Ala Thr Ser Gln Pro
                   100                 105                 110
   act ttt cct tct tca gta aca aat tcc aca cac tca tta ctt ccg ggt     1105
   Thr Phe Pro Ser Ser Val Thr Asn Ser Thr His Ser Leu Leu Pro Gly
               115                 120                 125
   aca gaa aac agt gga tat ttc cgt ggc tct tat tca aac tct cca tca     1153
   Thr Glu Asn Ser Gly Tyr Phe Arg Gly Ser Tyr Ser Asn Ser Pro Ser
           130                 135                 140
   aat cct gta aac tcc aga gca aat caa gat ttt tct gcc ttg atg aga      1201
   Asn Pro Val Asn Ser Arg Ala Asn Gln Asp Phe Ser Ala Leu Met Arg
       145                 150                 155
   agt tcc tac cac tgt gca atg aat aac gaa aat gcc aga tta ctt act      1249
   Ser Ser Tyr His Cys Ala Met Asn Asn Glu Asn Ala Arg Leu Leu Thr
   160                 165                 170                 175
   ttt cag aca tgg cca ttg act ttt ctg tcg cca aca gat ctg gca aaa      1297
   Phe Gln Thr Trp Pro Leu Thr Phe Leu Ser Pro Thr Asp Leu Ala Lys
                   180                 185                 190
   gca ggc ttt tac tac ata gga cct gga gac aga gtg gct tgc ttt gcc      1345
   Ala Gly Phe Tyr Tyr Ile Gly Pro Gly Asp Arg Val Ala Cys Phe Ala
               195                 200                 205
   tgt ggt gga aaa ttg agc aat tgg gaa ccg aag gat aat gct atg tca      1393
   Cys Gly Gly Lys Leu Ser Asn Trp Glu Pro Lys Asp Asn Ala Met Ser
           210                 215                 220
   gaa cac ctg aga cat ttt ccc aaa tgc cca ttt ata gaa aat cag ctt      1441
   Glu His Leu Arg His Phe Pro Lys Cys Pro Phe Ile Glu Asn Gln Leu
       225                 230                 235
   caa gac act tca aga tac aca gtt tct aat ctg agc atg cag aca cat      1489
   Gln Asp Thr Ser Arg Tyr Thr Val Ser Asn Leu Ser Met Gln Thr His
   240                 245                 250                 255
   gca gcc cgc ttt aaa aca ttc ttt aac tgg ccc tct agt gtt cta gtt      1537
   Ala Ala Arg Phe Lys Thr Phe Phe Asn Trp Pro Ser Ser Val Leu Val
                   260                 265                 270
   aat cct gag cag ctt gca agt gcg ggt ttt tat tat gtg ggt aac agt      1585
   Asn Pro Glu Gln Leu Ala Ser Ala Gly Phe Tyr Tyr Val Gly Asn Ser
               275                 280                 285
   gat gat gtc aaa tgc ttt tgc tgt gat ggt gga ctc agg tgt tgg gaa      1633
   Asp Asp Val Lys Cys Phe Cys Cys Asp Gly Gly Leu Arg Cys Trp Glu
           290                 295                 300
   tct gga gat gat cca tgg gtt caa cat gcc aag tgg ttt cca agg tgt      1681
   Ser Gly Asp Asp Pro Trp Val Gln His Ala Lys Trp Phe Pro Arg Cys
       305                 310                 315
   gag tac ttg ata aga att aaa gga cag gag ttc atc cgt caa gtt caa      1729
   Glu Tyr Leu Ile Arg Ile Lys Gly Gln Glu Phe Ile Arg Gln Val Gln
   320                 325                 330                 335
   gcc agt tac cct cat cta ctt gaa cag ctg cta tcc aca tca gac agc      1777
   Ala Ser Tyr Pro His Leu Leu Glu Gln Leu Leu Ser Thr Ser Asp Ser
                   340                 345                 350
   cca gga gat gaa aat gca gag tca tca att atc cat ttt gaa cct gga      1825
   Pro Gly Asp Glu Asn Ala Glu Ser Ser Ile Ile His Phe Glu Pro Gly
               355                 360                 365
   gaa gac cat tca gaa gat gca atc atg atg aat act cct gtg att aat      1873
   Glu Asp His Ser Glu Asp Ala Ile Met Met Asn Thr Pro Val Ile Asn
           370                 375                 380
   gct gcc gtg gaa atg ggc ttt agt aga agc ctg gta aaa cag aca gtt      1921
   Ala Ala Val Glu Met Gly Phe Ser Arg Ser Leu Val Lys Gln Thr Val
       385                 390                 395
   caa aga aaa atc cta gca act gga gag aat tat aga cta gtc aat gat      1969
   Gln Arg Lys Ile Leu Ala Thr Gly Glu Asn Tyr Arg Leu Val Asn Asp
   400                 405                 410                 415
   ctt gtg tta gac tta ctc aat gca gaa gat gaa ata agg gaa gag gag      2017
   Leu Val Leu Asp Leu Leu Asn Ala Glu Asp Glu Ile Arg Glu Glu Glu
                   420                 425                 430
   aga gaa aga gca act gag gaa aaa gaa tca aat gat tta tta tta atc      2065
   Arg Glu Arg Ala Thr Glu Glu Lys Glu Ser Asn Asp Leu Leu Leu Ile
               435                 440                 445
   cgg aag aat aga atg gca ctt ttt caa cat ttg act tgt gta att cca      2113
   Arg Lys Asn Arg Met Ala Leu Phe Gln His Leu Thr Cys Val Ile Pro
           450                 455                 460
   atc ctg gat agt cta cta act gcc gga att att aat gaa caa gaa cat      2161
   Ile Leu Asp Ser Leu Leu Thr Ala Gly Ile Ile Asn Glu Gln Glu His
       465                 470                 475
   gat gtt att aaa cag aag aca cag acg tct tta caa gca aga gaa ctg      2209
   Asp Val Ile Lys Gln Lys Thr Gln Thr Ser Leu Gln Ala Arg Glu Leu
   480                 485                 490                 495
   att gat acg att tta gta aaa gga aat att gca gcc act gta ttc aga      2257
   Ile Asp Thr Ile Leu Val Lys Gly Asn Ile Ala Ala Thr Val Phe Arg
                   500                 505                 510
   aac tct ctg caa gaa gct gaa gct gtg tta tat gag cat tta ttt gtg      2305
   Asn Ser Leu Gln Glu Ala Glu Ala Val Leu Tyr Glu His Leu Phe Val
               515                 520                 525
   caa cag gac ata aaa tat att ccc aca gaa gat gtt tca gat cta cca      2353
   Gln Gln Asp Ile Lys Tyr Ile Pro Thr Glu Asp Val Ser Asp Leu Pro
           530                 535                 540
   gtg gaa gaa caa ttg cgg aga cta caa gaa gaa aga aca tgt aaa gtg      2401
   Val Glu Glu Gln Leu Arg Arg Leu Gln Glu Glu Arg Thr Cys Lys Val
       545                 550                 555
   tgt atg gac aaa gaa gtg tcc ata gtg ttt att cct tgt ggt cat cta      2449
   Cys Met Asp Lys Glu Val Ser Ile Val Phe Ile Pro Cys Gly His Leu
   560                 565                 570                 575
   gta gta tgc aaa gat tgt gct cct tct tta aga aag tgt cct att tgt      2497
   Val Val Cys Lys Asp Cys Ala Pro Ser Leu Arg Lys Cys Pro Ile Cys
                   580                 585                 590
   agg agt aca atc aag ggt aca gtt cgt aca ttt ctt tca tga              2539
   Arg Ser Thr Ile Lys Gly Thr Val Arg Thr Phe Leu Ser
               595                 600
   agaagaacca aaacatcatc taaactttag aattaattta ttaaatgtat tataacttta   2599
   acttttatcc taatttggtt tccttaaaat ttttatttat ttacaactca aaaaacattg   2659
   ttttgtgtaa catatttata tatgtatcta aaccatatga acatatattt tttagaaact 2719
   aagagaatga taggcttttg ttcttatgaa cgaaaaagag gtagcactac aaacacaata 2779
   ttcaatcaaa atttcagcat tattgaaatt gtaagtgaag taaaacttaa gatatttgag 2839
   ttaaccttta agaattttaa atattttggc attgtactaa tacctggttt tttttttgtt 2899
   ttgttttttt gtacagacag ggcagcatac tgagaccctg cctttaaaaa caaacagaac 2959
   aaaaacaaaa caccagggac acatttctct gtcttttttg atcagtgtcc tatacatcga 3019
   aggtgtgcat atatgttgaa tgacatttta gggacatggt gtttttataa agaattc 3076
<210> 160
   <211> 604
   <212> PRT
   <213> Homo sapiens
<400> 160
   Met Asn Ile Val Glu Asn Ser Ile Phe Leu Ser Asn Leu Met Lys Ser
   1               5                   10                  15
   Ala Asn Thr Phe Glu Leu Lys Tyr Asp Leu Ser Cys Glu Leu Tyr Arg
               20                  25                  30
   Met Ser Thr Tyr Ser Thr Phe Pro Ala Gly Val Pro Val Ser Glu Arg
           35                  40                  45
   Ser Leu Ala Arg Ala Gly Phe Tyr Tyr Thr Gly Val Asn Asp Lys Val
       50                  55                  60
   Lys Cys Phe Cys Cys Gly Leu Met Leu Asp Asn Trp Lys Arg Gly Asp
   65                  70                  75                  80
   Ser Pro Thr Glu Lys His Lys Lys Leu Tyr Pro Ser Cys Arg Phe Val
                   85                  90                  95
   Gln Ser Leu Asn Ser Val Asn Asn Leu Glu Ala Thr Ser Gln Pro Thr
               100                 105                 110
   Phe Pro Ser Ser Val Thr Asn Ser Thr His Ser Leu Leu Pro Gly Thr
           115                 120                 125
   Glu Asn Ser Gly Tyr Phe Arg Gly Ser Tyr Ser Asn Ser Pro Ser Asn
       130                 135                 140
   Pro Val Asn Ser Arg Ala Asn Gln Asp Phe Ser Ala Leu Met Arg Ser
   145                 150                 155                 160
   Ser Tyr His Cys Ala Met Asn Asn Glu Asn Ala Arg Leu Leu Thr Phe
   165 170 175
   Gln Thr Trp Pro Leu Thr Phe Leu Ser Pro Thr Asp Leu Ala Lys Ala
   180 185 190
   Gly Phe Tyr Tyr Ile Gly Pro Gly Asp Arg Val Ala Cys Phe Ala Cys
   195 200 205
   Gly Gly Lys Leu Ser Asn Trp Glu Pro Lys Asp Asn Ala Met Ser Glu
   210 215 220
   His Leu Arg His Phe Pro Lys Cys Pro Phe Ile Glu Asn Gln Leu Gln
   225 230 235 240
   Asp Thr Ser Arg Tyr Thr Val Ser Asn Leu Ser Met Gln Thr His Ala
   245 250 255
   Ala Arg Phe Lys Thr Phe Phe Asn Trp Pro Ser Ser Val Leu Val Asn
   260 265 270
   Pro Glu Gln Leu Ala Ser Ala Gly Phe Tyr Tyr Val Gly Asn Ser Asp
   275 280 285
   Asp Val Lys Cys Phe Cys Cys Asp Gly Gly Leu Arg Cys Trp Glu Ser
   290 295 300
   Gly Asp Asp Pro Trp Val Gln His Ala Lys Trp Phe Pro Arg Cys Glu
   305 310 315 320
   Tyr Leu Ile Arg Ile Lys Gly Gln Glu Phe Ile Arg Gln Val Gln Ala
   325 330 335
   Ser Tyr Pro His Leu Leu Glu Gln Leu Leu Ser Thr Ser Asp Ser Pro
   340 345 350
   Gly Asp Glu Asn Ala Glu Ser Ser Ile Ile His Phe Glu Pro Gly Glu
   355 360 365
   Asp His Ser Glu Asp Ala Ile Met Met Asn Thr Pro Val Ile Asn Ala
   370 375 380
   Ala Val Glu Met Gly Phe Ser Arg Ser Leu Val Lys Gln Thr Val Gln
   385 390 395 400
   Arg Lys Ile Leu Ala Thr Gly Glu Asn Tyr Arg Leu Val Asn Asp Leu
                   405                 410                 415
   Val Leu Asp Leu Leu Asn Ala Glu Asp Glu Ile Arg Glu Glu Glu Arg
               420                 425                 430
   Glu Arg Ala Thr Glu Glu Lys Glu Ser Asn Asp Leu Leu Leu Ile Arg
           435                 440                 445
   Lys Asn Arg Met Ala Leu Phe Gln His Leu Thr Cys Val Ile Pro Ile
       450                 455                 460
   Leu Asp Ser Leu Leu Thr Ala Gly Ile Ile Asn Glu Gln Glu His Asp
   465                 470                 475                 480
   Val Ile Lys Gln Lys Thr Gln Thr Ser Leu Gln Ala Arg Glu Leu Ile
                   485                 490                 495
   Asp Thr Ile Leu Val Lys Gly Asn Ile Ala Ala Thr Val Phe Arg Asn
               500                 505                 510
   Ser Leu Gln Glu Ala Glu Ala Val Leu Tyr Glu His Leu Phe Val Gln
           515                 520                 525
   Gln Asp Ile Lys Tyr Ile Pro Thr Glu Asp Val Ser Asp Leu Pro Val
       530                 535                 540
   Glu Glu Gln Leu Arg Arg Leu Gln Glu Glu Arg Thr Cys Lys Val Cys
   545                 550                 555                 560
   Met Asp Lys Glu Val Ser Ile Val Phe Ile Pro Cys Gly His Leu Val
                   565                 570                 575
   Val Cys Lys Asp Cys Ala Pro Ser Leu Arg Lys Cys Pro Ile Cys Arg
               580                 585                 590
   Ser Thr Ile Lys Gly Thr Val Arg Thr Phe Leu Ser
           595                 600
<210> 161
   <211> 22
   <212> DNA
   <213> Artificial Sequence
<220>
   <221> misc_feature
   <223> PCR Primer
<400> 161
   ggactcaggt gttgggaatc tg 22
<210> 162
   <211> 24
   <212> DNA
   <213> Artificial Sequence
<220>
   <221> misc_feature
   <223> PCR Primer
<400> 162
   caagtactca caccttggaa acca 24
<210> 163
   <211> 27
   <212> DNA
   <213> Artificial Sequence
<220>
   <221> misc_feature
   <223> PCR Probe
<400> 163
   agatgatcca tgggttcaac atgccaa 27
<210> 164
   <211> 18
   <212> DNA
   <213> Artificial Sequence
<220>
   <221> misc_feature
   <223> Antisense Oligonucleotide
<400> 164
   actgaagaca ttttgaat 18
<210> 165
   <211> 18
   <212> DNA
   <213> Artificial Sequence
<220>
   <221> misc_feature
   <223> Antisense Oligonucleotide
<400> 165
   cttagaggta cgtaaaat 18
<210> 166
   <211> 18
   <212> DNA
   <213> Artificial Sequence
<220>
   <221> misc_feature
   <223> Antisense Oligonucleotide
<400> 166
   gcacttttat ttcttaga 18
<210> 167
   <211> 18
   <212> DNA
   <213> Artificial Sequence
<220>
   <221> misc_feature
   <223> Antisense Oligonucleotide
<400> 167
   attttaatta gaagcact 18
<210> 168
   <211> 18
   <212> DNA
   <213> Artificial Sequence
<220>
   <221> misc_feature
   <223> Antisense Oligonucleotide
<400> 168
   accatatttc actgattc 18
<210> 169
   <211> 18
   <212> DNA
   <213> Artificial Sequence
<220>
   <221> misc_feature
   <223> Antisense Oligonucleotide
<400> 169
   ctaactcaaa ggaggaaa 18
<210> 170
   <211> 18
   <212> DNA
   <213> Artificial Sequence
<220>
   <221> misc_feature
   <223> Antisense Oligonucleotide
<400> 170
   cacaagacct aactcaaa 18
<210> 171
   <211> 18
   <212> DNA
   <213> Artificial Sequence
<220>
   <221> misc_feature
   <223> Antisense Oligonucleotide
<400> 171
   gctctgctgt caagtgtt 18
<210> 172
   <211> 18
   <212> DNA
   <213> Artificial Sequence
<220>
   <221> misc_feature
   <223> Antisense Oligonucleotide
<400> 172
   tgtgtgactc atgaagct 18
<210> 173
   <211> 18
   <212> DNA
   <213> Artificial Sequence
<220>
   <221> misc_feature
   <223> Antisense Oligonucleotide
<400> 173
   ttcagtggca ttcaatca 18
<210> 174
   <211> 18
   <212> DNA
   <213> Artificial Sequence
<220>
   <221> misc_feature
   <223> Antisense Oligonucleotide
<400> 174
   cttctccagg ctactaga 18
<210> 175
   <211> 18
   <212> DNA
   <213> Artificial Sequence
<220>
   <221> misc_feature
   <223> Antisense Oligonucleotide
<400> 175
   ggtcaacttc tccaggct 18
<210> 176
   <211> 18
   <212> DNA
   <213> Artificial Sequence
<220>
   <221> misc_feature
   <223> Antisense Oligonucleotide
<400> 176
   taaaaccctt cacagaag 18
<210> 177
   <211> 18
   <212> DNA
   <213> Artificial Sequence
<220>
   <221> misc_feature
   <223> Antisense Oligonucleotide
<400> 177
   ttaaggaaga aatacaca 18
<210> 178
   <211> 18
   <212> DNA
   <213> Artificial Sequence
<220>
   <221> misc_feature
   <223> Antisense Oligonucleotide
<400> 178
   gcatggcttt gcttttat 18
<210> 179
   <211> 18
   <212> DNA
   <213> Artificial Sequence
<220>
   <221> misc_feature
   <223> Antisense Oligonucleotide
<400> 179
   caaacgtgtt ggcgcttt 18
<210> 180
   <211> 18
   <212> DNA
   <213> Artificial Sequence
<220>
   <221> misc_feature
   <223> Antisense Oligonucleotide
<400> 180
   agcaggaaaa gtggaata 18
<210> 181
   <211> 18
   <212> DNA
   <213> Artificial Sequence
<220>
   <221> misc_feature
   <223> Antisense Oligonucleotide
<400> 181
   ttaacggaat ttagactc 18
<210> 182
   <211> 18
   <212> DNA
   <213> Artificial Sequence
<220>
   <221> misc_feature
   <223> Antisense Oligonucleotide
<400> 182
   atttgttact gaagaagg 18
<210> 183
   <211> 18
   <212> DNA
   <213> Artificial Sequence
<220>
   <221> misc_feature
   <223> Antisense Oligonucleotide
<400> 183
   agagccacgg aaatatcc 18
<210> 184
   <211> 18
   <212> DNA
   <213> Artificial Sequence
<220>
   <221> misc_feature
   <223> Antisense Oligonucleotide
<400> 184
   aaatcttgat ttgctctg 18
<210> 185
   <211> 18
   <212> DNA
   <213> Artificial Sequence
<220>
   <221> misc_feature
   <223> Antisense Oligonucleotide
<400> 185
   gtaagtaatc tggcattt 18
<210> 186
   <211> 18
   <212> DNA
   <213> Artificial Sequence
<220>
   <221> misc_feature
   <223> Antisense Oligonucleotide
<400> 186
   agcaagccac tctgtctc 18
<210> 187
   <211> 18
   <212> DNA
   <213> Artificial Sequence
<220>
   <221> misc_feature
   <223> Antisense Oligonucleotide
<400> 187
   tgaagtgtct tgaagctg 18
<210> 188
   <211> 18
   <212> DNA
   <213> Artificial Sequence
<220>
   <221> misc_feature
   <223> Antisense Oligonucleotide
<400> 188
   tttgacatca tcactgtt 18
<210> 189
   <211> 18
   <212> DNA
   <213> Artificial Sequence
<220>
   <221> misc_feature
   <223> Antisense Oligonucleotide
<400> 189
   tggcttgaac ttgacgga 18
<210> 190
   <211> 18
   <212> DNA
   <213> Artificial Sequence
<220>
   <221> misc_feature
   <223> Antisense Oligonucleotide
<400> 190
   tcatctcctg ggctgtct 18
<210> 191
   <211> 18
   <212> DNA
   <213> Artificial Sequence
<220>
   <221> misc_feature
   <223> Antisense Oligonucleotide
<400> 191
   gcagcattaa tcacagga 18
<210> 192
   <211> 18
   <212> DNA
   <213> Artificial Sequence
<220>
   <221> misc_feature
   <223> Antisense Oligonucleotide
<400> 192
   tttctctctc ctcttccc 18
<210> 193
   <211> 18
   <212> DNA
   <213> Artificial Sequence
<220>
   <221> misc_feature
   <223> Antisense Oligonucleotide
<400> 193
   aacatcatgt tcttgttc 18
<210> 194
   <211> 18
   <212> DNA
   <213> Artificial Sequence
<220>
   <221> misc_feature
   <223> Antisense Oligonucleotide
<400> 194
   atataacaca gcttcagc 18
<210> 195
   <211> 18
   <212> DNA
   <213> Artificial Sequence
<220>
   <221> misc_feature
   <223> Antisense Oligonucleotide
<400> 195
   aattgttctt ccactggt 18
<210> 196
   <211> 18
   <212> DNA
   <213> Artificial Sequence
<220>
   <221> misc_feature
   <223> Antisense Oligonucleotide
<400> 196
   aagaaggagc acaatctt 18
<210> 197
   <211> 18
   <212> DNA
   <213> Artificial Sequence
<220>
   <221> misc_feature
   <223> Antisense Oligonucleotide
<400> 197
   gaaaccaaat taggataa 18
<210> 198
   <211> 18
   <212> DNA
   <213> Artificial Sequence
<220>
   <221> misc_feature
   <223> Antisense Oligonucleotide
<400> 198
   tgtagtgcta cctctttt 18
<210> 199
   <211> 18
   <212> DNA
   <213> Artificial Sequence
<220>
   <221> misc_feature
   <223> Antisense Oligonucleotide
<400> 199
   ctgaaatttt gattgaat 18
<210> 200
   <211> 18
   <212> DNA
   <213> Artificial Sequence
<220>
   <221> misc_feature
   <223> Antisense Oligonucleotide
<400> 200
   tacaatttca ataatgct 18
<210> 201
   <211> 18
   <212> DNA
   <213> Artificial Sequence
<220>
   <221> misc_feature
   <223> Antisense Oligonucleotide
<400> 201
   gggtctcagt atgctgcc 18
<210> 202
   <211> 18
   <212> DNA
   <213> Artificial Sequence
<220>
   <221> misc_feature
   <223> Antisense Oligonucleotide
<400> 202
   ccttcgatgt ataggaca 18
<210> 203
   <211> 18
   <212> DNA
   <213> Artificial Sequence
<220>
   <221> misc_feature
   <223> Antisense Oligonucleotide
<400> 203
   catgtcccta aaatgtca 18
<210> 204
   <211> 2266
   <212> DNA
   <213> Homo sapiens
<220>
   <221> CDS
   <222> (316)..(1602)
<400> 204
   aattccgagc tgtagggaaa cgcaggggcg gcttctaggt gctgccgccg ccaccgccac 60
   caccacctcc accgccgcct cggaacccag gcctgggggg cggtggggcc gcgtatggag 120
   cccccgcccc ccggagctgc caacattgcc aacgccaccg ccacgctaca cacagcctca 180
   actttcagga gacccgtccg tggccttatt tattccaccc ttcctgtaca tcgtagcgaa    240
   tcaatccgtg gcgccgcact cctccgcatc cctctttaac agtacccctg ggatggcgtg    300
   agcactcccc cagcg atg gac cca tct gtg acg ctg tgg cag ttt ctg ctg     351
                    Met Asp Pro Ser Val Thr Leu Trp Gln Phe Leu Leu
                    1               5                   10
   cag ctg ctg aga gag caa ggc aat ggc cac atc atc tcc tgg act tca      399
   Gln Leu Leu Arg Glu Gln Gly Asn Gly His Ile Ile Ser Trp Thr Ser
           15                  20                  25
   cgg gat ggt ggt gaa ttc aag ctg gtg gat gca gag gag gtg gcc cgg      447
   Arg Asp Gly Gly Glu Phe Lys Leu Val Asp Ala Glu Glu Val Ala Arg
       30                  35                  40
   ctg tgg gga cta cgc aag aac aag acc aac atg aat tac gac aag ctc      495
   Leu Trp Gly Leu Arg Lys Asn Lys Thr Asn Met Asn Tyr Asp Lys Leu
   45                  50                  55                  60
   agc cgg gcc ttg cgg tac tac tat gac aag aac atc atc cgc aag gtg      543
   Ser Arg Ala Leu Arg Tyr Tyr Tyr Asp Lys Asn Ile Ile Arg Lys Val
                   65                  70                  75
   agc ggc cag aag ttc gtc tac aag ttt gtg tcc tac cct gag gtc gca      591
   Ser Gly Gln Lys Phe Val Tyr Lys Phe Val Ser Tyr Pro Glu Val Ala
               80                  85                  90
   ggg tgc tcc act gag gac tgc ccg ccc cag cca gag gtg tct gtt acc      639
   Gly Cys Ser Thr Glu Asp Cys Pro Pro Gln Pro Glu Val Ser Val Thr
           95                  100                 105
   tcc acc atg cca aat gtg gcc cct gct gct ata cat gcc gcc cca ggg      687
   Ser Thr Met Pro Asn Val Ala Pro Ala Ala Ile His Ala Ala Pro Gly
       110                 115                 120
   gac act gtc tct gga aag cca ggc aca ccc aag ggt gca gga atg gca      735
   Asp Thr Val Ser Gly Lys Pro Gly Thr Pro Lys Gly Ala Gly Met Ala
   125                 130                 135                 140
   ggc cca ggc ggt ttg gca cgc agc agc cgg aac gag tac atg cgc tcg      783
   Gly Pro Gly Gly Leu Ala Arg Ser Ser Arg Asn Glu Tyr Met Arg Ser
                   145                 150                 155
   ggc ctc tat tcc acc ttc acc atc cag tct ctg cag ccg cag cca ccc      831
   Gly Leu Tyr Ser Thr Phe Thr Ile Gln Ser Leu Gln Pro Gln Pro Pro
               160                 165                 170
   cct cat cct cgg cct gct gtg gtg ctc ccc aat gca gct cct gca ggg      879
   Pro His Pro Arg Pro Ala Val Val Leu Pro Asn Ala Ala Pro Ala Gly
           175                 180                 185
   gca gca gcg ccc ccc tcg ggg agc agg agc acc agt cca agc ccc ttg      927
   Ala Ala Ala Pro Pro Ser Gly Ser Arg Ser Thr Ser Pro Ser Pro Leu
       190                 195                 200
   gag gcc tgt ctg gag gct gaa gag gcc ggc ttg cct ctg cag gtc atc      975
   Glu Ala Cys Leu Glu Ala Glu Glu Ala Gly Leu Pro Leu Gln Val Ile
   205                 210                 215                 220
   ctg acc ccg ccc gag gcc cca aac ctg aaa tcg gaa gag ctt aat gtg      1023
   Leu Thr Pro Pro Glu Ala Pro Asn Leu Lys Ser Glu Glu Leu Asn Val
                   225                 230                 235
   gag ccg ggt ttg ggc cgg gct ttg ccc cca gaa gtg aaa gta gaa ggg      1071
   Glu Pro Gly Leu Gly Arg Ala Leu Pro Pro Glu Val Lys Val Glu Gly
               240                 245                 250
   ccc aag gaa gag ttg gaa gtt gcg ggg gag aga ggg ttt gtg cca gaa      1119
   Pro Lys Glu Glu Leu Glu Val Ala Gly Glu Arg Gly Phe Val Pro Glu
           255                 260                 265
   acc acc aag gcc gag cca gaa gtc cct cca cag gag ggc gtg cca gcc      1167
   Thr Thr Lys Ala Glu Pro Glu Val Pro Pro Gln Glu Gly Val Pro Ala
       270                 275                 280
   cgg ctg ccc gcg gtt gtt atg gac acc gca ggg cag gcg ggc ggc cat      1215
   Arg Leu Pro Ala Val Val Met Asp Thr Ala Gly Gln Ala Gly Gly His
   285                 290                 295                 300
   gcg gct tcc agc cct gag atc tcc cag ccg cag aag ggc cgg aag ccc      1263
   Ala Ala Ser Ser Pro Glu Ile Ser Gln Pro Gln Lys Gly Arg Lys Pro
                   305                 310                 315
   cgg gac cta gag ctt cca ctc agc ccg agc ctg cta ggt ggg ccg gga      1311
   Arg Asp Leu Glu Leu Pro Leu Ser Pro Ser Leu Leu Gly Gly Pro Gly
               320                 325                 330
   ccc gaa cgg acc cca gga tcg gga agt ggc tcc ggc ctc cag gct ccg      1359
   Pro Glu Arg Thr Pro Gly Ser Gly Ser Gly Ser Gly Leu Gln Ala Pro
           335                 340                 345
   ggg ccg gcg ctg acc cca tcc ctg ctt cct acg cat aca ttg acc ccg      1407
   Gly Pro Ala Leu Thr Pro Ser Leu Leu Pro Thr His Thr Leu Thr Pro
       350                 355                 360
   gtg ctg ctg aca ccc agc tcg ctg cct cct agc att cac ttc tgg agc      1455
   Val Leu Leu Thr Pro Ser Ser Leu Pro Pro Ser Ile His Phe Trp Ser
   365                 370                 375                 380
   acc ctg agt ccc att gcg ccc cgt agc ccg gcc aag ctc tcc ttc cag      1503
   Thr Leu Ser Pro Ile Ala Pro Arg Ser Pro Ala Lys Leu Ser Phe Gln
                   385                 390                 395
   ttt cca tcc agt ggc agc gcc cag gtg cac atc cct tct atc agc gtg      1551
   Phe Pro Ser Ser Gly Ser Ala Gln Val His Ile Pro Ser Ile Ser Val
               400                 405                 410
   gat ggc ctc tcg acc ccc gtg gtg ctc tcc cca ggg ccc cag aag cca      1599
   Asp Gly Leu Ser Thr Pro Val Val Leu Ser Pro Gly Pro Gln Lys Pro
           415                 420                 425
   tga ctactaccac caccaccacc accccttctg gggtcactcc atccatgctc          1652
   tctccagcca gccatctcaa ggagaaacat agttcaactg aaagactcat gctctgattg    1712
   tggtggggtg gggatccttg ggaagaatta ctcccaagag taactctcat tatctcctcc    1772
   acagaaaaca cacagcttcc acaacttctc tgttttctgt cagtccccca gtggccgccc    1832
   ttacacgtct cctacttcaa tggtaggggc ggtttattta tttatttttt gaaggccact    1892
   gggatgagcc tgacctaacc ttttagggtg gttaggacat ctcccccacc tccccacttt 1952
   tttccccaag acaagacaat cgaggtctgg cttgagaacg acctttcttt ctttatttct 2012
   cagcctgccc ttggggagat gagggagccc tgtctgcgtt tttggatgtg agtagaagag 2072
   ttagtttgtt ttgttttatt attcctggcc atactcaggg gtccaggaag aatttgtacc 2132
   atttaatggg ttgggagtct tggccaagga agaatcacac ccttggaata gaaatttcca 2192
   cctcccccaa cctttctctc agacagctta tcctttttca accaactttt tggccaggga 2252
   ggaatgtccc tttt 2266
<210> 205
   <211> 428
   <212> PRT
   <213> Homo sapiens
<400> 205
   Met Asp Pro Ser Val Thr Leu Trp Gln Phe Leu Leu Gln Leu Leu Arg
   1               5                   10                  15
   Glu Gln Gly Asn Gly His Ile Ile Ser Trp Thr Ser Arg Asp Gly Gly
               20                  25                  30
   Glu Phe Lys Leu Val Asp Ala Glu Glu Val Ala Arg Leu Trp Gly Leu
           35                  40                  45
   Arg Lys Asn Lys Thr Asn Met Asn Tyr Asp Lys Leu Ser Arg Ala Leu
       50                  55                  60
   Arg Tyr Tyr Tyr Asp Lys Asn Ile Ile Arg Lys Val Ser Gly Gln Lys
   65                  70                  75                  80
   Phe Val Tyr Lys Phe Val Ser Tyr Pro Glu Val Ala Gly Cys Ser Thr
                   85                  90                  95
   Glu Asp Cys Pro Pro Gln Pro Glu Val Ser Val Thr Ser Thr Met Pro
               100                 105                 110
   Asn Val Ala Pro Ala Ala Ile His Ala Ala Pro Gly Asp Thr Val Ser
           115                 120                 125
   Gly Lys Pro Gly Thr Pro Lys Gly Ala Gly Met Ala Gly Pro Gly Gly
       130                 135                 140
   Leu Ala Arg Ser Ser Arg Asn Glu Tyr Met Arg Ser Gly Leu Tyr Ser
   145                 150                 155                 160
   Thr Phe Thr Ile Gln Ser Leu Gln Pro Gln Pro Pro Pro His Pro Arg
   165 170 175
   Pro Ala Val Val Leu Pro Asn Ala Ala Pro Ala Gly Ala Ala Ala Pro
   180 185 190
   Pro Ser Gly Ser Arg Ser Thr Ser Pro Ser Pro Leu Glu Ala Cys Leu
   195 200 205
   Glu Ala Glu Glu Ala Gly Leu Pro Leu Gln Val Ile Leu Thr Pro Pro
   210 215 220
   Glu Ala Pro Asn Leu Lys Ser Glu Glu Leu Asn Val Glu Pro Gly Leu
   225 230 235 240
   Gly Arg Ala Leu Pro Pro Glu Val Lys Val Glu Gly Pro Lys Glu Glu
   245 250 255
   Leu Glu Val Ala Gly Glu Arg Gly Phe Val Pro Glu Thr Thr Lys Ala
   260 265 270
   Glu Pro Glu Val Pro Pro Gln Glu Gly Val Pro Ala Arg Leu Pro Ala
   275 280 285
   Val Val Met Asp Thr Ala Gly Gln Ala Gly Gly His Ala Ala Ser Ser
   290 295 300
   Pro Glu Ile Ser Gln Pro Gln Lys Gly Arg Lys Pro Arg Asp Leu Glu
   305 310 315 320
   Leu Pro Leu Ser Pro Ser Leu Leu Gly Gly Pro Gly Pro Glu Arg Thr
   325 330 335
   Pro Gly Ser Gly Ser Gly Ser Gly Leu Gln Ala Pro Gly Pro Ala Leu
   340 345 350
   Thr Pro Ser Leu Leu Pro Thr His Thr Leu Thr Pro Val Leu Leu Thr
   355 360 365
   Pro Ser Ser Leu Pro Pro Ser Ile His Phe Trp Ser Thr Leu Ser Pro
   370 375 380
   Ile Ala Pro Arg Ser Pro Ala Lys Leu Ser Phe Gln Phe Pro Ser Ser
   385 390 395 400
   Gly Ser Ala Gln Val His Ile Pro Ser Ile Ser Val Asp Gly Leu Ser
                   405                 410                 415
   Thr Pro Val Val Leu Ser Pro Gly Pro Gln Lys Pro
               420                 425
<210> 206
   <211> 18
   <212> DNA
   <213> Artificial Sequence
<220>
   <221> misc_feature
   <223> PCR Primer
<400> 206
   gcaaggcaat ggccacat 18
<210> 207
   <211> 21
   <212> DNA
   <213> Artificial Sequence
<220>
   <221> misc_feature
   <223> PCR Primer
<400> 207
   ctcctctgca tccaccagct t 21
<210> 208
   <211> 26
   <212> DNA
   <213> Artificial Sequence
<220>
   <221> misc_feature
   <223> PCR Probe
<400> 208
   tctcctggac ttcacgggat ggtggt 26
<210> 209
   <211> 18
   <212> DNA
   <213> Artificial Sequence
<220>
   <221> misc_feature
   <223> Antisense Oligonucleotide
<400> 209
   cccctgcgtt tccctaca 18
<210> 210
   <211> 18
   <212> DNA
   <213> Artificial Sequence
<220>
   <221> misc_feature
   <223> Antisense Oligonucleotide
<400> 210
   ggtggtggtg gcggtggc 18
<210> 211
   <211> 18
   <212> DNA
   <213> Artificial Sequence
<220>
   <221> misc_feature
   <223> Antisense Oligonucleotide
<400> 211
   ggcgttggca atgttggc 18
<210> 212
   <211> 18
   <212> DNA
   <213> Artificial Sequence
<220>
   <221> misc_feature
   <223> Antisense Oligonucleotide
<400> 212
   aagttgaggc tgtgtgta 18
<210> 213
   <211> 18
   <212> DNA
   <213> Artificial Sequence
<220>
   <221> misc_feature
   <223> Antisense Oligonucleotide
<400> 213
   aggccacgga cgggtctc 18
<210> 214
   <211> 18
   <212> DNA
   <213> Artificial Sequence
<220>
   <221> misc_feature
   <223> Antisense Oligonucleotide
<400> 214
   gattgattcg ctacgatg 18
<210> 215
   <211> 18
   <212> DNA
   <213> Artificial Sequence
<220>
   <221> misc_feature
   <223> Antisense Oligonucleotide
<400> 215
   gggatgcgga ggagtgcg 18
<210> 216
   <211> 18
   <212> DNA
   <213> Artificial Sequence
<220>
   <221> misc_feature
   <223> Antisense Oligonucleotide
<400> 216
   agtgctcacg ccatccca 18
<210> 217
   <211> 18
   <212> DNA
   <213> Artificial Sequence
<220>
   <221> misc_feature
   <223> Antisense Oligonucleotide
<400> 217
   aaactgccac agcgtcac 18
<210> 218
   <211> 18
   <212> DNA
   <213> Artificial Sequence
<220>
   <221> misc_feature
   <223> Antisense Oligonucleotide
<400> 218
   gaagtccagg agatgatg 18
<210> 219
   <211> 18
   <212> DNA
   <213> Artificial Sequence
<220>
   <221> misc_feature
   <223> Antisense Oligonucleotide
<400> 219
   caccaccatc ccgtgaag 18
<210> 220
   <211> 18
   <212> DNA
   <213> Artificial Sequence
<220>
   <221> misc_feature
   <223> Antisense Oligonucleotide
<400> 220
   tcttgttctt gcgtagtc 18
<210> 221
   <211> 18
   <212> DNA
   <213> Artificial Sequence
<220>
   <221> misc_feature
   <223> Antisense Oligonucleotide
<400> 221
   tgttcttgtc atagtagt 18
<210> 222
   <211> 18
   <212> DNA
   <213> Artificial Sequence
<220>
   <221> misc_feature
   <223> Antisense Oligonucleotide
<400> 222
   tcaccttgcg gatgatgt 18
<210> 223
   <211> 18
   <212> DNA
   <213> Artificial Sequence
<220>
   <221> misc_feature
   <223> Antisense Oligonucleotide
<400> 223
   gagcaccctg cgacctca 18
<210> 224
   <211> 18
   <212> DNA
   <213> Artificial Sequence
<220>
   <221> misc_feature
   <223> Antisense Oligonucleotide
<400> 224
   ggcgggcagt cctcagtg 18
<210> 225
   <211> 18
   <212> DNA
   <213> Artificial Sequence
<220>
   <221> misc_feature
   <223> Antisense Oligonucleotide
<400> 225
   ggtgaaggtg gaatagag 18
<210> 226
   <211> 18
   <212> DNA
   <213> Artificial Sequence
<220>
   <221> misc_feature
   <223> Antisense Oligonucleotide
<400> 226
   tccgatttca ggtttggg 18
<210> 227
   <211> 18
   <212> DNA
   <213> Artificial Sequence
<220>
   <221> misc_feature
   <223> Antisense Oligonucleotide
<400> 227
   ttggtggttt ctggcaca 18
<210> 228
   <211> 18
   <212> DNA
   <213> Artificial Sequence
<220>
   <221> misc_feature
   <223> Antisense Oligonucleotide
<400> 228
   tggagggact tctggctc 18
<210> 229
   <211> 18
   <212> DNA
   <213> Artificial Sequence
<220>
   <221> misc_feature
   <223> Antisense Oligonucleotide
<400> 229
   gcgtaggaag cagggatg 18
<210> 230
   <211> 18
   <212> DNA
   <213> Artificial Sequence
<220>
   <221> misc_feature
   <223> Antisense Oligonucleotide
<400> 230
   gtgctccaga agtgaatg 18
<210> 231
   <211> 18
   <212> DNA
   <213> Artificial Sequence
<220>
   <221> misc_feature
   <223> Antisense Oligonucleotide
<400> 231
   actggatgga aactggaa 18
<210> 232
   <211> 18
   <212> DNA
   <213> Artificial Sequence
<220>
   <221> misc_feature
   <223> Antisense Oligonucleotide
<400> 232
   ggccatccac gctgatag 18
<210> 233
   <211> 18
   <212> DNA
   <213> Artificial Sequence
<220>
   <221> misc_feature
   <223> Antisense Oligonucleotide
<400> 233
   ccaccacaat cagagcat 18
<210> 234
   <211> 18
   <212> DNA
   <213> Artificial Sequence
<220>
   <221> misc_feature
   <223> Antisense Oligonucleotide
<400> 234
   gatccccacc ccaccaca 18
<210> 235
   <211> 18
   <212> DNA
   <213> Artificial Sequence
<220>
   <221> misc_feature
   <223> Antisense Oligonucleotide
<400> 235
   tgttttctgt ggaggaga 18
<210> 236
   <211> 18
   <212> DNA
   <213> Artificial Sequence
<220>
   <221> misc_feature
   <223> Antisense Oligonucleotide
<400> 236
   aaacagagaa gttgtgga 18
<210> 237
   <211> 18
   <212> DNA
   <213> Artificial Sequence
<220>
   <221> misc_feature
   <223> Antisense Oligonucleotide
<400> 237
   gggactgaca gaaaacag 18
<210> 238
   <211> 18
   <212> DNA
   <213> Artificial Sequence
<220>
   <221> misc_feature
   <223> Antisense Oligonucleotide
<400> 238
   ataaataaat aaaccgcc 18
<210> 239
   <211> 18
   <212> DNA
   <213> Artificial Sequence
<220>
   <221> misc_feature
   <223> Antisense Oligonucleotide
<400> 239
   gttaggtcag gctcatcc 18
<210> 240
   <211> 18
   <212> DNA
   <213> Artificial Sequence
<220>
   <221> misc_feature
   <223> Antisense Oligonucleotide
<400> 240
   gttctcaagc cagacctc 18
<210> 241
   <211> 18
   <212> DNA
   <213> Artificial Sequence
<220>
   <221> misc_feature
   <223> Antisense Oligonucleotide
<400> 241
   aataaagaaa gaaaggtc 18
<210> 242
   <211> 18
   <212> DNA
   <213> Artificial Sequence
<220>
   <221> misc_feature
   <223> Antisense Oligonucleotide
<400> 242
   agggcaggct gagaaata 18
<210> 243
   <211> 18
   <212> DNA
   <213> Artificial Sequence
<220>
   <221> misc_feature
   <223> Antisense Oligonucleotide
<400> 243
   cttctactca catccaaa 18
<210> 244
   <211> 18
   <212> DNA
   <213> Artificial Sequence
<220>
   <221> misc_feature
   <223> Antisense Oligonucleotide
<400> 244
   caaaacaaac taactctt 18
<210> 245
   <211> 18
   <212> DNA
   <213> Artificial Sequence
<220>
   <221> misc_feature
   <223> Antisense Oligonucleotide
<400> 245
   ggaataataa aacaaaac 18
<210> 246
   <211> 18
   <212> DNA
   <213> Artificial Sequence
<220>
   <221> misc_feature
   <223> Antisense Oligonucleotide
<400> 246
   ttcttcctgg acccctga 18
<210> 247
   <211> 18
   <212> DNA
   <213> Artificial Sequence
<220>
   <221> misc_feature
   <223> Antisense Oligonucleotide
<400> 247
   ccaagggtgt gattcttc 18
<210> 248
   <211> 18
   <212> DNA
   <213> Artificial Sequence
<400> 248
   tgtctgagag aaaggttg 18
<210> 249
   <211> 1080
   <212> DNA
   <213> Homo sapiens
<220>
   <221> CDS
   <222> (1)..(1080)
<400> 249
   atg act ctg gag tcc atc atg gcg tgt tgc ctg agc gat gag gtg aag        48
   gag tcc aag cgg atc aac gcc gag atc gag aag cag ctg cgg cgg gac        96
   aag cgc gac gcc cgg cgc gag ctc aag ctg ctg ctg ctc ggc acg ggc       144
   gag agc ggg aag agc acg ttc atc aag cag atg cgc atc atc cac ggc       192
   gcc ggc tac tcg gag gag gac aag cgc ggc ttc acc aag ctc gtc tac       240
   cag aac atc ttc acc gcc atg cag gcc atg atc cgg gcc atg gag acg       288
   ctc aag atc ctc tac aag tac gag cag aac aag gcc aat gcg ctc ctg       336
   atc cgg gag gtg gac gtg gag aag gtg acc acc ttc gag cat cag tac       384
   gtc agt gcc atc aag acc ctg tgg gag gac ccg ggc atc cag gaa tgc       432
   tac gac cgc agg cgc gag tac cag ctc tcc gac tct gcc aag tac tac       480
   ctg acc gac gtt gac cgc atc gcc acc ttg ggc tac ctg ccc acc cag       528
   cag gac gtg ctg cgg gtc cgc gtg ccc acc acc ggc atc atc gag tac       576
   cct ttc gac ctg gag aac atc atc ttc cgg atg gtg gat gtg ggg ggc       624
   cag cgg tcg gag cgg agg aag tgg atc cac tgc ttt gag aac gtg aca       672
   tcc atc atg ttt ctc gtc gcc ctc agc gaa tac gac caa gtc ctg gtg      720
   gag tcg gac aac gag aac cgg atg gag gag agc aaa gcc ctg ttc cgg      768
   acc atc atc acc tac ccc tgg ttc cag aac tcc tcc gtc atc ctc ttc      816
   ctc aac aag aag gac ctg ctg gag gac aag atc ctg tac tcg cac ctg      864
   gtg gac tac ttc ccc gag ttc gat ggt ccc cag cgg gac gcc cag gcg      912
   gcg cgg gag ttc atc ccg aag atg ttc gtg gac ctg aac ccc gac agc      960
   gac aag atc atc tac tca cac ttc acg tgt gcc acc gac acg gag aac     1008
   atc cgc ttc gtg ttc gcg gcc gtg aag gac acc atc ctg cag ctg aac     1056
   ctg aag gag tac aat ctg gtc taa                                     1080
<210> 250
   <211> 359
   <212> PRT
   <213> Homo sapiens
<400> 250
<210> 251
   <211> 20
   <212> DNA
   <213> Artificial Sequence
<220>
   <221> misc_feature
   <223> PCR Primer
<400> 251
   tgaccacctt cgagcatcag 20
<210> 252
   <211> 20
   <212> DNA
   <213> Artificial Sequence
<220>
   <221> misc_feature
   <223> PCR Primer
<400> 252
   cggtcgtagc attcctggat 20
<210> 253
   <211> 26
   <212> DNA
   <213> Artificial Sequence
<220>
   <221> misc_feature
   <223> PCR Probe
<400> 253
   tcagtgccat caagaccctg tgggag 26
<210> 254
   <211> 18
   <212> DNA
   <213> Artificial Sequence
<220>
   <221> misc_feature
   <223> Antisense Oligonucleotide
<400> 254
   gatggactcc agagtcat 18
<210> 255
   <211> 18
   <212> DNA
   <213> Artificial Sequence
<220>
   <221> misc_feature
   <223> Antisense Oligonucleotide
<400> 255
   gccatgatgg actccaga 18
<210> 256
   <211> 18
   <212> DNA
   <213> Artificial Sequence
<220>
   <221> misc_feature
   <223> Antisense Oligonucleotide
<400> 256
   cacgccatga tggactcc 18
<210> 257
   <211> 18
   <212> DNA
   <213> Artificial Sequence
<220>
   <221> misc_feature
   <223> Antisense Oligonucleotide
<400> 257
   ctcatcgctc aggcaaca 18
<210> 258
   <211> 18
   <212> DNA
   <213> Artificial Sequence
<220>
   <221> misc_feature
   <223> Antisense Oligonucleotide
<400> 258
   cttcacctca tcgctcag 18
<210> 259
   <211> 18
   <212> DNA
   <213> Artificial Sequence
<220>
   <221> misc_feature
   <223> Antisense Oligonucleotide
<400> 259
   gactccttca cctcatcg 18
<210> 260
   <211> 18
   <212> DNA
   <213> Artificial Sequence
<220>
   <221> misc_feature
   <223> Antisense Oligonucleotide
<400> 260
   atccgcttgg actccttc 18
<210> 261
   <211> 18
   <212> DNA
   <213> Artificial Sequence
<220>
   <221> misc_feature
   <223> Antisense Oligonucleotide
<400> 261
   cgttgatccg cttggact 18
<210> 262
   <211> 18
   <212> DNA
   <213> Artificial Sequence
<220>
   <221> misc_feature
   <223> Antisense Oligonucleotide
<400> 262
   ctcgatctcg gcgttgat 18
<210> 263
   <211> 18
   <212> DNA
   <213> Artificial Sequence
<220>
   <221> misc_feature
   <223> Antisense Oligonucleotide
<400> 263
   cccgccgcag ctgcttct 18
<210> 264
   <211> 18
   <212> DNA
   <213> Artificial Sequence
<220>
   <221> misc_feature
   <223> Antisense Oligonucleotide
<400> 264
   cttgagctcg cgccgggc 18
<210> 265
   <211> 18
   <212> DNA
   <213> Artificial Sequence
<220>
   <221> misc_feature
   <223> Antisense Oligonucleotide
<400> 265
   gcagcagcag cttgagct 18
<210> 266
   <211> 18
   <212> DNA
   <213> Artificial Sequence
<220>
   <221> misc_feature
   <223> Antisense Oligonucleotide
<400> 266
   gcccgtgccg agcagcag 18
<210> 267
   <211> 18
   <212> DNA
   <213> Artificial Sequence
<220>
   <221> misc_feature
   <223> Antisense Oligonucleotide
<400> 267
   acgtgctctt cccgctct 18
<210> 268
   <211> 18
   <212> DNA
   <213> Artificial Sequence
<220>
   <221> misc_feature
   <223> Antisense Oligonucleotide
<400> 268
   atctgcttga tgaacgtg 18
<210> 269
   <211> 18
   <212> DNA
   <213> Artificial Sequence
<220>
   <221> misc_feature
   <223> Antisense Oligonucleotide
<400> 269
   cgcatctgct tgatgaac 18
<210> 270
   <211> 18
   <212> DNA
   <213> Artificial Sequence
<220>
   <221> misc_feature
   <223> Antisense Oligonucleotide
<400> 270
   gtagccggcg ccgtggat 18
<210> 271
   <211> 18
   <212> DNA
   <213> Artificial Sequence
<220>
   <221> misc_feature
   <223> Antisense Oligonucleotide
<400> 271
   tgtcctcctc cgagtagc 18
<210> 272
   <211> 18
   <212> DNA
   <213> Artificial Sequence
<220>
   <221> misc_feature
   <223> Antisense Oligonucleotide
<400> 272
   cttgtcctcc tccgagta 18
<210> 273
   <211> 18
   <212> DNA
   <213> Artificial Sequence
<220>
   <221> misc_feature
   <223> Antisense Oligonucleotide
<400> 273
   aagccgcgct tgtcctcc 18
<210> 274
   <211> 18
   <212> DNA
   <213> Artificial Sequence
<220>
   <221> misc_feature
   <223> Antisense Oligonucleotide
<400> 274
   tagacgagct tggtgaag 18
<210> 275
   <211> 18
   <212> DNA
   <213> Artificial Sequence
<220>
   <221> misc_feature
   <223> Antisense Oligonucleotide
<400> 275
   tgttctggta gacgagct 18
<210> 276
   <211> 18
   <212> DNA
   <213> Artificial Sequence
<220>
   <221> misc_feature
   <223> Antisense Oligonucleotide
<400> 276
   tggcggtgaa gatgttct 18
<210> 277
   <211> 18
   <212> DNA
   <213> Artificial Sequence
<220>
   <221> misc_feature
   <223> Antisense Oligonucleotide
<400> 277
   cggatcatgg cctgcatg 18
<210> 278
   <211> 18
   <212> DNA
   <213> Artificial Sequence
<220>
   <221> misc_feature
   <223> Antisense Oligonucleotide
<400> 278
   cgtctccatg gcccggat 18
<210> 279
   <211> 18
   <212> DNA
   <213> Artificial Sequence
<220>
   <221> misc_feature
   <223> Antisense Oligonucleotide
<400> 279
   tagaggatct tgagcgtc 18
<210> 280
   <211> 18
   <212> DNA
   <213> Artificial Sequence
<220>
   <221> misc_feature
   <223> Antisense Oligonucleotide
<400> 280
   tgtagaggat cttgagcg 18
<210> 281
   <211> 18
   <212> DNA
   <213> Artificial Sequence
<220>
   <221> misc_feature
   <223> Antisense Oligonucleotide
<400> 281
   tgctcgtact tgtagagg 18
<210> 282
   <211> 18
   <212> DNA
   <213> Artificial Sequence
<220>
   <221> misc_feature
   <223> Antisense Oligonucleotide
<400> 282
   gccttgttct gctcgtac 18
<210> 283
   <211> 18
   <212> DNA
   <213> Artificial Sequence
<220>
   <221> misc_feature
   <223> Antisense Oligonucleotide
<400> 283
   ttggccttgt tctgctcg 18
<210> 284
   <211> 18
   <212> DNA
   <213> Artificial Sequence
<220>
   <221> misc_feature
   <223> Antisense Oligonucleotide
<400> 284
   caggagcgca ttggcctt 18
<210> 285
   <211> 18
   <212> DNA
   <213> Artificial Sequence
<220>
   <221> misc_feature
   <223> Antisense Oligonucleotide
<400> 285
   ctccacgtcc acctcccg 18
<210> 286
   <211> 18
   <212> DNA
   <213> Artificial Sequence
<220>
   <221> misc_feature
   <223> Antisense Oligonucleotide
<400> 286
   ggtcaccttc tccacgtc 18
<210> 287
   <211> 18
   <212> DNA
   <213> Artificial Sequence
<220>
   <221> misc_feature
   <223> Antisense Oligonucleotide
<400> 287
   gatgctcgaa ggtggtca 18
<210> 288
   <211> 18
   <212> DNA
   <213> Artificial Sequence
<220>
   <221> misc_feature
   <223> Antisense Oligonucleotide
<400> 288
   actgacgtac tgatgctc 18
<210> 289
   <211> 18
   <212> DNA
   <213> Artificial Sequence
<220>
   <221> misc_feature
   <223> Antisense Oligonucleotide
<400> 289
   cttgatggca ctgacgta 18
<210> 290
   <211> 18
   <212> DNA
   <213> Artificial Sequence
<220>
   <221> misc_feature
   <223> Antisense Oligonucleotide
<400> 290
   cagggtcttg atggcact 18
<210> 291
   <211> 18
   <212> DNA
   <213> Artificial Sequence
<220>
   <221> misc_feature
   <223> Antisense Oligonucleotide
<400> 291
   ctggatgccc gggtcctc 18
<210> 292
   <211> 18
   <212> DNA
   <213> Artificial Sequence
<220>
   <221> misc_feature
   <223> Antisense Oligonucleotide
<400> 292
   tcctggatgc ccgggtcc 18
<210> 293
   <211> 18
   <212> DNA
   <213> Artificial Sequence
<220>
   <221> misc_feature
   <223> Antisense Oligonucleotide
<400> 293
   cgcctgcggt cgtagcat 18
<210> 294
   <211> 18
   <212> DNA
   <213> Artificial Sequence
<220>
   <221> misc_feature
   <223> Antisense Oligonucleotide
<400> 294
   gctggtactc gcgcctgc 18
<210> 295
   <211> 18
   <212> DNA
   <213> Artificial Sequence
<220>
   <221> misc_feature
   <223> Antisense Oligonucleotide
<400> 295
   tacttggcag agtcggag 18
<210> 296
   <211> 18
   <212> DNA
   <213> Artificial Sequence
<220>
   <221> misc_feature
   <223> Antisense Oligonucleotide
<400> 296
   gtcaggtagt acttggca 18
<210> 297
   <211> 18
   <212> DNA
   <213> Artificial Sequence
<220>
   <221> misc_feature
   <223> Antisense Oligonucleotide
<400> 297
   ggtcaacgtc ggtcaggt 18
<210> 298
   <211> 18
   <212> DNA
   <213> Artificial Sequence
<220>
   <221> misc_feature
   <223> Antisense Oligonucleotide
<400> 298
   gtggcgatgc ggtcaacg 18
<210> 299
   <211> 18
   <212> DNA
   <213> Artificial Sequence
<220>
   <221> misc_feature
   <223> Antisense Oligonucleotide
<400> 299
   gcaggtagcc caaggtgg 18
<210> 300
   <211> 18
   <212> DNA
   <213> Artificial Sequence
<220>
   <221> misc_feature
   <223> Antisense Oligonucleotide
<400> 300
   cgtcctgctg ggtgggca 18
<210> 301
   <211> 18
   <212> DNA
   <213> Artificial Sequence
<220>
   <221> misc_feature
   <223> Antisense Oligonucleotide
<400> 301
   ggtggtgggc acgcggac 18
<210> 302
   <211> 18
   <212> DNA
   <213> Artificial Sequence
<220>
   <221> misc_feature
   <223> Antisense Oligonucleotide
<400> 302
   tcgatgatgc cggtggtg 18
<210> 303
   <211> 18
   <212> DNA
   <213> Artificial Sequence
<220>
   <221> misc_feature
   <223> Antisense Oligonucleotide
<400> 303
   ccaggtcgaa agggtact 18
<210> 304
   <211> 18
   <212> DNA
   <213> Artificial Sequence
<220>
   <221> misc_feature
   <223> Antisense Oligonucleotide
<400> 304
   tgttctccag gtcgaaag 18
<210> 305
   <211> 18
   <212> DNA
   <213> Artificial Sequence
<220>
   <221> misc_feature
   <223> Antisense Oligonucleotide
<400> 305
   agatgatgtt ctccaggt 18
<210> 306
   <211> 18
   <212> DNA
   <213> Artificial Sequence
<220>
   <221> misc_feature
   <223> Antisense Oligonucleotide
<400> 306
   atccggaaga tgatgttc 18
<210> 307
   <211> 18
   <212> DNA
   <213> Artificial Sequence
<220>
   <221> misc_feature
   <223> Antisense Oligonucleotide
<400> 307
   ctccgctccg accgctgg 18
<210> 308
   <211> 18
   <212> DNA
   <213> Artificial Sequence
<220>
   <221> misc_feature
   <223> Antisense Oligonucleotide
<400> 308
   gatccacttc ctccgctc 18
<210> 309
   <211> 18
   <212> DNA
   <213> Artificial Sequence
<220>
   <221> misc_feature
   <223> Antisense Oligonucleotide
<400> 309
   tgtcacgttc tcaaagca 18
<210> 310
   <211> 18
   <212> DNA
   <213> Artificial Sequence
<220>
   <221> misc_feature
   <223> Antisense Oligonucleotide
<400> 310
   atgatggatg tcacgttc 18
<210> 311
   <211> 18
   <212> DNA
   <213> Artificial Sequence
<220>
   <221> misc_feature
   <223> Antisense Oligonucleotide
<400> 311
   cgagaaacat gatggatg 18
<210> 312
   <211> 18
   <212> DNA
   <213> Artificial Sequence
<220>
   <221> misc_feature
   <223> Antisense Oligonucleotide
<400> 312
   gctgagggcg acgagaaa 18
<210> 313
   <211> 18
   <212> DNA
   <213> Artificial Sequence
<220>
   <221> misc_feature
   <223> Antisense Oligonucleotide
<400> 313
   cgactccacc aggacttg 18
<210> 314
   <211> 18
   <212> DNA
   <213> Artificial Sequence
<220>
   <221> misc_feature
   <223> Antisense Oligonucleotide
<400> 314
   atccggttct cgttgtcc 18
<210> 315
   <211> 18
   <212> DNA
   <213> Artificial Sequence
<220>
   <221> misc_feature
   <223> Antisense Oligonucleotide
<400> 315
   ccatccggtt ctcgttgt 18
<210> 316
   <211> 18
   <212> DNA
   <213> Artificial Sequence
<220>
   <221> misc_feature
   <223> Antisense Oligonucleotide
<400> 316
   agggctttgc tctcctcc 18
<210> 317
   <211> 18
   <212> DNA
   <213> Artificial Sequence
<220>
   <221> misc_feature
   <223> Antisense Oligonucleotide
<400> 317
   ggtccggaac agggcttt 18
<210> 318
   <211> 18
   <212> DNA
   <213> Artificial Sequence
<220>
   <221> misc_feature
   <223> Antisense Oligonucleotide
<400> 318
   gtaggtgatg atggtccg 18
<210> 319
   <211> 18
   <212> DNA
   <213> Artificial Sequence
<220>
   <221> misc_feature
   <223> Antisense Oligonucleotide
<400> 319
   ggaggagttc tggaacca 18
<210> 320
   <211> 18
   <212> DNA
   <213> Artificial Sequence
<220>
   <221> misc_feature
   <223> Antisense Oligonucleotide
<400> 320
   tgaggaagag gatgacgg 18
<210> 321
   <211> 18
   <212> DNA
   <213> Artificial Sequence
<220>
   <221> misc_feature
   <223> Antisense Oligonucleotide
<400> 321
   gcaggtcctt cttgttga 18
<210> 322
   <211> 18
   <212> DNA
   <213> Artificial Sequence
<220>
   <221> misc_feature
   <223> Antisense Oligonucleotide
<400> 322
   atcttgtcct ccagcagg 18
<210> 323
   <211> 18
   <212> DNA
   <213> Artificial Sequence
<220>
   <221> misc_feature
   <223> Antisense Oligonucleotide
<400> 323
   gcgagtacag gatcttgt 18
<210> 324
   <211> 18
   <212> DNA
   <213> Artificial Sequence
<220>
   <221> misc_feature
   <223> Antisense Oligonucleotide
<400> 324
   aagtagtcca ccaggtgc 18
<210> 325
   <211> 18
   <212> DNA
   <213> Artificial Sequence
<220>
   <221> misc_feature
   <223> Antisense Oligonucleotide
<400> 325
   gatgaactcc cgcgccgc 18
<210> 326
   <211> 18
   <212> DNA
   <213> Artificial Sequence
<220>
   <221> misc_feature
   <223> Antisense Oligonucleotide
<400> 326
   ggttcaggtc cacgaaca 18
<210> 327
   <211> 18
   <212> DNA
   <213> Artificial Sequence
<220>
   <221> misc_feature
   <223> Antisense Oligonucleotide
<400> 327
   gtagatgatc ttgtcgct 18
<210> 328
   <211> 18
   <212> DNA
   <213> Artificial Sequence
<220>
   <221> misc_feature
   <223> Antisense Oligonucleotide
<400> 328
   cacgtgaagt gtgagtag 18
<210> 329
   <211> 18
   <212> DNA
   <213> Artificial Sequence
<220>
   <221> misc_feature
   <223> Antisense Oligonucleotide
<400> 329
   atgttctccg tgtcggtg 18
<210> 330
   <211> 18
   <212> DNA
   <213> Artificial Sequence
<220>
   <221> misc_feature
   <223> Antisense Oligonucleotide
<400> 330
   acggccgcga acacgaag 18
<210> 331
   <211> 18
   <212> DNA
   <213> Artificial Sequence
<220>
   <221> misc_feature
   <223> Antisense Oligonucleotide
<400> 331
   gatggtgtcc ttcacggc 18
<210> 332
   <211> 18
   <212> DNA
   <213> Artificial Sequence
<220>
   <221> misc_feature
   <223> Antisense Oligonucleotide
<400> 332
   tcaggttcag ctgcagga 18
<210> 333
   <211> 18
   <212> DNA
   <213> Artificial Sequence
<220>
   <221> misc_feature
   <223> Antisense Oligonucleotide
<400> 333
   accagattgt actccttc 18
<210> 334
   <211> 2610
   <212> DNA
   <213> Homo sapiens
<220>
   <221> CDS
   <222> (199)..(1641)
<400> 334
   atcctgggac agggcacagg gccatctgtc accaggggct tagggaaggc cgagccagcc      60
   tgggtcaaag aagtcaaagg ggctgcctgg aggaggcagc ctgtcagctg gtgcatcaga     120
   ggctgtggcc aggccagctg ggctcgggga gcgccagcct gagaggagcg cgtgagcgtc     180
   gcgggagcct cgggcacc atg agc gac gtg gct att gtg aag gag ggt tgg       231
                       Met Ser Asp Val Ala Ile Val Lys Glu Gly Trp
                       1               5                   10
   ctg cac aaa cga ggg gag tac atc aag acc tgg cgg cca cgc tac ttc       279
   Leu His Lys Arg Gly Glu Tyr Ile Lys Thr Trp Arg Pro Arg Tyr Phe
               15                  20                  25
   ctc ctc aag aat gat ggc acc ttc att ggc tac aag gag cgg ccg cag       327
   Leu Leu Lys Asn Asp Gly Thr Phe Ile Gly Tyr Lys Glu Arg Pro Gln
           30                  35                  40
   gat gtg gac caa cgt gag gct ccc ctc aac aac ttc tct gtg gcg cag       375
   Asp Val Asp Gln Arg Glu Ala Pro Leu Asn Asn Phe Ser Val Ala Gln
       45                  50                  55
   tgc cag ctg atg aag acg gag cgg ccc cgg ccc aac acc ttc atc atc       423
   Cys Gln Leu Met Lys Thr Glu Arg Pro Arg Pro Asn Thr Phe Ile Ile
   60                  65                  70                  75
   cgc tgc ctg cag tgg acc act gtc atc gaa cgc acc ttc cat gtg gag       471
   Arg Cys Leu Gln Trp Thr Thr Val Ile Glu Arg Thr Phe His Val Glu
                   80                  85                  90
   act cct gag gag cgg gag gag tgg aca acc gcc atc cag act gtg gct       519
   Thr Pro Glu Glu Arg Glu Glu Trp Thr Thr Ala Ile Gln Thr Val Ala
               95                  100                 105
   gac ggc ctc aag aag cag gag gag gag gag atg gac ttc cgg tcg ggc      567
   Asp Gly Leu Lys Lys Gln Glu Glu Glu Glu Met Asp Phe Arg Ser Gly
           110                 115                 120
   tca ccc agt gac aac tca ggg gct gaa gag atg gag gtg tcc ctg gcc      615
   Ser Pro Ser Asp Asn Ser Gly Ala Glu Glu Met Glu Val Ser Leu Ala
       125                 130                 135
   aag ccc aag cac cgc gtg acc atg aac gag ttt gag tac ctg aag ctg      663
   Lys Pro Lys His Arg Val Thr Met Asn Glu Phe Glu Tyr Leu Lys Leu
   140                 145                 150                 155
   ctg ggc aag ggc act ttc ggc aag gtg atc ctg gtg aag gag aag gcc      711
   Leu Gly Lys Gly Thr Phe Gly Lys Val Ile Leu Val Lys Glu Lys Ala
                   160                 165                 170
   aca ggc cgc tac tac gcc atg aag atc ctc aag aag gaa gtc atc gtg      759
   Thr Gly Arg Tyr Tyr Ala Met Lys Ile Leu Lys Lys Glu Val Ile Val
               175                 180                 185
   gcc aag gac gag gtg gcc cac aca ctc acc gag aac cgc gtc ctg cag      807
   Ala Lys Asp Glu Val Ala His Thr Leu Thr Glu Asn Arg Val Leu Gln
           190                 195                 200
   aac tcc agg cac ccc ttc ctc aca gcc ctg aag tac tct ttc cag acc      855
   Asn Ser Arg His Pro Phe Leu Thr Ala Leu Lys Tyr Ser Phe Gln Thr
       205                 210                 215
   cac gac cgc ctc tgc ttt gtc atg gag tac gcc aac ggg ggc gag ctg      903
   His Asp Arg Leu Cys Phe Val Met Glu Tyr Ala Asn Gly Gly Glu Leu
   220                 225                 230                 235
   ttc ttc cac ctg tcc cgg gaa cgt gtg ttc tcc gag gac cgg gcc cgc      951
   Phe Phe His Leu Ser Arg Glu Arg Val Phe Ser Glu Asp Arg Ala Arg
                   240                 245                 250
   ttc tat ggc gct gag att gtg tca gcc ctg gac tac ctg cac tcg gag      999
   Phe Tyr Gly Ala Glu Ile Val Ser Ala Leu Asp Tyr Leu His Ser Glu
               255                 260                 265
   aag aac gtg gtg tac cgg gac ctc aag ctg gag aac ctc atg ctg gac     1047
   Lys Asn Val Val Tyr Arg Asp Leu Lys Leu Glu Asn Leu Met Leu Asp
           270                 275                 280
   aag gac ggg cac att aag atc aca gac ttc ggg ctg tgc aag gag ggg     1095
   Lys Asp Gly His Ile Lys Ile Thr Asp Phe Gly Leu Cys Lys Glu Gly
       285                 290                 295
   atc aag gac ggt gcc acc atg aag acc ttt tgc ggc aca cct gag tac     1143
   Ile Lys Asp Gly Ala Thr Met Lys Thr Phe Cys Gly Thr Pro Glu Tyr
   300                 305                 310                 315
   ctg gcc ccc gag gtg ctg gag gac aat gac tac ggc cgt gca gtg gac     1191
   Leu Ala Pro Glu Val Leu Glu Asp Asn Asp Tyr Gly Arg Ala Val Asp
                   320                 325                 330
   tgg tgg ggg ctg ggc gtg gtc atg tac gag atg atg tgc ggt cgc ctg     1239
   Trp Trp Gly Leu Gly Val Val Met Tyr Glu Met Met Cys Gly Arg Leu
               335                 340                 345
   ccc ttc tac aac cag gac cat gag aag ctt ttt gag ctc atc ctc atg     1287
   Pro Phe Tyr Asn Gln Asp His Glu Lys Leu Phe Glu Leu Ile Leu Met
           350                 355                 360
   gag gag atc cgc ttc ccg cgc acg ctt ggt ccc gag gcc aag tcc ttg      1335
   Glu Glu Ile Arg Phe Pro Arg Thr Leu Gly Pro Glu Ala Lys Ser Leu
       365                 370                 375
   ctt tca ggg ctg ctc aag aag gac ccc aag cag agg ctt ggc ggg ggc      1383
   Leu Ser Gly Leu Leu Lys Lys Asp Pro Lys Gln Arg Leu Gly Gly Gly
   380                 385                 390                 395
   tcc gag gac gcc aag gag atc atg cag cat cgc ttc ttt gcc ggt atc      1431
   Ser Glu Asp Ala Lys Glu Ile Met Gln His Arg Phe Phe Ala Gly Ile
                   400                 405                 410
   gtg tgg cag cac gtg tac gag aag aag ctc agc cca ccc ttc aag ccc      1479
   Val Trp Gln His Val Tyr Glu Lys Lys Leu Ser Pro Pro Phe Lys Pro
               415                 420                 425
   cag gtc acg tcg gag act gac acc agg tat ttt gat gag gag ttc acg      1527
   Gln Val Thr Ser Glu Thr Asp Thr Arg Tyr Phe Asp Glu Glu Phe Thr
           430                 435                 440
   gcc cag atg atc acc atc aca cca cct gac caa gat gac agc atg gag      1575
   Ala Gln Met Ile Thr Ile Thr Pro Pro Asp Gln Asp Asp Ser Met Glu
       445                 450                 455
   tgt gtg gac agc gag cgc agg ccc cac ttc ccc cag ttc tcc tac tcg      1623
   Cys Val Asp Ser Glu Arg Arg Pro His Phe Pro Gln Phe Ser Tyr Ser
   460                 465                 470                 475
   gcc agc agc acg gcc tga ggcggcggtg gactgcgctg gacgatagct            1671
   Ala Ser Ser Thr Ala
                   480
   tggagggatg gagaggcggc ctcgtgccat gatctgtatt taatggtttt tatttctcgg   1731
   gtgcatttga gagaagccac gctgtcctct cgagcccaga tggaaagacg tttttgtgct   1791
   gtgggcagca ccctcccccg cagcggggta gggaagaaaa ctatcctgcg ggttttaatt   1851
   tatttcatcc agtttgttct ccgggtgtgg cctcagccct cagaacaatc cgattcacgt   1911
   agggaaatgt taaggacttc tacagctatg cgcaatgtgg cattgggggg ccgggcaggt   1971
   cctgcccatg tgtcccctca ctctgtcagc cagccgccct gggctgtctg tcaccagcta   2031
   tctgtcatct ctctggggcc ctgggcctca gttcaacctg gtggcaccag atgcaacctc   2091
   actatggtat gctggccagc accctctcct gggggtggca ggcacacagc agccccccag   2151
   cactaaggcc gtgtctctga ggacgtcatc ggaggctggg cccctgggat gggaccaggg   2211
   atgggggatg ggccagggtt tacccagtgg gacagaggag caaggtttaa atttgttatt   2271
   gtgtattatg ttgttcaaat gcattttggg ggtttttaat ctttgtgaca ggaaagccct   2331
   cccccttccc cttctgtgtc acagttcttg gtgactgtcc caccggagcc tccccctcag    2391
   atgatctctc cacggtagca cttgaccttt tcgacgctta acctttccgc tgtcgcccca   2451
   ggccctccct gactccctgt gggggtggcc atccctgggc ccctccacgc ctcctggcca 2511
   gacgctgccg ctgccgctgc accacggcgt ttttttacaa cattcaactt tagtattttt 2571
   actattataa tataatatgg aaccttccct ccaaattct 2610
<210> 335
   <211> 480
   <212> PRT
   <213> Homo sapiens
<400> 335
   Met Ser Asp Val Ala Ile Val Lys Glu Gly Trp Leu His Lys Arg Gly
   1               5                   10                  15
   Glu Tyr Ile Lys Thr Trp Arg Pro Arg Tyr Phe Leu Leu Lys Asn Asp
               20                  25                  30
   Gly Thr Phe Ile Gly Tyr Lys Glu Arg Pro Gln Asp Val Asp Gln Arg
           35                  40                  45
   Glu Ala Pro Leu Asn Asn Phe Ser Val Ala Gln Cys Gln Leu Met Lys
       50                  55                  60
   Thr Glu Arg Pro Arg Pro Asn Thr Phe Ile Ile Arg Cys Leu Gln Trp
   65                  70                  75                  80
   Thr Thr Val Ile Glu Arg Thr Phe His Val Glu Thr Pro Glu Glu Arg
                   85                  90                  95
   Glu Glu Trp Thr Thr Ala Ile Gln Thr Val Ala Asp Gly Leu Lys Lys
               100                 105                 110
   Gln Glu Glu Glu Glu Met Asp Phe Arg Ser Gly Ser Pro Ser Asp Asn
           115                 120                 125
   Ser Gly Ala Glu Glu Met Glu Val Ser Leu Ala Lys Pro Lys His Arg
       130                 135                 140
   Val Thr Met Asn Glu Phe Glu Tyr Leu Lys Leu Leu Gly Lys Gly Thr
   145                 150                 155                 160
   Phe Gly Lys Val Ile Leu Val Lys Glu Lys Ala Thr Gly Arg Tyr Tyr
                   165                 170                 175
   Ala Met Lys Ile Leu Lys Lys Glu Val Ile Val Ala Lys Asp Glu Val
               180                 185                 190
   Ala His Thr Leu Thr Glu Asn Arg Val Leu Gln Asn Ser Arg His Pro
           195                 200                 205
   Phe Leu Thr Ala Leu Lys Tyr Ser Phe Gln Thr His Asp Arg Leu Cys
       210                 215                 220
   Phe Val Met Glu Tyr Ala Asn Gly Gly Glu Leu Phe Phe His Leu Ser
   225                 230                 235                 240
   Arg Glu Arg Val Phe Ser Glu Asp Arg Ala Arg Phe Tyr Gly Ala Glu
                   245                 250                 255
   Ile Val Ser Ala Leu Asp Tyr Leu His Ser Glu Lys Asn Val Val Tyr
               260                 265                 270
   Arg Asp Leu Lys Leu Glu Asn Leu Met Leu Asp Lys Asp Gly His Ile
           275                 280                 285
   Lys Ile Thr Asp Phe Gly Leu Cys Lys Glu Gly Ile Lys Asp Gly Ala
       290                 295                 300
   Thr Met Lys Thr Phe Cys Gly Thr Pro Glu Tyr Leu Ala Pro Glu Val
   305                 310                 315                 320
   Leu Glu Asp Asn Asp Tyr Gly Arg Ala Val Asp Trp Trp Gly Leu Gly
                   325                 330                 335
   Val Val Met Tyr Glu Met Met Cys Gly Arg Leu Pro Phe Tyr Asn Gln
               340                 345                 350
   Asp His Glu Lys Leu Phe Glu Leu Ile Leu Met Glu Glu Ile Arg Phe
           355                 360                 365
   Pro Arg Thr Leu Gly Pro Glu Ala Lys Ser Leu Leu Ser Gly Leu Leu
       370                 375                 380
   Lys Lys Asp Pro Lys Gln Arg Leu Gly Gly Gly Ser Glu Asp Ala Lys
   385                 390                 395                 400
   Glu Ile Met Gln His Arg Phe Phe Ala Gly Ile Val Trp Gln His Val
                   405                 410                 415
   Tyr Glu Lys Lys Leu Ser Pro Pro Phe Lys Pro Gln Val Thr Ser Glu
               420                 425                 430
   Thr Asp Thr Arg Tyr Phe Asp Glu Glu Phe Thr Ala Gln Met Ile Thr
           435                 440                 445
   Ile Thr Pro Pro Asp Gln Asp Asp Ser Met Glu Cys Val Asp Ser Glu
       450                 455                 460
   Arg Arg Pro His Phe Pro Gln Phe Ser Tyr Ser Ala Ser Ser Thr Ala
   465                 470                 475                 480
<210> 336
   <211> 21
   <212> DNA
   <213> Artificial Sequence
<220>
   <221> misc_feature
   <223> PCR Primer
<400> 336
   cgtgaccatg aacgagtttg a 21
<210> 337
   <211> 19
   <212> DNA
   <213> Artificial Sequence
<220>
   <221> misc_feature
   <223> PCR Probe
<400> 337
   caggatcacc ttgccgaaa 19
<210> 338
   <211> 22
   <212> DNA
   <213> Artificial Sequence
<220>
   <221> misc_feature
   <223> PCR Probe
<400> 338
   ctgaagctgc tgggcaaggg ca 22
<210> 339
   <211> 18
   <212> DNA
   <213> Artificial Sequence
<220>
   <221> misc_feature
   <223> Antisense Oligonucleotide
<400> 339
   ccctgtgccc tgtcccag 18
<210> 340
   <211> 18
   <212> DNA
   <213> Artificial Sequence
<220>
   <221> misc_feature
   <223> Antisense Oligonucleotide
<400> 340
   cctaagcccc tggtgaca 18
<210> 341
   <211> 18
   <212> DNA
   <213> Artificial Sequence
<220>
   <221> misc_feature
   <223> Antisense Oligonucleotide
<400> 341
   ctttgacttc tttgaccc 18
<210> 342
   <211> 18
   <212> DNA
   <213> Artificial Sequence
<220>
   <221> misc_feature
   <223> Antisense Oligonucleotide
<400> 342
   ggcagcccct ttgacttc 18
<210> 343
   <211> 18
   <212> DNA
   <213> Artificial Sequence
<220>
   <221> misc_feature
   <223> Antisense Oligonucleotide
<400> 343
   caaccctcct tcacaata 18
<210> 344
   <211> 18
   <212> DNA
   <213> Artificial Sequence
<220>
   <221> misc_feature
   <223> Antisense Oligonucleotide
<400> 344
   tactcccctc gtttgtgc 18
<210> 345
   <211> 18
   <212> DNA
   <213> Artificial Sequence
<220>
   <221> misc_feature
   <223> Antisense Oligonucleotide
<400> 345
   tgccatcatt cttgagga 18
<210> 346
   <211> 18
   <212> DNA
   <213> Artificial Sequence
<220>
   <221> misc_feature
   <223> Antisense Oligonucleotide
<400> 346
   agccaatgaa ggtgccat 18
<210> 347
   <211> 18
   <212> DNA
   <213> Artificial Sequence
<220>
   <221> misc_feature
   <223> Antisense Oligonucleotide
<400> 347
   cacagagaag ttgttgag 18
<210> 348
   <211> 18
   <212> DNA
   <213> Artificial Sequence
<220>
   <221> misc_feature
   <223> Antisense Oligonucleotide
<400> 348
   agtctggatg gcggttgt 18
<210> 349
   <211> 18
   <212> DNA
   <213> Artificial Sequence
<220>
   <221> misc_feature
   <223> Antisense Oligonucleotide
<400> 349
   tcctcctcct cctgcttc 18
<210> 350
   <211> 18
   <212> DNA
   <213> Artificial Sequence
<220>
   <221> misc_feature
   <223> Antisense Oligonucleotide
<400> 350
   cctgagttgt cactgggt 18
<210> 351
   <211> 18
   <212> DNA
   <213> Artificial Sequence
<220>
   <221> misc_feature
   <223> Antisense Oligonucleotide
<400> 351
   ccgaaagtgc ccttgccc 18
<210> 352
   <211> 18
   <212> DNA
   <213> Artificial Sequence
<220>
   <221> misc_feature
   <223> Antisense Oligonucleotide
<400> 352
   gccacgatga cttccttc 18
<210> 353
   <211> 18
   <212> DNA
   <213> Artificial Sequence
<220>
   <221> misc_feature
   <223> Antisense Oligonucleotide
<400> 353
   cggtcctcgg agaacaca 18
<210> 354
   <211> 18
   <212> DNA
   <213> Artificial Sequence
<220>
   <221> misc_feature
   <223> Antisense Oligonucleotide
<400> 354
   acgttcttct ccgagtgc 18
<210> 355
   <211> 18
   <212> DNA
   <213> Artificial Sequence
<220>
   <221> misc_feature
   <223> Antisense Oligonucleotide
<400> 355
   gtgccgcaaa aggtcttc 18
<210> 356
   <211> 18
   <212> DNA
   <213> Artificial Sequence
<220>
   <221> misc_feature
   <223> Antisense Oligonucleotide
<400> 356
   tactcaggtg tgccgcaa 18
<210> 357
   <211> 18
   <212> DNA
   <213> Artificial Sequence
<220>
   <221> misc_feature
   <223> Antisense Oligonucleotide
<400> 357
   ggcttgaagg gtgggctg 18
<210> 358
   <211> 18
   <212> DNA
   <213> Artificial Sequence
<220>
   <221> misc_feature
   <223> Antisense Oligonucleotide
<400> 358
   tcaaaatacc tggtgtca 18
<210> 359
   <211> 18
   <212> DNA
   <213> Artificial Sequence
<220>
   <221> misc_feature
   <223> Antisense Oligonucleotide
<400> 359
   gccgtgaact cctcatca 18
<210> 360
   <211> 18
   <212> DNA
   <213> Artificial Sequence
<220>
   <221> misc_feature
   <223> Antisense Oligonucleotide
<400> 360
   ggtcaggtgg tgtgatgg 18
<210> 361
   <211> 18
   <212> DNA
   <213> Artificial Sequence
<220>
   <221> misc_feature
   <223> Antisense Oligonucleotide
<400> 361
   ctcgctgtcc acacactc 18
   <210> 362
   <211> 18
   <212> DNA
   <213> Artificial Sequence
<220>
   <221> misc_feature
   <223> Antisense Oligonucleotide
<400> 362
   gcctctccat ccctccaa 18
<210> 363
   <211> 18
   <212> DNA
   <213> Artificial Sequence
<220>
   <221> misc_feature
   <223> Antisense Oligonucleotide
<400> 363
   acagcgtggc ttctctca 18
<210> 364
   <211> 18
   <212> DNA
   <213> Artificial Sequence
<220>
   <221> misc_feature
   <223> Antisense Oligonucleotide
<400> 364
   ttttcttccc taccccgc 18
<210> 365
   <211> 18
   <212> DNA
   <213> Artificial Sequence
<220>
   <221> misc_feature
   <223> Antisense Oligonucleotide
<400> 365
   gatagttttc ttccctac 18
<210> 366
   <211> 18
   <212> DNA
   <213> Artificial Sequence
<220>
   <221> misc_feature
   <223> Antisense Oligonucleotide
<400> 366
   taaaacccgc aggatagt 18
<210> 367
   <211> 18
   <212> DNA
   <213> Artificial Sequence
<220>
   <221> misc_feature
   <223> Antisense Oligonucleotide
<400> 367
   ggagaacaaa ctggatga 18
<210> 368
   <211> 18
   <212> DNA
   <213> Artificial Sequence
<220>
   <221> misc_feature
   <223> Antisense Oligonucleotide
<400> 368
   ctggctgaca gagtgagg 18
<210> 369
   <211> 18
   <212> DNA
   <213> Artificial Sequence
<220>
   <221> misc_feature
   <223> Antisense Oligonucleotide
<400> 369
   gcggctggct gacagagt 18
<210> 370
   <211> 18
   <212> DNA
   <213> Artificial Sequence
<220>
   <221> misc_feature
   <223> Antisense Oligonucleotide
<400> 370
   cccagagaga tgacagat 18
<210> 371
   <211> 18
   <212> DNA
   <213> Artificial Sequence
<220>
   <221> misc_feature
   <223> Antisense Oligonucleotide
<400> 371
   gctgctgtgt gcctgcca 18
<210> 372
   <211> 18
   <212> DNA
   <213> Artificial Sequence
<220>
   <221> misc_feature
   <223> Antisense Oligonucleotide
<400> 372
   cataatacac aataacaa 18
<210> 373
   <211> 18
   <212> DNA
   <213> Artificial Sequence
<220>
   <221> misc_feature
   <223> Antisense Oligonucleotide
<400> 373
   atttgaacaa cataatac 18
<210> 374
   <211> 18
   <212> DNA
   <213> Artificial Sequence
<220>
   <221> misc_feature
   <223> Antisense Oligonucleotide
<400> 374
   aagtgctacc gtggagag 18
<210> 375
   <211> 18
   <212> DNA
   <213> Artificial Sequence
<220>
   <221> misc_feature
   <223> Antisense Oligonucleotide
<400> 375
   cgaaaaggtc aagtgcta 18
<210> 376
   <211> 18
   <212> DNA
   <213> Artificial Sequence
<220>
   <221> misc_feature
   <223> Antisense Oligonucleotide
<400> 376
   cagggagtca gggagggc 18
<210> 377
   <211> 18
   <212> DNA
   <213> Artificial Sequence
<220>
   <221> misc_feature
   <223> Antisense Oligonucleotide
<400> 377
   aaagttgaat gttgtaaa 18
<210> 378
   <211> 18
   <212> DNA
   <213> Artificial Sequence
<220>
   <221> misc_feature
   <223> Antisense Oligonucleotide
   <400> 378
   aaaatactaa agttgaat 18